# EUROPEAN PATENT SPECIFICATION

(11) **EP 1 171 437 B1**
(45) Date of publication and mention of the grant of the patent: **08.10.2003**
(21) Application number: 00919009.1
(22) Date of filing: 04.04.2000
(51) Int. Cl.: C07D 417/12, C07D 401/12, C07D 403/12, A01N 43/56, A01N 43/647, A01N 43/74

(54) **PESTICIDAL INDAZOLE OR BENZOTRIAZOLE DERIVATIVES**
PESTIZIDE INDAZOL- ODER BENZOTRIAZOLDERIVATE
DERIVES INDAZOLE OU BENZOTRIAZOLE PESTICIDES

(30) Priority: 20.04.1999 GB 9909062; 28.01.2000 GB 0002039
(43) Date of publication of application: 16.01.2002
(73) Proprietor: Syngenta Limited, Guildford Surrey GU2 7YH (GB)
(72) Inventor: PILKINGTON, Brian, Leslie, deceased (GB); MATHEWS, Christopher John, Bracknell, Berkshire RG42 6ET (GB); BARNETT, Susan, Patricia, Bracknell, Berkshire RG42 6ET (GB); SMITH, Stephen, Christopher, Bracknell, Berkshire RG42 6ET (GB); BARNES, Nigel, John, Bracknell, Berkshire RG42 6ET (GB); WHITTINGHAM, William, Guy, Bracknell, Berkshire RG42 6ET (GB); WILLIAMS, John, Bracknell, Berkshire RG42 6ET (GB); CLARKE, Eric, Daniel, Bracknell, Berkshire RG42 6ET (GB); WHITTLE, Alan, John, Bracknell, Berkshire RG42 6ET (GB); HUGHES, David, John, Bracknell, Berkshire RG42 6ET (GB); ARMSTRONG, Sarah, Bracknell, Berkshire RG42 6ET (GB); VINER, Russell, Bracknell, Berkshire RG42 6ET (GB); FRASER, Torquil, Eoghan, Macleod, Bracknell, Berkshire RG42 6ET (GB); CROWLEY, Patrick, Jelf, Bracknell, Berkshire RG42 6ET (GB); SALMON, Roger, Bracknell, Berkshire RG42 6ET (GB)
(74) Representative: Waterman, John Richard
(86) International application number: PCT/GB00/01272
(87) International publication number: WO 00/063207

(56) References cited:
- WO-A-95/31448
- WO-A-98/17630

## Description

The present invention relates to azine and azole derivatives, to processes for preparing them, to fungicidal, insecticidal, acaricidal, molluscicidal and nematicidal compositions comprising them, to methods of using them to combat fungal diseases (especially fungal diseases of plants) and to methods of using them to combat and control insect, acarine, mollusc and nematode pests.

Azole and azine derivatives are disclosed in WO95/31448, WO97/18198, WO98/02424 and WO98/05670.

The present invention provides a compound of formula (I): 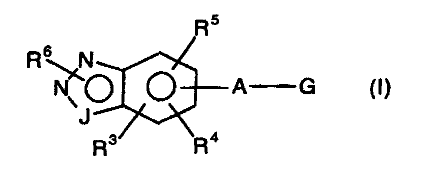 wherein G is either 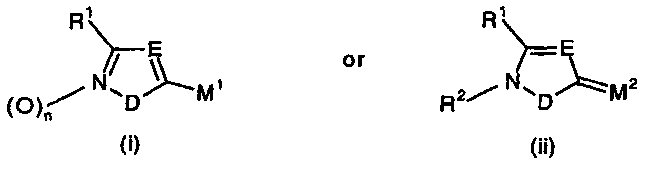 where M¹ or M² is bonded to A; n is 0 or 1; A is optionally substituted C₁₋₆ alkylene, optionally substituted C₂₋₆ alkenylene, optionally substituted C₂₋₆ alkynylene, optionally substituted C₁₋₆ alkylenoxy, optionally substituted oxy(C₁₋₆)alkylene, optionally substituted C₁₋₆ alkylenethio, optionally substituted thio(C₁₋₆)alkylene, optionally substituted C₁₋₆ alkylenamino, optionally substituted amino(C₁₋₆)alkylene, optionally substituted [C₁₋₆ alkyleneoxy(C₁₋₆)alkylene], optionally substituted [C₁₋₆ alkylenethio(C₁₋₆)alkylene], optionally substituted [C₁₋₆ alkylenesulfinyl(C₁₋₆)alkylene], optionally substituted [C₁₋₆ alkylenesulfonyl(C₁₋₆)alkylene] or optionally substituted [C₁₋₆ alkyleneamino(C₁₋₆)alkylene]; when G is (i), D is S, NR⁷, CR⁸=CR⁹, CR⁸=N, CR⁸=N(O), N=CR⁹ or N(O)=CR⁹; when G is (ii), D is S or NR⁷; E is N, N-oxide or CR¹⁰; M¹ is OC(=Y), N(R¹¹)C(=Y), N=C(OR¹²), N=C(SR¹³) or N=C(NR¹⁴R¹⁵) where O or N is the atom of attachment to the ring containing D and E; M² is N-C(=Y) where N is the atom of attachment to the ring containing D and E; Y is O, S or NR¹⁶; J is N or CR¹⁷; R¹ is hydrogen, halogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₁₋₆ alkylthio, optionally substituted C₃₋₇ cycloalkyl, cyano, nitro or SF₅; R² is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀ alkylaminocarbonyl, optionally substituted di(C₁₋₁₀)alkylaminocarbonyl, optionally substituted phenoxycarbonyl, optionally substituted C₁₋₆ alkylthio, optionally substituted C₁₋₆ alkylsulfinyl, optionally substituted C₁₋₆ alkylsulfonyl, optionally substituted arylthio, optionally substituted arylsulfinyl, optionally substituted arylsulfonyl or R¹⁸R¹⁹NS; R³, R⁴ and R⁵ are, independently, hydrogen, halogen, optionally substituted C₁₋₆ alkyl, optionally substituted C₁₋₆ alkoxy, optionally substituted C₁₋₆ alkylthio, optionally substituted C₁₋₆ alkylsulfinyl, optionally substituted C₁₋₆ alkylsulfonyl, cyano, nitro, optionally substituted C₁₋₆ alkylcarbonyl, optionally substituted C₁₋₆ alkoxycarbonyl or SF₅; R⁶ is hydrogen, cyano, optionally substituted C₁₋₂₀ alkyl, optionally substituted C₂₋₂₀ alkenyl(C₁₋₆)alkyl, optionally substituted C₂₋₂₀ alkynyl(C₁₋₆)alkyl, optionally substituted C₃₋₇ cycloalkyl, optionally substituted C₅₋₆ cycloalkenyl, formyl, optionally substituted C₁₋₂₀ alkoxycarbonyl, optionally substituted C₁₋₂₀ alkylcarbonyl, aminocarbonyl, optionally substituted C₁₋₂₀ alkylaminocarbonyl, optionally substituted di(C₁₋₂₀)alkylaminocarbonyl, optionally substituted aryloxycarbonyl, optionally substituted arylcarbonyl, optionally substituted arylaminocarbonyl, optionally substituted N-alkyl-N-arylaminocarbonyl, optionally substituted diarylaminocarbonyl, optionally substituted heteroaryloxycarbonyl, optionally substituted heteroarylcarbonyl, optionally substituted heteroarylaminocarbonyl, optionally substituted alkylheteroarylaminocarbonyl, optionally substituted diheteroarylaminocarbonyl, optionally substituted phenyl, optionally substituted heteroaryl, optionally substituted heterocyclyl, optionally substituted C₁₋₂₀ alkylsulfonyl or optionally substituted arylsulfonyl; R⁷ is C₁₋₆ alkyl; R⁸ and R⁹ are, independently, hydrogen, halogen, cyano, nitro, optionally substituted C₁₋₆ alkyl, optionally substituted C₂₋₆ alkenyl, optionally substituted C₂₋₆ alkynyl or optionally substituted C₁₋₆ alkoxy; R¹⁰ is hydrogen, halogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₁₋₆ alkylthio, optionally substituted C₁₋₆ alkylsulfinyl, optionally substituted C₁₋₆ alkylsulfonyl, cyano, nitro, formyl, R²⁰ON=C(R²¹), optionally substituted C₁₋₆ alkylcarbonyl, optionally substituted C₁₋₆ alkoxycarbonyl or SF₅; or R¹ and R¹⁰ together with the atoms to which they are attached may be joined to form a five, six or seven-membered saturated or unsaturated ring carbocylic or heterocyclic ring which may contain one or two hetero atoms selected from O, N and S and which may be optionally substituted by C₁₋₆ alkyl, C₁₋₆ haloalkyl or halogen; R¹¹ is hydrogen, optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀ alkylaminocarbonyl, optionally substituted di(C₁₋₁₀)alkylaminocarbonyl, optionally substituted phenoxycarbonyl, optionally substituted C₁₋₆ alkylthio, optionally substituted C₁₋₆ alkylsulfinyl, optionally substituted C₁₋₆ alkylsulfonyl, optionally substituted arylthio, optionally substituted arylsulfinyl, optionally substituted arylsulfonyl or R²²R²³NS; R¹² is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀ alkylaminocarbonyl, optionally substituted di(C₁₋₁₀)alkylaminocarbonyl, , amino, optionally substituted C₁₋₆ alkylamino, optionally substituted di(C₁₋₆)alkylamino, optionally substituted phenoxycarbonyl, tri(C₁₋₄)alkylsilyl, aryldi(C₁₋₄)alkylsilyl, (C₁₋₄)alkyldiarylsilyl or triarylsilyl; R¹³ is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, optionally substituted C₁₋₁₀ alkylaminocarbonyl, optionally substituted di(C₁₋₁₀)alkylaminocarbonyl or optionally substituted phenoxycarbonyl); R¹⁴ and R¹⁵ are, independently optionally substituted C₁₋₁₀ alkyl, optionally substituted C₁₋₆ alkoxy, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀alkylaminocarbonyl, optionally substituted di(C₁₋₁₀)alkylaminocarbonyl, hydroxy, amino, optionally substituted C₁₋₆ alkylamino, optionally substituted di(C₁₋₆)alkylamino, or optionally substituted phenoxycarbonyl; R¹⁶ is hydrogen, cyano, nitro, optionally substituted C₁₋₆ alkyl, optionally substituted C₃₋₇ cycloalkyl, optionally substituted (C₂₋₆)alkenyl(C₁₋₆)alkyl, optionally substituted (C₂₋₆)alkynyl(C₁₋₆)alkyl, optionally substituted phenyl, optionally substituted heteroaryl, optionally substituted C₁₋₆ alkylcarbonyl, optionally substituted C₁₋₆ alkoxycarbonyl, optionally substituted C₁₋₆ alkylamino, optionally substituted di(C₁₋₆)alkylamino, optionally substituted C₁₋₆ alkylcarbonylamino, optionally substituted C₁₋₆ alkoxycarbonylamino, optionally substituted C₁₋₆ alkoxy, optionally substituted C₁₋₆ alkylthio, optionally substituted C₁₋₆ alkylsulfinyl, optionally substituted C₁₋₆ alkylsulfonyl, optionally substituted arylthio, optionally substituted arylsulfinyl, optionally substituted arylsulfonyl or C₁₋₆ acyloxy; R¹⁷ is hydrogen, halogen, nitro, cyano, optionally substituted C₁₋₈ alkyl, optionally substituted C₂₋₆ alkenyl, optionally substituted C₂₋₆ alkynyl, optionally substituted C₃₋₇ cycloalkyl, optionally substituted C₁₋₆ alkoxycarbonyl, optionally substituted C₁₋₆ alkylcarbonyl, optionally substituted C₁₋₆ alkylaminocarbonyl, optionally substituted di(C₁₋₆)alkylaminocarbonyl, optionally substituted phenyl or optionally substituted heteroaryl; R¹⁸ and R¹⁹ are, independently, optionally substituted C₁₋₆ alkyl or R¹⁸ and R¹⁹ together with the N atom to which they are attached form a five, six or seven-membered heterocyclic ring which may contain one or two further hetero atoms selected from O, N or S and which may be optionally substituted by one or two C₁₋₆ alkyl groups; R²⁰ is hydrogen, optionally substituted phenyl, optionally substituted phenyl(C₁₋₄)alkyl or optionally substituted C₁₋₂₀ alkyl; R²¹ is hydrogen, optionally substituted phenyl or optionally substituted C₁₋₆ alkyl; and R²² and R²³ are, independently, optionally substituted C₁₋₆ alkyl or R²² and R²³ together with the N atom to which they are attached form a five, six or seven-membered heterocyclic ring which may contain one or two further hetero atoms selected from O, N or S and which may be optionally substituted by one or two C₁₋₆ alkyl groups.

The compounds of formula (I) may exist in different geometric or optical isomers or tautomeric forms. This invention covers all such isomers and tautomers and mixtures thereof in all proportions.

When present, optional substituents on alkylene, alkenylene or alkynylene moieties include, subject to valency constraints, one or more of halogen, C₁₋₆ alkyl, C₁₋₆ haloalkyl, C₁₋₆ cyanoalkyl, C₁₋₆ alkoxy(C₁₋₆) alkyl, C₁₋₆ alkoxy, cyano, =O, =NR²⁴ and =CR²⁵R²⁶, wherein R²⁴ is C₁₋₆ alkyl, C₁₋₆ haloalkyl, OR²⁷ or R²⁸R²⁹N; where R²⁵ and R²⁶ are, independently, hydrogen, C₁₋₆ alkyl, C₁₋₆ alkoxy, C₁₋₆ haloalkyl, cyano, C₁₋₆ alkoxycarbonyl, C₁₋₆ alkylcarbonyl or R³⁰R³¹N; R²⁷ is C₁₋₆ alkyl, C₁₋₆ haloalkyl or phenyl(C₁₋₂)alkyl; R²⁸ and R²⁹ are, independently, hydrogen, C₁₋₈ alkyl, C₃₋₇ cycloalkyl, C₂₋₆ alkenyl(C₁₋₆)alkyl, C₂₋₆ alkynyl(C₁₋₆)alkyl, C₂₋₆ haloalkyl, C₁₋₆ alkoxy(C₁₋₆)alkyl, C₁₋₆ alkoxycarbonyl(C₁₋₆)alkyl, carboxy(C₁₋₆)alkyl or phenyl(C₁₋₂)alkyl or R²⁸ and R²⁹ together with the N atom to which they are attached form a five, six or seven-membered heterocyclic ring which may contain one or two further hetero atoms selected from O, N or S and which may be optionally substituted by one or two C₁₋₆ alkyl groups; R³⁰ and R³¹ are, independently, hydrogen, C₁₋₈ alkyl, C₃₋₇ cycloalkyl, C₂₋₆ alkenyl(C₁₋₆)alkyl, C₂₋₆ alkynyl(C₁₋₆)alkyl, C₂₋₆ haloalkyl, C₁₋₆ alkoxy(C₁₋₆)alkyl, C₁₋₆ alkoxycarbonyl(C₁₋₆)alkyl, carboxy(C₁₋₆)alkyl or phenyl(C₁₋₂)alkyl; or R³⁰ and R³¹ together with the N atom to which they are attached form a five, six or seven-membered heterocyclic ring which may contain one or two further hetero atoms selected from O, N or S and which may be optionally substituted by one or two C₁₋₆ alkyl groups.

Each alkyl moiety is a straight or branched chain and is, for example, methyl, ethyl, *n*-propyl, *n*-butyl, *n*-pentyl, *n*-hexyl, *iso*-propyl, *n*-butyl, *sec*-butyl, *iso*-butyl, *tert*-butyl or *neo*-pentyl. When present, the optional substituents on alkyl include one or more of halogen, nitro, cyano, HO₂C, C₁₋₁₀ alkoxy (itself optionally substituted by C₁₋₁₀ alkoxy), aryl(C₁₋₄)alkoxy, C₁₋₁₀ alkylthio, C₁₋₁₀ alkylcarbonyl, C₁₋₁₀ alkoxycarbonyl, C₁₋₆ alkylaminocarbonyl, di(C₁₋₆ alkyl)aminocarbonyl, (C₁₋₆)alkylcarbonyloxy, optionally substituted phenyl, heteroaryl, aryloxy, arylcarbonyloxy, heteroaryloxy, heterocyclyl, heterocyclyloxy, C₃₋₇ cycloalkyl (itself optionally substituted with (C₁₋₆)alkyl or halogen), C₃₋₇ cycloalkyloxy, C₅₋₇ cycloalkenyl, C₁₋₆ alkylsulfonyl, C₁₋₆ alkylsulfinyl, tri(C₁₋₄)alkylsilyl, tri(C₁₋₄)alkylsilyl(C₁₋₆)alkoxy, aryldi(C₁₋₄)alkylsilyl, (C₁₋₄)alkyldiarylsilyl, triarylsilyl, =N-OR' and =N-NR'R"; where R' and R" are, independently, C₁₋₆ alkyl or C₁₋₆ haloalkyl.

Alkenyl and alkynyl moieties can be in the form of straight or branched chains, and the alkenyl moieties, where appropriate, can be of either the (E)- or (Z)-configuration. Examples are vinyl, allyl and propargyl. When present, the optional substituents on alkenyl or alkynyl include one or more of halogen, aryl and C₃₋₇ cycloalkyl.

In the context of this specification acyl is optionally substituted C₁₋₆ alkylcarbonyl (for example acetyl), optionally substituted C₂₋₆ alkenylcarbonyl, optionally substituted C₂₋₆ alkynylcarbonyl, optionally substituted arylcarbonyl (for example benzoyl) or optionally substituted heteroarylcarbonyl.

Halogen is fluorine, chlorine, bromine or iodine.

Haloalkyl groups are alkyl groups which are optionally substituted with one or more of the same or different halogen atoms and are, for example, CF₃, CF₂Cl, CF₃CH₂ or CHF₂CH₂.

Aryl includes naphthyl, anthracyl, fluorenyl and indenyl but is preferably phenyl.

The term heteroaryl refers to an aromatic ring containing up to 10 atoms including one or more heteroatoms (preferably one or two heteroatoms) selected from O, S and N. Examples of such rings include pyridine, pyrimidine, furan, quinoline, quinazoline, pyrazole, thiophene, thiazole, oxazole and isoxazole.

The terms heterocycle and heterocyclyl refers to a non-aromatic ring containing up to 10 atoms including one or more (preferably one or two) heteroatoms selected from O, S and N. Examples of such rings include 1,3-dioxolane, tetrahydrofuran and morpholine. It is preferred that heterocyclyl is optionally substituted by C₁₋₆ alkyl.

Cycloalkyl includes cyclopropyl, cyclopentyl and cyclohexyl. The optional substituents for cycloalkyl include halogen, cyano and C₁₋₃ alkyl.

Cycloalkenyl includes cyclopentenyl and cyclohexenyl. The optional substituents for cycloalkenyl include C₁₋₃ alkyl, halogen and cyano.

Carbocyclic rings include aryl, cycloalkyl and cycloalkenyl groups.

For substituted phenyl moieties, heterocyclyl and heteroaryl groups it is preferred that one or more substituents are independently selected from halogen, C₁₋₆ alkyl, C₁₋₆ haloalkyl, C₁₋₆ alkoxy(C₁₋₆)alkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₂₋₆ alkenyl, C₂₋₆ haloalkenyl, C₂₋₆ alkynyl, C₃₋₇ cycloalkyl, nitro, cyano, CO₂H, C₁₋₆ alkylcarbonyl, C₁₋₆ alkoxycarbonyl, R³²R³³N or R³⁴R³⁵NC(O) wherein R³², R³³, R³⁴ and R³⁵ are, independently, hydrogen or C₁₋₆ alkyl.

It is to be understood that dialkylamino substituents include those where the dialkyl groups together with the N atom to which they are attached form a five, six or seven-membered heterocyclic ring which may contain one or two further hetero atoms selected from O, N or S and which may be optionally substituted by one or two C₁₋₆ alkyl groups. When heterocyclic rings are formed by joining two groups on an N atom, the resulting rings are suitably pyrrolidine, piperidine, thiomorpholine and morpholine each of which may be substituted by one or two (C₁₋₆)alkyl groups.

In a further aspect, the present invention provides a compound of formula (IA): 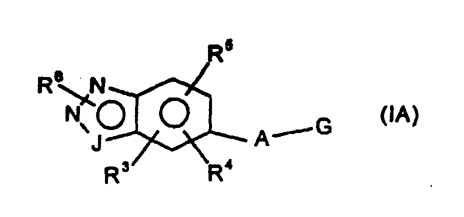 wherein A, G, J, R³, R⁴, R⁵ and R⁶ are as defined above for a compound of formula (I).

More preferred compounds of formula (IA) are those wherein n is 0; A is C₁₋₆ alkylene, C₁₋₆ alkenylene, C₁₋₆ alkylenoxy, oxy(C₁₋₆)alkylene, C₁₋₆ alkylenamino or C₁₋₆ alkylenethio, each of which is optionally substituted by C₁₋₃ alkyl, C₁₋₃ haloalkyl, C₁₋₃ cyanoalkyl, halogen, C₁₋₃ alkoxy, C₁₋₆ alkoxycarbonyl, cyano, =O, =NR³⁶ or =CR³⁷R³⁸; when G is (i), D is S, NR⁷, CR⁸=CR⁹, CR⁸=N, CR⁸=N(O), N=CR⁹ or N(O)=CR⁹; when G is (ii), D is S or NR⁷; E is N or CR¹⁰; M¹ is N(R¹¹)C(=Y) or N=C(SR¹³) where O or N is the atom of attachment to the ring containing D and E; M² is N-C(=Y) where N is the atom of attachment to the ring containing D and E; Y is O, S or NR¹⁶; J is N or CR¹⁷R¹ is hydrogen, halogen, C₁₋₆ alkyl, C₂₋₆ alkenyl, C₂₋₆ alkynyl, C₁₋₆ cyanoalkyl, C₁₋₆ haloalkyl, C₁₋₆ alkoxy, C₁₋₆ haloalkoxy, C₁₋₆ alkylthio, C₁₋₆ haloalkylthio, C₃₋₆ cycloalkyl, C₃₋₇ cycloalkyl(C₁₋₄)alkyl, C₁₋₆ alkoxy(C₁₋₆)alkyl, cyano, nitro or SF₅; R² is C₁₋₁₀ alkyl, benzyloxymethyl, benzoyloxymethyl, C₁₋₆alkoxy(C₁₋₆)alkyl, C₂₋₆ alkenyl(C₁₋₆)alkyl, C₂₋₆ alkynyl(C₁₋₆)alkyl, C₁₋₁₀ alkylcarbonyl or C₁₋₁₀ alkoxycarbonyl; R³, R⁴ and R⁵ are, independently, selected from hydrogen, halogen, C₁₋₆ alkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ haloalkyl, cyano, nitro, C₁₋₆ alkylcarbonyl, C₁₋₆ alkoxycarbonyl or SF₅; R⁶ is C₁₋₁₀ alkyl or C₁₋₁₀ haloalkyl (each one of which may be substituted with a C₁₋₆ alkyloxime, C₁₋₆ haloalkyloxime, C₁₋₆ alkylhydrazone or C₁₋₆ haloalkylhydrazone group) or cyano, C₁₋₆ cyanoalkyl, C₂₋₆ alkenyl(C₁₋₆)alkyl, C₂₋₆ alkynyl(C₁₋₆)alkyl, C₃₋₇ cycloalkyl, C₃₋₇ halocycloalkyl, C₃₋₇ cyanocycloalkyl, C₁₋₃ alkyl(C₃₋₇)cycloalkyl, C₁₋₃ alkyl(C₃₋₇)halocycloalkyl, C₅₋₆ cycloalkenyl, C₃₋₇ cycloalkyl(C₁₋₆)alkyl, C₅₋₆ cycloalkenyl(C₁₋₆)alkyl, C₂₋₆ haloalkenyl (C₁₋₆)alkyl, C₂₋₆ cyanoalkenyl(C₁₋₆)alkyl, C₁₋₆ alkoxy(C₁₋₆)alkyl, C₃₋₆ alkenyloxy(C₁₋₆)alkyl, C₃₋₆ alkynyloxy(C₁₋₆)alkyl, aryloxy(C₁₋₆)alkyl, formyl, C₁₋₆ carboxyalkyl, C₁₋₆ alkylcarbonyl(C₁₋₆)alkyl, C₂₋₆ alkenylcarbonyl(C₁₋₆)alkyl, C₂₋₆ alkynylcarbonyl(C₁₋₆)alkyl, C₁₋₆ alkoxycarbonyl(C₁₋₆)alkyl, C₃₋₆ alkenyloxycarbonyl(C₁₋₆)alkyl, C₃₋₆ alkynyloxycarbonyl(C₁₋₆)alkyl, aryloxycarbonyl(C₁₋₆)alkyl, C₁₋₆ alkylthio(C₁₋₆)alkyl, C₁₋₆ alkylsulfinyl(C₁₋₆)alkyl, C₁₋₆ alkylsulfonyl(C₁₋₆)alkyl, aminocarbonyl(C₁₋₆)alkyl, aminocarbonyl(C₂₋₆)alkenyl, aminocarbonyl(C₂₋₆)alkynyl, C₁₋₆alkylaminocarbonyl(C₁₋₆)alkyl, di(C₁₋₆)alkylaminocarbonyl(C₁₋₆)alkyl, C₁₋₆ alkylaminocarbonyl(C₂₋₆)alkenyl(C₁₋₆)alkyl, di(C₁₋₆)alkylaminocarbonyl(C₂₋₆)alkenyl(C₁₋₆)alkyl, alkylaminocarbonyl(C₂₋₆)alkynyl(C₁₋₆)alkyl, di(C₁₋₆)alkylaminocarbonyl(C₂₋₆)alkynyl(C₁₋₆)alkyl, C₁₋₆ alkoxycarbonyl, C₁₋₆ alkylcarbonyl, aminocarbonyl, C₁₋₆ alkylaminocarbonyl, di(C₁₋₆)alkylaminocarbonyl, phenyl (optionally substituted by halo, nitro, cyano, C₁₋₆ alkyl, C₁₋₆ haloalkyl, C₁₋₆ alkoxy or C₁₋₆ haloalkoxy), phenyl(C₁₋₄)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy), phenyl(C₂₋₄)alkenyl(C₁₋₆)alkyl, (wherein the phenyl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy), heteroaryl (optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy), heterocyclyl (optionally substituted by halo, nitro, cyano, C₁₋₆ alkyl, C₁₋₆ haloalkyl, C₁₋₆ alkoxy or C₁₋₆ haloalkoxy), heteroaryl(C₁₋₄)alkyl (where the heteroaryl may be substituted by halo, nitro, cyano, C₁₋₆ alkyl, C₁₋₆ haloalkyl, C₁₋₆ alkoxy or C₁₋₆ haloalkoxy),or heterocyclyl(C₁₋₄)alkyl (where the heterocyclyl may be substituted by halo, cyano, C₁₋₆ alkyl, C₁₋₆ haloalkyl, C₁₋₆ alkoxy or C₁₋₆ haloalkoxy); R⁷ is C₁₋₆ alkyl; R⁸ and R⁹ independently are, hydrogen, halogen, C₁₋₆ alkyl, C₁₋₆ haloalkyl C₂₋₆ alkenyl, C₂₋₆ alkynyl, C₁₋₆ alkoxy or C₁₋₆ haloalkoxy; R¹⁰ is hydrogen, halogen, C₁₋₆ alkyl, C₂₋₆ alkenyl, C₁₋₆ alkynyl, C₁₋₆ haloalkyl, C₁₋₆ alkoxy, C₁₋₆ alkoxy (C₁₋₆)alkyl, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ haloalkyl, cyano, nitro, formyl, R²⁰ON=CH, C₁₋₆ alkylcarbonyl, C₁₋₆ alkoxycarbonyl or SF₅; or together R¹ and R¹⁰ together with the atoms to which they are attached may be joined to form a five, six or seven-membered saturated or unsaturated ring carbocylic or heterocyclic ring which may contain one or two hetero atoms selected from O, N or S and which may be optionally substituted by C₁₋₆ alkyl, C₁₋₆ haloalkyl or halogen; R¹¹ is hydrogen, C₁₋₁₀ alkyl, benzyloxymethyl, benzoyloxymethyl, C₁₋₆alkoxy(C₁₋₆)alkyl, C₂₋₆ alkenyl(C₁₋₆)alkyl (especially allyl), C₂₋₆ alkynyl(C₁₋₆)alkyl (especially propargyl), C₁₋₁₀ alkylcarbonyl or C₁₋₁₀ alkoxycarbonyl (especially *iso*butoxycarbonyl); R¹³ is C₁₋₁₀ alkyl, benzyloxymethyl, benzoyloxymethyl, C₁₋₆alkoxy(C₁₋₆)alkyl, C₂₋₆ alkenyl(C₁₋₆)alkyl (especially allyl), C₂₋₆ alkynyl(C₁₋₆)alkyl (especially propargyl), C₁₋₁₀ alkylcarbonyl or C₁₋₁₀ alkoxycarbonyl (especially *iso*butoxycarbonyl); R¹⁶ is cyano, nitro, C₁₋₆ alkyl, C₁₋₆ haloalkyl, C₃₋₇ cycloalkyl, C₃₋₇ cycloalkyl(C₁₋₆)alkyl, CH₂(C₂₋₆)alkenyl, CH₂(C₂₋₆)alkynyl, phenyl (optionally substituted by halo, nitro, cyano, C₁₋₆ alkyl, C₁₋₆ haloalkyl, C₁₋₆ alkoxy or C₁₋₆ haloalkoxy) heteroaryl (optionally substituted by halo, nitro, cyano, C₁₋₆ alkyl, C₁₋₆ haloalkyl, C₁₋₆ alkoxy or C₁₋₆ haloalkoxy), C₁₋₆ alkylcarbonyl, C₁₋₆ alkoxycarbonyl, C₁₋₆ alkylamino, di(C₁₋₆)alkylamino, C₁₋₆ alkylcarbonylamino, C₁₋₆ alkoxycarbonylamino, C₁₋₆ alkoxy, C₁₋₆ alkylthio, C₁₋₆ alkylsulfinyl, C₁₋₆ alkylsulfonyl, C₁₋₆ haloalkylthio, C₁₋₆ haloalkylsulfinyl, C₁₋₆ haloalkylsulfonyl, arylthio, arylsulfinyl, arylsulfonyl or (C₁₋₆)alkylC(O)O; R¹⁷ is hydrogen, halogen, nitro, cyano, C₁₋₈ alkyl, C₁₋₆ haloalkyl, C₁₋₆ cyanoalkyl, C₂₋₆ alkenyl, C₂₋₆ alkynyl, C₃₋₇ cycloalkyl, C₂₋₆ haloalkenyl, C₃₋₇ cycloalkyl(C₁₋₆)alkyl, C₁₋₆alkox(C₁₋₆)alkyl, C₁₋₆ alkoxycarbonyl, C₁₋₆ alkylcarbonyl, C₁₋₆ alkylaminocarbonyl, di(C₁₋₆alkylaminocarbonyl, phenyl (optionally substituted by halo, nitro, cyano, C₁₋₆ alkyl, C₁₋₆ haloalkyl, C₁₋₆ alkoxy or C₁₋₆ haloalkoxy) or heteroaryl (optionally substituted by halo,nitro, cyano, C₁₋₆ alkyl, C₁₋₆ haloalkyl, C₁₋₆ alkoxy or C₁₋₆ haloalkoxy); R²⁰ is C₁₋₆ alkyl or phenyl(C₁₋₂)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy); R³⁶ is C₁₋₆ alkyl, OR³⁹ or NR⁴⁰R⁴¹; R³⁷ is hydrogen, C₁₋₆ alkyl or C₁₋₆ haloalkyl; R³⁸ is hydrogen, C₁₋₆ alkyl, C₁₋₆ haloalkyl, C₁₋₆ alkoxy, cyano, C₁₋₆ alkoxycarbonyl, C₁₋₆ alkylcarbonyl or NR⁴²R⁴³; R³⁹ is C₁₋₆ alkyl or optionally substituted phenyl(C₁₋₂)alkyl; R⁴⁰ and R⁴¹ independently are, hydrogen, C₁₋₈ alkyl or phenyl (optionally substituted by halo, nitro, cyano, C₁₋₆ alkyl, C₁₋₆ haloalkyl, C₁₋₆ alkoxy or C₁₋₆ haloalkoxy); and R⁴² and R⁴³ are, independently, hydrogen, C₁₋₈ alkyl, C₃₋₇ cycloalkyl, C₃₋₆ alkenyl, C₃₋₆ alkynyl, C₂₋₆ haloalkyl, C₁₋₆ alkoxy(C₁₋₆)alkyl, C₁₋₆ alkoxycarbonyl(C₁₋₆)alkyl, carboxy(C₁₋₆)alkyl or phenyl(C₁₋₂)alkyl; or R⁴² and R⁴³ together with the N atom to which they are attached form a five, six or seven-membered heterocyclic ring which may contain one or two further hetero atoms selected from O, N or S and which may be optionally substituted by one or two C₁₋₆ alkyl groups.

Preferably n is 0.

Preferably A is C₁₋₄ alkylene, -C(O)- or C₁₋₄ alkyleneoxy.

When G is (i), D is preferably S or CR⁸=CR⁹, where R⁸ and R⁹ are, independently, hydrogen, halogen, C₁₋₆ alkyl, C₁₋₆ haloalkyl C₂₋₆ alkenyl, C₁₋₆ alkynyl, C₁₋₆ alkoxy or C₁₋₆ haloalkoxy.

When G is (ii), D is preferably S.

Preferably E is N or CR¹⁰ where R¹⁰ is hydrogen, halogen, C₁₋₆ alkyl, C₁₋₆ haloalkyl, C₁₋₆ alkoxy, C₁₋₆ haloalkoxy, C₁₋₆ alkoxy (C₁₋₆)alkyl, C₁₋₆ alkylthio or SF₅; or R¹ and R¹⁰ together with the atoms to which they are attached form a benzene ring optionally substituted by C₁₋₆ alkyl, C₁₋₆ haloalkyl or halogen.

Preferably G is (i).

Preferably M¹ is N(R¹¹)C(=O) where R¹¹ is hydrogen, C₁₋₆ alkyl, C₁₋₆ alkoxy(C₁₋₆)alkyl, benzyloxymethyl or benzoyloxymethyl.

Preferably Y is O.

Preferably J is N or CR¹⁷ where R¹⁷ is hydrogen, C₁₋₆ alkyl, C₁₋₆ haloalkyl, cyano, halogen or nitro.

R¹ is preferably hydrogen, halogen, C₁₋₆ alkyl, C₂₋₆ alkenyl, C₁₋₆ haloalkyl, C₁₋₆ alkoxy, C₁₋₆ haloalkoxy, C₁₋₆ alkylthio, C₁₋₆ haloalkylmio, C₃₋₆ cycloalkyl, cyano, nitro or SF₅.

R² is preferably C₁₋₁₀ alkyl, C₁₋₆ alkoxy(C₁₋₆)alkyl, allyl or propargyl.

Preferably R³, R⁴ and R⁵ are, independently, hydrogen, C₁₋₃ alkyl or halogen.

R⁶ is preferably C₁₋₁₀ alkyl or C₁₋₁₀ haloalkyl (each one of which may be substituted with a C₁₋₆ alkyloxime, C₁₋₆ haloalkyloxime, C₁₋₆ alkylhydrazone or C₁₋₆ haloalkylhydrazone group) or C₁₋₆ cyanoalkyl, C₂₋₆ alkenyl(C₁₋₆)alkyl, C₂₋₆ alkynyl(C₁₋₆)alkyl, C₃₋₇ cycloalkyl, C₃₋₇ halocycloalkyl, C₃₋₇ cyanocycloalkyl, C₁₋₃ alkyl(C₃₋₇)cycloalkyl, C₁₋₃ alkyl(C₃₋₇)halocycloalkyl, C₅₋₆ cycloalkenyl, C₃₋₇ cycloalkyl(C₁₋₆)alkyl, C₅₋₆ cycloalkenyl(C₁₋₆)alkyl, C₂₋₆ haloalkenyl(C₁₋₆)alkyl, C₁₋₆cyanoalkenyl(C₁₋₆)alkyl, C₁₋₆ alkoxy(C₁₋₆)alkyl, C₃₋₆ alkenyloxy(C₁₋₆)alkyl, C₃₋₆ alkynyloxy(C₁₋₆)alkyl, aryloxy(C₁₋₆)alkyl, C₁₋₆ carboxyalkyl, C₁₋₆ alkylcarbonyl(C₁₋₆)alkyl, C₂₋₆ alkenylcarbonyl(C₁₋₆)alkyl, C₂₋₆ alkynylcarbonyl(C₁₋₆)alkyl, C₁₋₆ alkoxycarbonyl(C₁₋₆)alkyl, C₃₋₆ alkenyloxycarbonyl(C₁₋₆)alkyl, C₃₋₆ alkynyloxycarbonyl(C₁₋₆)alkyl, aryloxycarbonyl(C₁₋₆)alkyl, C₁₋₆ alkylthio(C₁₋₆)alkyl, C₁₋₆ alkylsulfinyl(C₁₋₆)alkyl, C₁₋₆ alkylsulfonyl(C₁₋₆)alkyl, aminocarbonyl(C₁₋₆)alkyl, aminocarbonyl(C₂₋₆)alkenyl, aminocarbonyl(C₂₋₆)alkynyl, C₁₋₆ alkylaminocarbonyl(C₁₋₆)alkyl, di(C₁₋₆)alkylaminocarbonyl(C₁₋₆)alkyl, C₁₋₆alkylaminocarbonyl(C₂₋₆)alkenyl(C₁₋₆)alkyl, di(C₁₋₆)alkylaminocarbonyl(C₂₋₆)alkenyl(C₁₋₆)alkyl, alkylaminocarbonyl(C₂₋₆)alkynyl(C₁₋₆)alkyl, di(C₁₋₆)alkylaminocarbonyl(C₂₋₆)alkynyl(C₁₋₆)alkyl, phenyl (optionally substituted by halo, nitro, cyano, C₁₋₆ alkyl, C₁₋₆ haloalkyl, C₁₋₆ alkoxy or C₁₋₆ haloalkoxy), phenyl(C₁₋₄)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy), phenyl(C₂₋₄)alkenyl(C₁₋₆)alkyl, (wherein the phenyl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy), heteroaryl (optionally substituted by halo, nitro, cyano, C₁₋₆ alkyl, C₁₋₆ haloalkyl, C₁₋₆ alkoxy or C₁₋₆ haloalkoxy), heterocyclyl (wherein the heterocyclyl group is optionally substituted by halo, nitro, cyano, C₁₋₆ alkyl, C₁₋₆ haloalkyl, C₁₋₆ alkoxy or C₁₋₆ haloalkoxy), heteroaryl(C₁₋₄)alkyl (wherein the heteroaryl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy)or heterocyclyl(C₁₋₄)alkyl (wherein the heterocyclyl group is optionally substituted by halo, nitro, cyano, C₁₋₆ alkyl, C₁₋₆ haloalkyl, C₁₋₆ alkoxy or C₁₋₆ haloalkoxy).

Preferably the optionally substituted ring of formula 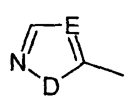 is optionally substituted isothiazolyl, optionally substituted pyridyl, optionally substituted pyrimidinyl, optionally substituted quinazolinyl and optionally substituted quinolinyl groups in which the optional substituents are chosen from halo, C₁₋₆ alkyl, C₁₋₆ haloalkyl, C₁₋₆ alkoxy, C₁₋₆ alkoxy(C₁₋₆)alkyl or C₁₋₆ haloalkoxy.

Preferably the optionally substituted ring of formula 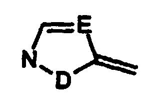 to

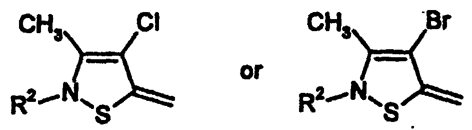

More preferably A is CH₂ or CH₂O, even more preferably CH₂.

More preferably M¹ is N(R¹¹)C(=O), where R¹¹ is hydrogen, C₁₋₄ alkyl, C₁₋₄ alkoxy(C₁₋₄)alkyl, benzyloxymethyl or benzoyloxymethyl. It is especially preferred that R¹¹ is hydrogen, ethyl, ethoxymethyl, allyl or propargyl.

More preferably J is N or CR¹⁷, where R¹⁷ is hydrogen, methyl or halogen.

More preferably R² is ethyl, ethoxymethyl, allyl or propargyl.

More preferably R³, R⁴ and R⁵ are independently, hydrogen, or halogen (especially fluorine); it is especially preferred that each of R³, R⁴ and R⁵ is hydrogen.

More preferably R⁶ is C₁₋₈ alkyl, C₂₋₈ haloalkyl, C₁₋₈ cyanoalkyl, C₃₋₇ cycloalkyl, C₁₋₃ alkyl (C₃₋₇) cycloalkyl, C₁₋₆ alkoxy (C₁₋₆) alkyl, heterocyclic (optionally substituted by halo, nitro, cyano, C₁₋₆ alkyl, C₁₋₆ haloalkyl, C₁₋₆ alkoxy or C₁₋₆ haloalkoxy), phenyl (optionally substituted by halogen, C₁₋₆ alkyl, C₁₋₆ haloalkyl, C₁₋₆ alkoxy, nitro, cyano or C₁₋₆ alkylsulfonyl) or heteroaryl (optionally substituted by halogen, C₁₋₆ alkyl, C₁₋₆ haloalkyl, C₁₋₆ alkoxy, nitro, cyano or C₁₋₆ alkylsulfonyl).

Even more preferably R⁶ is C₁₋₈ alkyl, C₂₋₆ haloalkyl, C₁₋₆alkoxy(C₁₋₆)alkyl, C₃₋₇cycloalkyl or C₃₋₇ cycloalkyl(C₁₋₆)alkyl.

Even more preferably the group 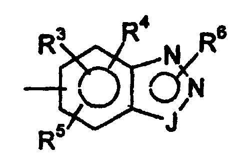 is

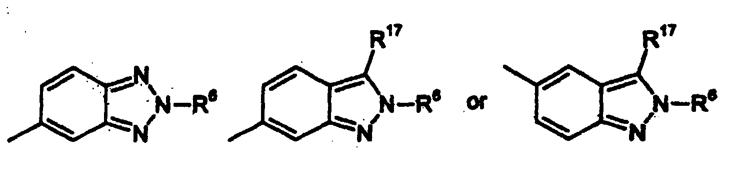

Most preferably the optionally substituted ring of formula

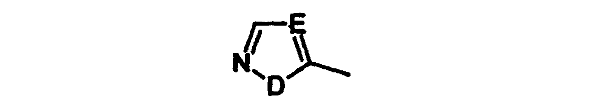

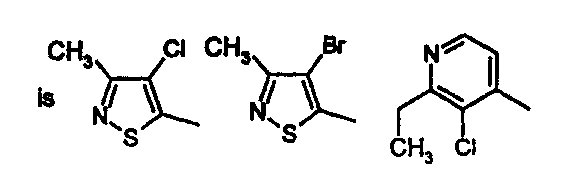

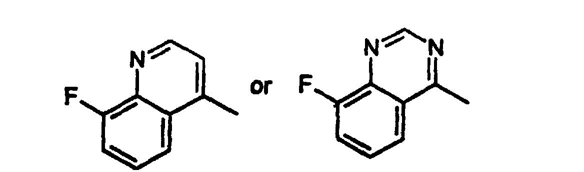

In a further aspect the present invention provides a compound of formula (IB): 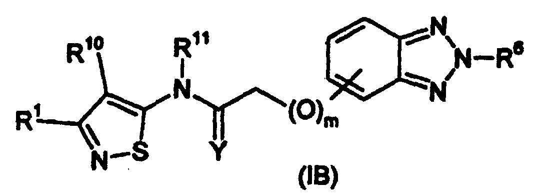 wherein R¹ is hydrogen, halogen, C₁₋₆ alkyl, C₁₋₆ haloalkyl, C₁₋₆ alkoxy, C₁₋₆ haloalkoxy, C₁₋₆ alkylthio, C₃₋₆ cycloalkyl or C₁₋₆ alkoxy(C₁₋₆)alkyl; R¹⁰ is hydrogen, halogen, C₁₋₆ alkyl, C₁₋₆ haloalkyl, cyano, nitro, CHO, CH=NOR²⁰, C₁₋₆ alkylcarbonyl, C₁₋₆ alkoxycarbonyl or C₁₋₆ alkylS(O)_{q}; or together R¹ and R¹⁰ form a five or six membered saturated or unsaturated carbocyclic ring, optionally substituted by one or two C₁₋₆ alkyl groups; R¹¹ is hydrogen, C₁₋₆ alkyl, CH₂(C₁₋₄ haloalkyl), C₃₋₆ alkenyl, C₃₋₆ alkynyl, C₁₋₆ alkoxy(C₁₋₆)alkyl, C₁₋₆ alkylcarbonyl, C₁₋₆ alkoxycarbonyl, formyl, optionally substituted phenoxycarbonyl, optionally substituted phenyl(C₁₋₄)alkyl or S(O)ₚR⁴⁴; R⁶ is hydrogen, C₁₋₈ alkyl, C₁₋₆ haloalkyl, C₁₋₆ cyanoalkyl, C₃₋₆ alkenyl, C₃₋₆ alkynyl, C₃₋₇ cycloalkyl, C₃₋₆ cycloalkyl(C₁₋₆)alkyl, C₃₋₆ haloalkenyl, C₁₋₆ alkoxy(C₁₋₆)alkyl, C₁₋₆ carboxyalkyl, C₁₋₆ alkylcarbonyl(C₁₋₆)alkyl, C₁₋₆ alkoxycarbonyl(C₁₋₆)alkyl, C₁₋₆ alkylthio(C₁₋₆)alkyl, C₁₋₆ alkylsulfinyl(C₁₋₆)alkyl, C₁₋₆ alkylsulfonyl(C₁₋₆)alkyl, aminocarbonyl(C₁₋₆)alkyl, C₁₋₆alkylaminocarbonyl(C₁₋₆)alkyl, di(C₁₋₆)alkylaminocarbonyl(C₁₋₆)alkyl, optionally substituted phenyl, optionally substituted phenyl(C₁₋₄)alkyl or optionally substituted heteroaryl; R²⁰ is hydrogen, C₁₋₆ alkyl, optionally substituted phenyl or optionally substituted phenyl(C₁₋₄)alkyl; R⁴⁴ is C₁₋₆ alkyl, C₁₋₆ haloalkyl or optionally substituted phenyl; Y is O or S; m is 0 or 1; and p and q are, independently, 0, 1 or 2; provided that when R⁶ is C₃₋₆ alkenyl, C₃₋₆ alkynyl or C₃₋₆ haloalkenyl, R⁶ does not have an unsaturated carbon atom attached directly to N and provided that when R¹¹ is C₃₋₆ alkenyl or C₃₋₆ alkynyl, R¹¹ does not have an unsaturated carbon atom attached directly to N.

It is preferred that R¹ is ethyl or, especially, methyl.

It is preferred that R¹⁰ is hydrogen, halogen (especially chloro) or cyano.

It is preferred that m is 0.

It is preferred that the compounds of the invention are of formula (Ia).

In one particular aspect the present invention provides a compound of formula (Ia) wherein R¹ is C₁₋₄ alkyl (especially methyl or ethyl); R¹⁰ is hydrogen, halogen (especially chloro or bromo) or cyano; or R¹ and R¹⁰ together form a cyclopentyl, cyclohexyl or phenyl ring; R¹¹ is hydrogen, C₁₋₄ alkyl (especially methyl or ethyl), phenyl(C₁₋₄)alkyl (especially benzyl), C₁₋₄ alkylcarbonyl (especially acetyl) or C₁₋₄ alkylsulfonyl (especially methanesulfonyl); m is 0; Y is oxygen; and R⁶ is C₁₋₆ alkyl [optionally substituted with halogen, C₁₋₄ alkoxy, phenyl (itself optionally substituted with halogen), CO₂H, CONH₂ (itself optionally substituted with C₁₋₄ alkyl), cyano, C₃₋₆ cycloalkyl or CO(C₁₋₄ alkoxy)], C₃₋₆ alkenyl, C₃₋₆ alkynyl, C₃₋₆ cycloalkyl, phenyl [optionally substituted with halogen, C₁₋₄ haloalkyl (especially CF₃), nitro, CO₂H, or cyano] or heteroaryl (especially pyridyl or pyrimidinyl) [optionally substituted with C₁₋₄ alkyl or C₁₋₄ haloalkyl].

In a further aspect the present invention provides a compound of formula (IC): 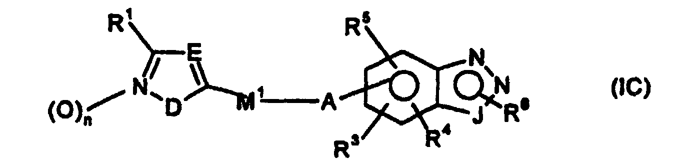 wherein n is 0 or 1; A is optionally substituted C₁₋₆ alkylene, optionally substituted C₂₋₆ alkenylene, optionally substituted C₂₋₆ alkynylene, optionally substituted C₁₋₆ alkylenoxy, optionally substituted oxy(C₁₋₆)alkylene, optionally substituted C₁₋₆ alkylenethio, optionally substituted thio(C₁₋₆)alkylene, optionally substituted C₁₋₆ alkylenamino, optionally substituted amino(C₁₋₆)alkylene, optionally substituted [C₁₋₆ alkyleneoxy(C₁₋₆)alkylene], optionally substituted [C₁₋₆ alkylenethio(C₁₋₆)alkylene], optionally substituted [C₁₋₆ alkylenesulfinyl(C₁₋₆)alkylene], optionally substituted [C₁₋₆ alkylenesulfonyl(C₁₋₆)alkylene] or optionally substituted [C₁₋₆ alkyleneamino(C₁₋₆)alkylene]; D is S, NR⁷, CR⁸=CR⁹, CR⁸=N, CR⁸=N(O), N=CR⁹ or N(O)=CR⁹; R⁷ is C₁₋₆ alkyl; E is N, N-oxide or CR¹⁰; M¹ is OC(=Y), N(R¹¹)C(=Y), N=C(OR¹²), N=C(SR¹³) or N=C(NR¹⁴R¹⁵) where O or N is the atom of attachment to the ring containing E and D; Y is O, S or NR¹⁶; J is N or CR¹⁷; R¹ is hydrogen, halogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₁₋₆ alkylthio, optionally substituted C₃₋₇ cycloalkyl, cyano, nitro or SF₅; R¹¹ is hydrogen, optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀ alkylaminocarbonyl, optionally substituted di(C₁₋₁₀)alkylaminocarbonyl, optionally substituted phenoxycarbonyl, optionally substituted C₁₋₆ alkylthio, optionally substituted C₁₋₆ alkylsulfinyl, optionally substituted C₁₋₆ alkylsulfonyl, optionally-substituted C₁₋₆ arylthio, optionally substituted C₁₋₆ arylsulfinyl, optionally substituted C₁₋₆ arylsulfonyl or R²²R²³NS; R¹² is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀alkylaminocarbonyl, optionally substituted di(C₁₋₁₀)alkylaminocarbonyl, , amino, optionally substituted C₁₋₆ alkylamino, optionally substituted di(C₁₋₆)alkylamino, optionally substituted phenoxycarbonyl, tri(C₁₋₄)alkylsilyl, aryldi(C₁₋₄)alkylsilyl, (C₁₋₄)alkyldiarylsilyl or triarylsilyl; R¹³ is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, optionally substituted C₁₋₁₀ alkylaminocarbonyl, optionally substituted di(C₁₋₁₀)alkylaminocarbonyl or optionally substituted phenoxycarbonyl); R¹⁴ and R¹⁵ are, independently optionally substituted C₁₋₁₀ alkyl, optionally substituted C₁₋₆ alkoxy, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀ alkylaminocarbonyl, optionally substituted di(C₁₋₁₀)alkylaminocarbonyl, hydroxy, amino, optionally substituted C₁₋₆ alkylamino, optionally substituted di(C₁₋₆)alkylamino, or optionally substituted phenoxycarbonyl; R³, R⁴ and R⁵ are, independently, hydrogen, halogen, optionally substituted C₁₋₆ alkyl, optionally substituted - C₁₋₆ alkoxy, optionally substituted C₁₋₆ alkylthio, optionally substituted C₁₋₆ alkylsulfinyl, optionally substituted C₁₋₆ alkylsulfonyl, cyano, nitro, optionally substituted C₁₋₆ alkylcarbonyl, optionally substituted C₁₋₆ alkoxycarbonyl or SF₅; R⁶ is hydrogen, cyano, optionally substituted C₁₋₂₀ alkyl, optionally substituted C₂₋₂₀ alkenyl(C₁₋₆)alkyl, optionally substituted C₂₋₂₀ alkynyl(C₁₋₆)alkyl, optionally substituted C₃₋₇ cycloalkyl, optionally substituted C₅₋₆ cycloalkenyl, formyl, optionally substituted C₁₋₂₀ alkoxycarbonyl, optionally substituted C₁₋₂₀ alkylcarbonyl, aminocarbonyl, optionally substituted C₁₋₂₀ alkylaminocarbonyl, optionally substituted di(C₁₋₂₀)alkylaminocarbonyl, optionally substituted aryloxycarbonyl, optionally substituted arylcarbonyl, optionally substituted arylaminocarbonyl, optionally substituted N-alkyl-N-arylaminocarbonyl, optionally substituted diarylaminocarbonyl, optionally substituted heteroaryloxycarbonyl, optionally substituted heteoarylcarbonyl, optionally substituted hetoroarylaminocarbonyl, optionally substituted alkylheteroarylaminocarbonyl, optionally substituted diheteroarylaminocarbonyl, optionally substituted phenyl, optionally substituted heteroaryl, optionally substituted heterocyclyl, optionally substituted C₁₋₂₀ alkylsulfonyl, or optionally substituted arylsulfonyl; R⁸ and R⁹ are, independently, hydrogen, halogen, cyano, nitro, optionally substituted C₁₋₆ alkyl, optionally substituted C₂₋₆ alkenyl, optionally substituted C₂₋₆ alkynyl or optionally substituted C₁₋₆ alkoxy; R¹⁰ is hydrogen, halogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₁₋₆ alkylthio, optionally substituted C₁₋₆ alkylsulfinyl, optionally substituted C₁₋₆ alkylsulfonyl, cyano, nitro, formyl, R²⁰ON=C(R²¹), optionally substituted C₁₋₆ alkylcarbonyl, optionally substituted C₁₋₆ alkoxycarbonyl or SF₅; or R¹ and R¹⁰ together with the atoms to which they are attached may be joined to form a five, six or seven-membered saturated or unsaturated ring carbocylic or heterocyclic ring which may contain one or two hetero atoms selected from O, N or S and which may be optionally substituted by C₁₋₆ alkyl, C₁₋₆ haloalkyl or halogen; R¹⁶ is hydrogen, cyano, nitro, optionally substituted C₁₋₆ alkyl, optionally substituted C₃₋₇ cycloalkyl, optionally substituted (C₂₋₆)alkenyl(C₁₋₆)alkyl, optionally substituted (C₂₋₆)alkynyl(C₁₋₆)alkyl, optionally substituted phenyl, optionally substituted heteroaryl, optionally substituted C₁₋₆ alkylcarbonyl, optionally substituted C₁₋₆ alkoxycarbonyl, optionally substituted C₁₋₆ alkylamino, optionally substituted di(C₁₋₆)alkylamino, optionally substituted C₁₋₆ alkylcarbonylamino, optionally substituted C₁₋₆ alkoxycarbonylamino, optionally substituted C₁₋₆ alkoxy, optionally substituted C₁₋₆ alkylthio, optionally substituted C₁₋₆ alkylsulfinyl, optionally substituted C₁₋₆ alkylsulfonyl, optionally substituted arylthio, optionally substituted arylsulfinyl, optionally substituted arylsulfonyl or C₁₋₆ acyloxy; R¹⁷ is hydrogen, halogen, nitro, cyano, optionally substituted - C₁₋₈ alkyl, optionally substituted C₂₋₆ alkenyl, optionally substituted C₂₋₆ alkynyl, optionally substituted C₃₋₇ cycloalkyl, optionally substituted C₁₋₆ alkoxycarbonyl, optionally substituted C₁₋₆ alkylcarbonyl, optionally substituted C₁₋₆ alkylaminocarbonyl, optionally substituted di(C₁₋₆)alkylaminocarbonyl, optionally substituted phenyl or optionally substituted heteroaryl; R²² and R²³ are, independently, optionally substituted C₁₋₆ alkyl or R²² and R²³ together with the N atom to which they are attached form a five, six or seven-membered heterocyclic ring which may contain one or two further hetero atoms selected from O, N or S and which may be optionally substituted by one or two C₁₋₆ alkyl groups; R²¹ is hydrogen, optionally substituted phenyl or optionally substituted C₁₋₆ alkyl; and R²⁰ is hydrogen, optionally substituted phenyl (C₁₋₂)alkyl or optionally substituted C₁₋₂₀ alkyl.

The compounds of formula (IC) may exist in different geometric or optical isomers or tautomeric forms. This invention covers all such isomers and tautomers and mixtures thereof in all proportions.

In a further aspect, the present invention provides a compound of formula (ID): 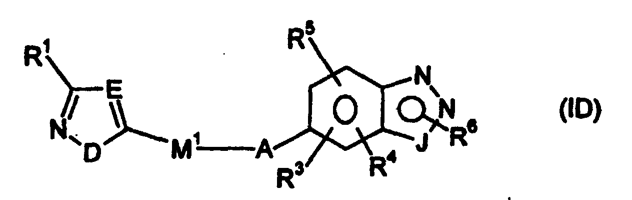 wherein A, D, E, M¹, Y, J, R¹, R³, R⁴, R⁵ and R⁶ are as defined above for a compound of formula (IC). More preferred compounds of formula (ID) are those of formula (IE) 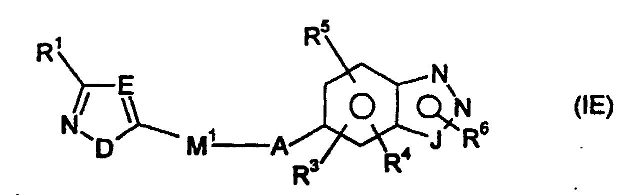 wherein D is S, NR⁷, CR⁸=CR⁹, CR⁸=N, CR⁸=N(O), N=CR⁹ or N(O)=CR⁹; E is N or CR¹⁰; R¹ is hydrogen, halogen, C₁₋₆ alkyl, C₂₋₆ alkenyl, C₂₋₆ alkynyl, C₁₋₆ cyanoalkyl, C₁₋₆ haloalkyl, C₁₋₆ alkoxy, C₁₋₆ haloalkoxy, C₁₋₆ alkylthio, C₁₋₆ haloalkylthio, C₃₋₆ cycloalkyl, C₃₋₇ cycloalkyl(C₁₋₄)alkyl, C₁₋₆ alkoxy(C₁₋₆)alkyl, cyano, nitro or SF₅; A is C₁₋₆ alkylene, C₁₋₆ alkenylene, C₁₋₆ alkylenoxy, oxy(C₁₋₆)alkylene, C₁₋₆ alkylenamino or C₁₋₆ alkylenethio, each of which is optionally substituted by C₁₋₃ alkyl, C₁₋₃ haloalkyl, C₁₋₃ cyanoalkyl, halogen, C₁₋₃ alkoxy, C₁₋₆ alkoxycarbonyl, cyano, =O, =NR³⁶ or =CR³⁷R³⁸; M¹ is N(R¹¹)C(=Y) or N=C(SR¹³) where O or N is the atom of attachment to the ring containing E and D; Y is O, S or NR¹⁶; J is N or CR¹⁷; R¹¹ is hydrogen, C₁₋₁₀ alkyl, benzyloxymethyl, benzoyloxymethyl, C₁₋₆alkoxy(C₁₋₆)alkyl, C₂₋₆ alkenyl(C₁₋₆)alkyl (especially allyl), C₂₋₆ alkynyl(C₁₋₆)alkyl (especially propargyl), C₁₋₁₀ alkylcarbonyl or C₁₋₁₀ alkoxycarbonyl (especially *iso*butoxycarbonyl); R¹³ is C₁₋₁₀ alkyl, benzyloxymethyl, benzoyloxymethyl, C₁₋₆alkoxy(C₁₋₆)alkyl, C₂₋₆ alkenyl(C₁₋₆)alkyl (especially allyl), C₂₋₆ alkynyl(C₁₋₆)alkyl (especially propargyl), C₁₋₁₀ alkylcarbonyl or C₁₋₁₀ alkoxycarbonyl (especially *iso*butoxycarbonyl); R³, R⁴ and R⁵ are independently selected from hydrogen, halogen, C₁₋₆ alkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ haloalkyl, cyano, nitro, C₁₋₆ alkylcarbonyl, C₁₋₆ alkoxycarbonyl or SF₅; R⁶ is cyano, C₁₋₈ alkyl, C₂₋₆ haloalkyl, C₁₋₆ cyanoalkyl, C₂₋₆ alkenyl(C₁₋₆)alkyl, C₂₋₆ alkynyl(C₁₋₆)alkyl, C₃₋₇ cycloalkyl, C₃₋₇ halocycloalkyl, C₃₋₇ cyanocycloalkyl, C₁₋₃alkyl(C₃₋₇)cycloalkyl, C₁₋₃ alkyl(C₃₋₇)halocycloalkyl, C₅₋₆ cycloalkenyl, C₃₋₇ cycloalkyl(C₁₋₆)alkyl, C₅₋₆ cycloalkenyl(C₁₋₆)alkyl, C₂₋₆ haloalkenyl(C₁₋₆)alkyl, C₁₋₆ cyanoalkenyl(C₁₋₆)alkyl, C₁₋₆ alkoxy(C₁₋₆)alkyl, C₃₋₆ alkenyloxy(C₁₋₆)alkyl, C₃₋₆ alkynyloxy(C₁₋₆)alkyl, aryloxy(C₁₋₆)alkyl, formyl, C₁₋₆ carboxyalkyl, C₁₋₆ alkylcarbonyl(C₁₋₆)alkyl, C₂₋₆ alkenylcarbonyl(C₁₋₆)alkyl, C₂₋₆ alkynylcarbonyl(C₁₋₆)alkyl, C₁₋₆ alkoxycarbonyl(C₁₋₆)alkyl, C₃₋₆ alkenyloxycarbonyl(C₁₋₆)alkyl, C₃₋₆ alkynyloxycarbonyl(C₁₋₆)alkyl, aryloxycarbonyl(C₁₋₆)alkyl, C₁₋₆ alkylthio(C₁₋₆)alkyl, C₁₋₆ alkylsulfinyl(C₁₋₆)alkyl, C₁₋₆ alkylsulfonyl(C₁₋₆)alkyl, aminocarbonyl(C₁₋₆)alkyl, amiocarbonyl(C₂₋₆)alkenyl, aminocarbonyl(C₂₋₆)alkynyl, C₁₋₆ alkylaminocarbonyl(C₁₋₆)alkyl, di(C₁₋₆)alkylaminocarbonyl(C₁₋₆)alkyl, C₁₋₆ alkylaminocarbonyl(C₂₋₆)alkenyl(C₁₋₆)alkyl, di(C₁₋₆)alkylaminocarbonyl(C₂₋₆)alkenyl(C₁₋₆)alkyl, alkylaminocarbonyl(C₂₋₆)alkynyl(C₁₋₆)alkyl, di(C₁₋₆)alkylaminocarbonyl(C₁₋₆)alkynyl(C₁₋₆)alkyl, C₁₋₆ alkoxycarbonyl, C₁₋₆ alkylcarbonyl, aminocarbonyl, C₁₋₆ alkylaminocarbonyl, di(C₁₋₆)alkylaminocabonyl, phenyl (optionally substituted by halo, nitro, cyano, C₁₋₆ alkyl, C₁₋₆ haloalkyl, C₁₋₆ alkoxy or C₁₋₆ haloalkoxy), phenyl(C₁₋₄)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy), phenyl(C₂₋₄)alkenyl(C₁₋₆)alkyl, (wherein the phenyl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy), heteroaryl (optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy), heterocyclyl (optionally substituted by halo, nitro, cyano, C₁₋₆ alkyl, C₁₋₆ haloalkyl, C₁₋₆ alkoxy or C₁₋₆ haloalkoxy), heteroaryl(C₁₋₄)alkyl (where the heteroaryl may be substituted by halo, nitro, cyano, C₁₋₆ alkyl, C₁₋₆ haloalkyl, C₁₋₆ alkoxy or C₁₋₆ haloalkoxy),or heterocyclyl(C₁₋₄)alkyl (where the heterocyclyl may be substituted by halo, cyano, C₁₋₆ alkyl, C₁₋₆ haloalkyl, C₁₋₆ alkoxy or C₁₋₆ haloalkoxy); R⁸ and R⁹ independently are, hydrogen, halogen, C₁₋₆ alkyl, C₁₋₆ haloalkyl C₂₋₆ alkenyl, C₁₋₆ alkynyl, C₁₋₆ alkoxy or C₁₋₆ haloalkoxy; R¹⁰ is hydrogen, halogen, C₁₋₆ alkyl, C₂₋₆ alkenyl, C₁₋₆ alkynyl, C₁₋₆ haloalkyl, C₁₋₆ alkoxy, C₁₋₆ alkoxy (C₁₋₆)alkyl, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ haloalkyl, cyano, nitro, formyl, CH=NOR²⁰, C₁₋₆ alkylcarbonyl, C₁₋₆ alkoxycarbonyl or SF₅; or together R¹ and R¹⁰ together with the atoms to which they are attached may be joined to form a five, six or seven-membered saturated or unsaturated ring carbocylic or heterocyclic ring which may contain one or two hetero atoms selected from O, N or S and which may be optionally substituted by C₁₋₆ alkyl, C₁₋₆ haloalkyl or halogen; R¹⁶ is cyano, nitro, C₁₋₆ alkyl, C₁₋₆ haloalkyl, C₃₋₇ cycloalkyl, C₃₋₇ cycloalkyl(C₁₋₆)alkyl, CH₂(C₂₋₆)alkenyl, CH₂(C₂₋₆)alkynyl, phenyl (optionally substituted by halo, nitro, cyano, C₁₋₆ alkyl, C₁₋₆ haloalkyl, C₁₋₆ alkoxy or C₁₋₆ haloalkoxy) heteroaryl (optionally substituted by halo, nitro, cyano, C₁₋₆ alkyl, C₁₋₆ haloalkyl, C₁₋₆ alkoxy or C₁₋₆ haloalkoxy), C₁₋₆ alkylcarbonyl, C₁₋₆ alkoxycarbonyl, C₁₋₆ alkylamino, di(C₁₋₆)alkylamino, C₁₋₆ alkylcarbonylamino, C₁₋₆ alkoxycarbonylamino, C₁₋₆ alkoxy, C₁₋₆ alkylthio, C₁₋₆ alkylsulfinyl, C₁₋₆ alkylsulfonyl, C₁₋₆ haloalkylthio, C₁₋₆ haloalkylsulfinyl, C₁₋₆ haloalkylsulfonyl, arylthio, arylsulfinyl, arylsulfonyl or OCO(C₁₋₆)alkyl; R¹⁷ is hydrogen, halogen, nitro, cyano, C₁₋₈ alkyl, C₁₋₆ haloalkyl, C₁₋₆ cyanoalkyl, C₂₋₆ alkenyl, C₂₋₆ alkynyl, C₃₋₇ cycloalkyl, C₂₋₆ haloalkenyl, C₃₋₇ cycloalkyl(C₁₋₆)alkyl, C₁₋₆alkox(C₁₋₆)alkyl, C₁₋₆ alkoxycarbonyl, C₁₋₆ alkylcarbonyl, C₁₋₆ alkylaminocarbonyl, di(C₁₋₆alkylaminocarbonyl, phenyl (optionally substituted by halo, nitro, cyano, C₁₋₆ alkyl, C₁₋₆ haloalkyl, C₁₋₆ alkoxy or C₁₋₆ haloalkoxy) or heteroaryl (optionally substituted by halo, nitro, cyano, C₁₋₆ alky, C₁₋₆ haloalkyl, C₁₋₆ alkoxy or C₁₋₆ haloalkoxy); R³⁶ is C₁₋₆ alkyl, OR³⁹ or NR⁴⁰R⁴¹; R³⁷ is hydrogen, C₁₋₆ alkyl or C₁₋₆ haloalkyl; R³⁸ is hydrogen, C₁₋₆ alkyl, C₁₋₆ haloalkyl, C₁₋₆ alkoxy, cyano, C₁₋₆ alkoxycarbonyl, C₁₋₆ alkylcarbonyl or NR⁴²R⁴³; R²² and R²³ independently are, hydrogen, C₁₋₆ alkyl, CH₂(C₁₋₄ haloalkyl), C₁₋₆ cyanoalkyl, C₁₋₆ alkoxy(C₁₋₆)alkyl, C₁₋₆ alkylthio(C₁₋₆)alkyl, C₁₋₆ alkoxy(C₁₋₆)alkoxy(C₁₋₆)alkyl, phenyl(C₁₋₄)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy), heteroaryl(C₁₋₄)alkyl (wherein the heteroaryl group is optionally substituted by halo, nitro, cyano, C₁₋₆ alkyl, C₁₋₆ haloalkyl, C₁₋₆ alkoxy or C₁₋₆ haloalkoxy), heterocyclyl(C₁₋₄)alkyl (wherein the heterocyclyl group is optionally substituted by halo, nitro, cyano, C₁₋₆ alkyl, C₁₋₆ haloalkyl, C₁₋₆ alkoxy or C₁₋₆ haloalkoxy), or R²² and R²³ together with the N atom to which they are attached form a five, six or seven-membered heterocyclic ring which may contain one or two further hetero atoms selected from O, N or S and which may be optionally substituted by one or two C₁₋₆ alkyl groups; R³⁹ is C₁₋₆ alkyl or optionally substituted phenyl(C₁₋₂)alkyl; R⁴⁰ and R⁴¹ independently are, hydrogen, C₁₋₈ alkyl or phenyl (optionally substituted by halo, nitro, cyano, C₁₋₆ alkyl, C₁₋₆ haloalkyl, C₁₋₆ alkoxy or C₁₋₆ haloalkoxy); R²¹ is hydrogen or C₁₋₃ alkyl; R²⁰ is C₁₋₆ alkyl or phenyl(C₁₋₂)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy); and R⁴² and R⁴³ independently, are, hydrogen, C₁₋₈ alkyl, C₃₋₇ cycloalkyl, C₃₋₆ alkenyl, C₃₋₆ alkynyl, C₂₋₆ haloalkyl, C₁₋₆ alkoxy(C₁₋₆)alkyl, C₁₋₆ alkoxycarbonyl(C₁₋₆)alkyl, carboxy(C₁₋₆)alkyl or phenyl(C₁₋₂)alkyl; or R⁴² and R⁴³ together with the N atom to which they are attached form a five, six or seven-membered heterocyclic ring which may contain one or two farther hetero atoms selected from O, N or S and which may be optionally substituted by one or two C₁₋₆ alkyl groups.

Preferably A is C₁₋₄ alkylene -C(O)- or C₁₋₄ alkyleneoxy.

Preferably D is S or CR⁸=CR⁹, where R⁸ and R⁹ are, independently, hydrogen, halogen, C₁₋₆ alkyl, C₁₋₆ haloalkyl C₂₋₆ alkenyl, C₁₋₆ alkynyl, C₁₋₆ alkoxy or C₁₋₆ haloalkoxy.

Preferably E is N or CR¹⁰ where R¹⁰ is hydrogen, halogen, C₁₋₆ alkyl, C₁₋₆ haloalkyl, C₁₋₆ alkoxy, C₁₋₆ haloalkoxy, C₁₋₆ alkoxy (C₁₋₆)alkyl, C₁₋₆ alkylthio or SF₅; or R¹ and R¹⁰ together with the atoms to which they are attached form a benzene ring optionally substituted by C₁₋₆ alkyl, C₁₋₆ haloalkyl or halogen.

A preferred value of M¹ is N(R¹¹)C(=O) where N is the atom of attachment to the ring containing E and D; and R¹¹ is hydrogen, C₁₋₆ alkyl, C₁₋₆ alkoxy(C₁₋₆ )alkyl, benzyloxymethyl or benzoyloxymethyl.

Preferably Y is O.

Preferably J is N or CR¹⁷ where R¹⁷ is hydrogen, C₁₋₆ alkyl, C₁₋₆ haloalkyl, cyano, halogen or nitro.

R¹ is preferably hydrogen, halogen, C₁₋₆ alkyl, C₂₋₆ alkenyl, C₁₋₆ haloalkyl, C₁₋₆ alkoxy, C₁₋₆ haloalkoxy, C₁₋₆ alkylthio, C₁₋₆ haloalkylthio, C₃₋₆ cycloalkyl, cyano, nitro or SF₅.

Preferably R³, R⁴ and R⁵ are independently hydrogen, C₁₋₃ alkyl or halogen.

R⁶ is preferably C₁₋₈ alkyl, C₁₋₆ haloalkyl, C₁₋₆ cyanoalkyl, C₂₋₆ alkenyl(C₁₋₆)alkyl, C₂₋₆ alkynyl (C₁₋₆)alkyl, C₃₋₇ cycloalkyl, C₃₋₇ halocycloalkyl, C₃₋₇ cyanocycloalkyl, C₁₋₃ alkyl(C₃₋₇)cycloalkyl, C₁₋₃ alkyl(C₃₋₇)halocycloalkyl, C₅₋₆ cycloalkenyl, C₃₋₇ cycloalkyl(C₁₋₆)alkyl, C₅₋₆ cycloalkenyl(C₁₋₆)alkyl, C₂₋₆ haloalkenyl(C₁₋₆)alkyl, C₁₋₆ cyanoalkenyl(C₁₋₆)alkyl, C₁₋₆ alkoxy(C₁₋₆)alkyl, C₃₋₆ alkenyloxy(C₁₋₆)alkyl, C₃₋₆ alkynyloxy(C₁₋₆)alkyl, aryloxy(C₁₋₆)alkyl, C₁₋₆ carboxyalkyl, C₁₋₆ alkylcarbonyl(C₁₋₆)alkyl, C₂₋₆ alkenylcarbonyl(C₁₋₆)alkyl, C₂₋₆ alkynylcarbonyl(C₁₋₆)alkyl, C₁₋₆ alkoxycarbonyl(C₁₋₆)alkyl, C₃₋₆ alkenyloxycarbonyl(C₁₋₆)-alkyl, C₃₋₆ alkynyloxycarbonyl(C₁₋₆)alkyl, aryloxycarbonyl(C₁₋₆)alkyl, C₁₋₆ alkylthio(C₁₋₆)-alkyl, C₁₋₆ alkylsulfinyl(C₁₋₆)alkyl, C₁₋₆ alkylsulfonyl(C₁₋₆)alkyl, amiocarbonyl(C₁₋₆)alkyl, aminocarbonyl(C₂₋₆)alkenyl, aminocarbonyl(C₂₋₆)alkynyl, C₁₋₆ alkylaminocarbonyl(C₁₋₆)alkyl, di(C₁₋₆)alkylaminocarbonyl(C₁₋₆)alkyl, C₁₋₆ alkylaminocarbonyl(C₁₋₆)alkenyl(C₁₋₆)alkyl, di (C₁₋₆)alkylaminocarbonyl(C₁₋₆)alkenyl(C₁₋₆)alkyl, alkylaminocarbonyl(C₁₋₆)alkynyl(C₁₋₆)alkyl, di(C₁₋₆)alkylaminocarbonyl(C₁₋₆)alkynyl(C₁₋₆)alkyl, phenyl (optionally substituted by halo, nitro, cyano, C₁₋₆ alkyl, C₁₋₆ haloalkyl, C₁₋₆ alkoxy or C₁₋₆ haloalkoxy), phenyl(C₁₋₄)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy), phenyl(C₂₋₄)alkenyl(C₁₋₆)alkyl, (wherein the phenyl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy), heteroaryl (optionally substituted by halo, nitro, cyano, C₁₋₆ alkyl, C₁₋₆ haloalkyl, C₁₋₆ alkoxy or C₁₋₆ haloalkoxy), heterocyclyl (wherein the heterocyclyl group is optionally substituted by halo, nitro, cyano, C₁₋₆ alkyl, C₁₋₆ haloalkyl, C₁₋₆ alkoxy or C₁₋₆ haloalkoxy), heteroaryl(C₁₋₄)alkyl (wherein the heteroaryl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy)or heterocyclyl(C₁₋₄)alkyl (wherein the heterocyclyl group is optionally substituted by halo, nitro, cyano, C₁₋₆ alkyl, C₁₋₆ haloalkyl, C₁₋₆ alkoxy or C₁₋₆ haloalkoxy).

More preferred optionally substituted rings of formula 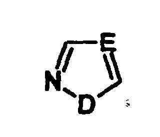 include optionally substituted isothiazolyl, optionally substituted pyridyl, optionally substituted pyrimidinyl, optionally substituted quinazolinyl and optionally substituted quinolinyl groups in which the optional substituents are chosen from halo, C₁₋₆ alkyl, C₁₋₆ haloalkyl, C₁₋₆ alkoxy, C₁₋₆ alkoxy(C₁₋₆)alkyl or C₁₋₆ haloalkoxy.

Most preferred optionally substituted rings of formula 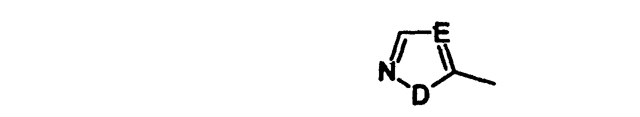 are

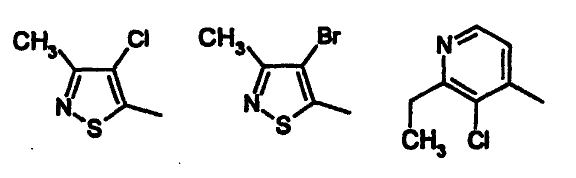

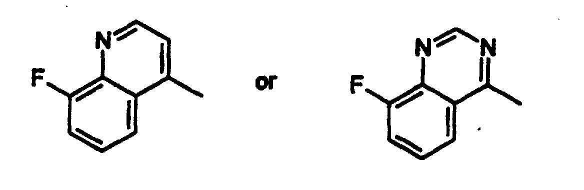

More preferably A is CH₂ or CH₂O, even more preferably CH₂.

It is more preferred that M¹ is NR¹¹C(=O), where R¹¹ is hydrogen, C₁₋₄ alkyl, C₁₋₄ alkoxy (C₁₋₄)alkyl, benzyloxymethyl or benzoyloxymethyl. It is especially preferred that R¹¹ is hydrogen, ethyl, ethoxymethyl, allyl or propargyl.

It is more preferred that R³, R⁴ and R⁵ are independently, hydrogen, or halogen (especially fluorine); it is especially preferred that each of R³, R⁴ and R⁵ is hydrogen.

More preferably J is N or CR¹⁷ where R¹⁷ is hydrogen, methyl, or halogen.

More preferably R⁶ is C₁₋₈ alkyl, C₂₋₈ haloalkyl, C₁₋₈ cyanoalkyl, C₃₋₇ cycloalkyl, C₁₋₃ alkyl (C₃₋₇) cycloalkyl, C₁₋₆ alkoxy (C₁₋₆) alkyl, heterocyclic (optionally substituted by halo, nitro, cyano, C₁₋₆ alkyl, C₁₋₆ haloalkyl, C₁₋₆ alkoxy or C₁₋₆ haloalkoxy), phenyl (optionally substituted by halogen, C₁₋₆ alkyl, C₁₋₆ haloalkyl, C₁₋₆ alkoxy, nitro, cyano or C₁₋₆ alkylsulfonyl) or heteroaryl (optionally substituted by halogen, C₁₋₆ alkyl, C₁₋₆ haloalkyl, C₁₋₆ alkoxy, nitro, cyano or C₁₋₆ alkylsulfonyl).

Particularly preferred values of R₆ are C₁₋₈ alkyl, C₂₋₆ haloalkyl, C₁₋₆alkoxy(C₁₋₆)alkyl, C ₃₋₇cycloalkyl or C₃₋₇ cycloalkyl(C₁₋₆)alkyl.

Particularly preferred values of the group: 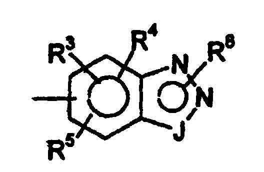 are

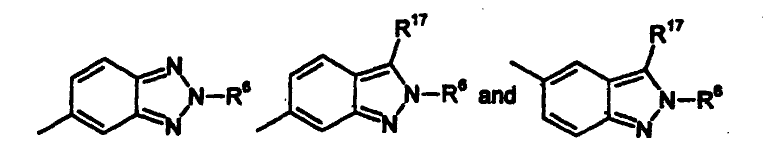

The compounds in the following Tables illustrate compounds of the invention.

### Table A1

Table A1 provides 432 compounds of Formula A1 wherein R¹, D, E, R¹¹, A and J are as defined in Table A1 and R⁶ is methyl.

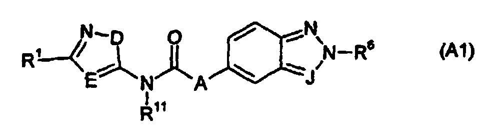

**Table A1**

| **Compound No.** | **R**^{**1**} | **D** | **E** | **R**^{**11**} | **A** | **J** |
|---|---|---|---|---|---|---|
| A1.1 | CH₃ | S | C-Cl | H | CH₂ | N |
| A1.2 | CH₃ | S | C-Cl | CH₂CH₃ | CH₂ | N |
| A1.3 | CH₃ | S | C-Cl | CH₂C=CH₂ | CH₂ | N |
| A1.4 | CH₃ | S | C-Cl | CH₂C.CH | CH₂ | N |
| A1.5 | CH₃ | S | C-Cl | CH₂OCH₃ | CH₂ | N |
| A1.6 | CH₃ | S | C-Cl | CH₂OCH₂CH₃ | CH₂ | N |
| A1.7 | CH₃ | S | C-Br | H | CH₂ | N |
| A1.8 | CH₃ | S | C-Br | CH₂CH₃ | CH₂ | N |
| A1.9 | CH₃ | S | C-Br | CH₂C=CH₂ | CH₂ | N |
| A1.10 | CH₃ | S | C-Br | CH₂C.CH | CH₂ | N |
| A1.11 | CH₃ | S | C-Br | CH₂OCH₃ | CH₂ | N |
| A1.12 | CH₃ | S | C-Br | CH₂OCH₂CH₃ | CH₂ | N |
| A1.13 | CH₂CH₃ | S | C-Cl | H | CH₂ | N |
| A1.14 | CH₂CH₃ | S | C-Cl | CH₂CH₃ | CH₂ | N |
| A1.15 | CH₂CH₃ | S | C-Cl | CH₂C=CH₂ | CH₂ | N |
| A1.16 | CH₂CH₃ | S | C-Cl | CH₂C.CH | CH₂ | N |
| A1.17 | CH₂CH₃ | S | C-Cl | CH₂OCH₃ | CH₂ | N |
| A1.18 | CH₂CH₃ | S | C-Cl | CH₂OCH₂CH₃ | CH₂ | N |
| A1.19 | CH₂CH₃ | S | C-Br | H | CH₂ | N |
| A1.20 | CH₂CH₃ | S | C-Br | CH₂CH₃ | CH₂ | N |
| A1.21 | CH₂CH₃ | S | C-Br | CH₂C=CH₂ | CH₂ | N |
| A1.22 | CH₂CH₃ | S | C-Br | CH₂C.CH | CH₂ | N |
| A1.23 | CH₂CH₃ | S | C-Br | CH₂OCH₃ | CH₂ | N |
| A1.24 | CH₂CH₃ | S | C-Br | CH₂OCH₂CH₃ | CH₂ | N |
| A1.25 | CH₃ | S | C-Cl | H | CH(CH₃) | N |
| A1.26 | CH₃ | S | C-Cl | CH₂CH₃ | CH(CH₃) | N |
| A1.27 | CH₃ | S | C-Cl | CH₂C=CH₂ | CH(CH₃) | N |
| A1.28 | CH₃ | S | C-Cl | CH₂C.CH | CH(CH₃) | N |
| A1.29 | CH₃ | S | C-Cl | CH₂OCH₃ | CH(CH₃) | N |
| A1.30 | CH₃ | S | C-Cl | CH₂OCH₂CH₃ | CH(CH₃) | N |
| A1.31 | CH₃ | S | C-Br | H | CH(CH₃) | N |
| A1.32 | CH₃ | S | C-Br | CH₂CH₃ | CH(CH₃) | N |
| A1.33 | CH₃ | S | C-Br | CH₂C=CH₂ | CH(CH₃) | N |
| A1.34 | CH₃ | S | C-Br | CH₂C.CH | CH(CH₃) | N |
| A1.35 | CH₃ | S | C-Br | CH₂OCH₃ | CH(CH₃) | N |
| A1.36 | CH₃ | S | C-Br | CH₂OCH₂CH₃ | CH(CH₃) | N |
| A1.37 | CH₂CH₃ | S | C-Cl | H | CH(CH₃) | N |
| A1.38 | CH₂CH₃ | S | C-Cl | CH₂CH₃ | CH(CH₃) | N |
| A1.39 | CH₂CH₃ | S | C-Cl | CH₂C=CH₂ | CH(CH₃) | N |
| A1.40 | CH₂CH₃ | S | C-Cl | CH₂C.CH | CH(CH₃) | N |
| A1.41 | CH₂CH₃ | S | C-Cl | CH₂OCH₃ | CH(CH₃) | N |
| A1.42 | CH₂CH₃ | S | C-Cl | CH₂OCH₂CH₃ | CH(CH₃) | N |
| A1.43 | CH₂CH₃ | S | C-Br | H | CH(CH₃) | N |
| A1.44 | CH₂CH₃ | S | C-Br | CH₂CH₃ | CH(CH₃) | N |
| A1.45 | CH₂CH₃ | S | C-Br | CH₂C=CH₂ | CH(CH₃) | N |
| A1.46 | CH₂CH₃ | S | C-Br | CH₂C.CH | CH(CH₃) | N |
| A1.47 | CH₂CH₃ | S | C-Br | CH₂OCH₃ | CH(CH₃) | N |
| A1.48 | CH₂CH₃ | S | C-Br | CH₂OCH₂CH₃ | CH(CH₃) | N |
| A1.49 | CH₃ | S | C-Cl | H | CHF | N |
| A1.50 | CH₃ | S | C-Cl | CH₂CH₃ | CHF | N |
| A1.51 | CH₃ | S | C-Cl | CH₂C=CH₂ | CHF | N |
| A1.52 | CH₃ | S | C-Cl | CH₂C.CH | CHF | N |
| A1.53 | CH₃ | S | C-Cl | CH₂OCH₃ | CHF | N |
| A1.54 | CH₃ | S | C-Cl | CH₂OCH₂CH₃ | CHF | N |
| A1.55 | CH₃ | S | C-Br | H | CHF | N |
| A1.56 | CH₃ | S | C-Br | CH₂CH₃ | CHF | N |
| A1.57 | CH₃ | S | C-Br | CH₂C=CH₂ | CHF | N |
| A1.58 | CH₃ | S | C-Br | CH₂C.CH | CHF | N |
| A1.59 | CH₃ | S | C-Br | CH₂OCH₃ | CHF | N |
| A1.60 | CH₃ | S | C-Br | CH₂OCH₂CH₃ | CHF | N |
| A1.61 | CH₂CH₃ | S | C-Cl | H | CHF | N |
| A1.62 | CH₂CH₃ | S | C-Cl | CH₂CH₃ | CHF | N |
| A1.63 | CH₂CH₃ | S | C-Cl | CH₂C=CH₂ | CHF | N |
| A1.64 | CH₂CH₃ | S | C-Cl | CH₂C.CH | CHF | N |
| A1.65 | CH₂CH₃ | S | C-Cl | CH₂OCH₃ | CHF | N |
| A1.66 | CH₂CH₃ | S | C-Cl | CH₂OCH₂CH₃ | CHF | N |
| A1.67 | CH₂CH₃ | S | C-Br | H | CHF | N |
| A1.68 | CH₂CH₃ | S | C-Br | CH₂CH₃ | CHF | N |
| A1.69 | CH₂CH₃ | S | C-Br | CH₂C=CH₂ | CHF | N |
| A1.70 | CH₂CH₃ | S | C-Br | CH₂C.CH | CHF | N |
| A1.71 | CH₂CH₃ | S | C-Br | CH₂OCH₃ | CHF | N |
| A1.72 | CH₂CH₃ | S | C-Br | CH₂OCH₂CH₃ | CHF | N |
| A1.73 | CH₃ | S | C-Cl | H | CH₂O | N |
| A1.74 | CH₃ | S | C-Cl | CH₂CH₃ | CH₂O | N |
| A1.75 | CH₃ | S | C-Cl | CH₂C=CH₂ | CH₂O | N |
| A1.76 | CH₃ | S | C-Cl | CH₂C.CH | CH₂O | N |
| A1.77 | CH₃ | S | C-Cl | CH₂OCH₃ | CH₂O | N |
| A1.78 | CH₃ | S | C-Cl | CH₂OCH₂CH₃ | CH₂O | N |
| A1.79 | CH₃ | S | C-Br | H | CH₂O | N |
| A1.80 | CH₃ | S | C-Br | CH₂CH₃ | CH₂O | N |
| A1.81 | CH₃ | S | C-Br | CH₂C=CH₂ | CH₂O | N |
| A1.82 | CH₃ | S | C-Br | CH₂C.CH | CH₂O | N |
| A1.83 | CH₃ | S | C-Br | CH₂OCH₃ | CH₂O | N |
| A1.84 | CH₃ | S | C-Br | CH₂OCH₂CH₃ | CH₂O | N |
| A1.85 | CH₂CH₃ | S | C-Cl | H | CH₂O | N |
| A1.86 | CH₂CH₃ | S | C-Cl | CH₂CH₃ | CH₂O | N |
| A1.87 | CH₂CH₃ | S | C-Cl | CH₂C=CH₂ | CH₂O | N |
| A1.88 | CH₂CH₃ | S | C-Cl | CH₂C.CH | CH₂O | N |
| A1.89 | CH₂CH₃ | S | C-Cl | CH₂OCH₃ | CH₂O | N |
| A1.90 | CH₂CH₃ | S | C-Cl | CH₂OCH₂CH₃ | CH₂O | N |
| A1.91 | CH₂CH₃ | S | C-Br | H | CH₂O | N |
| A1.92 | CH₂CH₃ | S | C-Br | CH₂CH₃ | CH₂O | N |
| A1.93 | CH₂CH₃ | S | C-Br | CH₂C=CH₂ | CH₂O | N |
| A1.94 | CH₂CH₃ | S | C-Br | CH₂C.CH | CH₂O | N |
| A1.95 | CH₂CH₃ | S | C-Br | CH₂OCH₃ | CH₂O | N |
| A1.96 | CH₂CH₃ | S | C-Br | CH₂OCH₂CH₃ | CH₂O | N |
| A1.97 | CH₃ | CH=CH | C-Cl | H | CH₂ | N |
| A1.98 | CH₃ | CH=CH | C-Cl | CH₂CH₃ | CH₂ | N |
| A1.99 | CH₃ | CH=CH | C-Cl | CH₂C=CH₂ | CH₂ | N |
| A1.100 | CH₃ | CH=CH | C-Cl | CH₂C.CH | CH₂ | 'N |
| A1.101 | CH₃ | CH=CH | C-Cl | CH₂OCH₃ | CH₂ | N |
| A1.102 | CH₃ | CH=CH | C-Cl | CH₂OCH₂CH₃ | CH₂ | N |
| A1.103 | CH₂CH₃ | CH=CH | C-Cl | H | CH₂ | N |
| A1.104 | CH₂CH₃ | CH=CH | C-Cl | CH₂CH₃ | CH₂ | N |
| A1.105 | CH₂CH₃ | CH=CH | C-Cl | CH₂C=CH₂ | CH₂ | N |
| A1.106 | CH₂CH₃ | CH=CH | C-Cl | CH₂C.CH | CH₂ | N |
| A1.107 | CH₂CH₃ | CH=CH | C-Cl | CH₂OCH₃ | CH₂ | N |
| A1.108 | CH₂CH₃ | CH=CH | C-Cl | CH₂OCH₂CH₃ | CH₂ | N |
| A1.109 | CH₃ | CH=CH | C-Cl | H | CH(CH₃) | N |
| A1.110 | CH₃ | CH=CH | C-Cl | CH₂CH₃ | CH(CH₃) | N |
| A1.111 | CH₃ | CH=CH | C-Cl | CH₂C=CH₂ | CH(CH₃) | N |
| A1.112 | CH₃ | CH=CH | C-Cl | CH₂C.CH | CH(CH₃) | N |
| A1.113 | CH₃ | CH=CH | C-Cl | CH₂OCH₃ | CH(CH₃) | N |
| A1.114 | CH₃ | CH=CH | C-Cl | CH₂OCH₂CH₃ | CH(CH₃) | N |
| A1.115 | CH₂CH₃ | CH=CH | C-Cl | H | CH(CH₃) | N |
| A1.116 | CH₂CH₃ | CH=CH | C-Cl | CH₂CH₃ | CH(CH₃) | N |
| A1.117 | CH₂CH₃ | CH=CH | C-Cl | CH₂C=CH₂ | CH(CH₃) | N |
| A1.118 | CH₂CH₃ | CH=CH | C-Cl | CH₂C.CH | CH(CH₃) | N |
| A1.119 | CH₂CH₃ | CH=CH | C-Cl | CH₂OCH₃ | CH(CH₃) | N |
| A1.120 | CH₂CH₃ | CH=CH | C-Cl | CH₂OCH₂CH₃ | CH(CH₃) | N |
| A1.121 | CH₃ | CH=CH | C-Cl | H | CHF | N |
| A1.122 | CH₃ | CH=CH | C-Cl | CH₂CH₃ | CHF | N |
| A1.123 | CH₃ | CH=CH | C-Cl | CH₂C=CH₂ | CHF | N |
| A1.124 | CH₃ | CH=CH | C-Cl | CH₂C.CH | CHF | N |
| A1.125 | CH₃ | CH=CH | C-Cl | CH₂OCH₃ | CHF | N |
| A1.126 | CH₃ | CH=CH | C-Cl | CH₂OCH₂CH₃ | CHF | N |
| A1.127 | CH₂CH₃ | CH=CH | C-Cl | H | CHF | N |
| A1.128 | CH₂CH₃ | CH=CH | C-Cl | CH₂CH₃ | CHF | N |
| A1.129 | CH₂CH₃ | CH=CH | C-Cl | CH₂C=CH₂ | CHF | N |
| A1.130 | CH₂CH₃ | CH=CH | C-Cl | CH₂C.CH | CHF | N |
| A1.131 | CH₂CH₃ | CH=CH | C-Cl | CH₂OCH₃ | CHF | N |
| A1.132 | CH₂CH₃ | CH=CH | C-Cl | CH₂OCH₂CH₃ | CHF | N |
| A1.133 | CH₃ | CH=CH | C-Cl | H | CH₂O | N |
| A1.134 | CH₃ | CH=CH | C-Cl | CH₂CH₃ | CH₂O | N |
| A1.135 | CH₃ | CH=CH | C-Cl | CH₂C=CH₂ | CH₂O | N |
| A1.136 | CH₃ | CH=CH | C-Cl | CH₂C.CH | CH₂O | N |
| A1.137 | CH₃ | CH=CH | C-Cl | CH₂OCH₃ | CH₂O | N |
| A1.138 | CH₃ | CH=CH | C-Cl | CH₂OCH₂CH₃ | CH₂O | N |
| A1.139 | CH₂CH₃ | CH=CH | C-Cl | H | CH₂O | N |
| A1.140 | CH₂CH₃ | CH=CH | C-Cl | CH₂CH₃ | CH₂O | N |
| A1.141 | CH₂CH₃ | CH=CH | C-Cl | CH₂C=CH₂ | CH₂O | N |
| A1.142 | CH₂CH₃ | CH=CH | C-Cl | CH₂C.CH | CH₂O | N |
| A1.143 | CH₂CH₃ | CH=CH | C-Cl | CH₂OCH₃ | CH₂O | N |
| A1.144 | CH₂CH₃ | CH=CH | C-Cl | CH₂OCH₂CH₃ | CH₂O | N |
| A1.145 | CH₃ | S | C-Cl | H | CH₂ | C-H |
| A1.146 | CH₃ | S | C-Cl | CH₂CH₃ | CH₂ | C-H |
| A1.147 | CH₃ | S | C-Cl | CH₂C=CH₂ | CH₂ | C-H |
| A1.148 | CH₃ | S | C-Cl | CH₂C.CH | CH₂ | C-H |
| A1.149 | CH₃ | S | C-Cl | CH₂OCH₃ | CH₂ | C-H |
| A1.150 | CH₃ | S | C-Cl | CH₂OCH₂CH₃ | CH₂ | C-H |
| A1.151 | CH₃ | S | C-Br | H | CH₂ | C-H |
| A1.152 | CH₃ | S | C-Br | CH₂CH₃ | CH₂ | C-H |
| A1.153 | CH₃ | S | C-Br | CH₂C=CH₂ | CH₂ | C-H |
| A1.154 | CH₃ | S | C-Br | CH₂C.CH | CH₂ | C-H |
| A1.155 | CH₃ | S | C-Br | CH₂OCH₃ | CH₂ | C-H |
| A1.156 | CH₃ | S | C-Br | CH₂OCH₂CH₃ | CH₂ | C-H |
| A1.157 | CH₂CH₃ | S | C-Cl | H | CH₂ | C-H |
| A1.158 | CH₂CH₃ | S | C-Cl | CH₂CH₃ | CH₂ | C-H |
| A1.159 | CH₂CH₃ | S | C-Cl | CH₂C=CH₂ | CH₂ | C-H |
| A1.160 | CH₂CH₃ | S | C-Cl | CH₂C.CH | CH₂ | C-H |
| A1.161 | CH₂CH₃ | S | C-Cl | CH₂OCH₃ | CH₂ | C-H |
| A1.162 | CH₂CH₃ | S | C-Cl | CH₂OCH₂CH₃ | CH₂ | C-H |
| A1.163 | CH₂CH₃ | S | C-Br | H | CH₂ | C-H |
| A1.164 | CH₂CH₃ | S | C-Br | CH₂CH₃ | CH₂ | C-H |
| A1.165 | CH₂CH₃ | S | C-Br | CH₂C=CH₂ | CH₂ | C-H |
| A1.166 | CH₂CH₃ | S | C-Br | CH₂C.CH | CH₂ | C-H |
| A1.167 | CH₂CH₃ | S | C-Br | CH₂OCH₃ | CH₂ | C-H |
| A1.168 | CH₂CH₃ | S | C-Br | CH₂OCH₂CH₃ | CH₂ | C-H |
| A1.169 | CH₃ | S | C-Cl | H | CH₂ | C-Cl |
| A1.170 | CH₃ | S | C-Cl | CH₂CH₃ | CH₂ | C-Cl |
| A1.171 | CH₃ | S | C-Cl | CH₂C=CH₂ | CH₂ | C-Cl |
| A1.172 | CH₃ | S | C-Cl | CH₂C.CH | CH₂ | C-Cl |
| A1.173 | CH₃ | S | C-Cl | CH₂OCH₃ | CH₂ | C-Cl |
| A1.174 | CH₃ | S | C-Cl | CH₂OCH₂CH₃ | CH₂ | C-Cl |
| A1.175 | CH₃ | S | C-Br | H | CH₂ | C-Cl |
| A1.176 | CH₃ | S | C-Br | CH₂CH₃ | CH₂ | C-Cl |
| A1.177 | CH₃ | S | C-Br | CH₂C=CH₂ | CH₂ | C-Cl |
| A1.178 | CH₃ | S | C-Br | CH₂C.CH | CH₂ | C-Cl |
| A1.179 | CH₃ | S | C-Br | CH₂OCH₃ | CH₂ | C-Cl |
| A1.180 | CH₃ | S | C-Br | CH₂OCH₂CH₃ | CH₂ | C-Cl |
| A1.181 | CH₂CH₃ | S | C-Cl | H | CH₂ | C-Cl |
| A1.182 | CH₂CH₃ | S | C-Cl | CH₂CH₃ | CH₂ | C-Cl |
| A1.183 | CH₂CH₃ | S | C-Cl | CH₂C=CH₂ | CH₂ | C-Cl |
| A1.184 | CH₂CH₃ | S | C-Cl | CH₂C.CH | CH₂ | C-Cl |
| A1.185 | CH₂CH₃ | S | C-Cl | CH₂OCH₃ | CH₂ | C-Cl |
| A1.186 | CH₂CH₃ | S | C-Cl | CH₂OCH₂CH₃ | CH₂ | C-Cl |
| A1.187 | CH₂CH₃ | S | C-Br | H | CH₂ | C-Cl |
| A1.188 | CH₂CH₃ | S | C-Br | CH₂CH₃ | CH₂ | C-Cl |
| A1.189 | CH₂CH₃ | S | C-Br | CH₂C=CH₂ | CH₂ | C-Cl |
| A1.190 | CH₂CH₃ | S | C-Br | CH₂C.CH | CH₂ | C-Cl |
| A1.191 | CH₂CH₃ | S | C-Br | CH₂OCH₃ | CH₂ | C-Cl |
| A1.192 | CH₂CH₃ | S | C-Br | CH₂OCH₂CH₃ | CH₂ | C-Cl |
| A1.193 | CH₃ | S | C-Cl | H | CH(CH₃) | C-H |
| A1.194 | CH₃ | S | C-Cl | CH₂CH₃ | CH(CH₃) | C-H |
| A1.195 | CH₃ | S | C-Cl | CH₂C=CH₂ | CH(CH₃) | C-H |
| A1.196 | CH₃ | S | C-Cl | CH₂C.CH | CH(CH₃) | C-H |
| A1.197 | CH₃ | S | C-Cl | CH₂OCH₃ | CH(CH₃) | C-H |
| A1.198 | CH₃ | S | C-Cl | CH₂OCH₂CH₃ | CH(CH₃) | C-H |
| A1.199 | CH₃ | S | C-Br | H | CH(CH₃) | C-H |
| A1.200 | CH₃ | S | C-Br | CH₂CH₃ | CH(CH₃) | C-H |
| A1.201 | CH₃ | S | C-Br | CH₂C=CH₂ | CH(CH₃) | C-H |
| A1.202 | CH₃ | S | C-Br | CH₂C.CH | CH(CH₃) | C-H |
| A1.203 | CH₃ | S | C-Br | CH₂OCH₃ | CH(CH₃) | C-H |
| A1.204 | CH₃ | S | C-Br | CH₂OCH₂CH₃ | CH(CH₃) | C-H |
| A1.205 | CH₂CH₃ | S | C-Cl | H | CH(CH₃) | C-H |
| A1.206 | CH₂CH₃ | S | C-Cl | CH₂CH₃ | CH(CH₃) | C-H |
| A1.207 | CH₂CH₃ | S | C-Cl | CH₂C=CH₂ | CH(CH₃) | C-H |
| A1.208 | CH₂CH₃ | S | C-Cl | CH₂C.CH | CH(CH₃) | C-H |
| A1.209 | CH₂CH₃ | S | C-Cl | CH₂OCH₃ | CH(CH₃) | C-H |
| A1.210 | CH₂CH₃ | S | C-Cl | CH₂OCH₂CH₃ | CH(CH₃) | C-H |
| A1.211 | CH₂CH₃ | S | C-Br | H | CH(CH₃) | C-H |
| A1.212 | CH₂CH₃ | S | C-Br | CH₂CH₃ | CH(CH₃) | C-H |
| A1.213 | CH₂CH₃ | S | C-Br | CH₂C=CH₂ | CH(CH₃) | C-H |
| A1.214 | CH₂CH₃ | S | C-Br | CH₂C.CH | CH(CH₃) | C-H |
| A1.215 | CH₂CH₃ | S | C-Br | CH₂OCH₃ | CH(CH₃) | C-H |
| A1.216 | CH₂CH₃ | S | C-Br | CH₂OCH₂CH₃ | CH(CH₃) | C-H |
| A1.217 | CH₃ | S | C-Cl | H | CH(CH₃) | C-Cl |
| A1.218 | CH₃ | S | C-Cl | CH₂CH₃ | CH(CH₃) | C-Cl |
| A1.219 | CH₃ | S | C-Cl | CH₂C=CH₂ | CH(CH₃) | C-Cl |
| A1.220 | CH₃ | S | C-Cl | CH₂C.CH | CH(CH₃) | C-Cl |
| A1.221 | CH₃ | S | C-Cl | CH₂OCH₃ | CH(CH₃) | C-Cl |
| A1.222 | CH₃ | S | C-Cl | CH₂OCH₂CH₃ | CH(CH₃) | C-Cl |
| A1.223 | CH₃ | S | C-Br | H | CH(CH₃) | C-Cl |
| A1.224 | CH₃ | S | C-Br | CH₂CH₃ | CH(CH₃) | C-Cl |
| A1.225 | CH₃ | S | C-Br | CH₂C=CH₂ | CH(CH₃) | C-Cl |
| A1.226 | CH₃ | S | C-Br | CH₂C.CH | CH(CH₃) | C-Cl |
| A1.227 | CH₃ | S | C-Br | CH₂OCH₃ | CH(CH₃) | C-Cl |
| A1.228 | CH₃ | S | C-Br | CH₂OCH₂CH₃ | CH(CH₃) | C-Cl |
| A1.229 | CH₂CH₃ | S | C-Cl | H | CH(CH₃) | C-Cl |
| A1.230 | CH₂CH₃ | S | C-Cl | CH₂CH₃ | CH(CH₃) | C-Cl |
| A1.231 | CH₂CH₃ | S | C-Cl | CH₂C=CH₂ | CH(CH₃) | C-Cl |
| A1.232 | CH₂CH₃ | S | C-Cl | CH₂C.CH | CH(CH₃) | C-Cl |
| A1.233 | CH₂CH₃ | S | C-Cl | CH₂OCH₃ | CH(CH₃) | C-Cl |
| A1.234 | CH₂CH₃ | S | C-Cl | CH₂OCH₂CH₃ | CH(CH₃) | C-Cl |
| A1.235 | CH₂CH₃ | S | C-Br | H | CH(CH₃) | C-Cl |
| A1.236 | CH₂CH₃ | S | C-Br | CH₂CH₃ | CH(CH₃) | C-Cl |
| A1.237 | CH₂CH₃ | S | C-Br | CH₂C=CH₂ | CH(CH₃) | C-Cl |
| A1.238 | CH₂CH₃ | S | C-Br | CH₂C.CH | CH(CH₃) | C-Cl |
| A1.239 | CH₂CH₃ | S | C-Br | CH₂OCH₃ | CH(CH₃) | C-Cl |
| A1.240 | CH₂CH₃ | S | C-Br | CH₂OCH₂CH₃ | CH(CH₃) | C-Cl |
| A1.241 | CH₃ | S | C-Cl | H | CHF | C-H |
| A1.242 | CH₃ | S | C-Cl | CH₂CH₃ | CHF | C-H |
| A1.243 | CH₃ | S | C-Cl | CH₂C=CH₂ | CHF | C-H |
| A1.244 | CH₃ | S | C-Cl | CH₂C.CH | CHF | C-H |
| A1.245 | CH₃ | S | C-Cl | CH₂OCH₃ | CHF | C-H |
| A1.246 | CH₃ | S | C-Cl | CH₂OCH₂CH₃ | CHF | C-H |
| A1.247 | CH₃ | S | C-Br | H | CHF | C-H |
| A1.248 | CH₃ | S | C-Br | CH₂CH₃ | CHF | C-H |
| A1.249 | CH₃ | S | C-Br | CH₂C=CH₂ | CHF | C-H |
| A1.250 | CH₃ | S | C-Br | CH₂C.CH | CHF | C-H |
| A1.251 | CH₃ | S | C-Br | CH₂OCH₃ | CHF | C-H |
| A1.252 | CH₃ | S | C-Br | CH₂OCH₂CH₃ | CHF | C-H |
| A1.253 | CH₂CH₃ | S | C-Cl | H | CHF | C-H |
| A1.254 | CH₂CH₃ | S | C-Cl | CH₂CH₃ | CHF | C-H |
| A1.255 | CH₂CH₃ | S | C-Cl | CH₂C=CH₂ | CHF | C-H |
| A1.256 | CH₂CH₃ | S | C-Cl | CH₂C.CH | CHF | C-H |
| A1.257 | CH₂CH₃ | S | C-Cl | CH₂OCH₃ | CHF | C-H |
| A1.258 | CH₂CH₃ | S | C-Cl | CH₂OCH₂CH₃ | CHF | C-H |
| A1.259 | CH₂CH₃ | S | C-Br | H | CHF | C-H |
| A1.260 | CH₂CH₃ | S | C-Br | CH₂CH₃ | CHF | C-H |
| A1.261 | CH₂CH₃ | S | C-Br | CH₂C=CH₂ | CHF | C-H |
| A1.262 | CH₂CH₃ | S | C-Br | CH₂C.CH | CHF | C-H |
| A1.263 | CH₂CH₃ | S | C-Br | CH₂OCH₃ | CHF | C-H |
| A1.264 | CH₂CH₃ | S | C-Br | CH₂OCH₂CH₃ | CHF | C-H |
| A1.265 | CH₃ | S | C-Cl | H | CHF | C-Cl |
| A1.266 | CH₃ | S | C-Cl | CH₂CH₃ | CHF | C-Cl |
| A1.267 | CH₃ | S | C-Cl | CH₂C=CH₂ | CHF | C-Cl |
| A1.268 | CH₃ | S | C-Cl | CH₂C.CH | CHF | C-Cl |
| A1.269 | CH₃ | S | C-Cl | CH₂OCH₃ | CHF | C-Cl |
| A1.270 | CH₃ | S | C-Cl | CH₂OCH₂CH₃ | CHF | C-Cl |
| A1.271 | CH₃ | S | C-Br | H | CHF | C-Cl |
| A1.272 | CH₃ | S | C-Br | CH₂CH₃ | CHF | C-Cl |
| A1.273 | CH₃ | S | C-Br | CH₂C=CH₂ | CHF | C-Cl |
| A1.274 | CH₃ | S | C-Br | CH₂C.CH | CHF | C-Cl |
| A1.275 | CH₃ | S | C-Br | CH₂OCH₃ | CHF | C-Cl |
| A1.276 | CH₃ | S | C-Br | CH₂OCH₂CH₃ | CHF | C-Cl |
| A1.277 | CH₂CH₃ | S | C-Cl | H | CHF | C-Cl |
| A1.278 | CH₂CH₃ | S | C-Cl | CH₂CH₃ | CHF | C-Cl |
| A1.279 | CH₂CH₃ | S | C-Cl | CH₂C=CH₂ | CHF | C-Cl |
| A1.280 | CH₂CH₃ | S | C-Cl | CH₂C.CH | CHF | C-Cl |
| A1.281 | CH₂CH₃ | S | C-Cl | CH₂OCH₃ | CHF | C-Cl |
| A1.282 | CH₂CH₃ | S | C-Cl | CH₂OCH₂CH₃ | CHF | C-Cl |
| A1.283 | CH₂CH₃ | S | C-Br | H | CHF | C-Cl |
| A1.284 | CH₂CH₃ | S | C-Br | CH₂CH₃ | CHF | C-Cl |
| A1.285 | CH₂CH₃ | S | C-Br | CH₂C=CH₂ | CHF | C-Cl |
| A1.286 | CH₂CH₃ | S | C-Br | CH₂C.CH | CHF | C-Cl |
| A1.287 | CH₂CH₃ | S | C-Br | CH₂OCH₃ | CHF | C-Cl |
| A1.288 | CH₂CH₃ | S | C-Br | CH₂OCH₂CH₃ | CHF | C-Cl |
| A1.289 | CH₃ | S | C-Cl | H | CH₂O | C-H |
| A1.290 | CH₃ | S | C-Cl | CH₂CH₃ | CH₂O | C-H |
| A1.291 | CH₃ | S | C-Cl | CH₂C=CH₂ | CH₂O | C-H |
| A1.292 | CH₃ | S | C-Cl | CH₂C.CH | CH₂O | C-H |
| A1.293 | CH₃ | S | C-Cl | CH₂OCH₃ | CH₂O | C-H |
| A1.294 | CH₃ | S | C-Cl | CH₂OCH₂CH₃ | CH₂O | C-H |
| A1.295 | CH₃ | S | C-Br | H | CH₂O | C-H |
| A1.296 | CH₃ | S | C-Br | CH₂CH₃ | CH₂O | C-H |
| A1.297 | CH₃ | S | C-Br | CH₂C=CH₂ | CH₂O | C-H |
| A1.298 | CH₃ | S | C-Br | CH₂C.CH | CH₂O | C-H |
| A1.299 | CH₃ | S | C-Br | CH₂OCH₃ | CH₂O | C-H |
| A1.300 | CH₃ | S | C-Br | CH₂OCH₂CH₃ | CH₂O | C-H |
| A1.301 | CH₂CH₃ | S | C-Cl | H | CH₂O | C-H |
| A1.302 | CH₂CH₃ | S | C-Cl | CH₂CH₃ | CH₂O | C-H |
| A1.303 | CH₂CH₃ | S | C-Cl | CH₂C=CH₂ | CH₂O | C-H |
| A1.304 | CH₂CH₃ | S | C-Cl | CH₂C.CH | CH₂O | C-H |
| A1.305 | CH₂CH₃ | S | C-Cl | CH₂OCH₃ | CH₂O | C-H |
| A1.306 | CH₂CH₃ | S | C-Cl | CH₂OCH₂CH₃ | CH₂O | C-H |
| A1.307 | CH₂CH₃ | S | C-Br | H | CH₂O | C-H |
| A1.308 | CH₂CH₃ | S | C-Br | CH₂CH₃ | CH₂O | C-H |
| A1.309 | CH₂CH₃ | S | C-Br | CH₂C=CH₂ | CH₂O | C-H |
| A1.310 | CH₂CH₃ | S | C-Br | CH₂C.CH | CH₂O | C-H |
| A1.311 | CH₂CH₃ | S | C-Br | CH₂OCH₃ | CH₂O | C-H |
| A1.312 | CH₂CH₃ | S | C-Br | CH₂OCH₂CH₃ | CH₂O | C-H |
| A1.313 | CH₃ | S | C-Cl | H | CH₂O | C-H |
| A1.314 | CH₃ | S | C-Cl | CH₂CH₃ | CH₂O | C-H |
| A1.315 | CH₃ | S | C-Cl | CH₂C=CH₂ | CH₂O | C-H |
| A1.316 | CH₃ | S | C-Cl | CH₂C.CH | CH₂O | C-H |
| A1.317 | CH₃ | S | C-Cl | CH₂OCH₃ | CH₂O | C-H |
| A1.318 | CH₃ | S | C-Cl | CH₂OCH₂CH₃ | CH₂O | C-H |
| A1.319 | CH₃ | S | C-Br | H | CH₂O | C-H |
| A1.320 | CH₃ | S | C-Br | CH₂CH₃ | CH₂O | C-H |
| A1.321 | CH₃ | S | C-Br | CH₂C=CH₂ | CH₂O | C-H |
| A1.322 | CH₃ | S | C-Br | CH₂C.CH | CH₂O | C-H |
| A1.323 | CH₃ | S | C-Br | CH₂OCH₃ | CH₂O | C-H |
| A1.324 | CH₃ | S | C-Br | CH₂OCH₂CH₃ | CH₂O | C-H |
| A1.325 | CH₂CH₃ | S | C-Cl | H | CH₂O | C-Cl |
| A1.326 | CH₂CH₃ | S | C-Cl | CH₂CH₃ | CH₂O | C-Cl |
| A1.327 | CH₂CH₃ | S | C-Cl | CH₂C=CH₂ | CH₂O | C-Cl |
| A1.328 | CH₂CH₃ | S | C-Cl | CH₂C.CH | CH₂O | C-Cl |
| A1.329 | CH₂CH₃ | S | C-Cl | CH₂OCH₃ | CH₂O | C-Cl |
| A1.330 | CH₂CH₃ | S | C-Cl | CH₂OCH₂CH₃ | CH₂O | C-Cl |
| A1.331 | CH₂CH₃ | S | C-Br | H | CH₂O | C-Cl |
| A1.332 | CH₂CH₃ | S | C-Br | CH₂CH₃ | CH₂O | C-Cl |
| A1.333 | CH₂CH₃ | S | C-Br | CH₂C=CH₂ | CH₂O | C-Cl |
| A1.334 | CH₂CH₃ | S | C-Br | CH₂C.CH | CH₂O | C-Cl |
| A1.335 | CH₂CH₃ | S | C-Br | CH₂OCH₃ | CH₂O | C-Cl |
| A1.336 | CH₂CH₃ | S | C-Br | CH₂OCH₂CH₃ | CH₂O | C-Cl |
| A1.337 | CH₃ | CH=CH | C-Cl | H | CH₂ | C-H |
| A1.338 | CH₃ | CH=CH | C-Cl | CH₂CH₃ | CH₂ | C-H |
| A1.339 | CH₃ | CH=CH | C-Cl | CH₂C=CH₂ | CH₂ | C-H |
| A1.340 | CH₃ | CH=CH | C-Cl | CH₂C.CH | CH₂ | C-H |
| A1.341 | CH₃ | CH=CH | C-Cl | CH₂OCH₃ | CH₂ | C-H |
| A1.342 | CH₃ | CH=CH | C-Cl | CH₂OCH₂CH₃ | CH₂ | C-H |
| A1.343 | CH₂CH₃ | CH=CH | C-Cl | H | CH₂ | C-H |
| A1.344 | CH₂CH₃ | CH=CH | C-Cl | CH₂CH₃ | CH₂ | C-H |
| A1.345 | CH₂CH₃ | CH=CH | C-Cl | CH₂C=CH₂ | CH₂ | C-H |
| A1.346 | CH₂CH₃ | CH=CH | C-Cl | CH₂C.CH | CH₂ | C-H |
| A1.347 | CH₂CH₃ | CH=CH | C-Cl | CH₂OCH₃ | CH₂ | C-H |
| A1.348 | CH₂CH₃ | CH=CH | C-Cl | CH₂OCH₂CH₃ | CH₂ | C-H |
| A1.349 | CH₃ | CH=CH | C-Cl | H | CH₂ | C-Cl |
| A1.350 | CH₃ | CH=CH | C-Cl | CH₂CH₃ | CH₂ | C-Cl |
| A1.351 | CH₃ | CH=CH | C-Cl | CH₂C=CH₂ | CH₂ | C-Cl |
| A1.352 | CH₃ | CH=CH | C-Cl | CH₂C.CH | CH₂ | C-Cl |
| A1.353 | CH₃ | CH=CH | C-Cl | CH₂OCH₃ | CH₂ | C-Cl |
| A1.354 | CH₃ | CH=CH | C-Cl | CH₂OCH₂CH₃ | CH₂ | C-Cl |
| A1.355 | CH₂CH₃ | CH=CH | C-Cl | H | CH₂ | C-Cl |
| A1.356 | CH₂CH₃ | CH=CH | C-Cl | CH₂CH₃ | CH₂ | C-Cl |
| A1.357 | CH₂CH₃ | CH=CH | C-Cl | CH₂C=CH₂ | CH₂ | C-Cl |
| A1.358 | CH₂CH₃ | CH=CH | C-Cl | CH₂C.CH | CH₂ | C-Cl |
| A1.359 | CH₂CH₃ | CH=CH | C-Cl | CH₂OCH₃ | CH₂ | C-Cl |
| A1.360 | CH₂CH₃ | CH=CH | C-Cl | CH₂OCH₂CH₃ | CH₂ | C-Cl |
| A1.361 | CH₃ | CH=CH | C-Cl | H | CH(CH₃) | C-H |
| A1.362 | CH₃ | CH=CH | C-Cl | CH₂CH₃ | CH(CH₃) | C-H |
| A1.363 | CH₃ | CH=CH | C-Cl | CH₂C=CH₂ | CH(CH₃) | C-H |
| A1.364 | CH₃ | CH=CH | C-Cl | CH₂C.CH | CH(CH₃) | C-H |
| A1.365 | CH₃ | CH=CH | C-Cl | CH₂OCH₃ | CH(CH₃) | C-H |
| A1.366 | CH₃ | CH=CH | C-Cl | CH₂OCH₂CH₃ | CH(CH₃) | C-H |
| A1.367 | CH₂CH₃ | CH=CH | C-Cl | H | CH(CH₃) | C-H |
| A1.368 | CH₂CH₃ | CH=CH | C-Cl | CH₂CH₃ | CH(CH₃) | C-H |
| A1.369 | CH₂CH₃ | CH=CH | C-Cl | CH₂C=CH₂ | CH(CH₃) | C-H |
| A1.370 | CH₂CH₃ | CH=CH | C-Cl | CH₂C.CH | CH(CH₃) | C-H |
| A1.371 | CH₂CH₃ | CH=CH | C-Cl | CH₂OCH₃ | CH(CH₃) | C-H |
| A1.372 | CH₂CH₃ | CH=CH | C-Cl | CH₂OCH₂CH₃ | CH(CH₃) | C-H |
| A1.373 | CH₃ | CH=CH | C-Cl | H | CH(CH₃) | C-Cl |
| A1.374 | CH₃ | CH=CH | C-Cl | CH₂CH₃ | CH(CH₃) | C-Cl |
| A1.375 | CH₃ | CH=CH | C-Cl | CH₂C=CH₂ | CH(CH₃) | C-Cl |
| A1.376 | CH₃ | CH=CH | C-Cl | CH₂C.CH | CH(CH₃) | C-Cl |
| A1.377 | CH₃ | CH=CH | C-Cl | CH₂OCH₃ | CH(CH₃) | C-Cl |
| A1.378 | CH₃ | CH=CH | C-Cl | CH₂OCH₂CH₃ | CH(CH₃) | C-Cl |
| A1.379 | CH₂CH₃ | CH=CH | C-Cl | H | CH(CH₃) | C-Cl |
| A1.380 | CH₂CH₃ | CH=CH | C-Cl | CH₂CH₃ | CH(CH₃) | C-Cl |
| A1.381 | CH₂CH₃ | CH=CH | C-Cl | CH₂C=CH₂ | CH(CH₃) | C-Cl |
| A1.382 | CH₂CH₃ | CH=CH | C-Cl | CH₂C.CH | CH(CH₃) | C-Cl |
| A1.383 | CH₂CH₃ | CH=CH | C-Cl | CH₂OCH₃ | CH(CH₃) | C-Cl |
| A1.384 | CH₂CH₃ | CH=CH | C-Cl | CH₂OCH₂CH₃ | CH(CH₃) | C-Cl |
| A1.385 | CH₃ | CH=CH | C-Cl | H | CHF | C-H |
| A1.386 | CH₃ | CH=CH | C-Cl | CH₂CH₃ | CHF | C-H |
| A1.387 | CH₃ | CH=CH | C-Cl | CH₂C=CH₂ | CHF | C-H |
| A1.388 | CH₃ | CH=CH | C-Cl | CH₂C.CH | CHF | C-H |
| A1.389 | CH₃ | CH=CH | C-Cl | CH₂OCH₃ | CHF | C-H |
| A1.390 | CH₃ | CH=CH | C-Cl | CH₂OCH₂CH₃ | CHF | C-H |
| A1.391 | CH₂CH₃ | CH=CH | C-Cl | H | CHF | C-H |
| A1.392 | CH₂CH₃ | CH=CH | C-Cl | CH₂CH₃ | CHF | C-H |
| A1.393 | CH₂CH₃ | CH=CH | C-Cl | CH₂C=CH₂ | CHF | C-H |
| A1.394 | CH₂CH₃ | CH=CH | C-Cl | CH₂C.CH | CHF | C-H |
| A1.395 | CH₂CH₃ | CH=CH | C-Cl | CH₂OCH₃ | CHF | C-H |
| A1.396 | CH₂CH₃ | CH=CH | C-Cl | CH₂OCH₂CH₃ | CHF | C-H |
| A1.397 | CH₃ | CH=CH | C-Cl | H | CHF | C-Cl |
| A1.398 | CH₃ | CH=CH | C-Cl | CH₂CH₃ | CHF | C-Cl |
| A1.399 | CH₃ | CH=CH | C-Cl | CH₂C=CH₂ | CHF | C-Cl |
| A1.400 | CH₃ | CH=CH | C-Cl | CH₂C.CH | CHF | C-Cl |
| A1.401 | CH₃ | CH=CH | C-Cl | CH₂OCH₃ | CHF | C-Cl |
| A1.402 | CH₃ | CH=CH | C-Cl | CH₂OCH₂CH₃ | CHF | C-Cl |
| A1.403 | CH₂CH₃ | CH=CH | C-Cl | H | CHF | C-Cl |
| A1.404 | CH₂CH₃ | CH=CH | C-Cl | CH₂CH₃ | CHF | C-Cl |
| A1.405 | CH₂CH₃ | CH=CH | C-Cl | CH₂C=CH₂ | CHF | C-Cl |
| A1.406 | CH₂CH₃ | CH=CH | C-Cl | CH₂C.CH | CHF | C-Cl |
| A1.407 | CH₂CH₃ | CH=CH | C-Cl | CH₂OCH₃ | CHF | C-Cl |
| A1.408 | CH₂CH₃ | CH=CH | C-Cl | CH₂OCH₂CH₃ | CHF | C-Cl |
| A1.409 | CH₃ | CH=CH | C-Cl | H | CH₂O | C-H |
| A1.410 | CH₃ | CH=CH | C-Cl | CH₂CH₃ | CH₂O | C-H |
| A1.411 | CH₃ | CH=CH | C-Cl | CH₂C=CH₂ | CH₂O | C-H |
| A1.412 | CH₃ | CH=CH | C-Cl | CH₂C.CH | CH₂O | C-H |
| A1.413 | CH₃ | CH=CH | C-Cl | CH₂OCH₃ | CH₂O | C-H |
| A1.414 | CH₃ | CH=CH | C-Cl | CH₂OCH₂CH₃ | CH₂O | C-H |
| A1.415 | CH₂CH₃ | CH=CH | C-Cl | H | CH₂O | C-H |
| A1.416 | CH₂CH₃ | CH=CH | C-Cl | CH₂CH₃ | CH₂O | C-H |
| A1.417 | CH₂CH₃ | CH=CH | C-Cl | CH₂C=CH₂ | CH₂O | C-H |
| A1.418 | CH₂CH₃ | CH=CH | C-Cl | CH₂C.CH | CH₂O | C-H |
| A1.419 | CH₂CH₃ | CH=CH | C-Cl | CH₂OCH₃ | CH₂O | C-H |
| A1.420 | CH₂CH₃ | CH=CH | C-Cl | CH₂OCH₂CH₃ | CH₂O | C-H |
| A1.421 | CH₃ | CH=CH | C-Cl | H | CH₂O | C-Cl |
| A1.422 | CH₃ | CH=CH | C-Cl | CH₂CH₃ | CH₂O | C-Cl |
| A1.423 | CH₃ | CH=CH | C-Cl | CH₂C=CH₂ | CH₂O | C-Cl |
| A1.424 | CH₃ | CH=CH | C-Cl | CH₂C.CH | CH₂O | C-Cl |
| A1.425 | CH₃ | CH=CH | C-Cl | CH₂OCH₃ | CH₂O | C-Cl |
| A1.426 | CH₃ | CH=CH | C-Cl | CH₂OCH₂CH₃ | CH₂O | C-Cl |
| A1.427 | CH₂CH₃ | CH=CH | C-Cl | H | CH₂O | C-Cl |
| A1.428 | CH₂CH₃ | CH=CH | C-Cl | CH₂CH₃ | CH₂O | C-Cl |
| A1.429 | CH₂CH₃ | CH=CH | C-Cl | CH₂C=CH₂ | CH₂O | C-Cl |
| A1.430 | CH₂CH₃ | CH=CH | C-Cl | CH₂C.CH | CH₂O | C-Cl |
| A1.431 | CH₂CH₃ | CH=CH | C-Cl | CH₂OCH₃ | CH₂O | C-Cl |
| A1.432 | CH₂CH₃ | CH=CH | C-Cl | CH₂OCH₂CH₃ | CH₂O | C-Cl |

### Table A2

Table A2 provides 432 compounds of Formula A1 wherein R¹, D, E, R¹¹, A and J are as defined in Table A1 and R⁶ is ethyl.

### Table A3

Table A3 provides 432 compounds of Formula A1 wherein R¹, D, E, R¹¹, A and J are as defined in Table A1 and R⁶ is *n*-propyl.

### Table A4

Table A4 provides 432 compounds of Formula A1 wherein R¹, D, E, R¹¹, A and J are as defined in Table A1 and R⁶ is *iso*-propyl.

### Table A5

Table A5 provides 432 compounds of Formula A1 wherein R¹, D, E, R¹¹, A and J are as defined in Table A1 and R⁶ is *n*-butyl.

### Table A6

Table A6 provides 432 compounds of Formula A1 wherein R¹, D, E, R¹¹, A and J are as defined in Table A1 and R⁶ is *sec*-butyl.

### Table A7

Table A7 provides 432 compounds of Formula A1 wherein R¹, D, E, R¹¹, A and J are as defined in Table A1 and R⁶ is *iso*-butyl.

### Table A8

Table A8 provides 432 compounds of Formula A1 wherein R¹, D, E, R¹¹, A and J are as defined in Table A1 and R⁶ is *tert*-butyl.

### Table A9

Table A9 provides 432 compounds of Formula A1 wherein R¹, D, E, R¹¹, A and J are as defined in Table A1 and R⁶ is *n*-pentyl.

### Table A10

Table A10 provides 432 compounds of Formula A1 wherein R¹, D, E, R¹¹, A and J are as defined in Table A1 and R⁶ is 1-methylbutyl.

### Table A11

Table A11 provides 432 compounds of Formula A1 wherein R¹, D, E, R¹¹, A and J are as defined in Table A1 and R⁶ is 2-methylbutyl.

### Table A12

Table A12 provides 432 compounds of Formula A1 wherein R¹, D, E, R¹¹, A and J are as defined in Table A1 and R⁶ is 3-methylbutyl.

### Table A13

Table A13 provides 432 compounds of Formula A1 wherein R¹, D, E, R¹¹, A and J are as defined in Table A1 and R⁶ is neopentyl.

### Table A14

Table A14 provides 432 compounds of Formula A1 wherein R¹, D, E, R¹¹, A and J are as defined in Table A1 and R⁶ is 2,2-dimethylbutyl.

### Table A15

Table A15 provides 432 compounds of Formula A1 wherein R¹, D, E, R¹¹, A and J are as defined in Table A1 and R⁶ is 2,2,2-trifluoroethyl.

### Table A16

Table A16 provides 432 compounds of Formula A1 wherein R¹, D, E, R¹¹, A and J are as defined in Table A1 and R⁶ is 2,2-difluoro-2-methoxyethyl.

### Table A17

Table A17 provides 432 compounds of Formula A1 wherein R¹, D, E, R¹¹, A and J are as defined in Table A1 and R⁶ is 3,3,3-trifluoropropyl.

### Table A18

Table A18 provides 432 compounds of Formula A1 wherein R¹, D, E, R¹¹, A and J are as defined in Table A1 and R⁶ is *para*-fluorobenzyl.

### Table A19

Table A19 provides 432 compounds of Formula A1 vherein R¹, D, E, R¹¹, A and J are as defined in Table A1 and R⁶ is 2,4-dichlorophenyl.

### Table A20

Table A20 provides 432 compounds of Formula A1 wherein R¹, D, E, R¹¹, A and J are as defined in Table A1 and R⁶ is 2-chloro-4-trifluoromethylphenyl.

### Table A21

Table A21 provides 432 compounds of Formula A1 wherein R¹, D, E, R¹¹, A and J are as defined in Table A1 and R⁶ is 4-trifluoromethylphenyl.

### Table B1

Table B1 provides 288 compounds of Formula B1 wherein R¹, D, E, R¹¹, A and J are as defined in Table B1 and R⁶ is methyl.

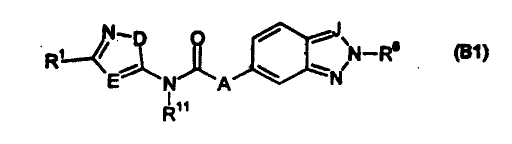

**Table B1**

| **Compound** No. | **R**^{**1**} | **D** | **E** | **R**^{**11**} | **A** | **J** |
|---|---|---|---|---|---|---|
| B1.1 | CH₃ | S | C-Cl | H | CH₂ | C-H |
| B1.2 | CH₃ | S | C-Cl | CH₂CH₃ | CH₂ | C-H |
| B1.3 | CH₃ | S | C-Cl | CH₂C=CH₂ | CH₂ | C-H |
| B1.4 | CH₃ | S | C-Cl | CH₂C.CH | CH₂ | C-H |
| B1.5 | CH₃ | S | C-Cl | CH₂OCH₃ | CH₂ | C-H |
| B1.6 | CH₃ | S | C-Cl | CH₂OCH₂CH₃ | CH₂ | C-H |
| B1.7 | CH₃ | S | C-Br | H | CH₂ | C-H |
| B1.8 | CH₃ | S | C-Br | CH₂CH₃ | CH₂ | C-H |
| B1.9 | CH₃ | S | C-Br | CH₂C=CH₂ | CH₂ | C-H |
| B1.10 | CH₃ | S | C-Br | CH₂C.CH | CH₂ | C-H |
| B1.11 | CH₃ | S | C-Br | CH₂OCH₃ | CH₂ | C-H |
| B1.12 | CH₃ | S | C-Br | CH₂OCH₂CH₃ | CH₂ | C-H |
| B1.13 | CH₂CH₃ | S | C-Cl | H | CH₂ | C-H |
| B1.14 | CH₂CH₃ | S | C-Cl | CH₂CH₃ | CH₂ | C-H |
| B1.15 | CH₂CH₃ | S | C-Cl | CH₂C=CH₂ | CH₂ | C-H |
| B1.16 | CH₂CH₃ | S | C-Cl | CH₂C.CH | CH₂ | C-H |
| B1.17 | CH₂CH₃ | S | C-Cl | CH₂OCH₃ | CH₂ | C-H |
| B1.18 | CH₂CH₃ | S | C-Cl | CH₂OCH₂CH₃ | CH₂ | C-H |
| B1.19 | CH₂CH₃ | S | C-Br | H | CH₂ | C-H |
| B1.20 | CH₂CH₃ | S | C-Br | CH₂CH₃ | CH₂ | C-H |
| B1.21 | CH₂CH₃ | S | C-Br | CH₂C=CH₂ | CH₂ | C-H |
| B1.22 | CH₂CH₃ | S | C-Br | CH₂C.CH | CH₂ | C-H |
| B1.23 | CH₂CH₃ | S | C-Br | CH₂OCH₃ | CH₂ | C-H |
| B1.24 | CH₂CH₃ | S | C-Br | CH₂OCH₂CH₃ | CH₂ | C-H |
| B1.25 | CH₃ | S | C-Cl | H | CH(CH₃) | C-H |
| B1.26 | CH₃ | S | C-Cl | CH₂CH₃ | CH(CH₃) | C-H |
| B1.27 | CH₃ | S | C-Cl | CH₂C=CH₂ | CH(CH₃) | C-H |
| B1.28 | CH₃ | S | C-Cl | CH₂C.CH | CH(CH₃) | C-H |
| B1.29 | CH₃ | S | C-Cl | CH₂OCH₃ | CH(CH₃) | C-H |
| B1.30 | CH₃ | S | C-Cl | CH₂OCH₂CH₃ | CH(CH₃) | C-H |
| B1.31 | CH₃ | S | C-Br | H | CH(CH₃) | C-H |
| B1.32 | CH₃ | S | C-Br | CH₂CH₃ | CH(CH₃) | C-H |
| B1.33 | CH₃ | S | C-Br | CH₂C=CH₂ | CH(CH₃) | C-H |
| B1.34 | CH₃ | S | C-Br | CH₂C.CH | CH(CH₃) | C-H |
| B1.35 | CH₃ | S | C-Br | CH₂OCH₃ | CH(CH₃) | C-H |
| B1.36 | CH₃ | S | C-Br | CH₂OCH₂CH₃ | CH(CH₃) | C-H |
| B1.37 | CH₂CH₃ | S | C-Cl | H | CH(CH₃) | C-H |
| B1.38 | CH₂CH₃ | S | C-Cl | CH₂CH₃ | CH(CH₃) | C-H |
| B1.39 | CH₂CH₃ | S | C-Cl | CH₂C=CH₂ | CH(CH₃) | C-H |
| B1.40 | CH₂CH₃ | S | C-Cl | CH₂C.CH | CH(CH₃) | C-H |
| B1.41 | CH₂CH₃ | S | C-Cl | CH₂OCH₃ | CH(CH₃) | C-H |
| B1.42 | CH₂CH₃ | S | C-Cl | CH₂OCH₂CH₃ | CH(CH₃) | C-H |
| B1.43 | CH₂CH₃ | S | C-Br | H | CH(CH₃) | C-H |
| B1.44 | CH₂CH₃ | S | C-Br | CH₂CH₃ | CH(CH₃) | C-H |
| B1.45 | CH₂CH₃ | S | C-Br | CH₂C=CH₂ | CH(CH₃) | C-H |
| B1.46 | CH₂CH₃ | S | C-Br | CH₂C.CH | CH(CH₃) | C-H |
| B1.47 | CH₂CH₃ | S | C-Br | CH₂OCH₃ | CH(CH₃) | C-H |
| B1.48 | CH₂CH₃ | S | C-Br | CH₂OCH₂CH₃ | CH(CH₃) | C-H |
| B1.49 | CH₃ | S | C-Cl | H | CHF | C-H |
| B1.50 | CH₃ | S | C-Cl | CH₂CH₃ | CHF | C-H |
| B1.51 | CH₃ | S | C-Cl | CH₂C=CH₂ | CHF | C-H |
| B1.52 | CH₃ | S | C-Cl | CH₂C.CH | CHF | C-H |
| B1.53 | CH₃ | S | C-Cl | CH₂OCH₃ | CHF | C-H |
| B1.54 | CH₃ | S | C-Cl | CH₂OCH₂CH₃ | CHF | C-H |
| B1.55 | CH₃ | S | C-Br | H | CHF | C-H |
| B1.56 | CH₃ | S | C-Br | CH₂CH₃ | CHF | C-H |
| B1.57 | CH₃ | S | C-Br | CH₂C=CH₂ | CHF | C-H |
| B1.58 | CH₃ | S | C-Br | CH₂C.CH | CHF | C-H |
| B1.59 | CH₃ | S | C-Br | CH₂OCH₃ | CHF | C-H |
| B1.60 | CH₃ | S | C-Br | CH₂OCH₂CH₃ | CHF | C-H |
| B1.61 | CH₂CH₃ | S | C-Cl | H | CHF | C-H |
| B1.62 | CH₂CH₃ | S | C-Cl | CH₂CH₃ | CHF | C-H |
| B1.63 | CH₂CH₃ | S | C-Cl | CH₂C=CH₂ | CHF | C-H |
| B1.64 | CH₂CH₃ | S | C-Cl | CH₂C.CH | CHF | C-H |
| B1.65 | CH₂CH₃ | S | C-Cl | CH₂OCH₃ | CHF | C-H |
| B1.66 | CH₂CH₃ | S | C-Cl | CH₂OCH₂CH₃ | CHF | C-H |
| B1.67 | CH₂CH₃ | S | C-Br | H | CHF | C-H |
| B1.68 | CH₂CH₃ | S | C-Br | CH₂CH₃ | CHF | C-H |
| B1.69 | CH₂CH₃ | S | C-Br | CH₂C=CH₂ | CHF | C-H |
| B1.70 | CH₂CH₃ | S | C-Br | CH₂C.CH | CHF | C-H |
| B1.71 | CH₂CH₃ | S | C-Br | CH₂OCH₃ | CHF | C-H |
| B1.72 | CH₂CH₃ | S | C-Br | CH₂OCH₂CH₃ | CHF | C-H |
| B1.73 | CH₃ | S | C-Cl | H | CH₂O | C-H |
| B1.74 | CH₃ | S | C-Cl | CH₂CH₃ | CH₂O | C-H |
| B1.75 | CH₃ | S | C-Cl | CH₂C=CH₂ | CH₂O | C-H |
| B1.76 | CH₃ | S | C-Cl | CH₂C.CH | CH₂O | C-H |
| B1.77 | CH₃ | S | C-Cl | CH₂OCH₃ | CH₂O | C-H |
| B1.78 | CH₃ | S | C-Cl | CH₂OCH₂CH₃ | CH₂O | C-H |
| B1.79 | CH₃ | S | C-Br | H | CH₂O | C-H |
| B1.80 | CH₃ | S | C-Br | CH₂CH₃ | CH₂O | C-H |
| B1.81 | CH₃ | S | C-Br | CH₂C=CH₂ | CH₂O | C-H |
| B1.82 | CH₃ | S | C-Br | CH₂C.CH | CH₂O | C-H |
| B1.83 | CH₃ | S | C-Br | CH₂OCH₃ | CH₂O | C-H |
| B1.84 | CH₃ | S | C-Br | CH₂OCH₂CH₃ | CH₂O | C-H |
| B1.85 | CH₂CH₃ | S | C-Cl | H | CH₂O | C-H |
| B1.86 | CH₂CH₃ | S | C-Cl | CH₂CH₃ | CH₂O | C-H |
| B1.87 | CH₂CH₃ | S | C-Cl | CH₂C=CH₂ | CH₂O | C-H |
| B1.88 | CH₂CH₃ | S | C-Cl | CH₂C.CH | CH₂O | C-H |
| B1.89 | CH₂CH₃ | S | C-Cl | CH₂OCH₃ | CH₂O | C-H |
| B1.90 | CH₂CH₃ | S | C-Cl | CH₂OCH₂CH₃ | CH₂O | C-H |
| B1.91 | CH₂CH₃ | S | C-Br | H | CH₂O | C-H |
| B1.92 | CH₂CH₃ | S | C-Br | CH₂CH₃ | CH₂O | C-H |
| B1.93 | CH₂CH₃ | S | C-Br | CH₂C=CH₂ | CH₂O | C-H |
| B1.94 | CH₂CH₃ | S | C-Br | CH₂C.CH | CH₂O | C-H |
| B1.95 | CH₂CH₃ | S | C-Br | CH₂OCH₃ | CH₂O | C-H |
| B1.96 | CH₂CH₃ | S | C-Br | CH₂OCH₂CH₃ | CH₂O | C-H |
| B1.97 | CH₃ | S | C-Cl | H | CH₂ | C-Cl |
| B1.98 | CH₃ | S | C-Cl | CH₂CH₃ | CH₂ | C-Cl |
| B1.99 | CH₃ | S | C-Cl | CH₂C=CH₂ | CH₂ | C-Cl |
| B1.100 | CH₃ | S | C-Cl | CH₂C.CH | CH₂ | C-Cl |
| B1.101 | CH₃ | S | C-Cl | CH₂OCH₃ | CH₂ | C-Cl |
| B1.102 | CH₃ | S | C-Cl | CH₂OCH₂CH₃ | CH₂ | C-Cl |
| B1.103 | CH₃ | S | C-Br | H | CH₂ | C-Cl |
| B1.104 | CH₃ | S | C-Br | CH₂CH₃ | CH₂ | C-Cl |
| B1.105 | CH₃ | S | C-Br | CH₂C=CH₂ | CH₂ | C-Cl |
| B1.106 | CH₃ | S | C-Br | CH₂C.CH | CH₂ | C-Cl |
| B1.107 | CH₃ | S | C-Br | CH₂OCH₃ | CH₂ | C-Cl |
| B1.108 | CH₃ | S | C-Br | CH₂OCH₂CH₃ | CH₂ | C-Cl |
| B1.109 | CH₂CH₃ | S | C-Cl | H | CH₂ | C-Cl |
| B1.110 | CH₂CH₃ | S | C-Cl | CH₂CH₃ | CH₂ | C-Cl |
| B1.111 | CH₂CH₃ | S | C-Cl | CH₂C=CH₂ | CH₂ | C-Cl |
| B1.112 | CH₂CH₃ | S | C-Cl | CH₂C.CH | CH₂ | C-Cl |
| B1.113 | CH₂CH₃ | S | C-Cl | CH₂OCH₃ | CH₂ | C-Cl |
| B1.114 | CH₂CH₃ | S | C-Cl | CH₂OCH₂CH₃ | CH₂ | C-Cl |
| B1.115 | CH₂CH₃ | S | C-Br | H | CH₂ | C-Cl |
| B1.116 | CH₂CH₃ | S | C-Br | CH₂CH₃ | CH₂ | C-Cl |
| B1.117 | CH₂CH₃ | S | C-Br | CH₂C=CH₂ | CH₂ | C-Cl |
| B1.118 | CH₂CH₃ | S | C-Br | CH₂C.CH | CH₂ | C-Cl |
| B1.119 | CH₂CH₃ | S | C-Br | CH₂OCH₃ | CH₂ | C-Cl |
| B1.120 | CH₂CH₃ | S | C-Br | CH₂OCH₂CH₃ | CH₂ | C-Cl |
| B1.121 | CH₃ | S | C-Cl | H | CH(CH₃) | C-Cl |
| B1.122 | CH₃ | S | C-Cl | CH₂CH₃ | CH(CH₃) | C-Cl |
| B1.123 | CH₃ | S | C-Cl | CH₂C=CH₂ | CH(CH₃) | C-Cl |
| B1.124 | CH₃ | S | C-Cl | CH₂C.CH | CH(CH₃) | C-Cl |
| B1.125 | CH₃ | S | C-Cl | CH₂OCH₃ | CH(CH₃) | C-Cl |
| B1.126 | CH₃ | S | C-Cl | CH₂OCH₂CH₃ | CH(CH₃) | C-Cl |
| B1.127 | CH₃ | S | C-Br | H | CH(CH₃) | C-Cl |
| B1.128 | CH₃ | S | C-Br | CH₂CH₃ | CH(CH₃) | C-Cl |
| B1.129 | CH₃ | S | C-Br | CH₂C=CH₂ | CH(CH₃) | C-Cl |
| B1.130 | CH₃ | S | C-Br | CH₂C.CH | CH(CH₃) | C-Cl |
| B1.131 | CH₃ | S | C-Br | CH₂OCH₃ | CH(CH₃) | C-Cl |
| B1.132 | CH₃ | S | C-Br | CH₂OCH₂CH₃ | CH(CH₃) | C-Cl |
| B1.133 | CH₂CH₃ | S | C-Cl | H | CH(CH₃) | C-Cl |
| B1.134 | CH₂CH₃ | S | C-Cl | CH₂CH₃ | CH(CH₃) | C-Cl |
| B1.135 | CH₂CH₃ | S | C-Cl | CH₂C=CH₂ | CH(CH₃) | C-Cl |
| B1.136 | CH₂CH₃ | S | C-Cl | CH₂C.CH | CH(CH₃) | C-Cl |
| B1.137 | CH₂CH₃ | S | C-Cl | CH₂OCH₃ | CH(CH₃) | C-Cl |
| B1.138 | CH₂CH₃ | S | C-Cl | CH₂OCH₂CH₃ | CH(CH₃) | C-Cl |
| B1.139 | CH₂CH₃ | S | C-Br | H | CH(CH₃) | C-Cl |
| B1.140 | CH₂CH₃ | S | C-Br | CH₂CH₃ | CH(CH₃) | C-Cl |
| B1.141 | CH₂CH₃ | S | C-Br | CH₂C=CH₂ | CH(CH₃) | C-Cl |
| B1.142 | CH₂CH₃ | S | C-Br | CH₂C.CH | CH(CH₃) | C-Cl |
| B1.143 | CH₂CH₃ | S | C-Br | CH₂OCH₃ | CH(CH₃) | C-Cl |
| B1.144 | CH₂CH₃ | S | C-Br | CH₂OCH₂CH₃ | CH(CH₃) | C-Cl |
| B1.145 | CH₃ | S | C-Cl | H | CHF | C-Cl |
| B1.146 | CH₃ | S | C-Cl | CH₂CH₃ | CHF | C-Cl |
| B1.147 | CH₃ | S | C-Cl | CH₂C=CH₂ | CHF | C-Cl |
| B1.148 | CH₃ | S | C-Cl | CH₂C.CH | CHF | C-Cl |
| B1.149 | CH₃ | S | C-Cl | CH₂OCH₃ | CHF | C-Cl |
| B1.150 | CH₃ | S | C-Cl | CH₂OCH₂CH₃ | CHF | C-Cl |
| B1.151 | CH₃ | S | C-Br | H | CHF | C-Cl |
| B1.152 | CH₃ | S | C-Br | CH₂CH₃ | CHF | C-Cl |
| B1.153 | CH₃ | S | C-Br | CH₂C=CH₂ | CHF | C-Cl |
| B1.154 | CH₃ | S | C-Br | CH₂C.CH | CHF | C-Cl |
| B1.155 | CH₃ | S | C-Br | CH₂OCH₃ | CHF | C-Cl |
| B1.156 | CH₃ | S | C-Br | CH₂OCH₂CH₃ | CHF | C-Cl |
| B1.157 | CH₂CH₃ | S | C-Cl | H | CHF | C-Cl |
| B1.158 | CH₂CH₃ | S | C-Cl | CH₂CH₃ | CHF | C-Cl |
| B1.159 | CH₂CH₃ | S | C-Cl | CH₂C=CH₂ | CHF | C-Cl |
| B1.160 | CH₂CH₃ | S | C-Cl | CH₂C.CH | CHF | C-Cl |
| B1.161 | CH₂CH₃ | S | C-Cl | CH₂OCH₃ | CHF | C-Cl |
| B1.162 | CH₂CH₃ | S | C-Cl | CH₂OCH₂CH₃ | CHF | C-Cl |
| B1.163 | CH₂CH₃ | S | C-Br | H | CHF | C-Cl |
| B1.164 | CH₂CH₃ | S | C-Br | CH₂CH₃ | CHF | C-Cl |
| B1.165 | CH₂CH₃ | S | C-Br | CH₂C=CH₂ | CHF | C-Cl |
| B1.166 | CH₂CH₃ | S | C-Br | CH₂C.CH | CHF | C-Cl |
| B1.167 | CH₂CH₃ | S | C-Br | CH₂OCH₃ | CHF | C-Cl |
| B1.168 | CH₂CH₃ | S | C-Br | CH₂OCH₂CH₃ | CHF | C-Cl |
| B1.169 | CH₃ | S | C-Cl | H | CH₂O | C-Cl |
| B1.170 | CH₃ | S | C-Cl | CH₂CH₃ | CH₂O | C-Cl |
| B1.171 | CH₃ | S | C-Cl | CH₂C=CH₂ | CH₂O | C-Cl |
| B1.172 | CH₃ | S | C-Cl | CH₂C.CH | CH₂O | C-Cl |
| B1.173 | CH₃ | S | C-Cl | CH₂OCH₃ | CH₂O | C-Cl |
| B1.174 | CH₃ | S | C-Cl | CH₂OCH₂CH₃ | CH₂O | C-Cl |
| B1.175 | CH₃ | S | C-Br | H | CH₂O | C-Cl |
| B1.176 | CH₃ | S | C-Br | CH₂CH₃ | CH₂O | C-Cl |
| B1.177 | CH₃ | S | C-Br | CH₂C=CH₂ | CH₂O | C-Cl |
| B1.178 | CH₃ | S | C-Br | CH₂C.CH | CH₂O | C-Cl |
| B1.179 | CH₃ | S | C-Br | CH₂OCH₃ | CH₂O | C-Cl |
| B1.180 | CH₃ | S | C-Br | CH₂OCH₂CH₃ | CH₂O | C-Cl |
| B1.181 | CH₂CH₃ | S | C-Cl | H | CH₂O | C-Cl |
| B1.182 | CH₂CH₃ | S | C-Cl | CH₂CH₃ | CH₂O | C-Cl |
| B1.183 | CH₂CH₃ | S | C-Cl | CH₂C=CH₂ | CH₂O | C-Cl |
| B1.184 | CH₂CH₃ | S | C-Cl | CH₂C.CH | CH₂O | C-Cl |
| B1.185 | CH₂CH₃ | S | C-Cl | CH₂OCH₃ | CH₂O | C-Cl |
| B1.186 | CH₂CH₃ | S | C-Cl | CH₂OCH₂CH₃ | CH₂O | C-Cl |
| B1.187 | CH₂CH₃ | S | C-Br | H | CH₂O | C-Cl |
| B1.188 | CH₂CH₃ | S | C-Br | CH₂CH₃ | CH₂O | C-Cl |
| B1.189 | CH₂CH₃ | S | C-Br | CH₂C=CH₂ | CH₂O | C-Cl |
| B1.190 | CH₂CH₃ | S | C-Br | CH₂C.CH | CH₂O | C-Cl |
| B1.191 | CH₂CH₃ | S | C-Br | CH₂OCH₃ | CH₂O | C-Cl |
| B1.192 | CH₂CH₃ | S | C-Br | CH₂OCH₂CH₃ | CH₂O | C-Cl |
| B1.193 | CH₃ | CH=CH | C-Cl | H | CH₂ | C-H |
| B1.194 | CH₃ | CH=CH | C-Cl | CH₂CH₃ | CH₂ | C-H |
| B1.195 | CH₃ | CH=CH | C-Cl | CH₂C=CH₂ | CH₂ | C-H |
| B1.196 | CH₃ | CH=CH | C-Cl | CH₂C.CH | CH₂ | C-H |
| B1.197 | CH₃ | CH=CH | C-Cl | CH₂OCH₃ | CH₂ | C-H |
| B1.198 | CH₃ | CH=CH | C-Cl | CH₂OCH₂CH₃ | CH₂ | C-H |
| B1.199 | CH₂CH₃ | CH=CH | C-Cl | H | CH₂ | C-H |
| B1.200 | CH₂CH₃ | CH=CH | C-Cl | CH₂CH₃ | CH₂ | C-H |
| B1.201 | CH₂CH₃ | CH=CH | C-Cl | CH₂C=CH₂ | CH₂ | C-H |
| B1.202 | CH₂CH₃ | CH=CH | C-Cl | CH₂C.CH | CH₂ | C-H |
| B1.203 | CH₂CH₃ | CH=CH | C-Cl | CH₂OCH₃ | CH₂ | C-H |
| B1.204 | CH₂CH₃ | CH=CH | C-Cl | CH₂OCH₂CH₃ | CH₂ | C-H |
| B1.205 | CH₃ | CH=CH | C-Cl | H | CH₂ | C-Cl |
| B1.206 | CH₃ | CH=CH | C-Cl | CH₂CH₃ | CH₂ | C-Cl |
| B1.207 | CH₃ | CH=CH | C-Cl | CH₂C=CH₂ | CH₂ | C-Cl |
| B1.208 | CH₃ | CH=CH | C-Cl | CH₂C.CH | CH₂ | C-Cl |
| B1.209 | CH₃ | CH=CH | C-Cl | CH₂OCH₃ | CH₂ | C-Cl |
| B1.210 | CH₃ | CH=CH | C-Cl | CH₂OCH₂CH₃ | CH₂ | C-Cl |
| B1.211 | CH₂CH₃ | CH=CH | C-Cl | H | CH₂ | C-Cl |
| B1.212 | CH₂CH₃ | CH=CH | C-Cl | CH₂CH₃ | CH₂ | C-Cl |
| B1.213 | CH₂CH₃ | CH=CH | C-Cl | CH₂C=CH₂ | CH₂ | C-Cl |
| B1.214 | CH₂CH₃ | CH=CH | C-Cl | CH₂C.CH | CH₂ | C-Cl |
| B1.215 | CH₂CH₃ | CH=CH | C-Cl | CH₂OCH₃ | CH₂ | C-Cl |
| B1.216 | CH₂CH₃ | CH=CH | C-Cl | CH₂OCH₂CH₃ | CH₂ | C-Cl |
| B1.217 | CH₃ | CH=CH | C-Cl | H | CH(CH₃) | C-H |
| B1.218 | CH₃ | CH=CH | C-Cl | CH₂CH₃ | CH(CH₃) | C-H |
| B1.219 | CH₃ | CH=CH | C-Cl | CH₂C=CH₂ | CH(CH₃) | C-H |
| B1.220 | CH₃ | CH=CH | C-Cl | CH₂C.CH | CH(CH₃) | C-H |
| B1.221 | CH₃ | CH=CH | C-Cl | CH₂OCH₃ | CH(CH₃) | C-H |
| B1.222 | CH₃ | CH=CH | C-Cl | CH₂OCH₂CH₃ | CH(CH₃) | C-H |
| B1.223 | CH₂CH₃ | CH=CH | C-Cl | H | CH(CH₃) | C-H |
| B1.224 | CH₂CH₃ | CH=CH | C-Cl | CH₂CH₃ | CH(CH₃) | C-H |
| B1.225 | CH₂CH₃ | CH=CH | C-Cl | CH₂C=CH₂ | CH(CH₃) | C-H |
| B1.226 | CH₂CH₃ | CH=CH | C-Cl | CH₂C.CH | CH(CH₃) | C-H |
| B1.227 | CH₂CH₃ | CH=CH | C-Cl | CH₂OCH₃ | CH(CH₃) | C-H |
| B1.228 | CH₂CH₃ | CH=CH | C-Cl | CH₂OCH₂CH₃ | CH(CH₃) | C-H |
| B1.229 | CH₃ | CH=CH | C-Cl | H | CH(CH₃) | C-Cl |
| B1.230 | CH₃ | CH=CH | C-Cl | CH₂CH₃ | CH(CH₃) | C-Cl |
| B1.231 | CH₃ | CH=CH | C-Cl | CH₂C=CH₂ | CH(CH₃) | C-Cl |
| B1.232 | CH₃ | CH=CH | C-Cl | CH₂C.CH | CH(CH₃) | C-Cl |
| B1.233 | CH₃ | CH=CH | C-Cl | CH₂OCH₃ | CH(CH₃) | C-Cl |
| B1.234 | CH₃ | CH=CH | C-Cl | CH₂OCH₂CH₃ | CH(CH₃) | C-Cl |
| B1.235 | CH₂CH₃ | CH=CH | C-Cl | H | CH(CH₃) | C-Cl |
| B1.236 | CH₂CH₃ | CH=CH | C-Cl | CH₂CH₃ | CH(CH₃) | C-Cl |
| B1.237 | CH₂CH₃ | CH=CH | C-Cl | CH₂C=CH₂ | CH(CH₃) | C-Cl |
| B1.238 | CH₂CH₃ | CH=CH | C-Cl | CH₂C.CH | CH(CH₃) | C-Cl |
| B1.239 | CH₂CH₃ | CH=CH | C-Cl | CH₂OCH₃ | CH(CH₃) | C-Cl |
| B1.240 | CH₂CH₃ | CH=CH | C-Cl | CH₂OCH₂CH₃ | CH(CH₃) | C-Cl |
| B1.241 | CH₃ | CH=CH | C-Cl | H | CHF | C-H |
| B1.242 | CH₃ | CH=CH | C-Cl | CH₂CH₃ | CHF | C-H |
| B1.243 | CH₃ | CH=CH | C-Cl | CH₂C=CH₂ | CHF | C-H |
| B1.244 | CH₃ | CH=CH | C-Cl | CH₂C.CH | CHF | C-H |
| B1.245 | CH₃ | CH=CH | C-Cl | CH₂OCH₃ | CHF | C-H |
| B1.246 | CH₃ | CH=CH | C-Cl | CH₂OCH₂CH₃ | CHF | C-H |
| B1.247 | CH₂CH₃ | CH=CH | C-Cl | H | CHF | C-H |
| B1.248 | CH₂CH₃ | CH=CH | C-Cl | CH₂CH₃ | CHF | C-H |
| B1.249 | CH₂CH₃ | CH=CH | C-Cl | CH₂C=CH₂ | CHF | C-H |
| B1.250 | CH₂CH₃ | CH=CH | C-Cl | CH₂C.CH | CHF | C-H |
| B1.251 | CH₂CH₃ | CH=CH | C-Cl | CH₂OCH₃ | CHF | C-H |
| B1.252 | CH₂CH₃ | CH=CH | C-Cl | CH₂OCH₂CH₃ | CHF | C-H |
| B1.253 | CH₃ | CH=CH | C-Cl | H | CHF | C-Cl |
| B1.254 | CH₃ | CH=CH | C-Cl | CH₂CH₃ | CHF | C-Cl |
| B1.255 | CH₃ | CH=CH | C-Cl | CH₂C=CH₂ | CHF | C-Cl |
| B1.256 | CH₃ | CH=CH | C-Cl | CH₂C.CH | CHF | C-Cl |
| B1.257 | CH₃ | CH=CH | C-Cl | CH₂OCH₃ | CHF | C-Cl |
| B1.258 | CH₃ | CH=CH | C-Cl | CH₂OCH₂CH₃ | CHF | C-Cl |
| B1.259 | CH₂CH₃ | CH=CH | C-Cl | H | CHF | C-Cl |
| B1.260 | CH₂CH₃ | CH=CH | C-Cl | CH₂CH₃ | CHF | C-Cl |
| B1.261 | CH₂CH₃ | CH=CH | C-Cl | CH₂C=CH₂ | CHF | C-Cl |
| B1.262 | CH₂CH₃ | CH=CH | C-Cl | CH₂C.CH | CHF | C-Cl |
| B1.263 | CH₂CH₃ | CH=CH | C-Cl | CH₂OCH₃ | CHF | C-Cl |
| B1.264 | CH₂CH₃ | CH=CH | C-Cl | CH₂OCH₂CH₃ | CHF | C-Cl |
| B1.265 | CH₃ | CH=CH | C-Cl | H | CH₂O | C-H |
| B1.266 | CH₃ | CH=CH | C-Cl | CH₂CH₃ | CH₂O | C-H |
| B1.267 | CH₃ | CH=CH | C-Cl | CH₂C=CH₂ | CH₂O | C-H |
| B1.268 | CH₃ | CH=CH | C-Cl | CH₂C.CH | CH₂O | C-H |
| B1.269 | CH₃ | CH=CH | C-Cl | CH₂OCH₃ | CH₂O | C-H |
| B1.270 | CH₃ | CH=CH | C-Cl | CH₂OCH₂CH₃ | CH₂O | C-H |
| B1.271 | CH₂CH₃ | CH=CH | C-Cl | H | CH₂O | C-H |
| B1.272 | CH₂CH₃ | CH=CH | C-Cl | CH₂CH₃ | CH₂O | C-H |
| B1.273 | CH₂CH₃ | CH=CH | C-Cl | CH₂C=CH₂ | CH₂O | C-H |
| B1.274 | CH₂CH₃ | CH=CH | C-Cl | CH₂C.CH | CH₂O | C-H |
| B1.275 | CH₂CH₃ | CH=CH | C-Cl | CH₂OCH₃ | CH₂O | C-H |
| B1.276 | CH₂CH₃ | CH=CH | C-Cl | CH₂OCH₂CH₃ | CH₂O | C-H |
| B1.277 | CH₃ | CH=CH | C-Cl | H | CH₂O | C-Cl |
| B1.278 | CH₃ | CH=CH | C-Cl | CH₂CH₃ | CH₂O | C-Cl |
| B1.279 | CH₃ | CH=CH | C-Cl | CH₂C=CH₂ | CH₂O | C-Cl |
| B1.280 | CH₃ | CH=CH | C-Cl | CH₂C.CH | CH₂O | C-Cl |
| B1.281 | CH₃ | CH=CH | C-Cl | CH₂OCH₃ | CH₂O | C-Cl |
| B1.282 | CH₃ | CH=CH | C-Cl | CH₂OCH₂CH₃ | CH₂O | C-Cl |
| B1.283 | CH₂CH₃ | CH=CH | C-Cl | H | CH₂O | C-Cl |
| B1.284 | CH₂CH₃ | CH=CH | C-Cl | CH₂CH₃ | CH₂O | C-Cl |
| B1.285 | CH₂CH₃ | CH=CH | C-Cl | CH₂C=CH₂ | CH₂O | C-Cl |
| B1.286 | CH₂CH₃ | CH=CH | C-Cl | CH₂C.CH | CH₂O | C-Cl |
| B1.287 | CH₂CH₃ | CH=CH | C-Cl | CH₂OCH₃ | CH₂O | C-Cl |
| B1.288 | CH₂CH₃ | CH=CH | C-Cl | CH₂OCH₂CH₃ | CH₂O | C-Cl |

### Table B2

Table B2 provides 288 compounds of Formula B1 wherein R¹, D, E, R¹¹, A and J are as defined in Table B1 and R⁶ is ethyl.

### Table B3

Table B3 provides 288 compounds of Formula B1 wherein R¹, D, E, R¹¹, A and J are as defined in Table B1 and R⁶ is *n*-propyl.

### Table B4

Table B4 provides 288 compounds of Formula B1 wherein R¹, D, E, R¹¹, A and J are as defined in Table B1 and R⁶ is *iso*-propyl.

### Table B5

Table B5 provides 288 compounds of Formula B1 wherein R¹, D, E, R¹¹, A and J are as defined in Table B1 and R⁶ is *n*-butyl.

### Table B6

Table B6 provides 288 compounds of Formula B1 wherein R¹, D, E, R¹¹, A and J are as defined in Table B1 and R⁶ is *sec*-butyl.

### Table B7

Table B7 provides 288 compounds of Formula B1 wherein R¹, D, E, R¹¹, A and J are as defined in Table B1 and R⁶ is *iso*-butyl.

### Table B8

Table B8 provides 288 compounds of Formula B1 wherein R¹, D, E, R¹¹, A and J are as defined in Table B1 and R⁶ is *tert*-butyl.

### Table B9

Table B9 provides 288 compounds of Formula B1 wherein R¹, D, E, R¹¹, A and J are as defined in Table B1 and R⁶ is *n*-pentyl.

### Table B10

Table B10 provides 288 compounds of Formula B1 wherein R¹, D, E, R¹¹, A and J are as defined in Table B1 and R⁶ is 1-methylbutyl.

### Table B11

Table B11 provides 288 compounds of Formula B1 wherein R¹, D, E, R¹¹, A and J are as defined in Table B1 and R⁶ is 2-methylbutyl.

### Table B12

Table B12 provides 288 compounds of Formula B1 wherein R¹, D, E, R¹¹, A and J are as defined in Table B1 and R⁶ is 3-methylbutyl.

### Table B13

Table B13 provides 288 compounds of Formula B1 wherein R¹, D, E, R¹¹, A and J are as defined in Table B1 and R⁶ is neopentyl.

### Table B14

Table B14 provides 288 compounds of Formula B1 wherein R¹, D, E, R¹¹, A and J are as defined in Table B1 and R⁶ is 2,2-dimethylbutyl.

### Table B15

Table B15 provides 288 compounds of Formula B1 wherein R¹, D, E, R¹¹, A and J are as defined in Table B1 and R⁶ is 2,2,2-trifluoroethyl.

### Table B16

Table B16 provides 288 compounds of Formula B1 wherein R¹, D, E, R¹¹, A and J are as defined in Table B1 and R⁶ is 2,2-dilluoro-2-methoxyethyl.

### Table B17

Table B17 provides 288 compounds of Formula B1 wherein R¹, D, E, R¹¹, A and J are as defined in Table B1 and R⁶ is 3,3,3-trifluoropropyl.

### Table B18

Table B18 provides 288 compounds of Formula B1 wherein R¹, D, E, R¹¹, A and J are as defined in Table B1 and R⁶ is *para*-fluorobenzyl.

### Table B19

Table B19 provides 288 compounds of Formula B1 wherein R¹, D, E, R¹¹, A and J are as defined in Table B1 and R⁶ is 2,4-dichlorophenyl.

### Table B20

Table B20 provides 288 compounds of Formula B1 wherein R¹, D, E, R¹¹, A and J are as defined in Table B1 and R⁶ is 2-chloro-4-trifluoromethylphenyl.

### Table C1

Table C1 provides 144 compounds of Formula C1 wherein X, R¹¹, A and J are as defined in Table C1 and R⁶ is methyl.

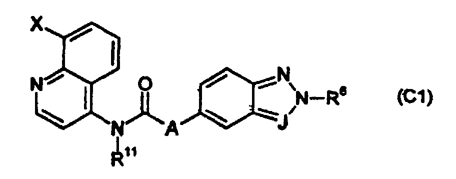

**Table C1**

| **Compound No** | **X** | **R**^{**11**} | **A** | **J** |
|---|---|---|---|---|
| C1.1 | H | H | CH₂ | N |
| C1.2 | H | CH₂CH₃ | CH₂ | N |
| C1.3 | H | CH₂C=CH₂ | CH₂ | N |
| C1.4 | H | CH₂C.CH | CH₂ | N |
| C1.5 | H | CH₂OCH₃ | CH₂ | N |
| C1.6 | H | CH₂OCH₂CH₃ | CH₂ | N |
| C1.7 | H | H | CH(CH₃) | N |
| C1.8 | H | CH₂CH₃ | CH(CH₃) | N |
| C1.9 | H | CH₂C=CH₂ | CH(CH₃) | N |
| C1.10 | H | CH₂C.CH | CH(CH₃) | N |
| C1.11 | H | CH₂OCH₃ | CH(CH₃) | N |
| C1.12 | H | CH₂OCH₂CH₃ | CH(CH₃) | N |
| C1.13 | H | H | CHF | N |
| C1.14 | H | CH₂CH₃ | CHF | N |
| C1.15 | H | CH₂C=CH₂ | CHF | N |
| C1.16 | H | CH₂C.CH | CHF | N |
| C1.17 | H | CH₂OCH₃ | CHF | N |
| C1.18 | H | CH₂OCH₂CH₃ | CHF | N |
| C1.19 | H | H | CH₂O | N |
| C1.20 | H | CH₂CH₃ | CH₂O | N |
| C1.21 | H | CH₂C=CH₂ | CH₂O | N |
| C1.22 | H | CH₂C.CH | CH₂O | N |
| C1.23 | H | CH₂OCH₃ | CH₂O | N |
| C1.24 | H | CH₂OCH₂CH₃ | CH₂O | N |
| C1.25 | F | H | CH₂ | N |
| C1.26 | F | CH₂CH₃ | CH₂ | N |
| C1.27 | F | CH₂C=CH₂ | CH₂ | N |
| C1.28 | F | CH₂C.CH | CH₂ | N |
| C1.29 | F | CH₂OCH₃ | CH₂ | N |
| C1.30 | F | CH₂OCH₂CH₃ | CH₂ | N |
| C1.31 | F | H | CH(CH₃) | N |
| C1.32 | F | CH₂CH₃ | CH(CH₃) | N |
| C1.33 | F | CH₂C=CH₂ | CH(CH₃) | N |
| C1.34 | F | CH₂C.CH | CH(CH₃) | N |
| C1.35 | F | CH₂OCH₃ | CH(CH₃) | N |
| C1.36 | F | CH₂OCH₂CH₃ | CH(CH₃) | N |
| C1.37 | F | H | CHF | N |
| C1.38 | F | CH₂CH₃ | CHF | N |
| C1.39 | F | CH₂C=CH₂ | CHF | N |
| C1.40 | F | CH₂C.CH | CHF | N |
| C1.41 | F | CH₂OCH₃ | CHF | N |
| C1.42 | F | CH₂OCH₂CH₃ | CHF | N |
| C1.43 | F | H | CH₂O | N |
| C1.44 | F | CH₂CH₃ | CH₂O | N |
| C1.45 | F | CH₂C=CH₂ | CH₂O | N |
| C1.46 | F | CH₂C.CH | CH₂O | N |
| C1.47 | F | CH₂OCH₃ | CH₂O | N |
| C1.48 | F | CH₂OCH₂CH₃ | CH₂O | N |
| C1.49 | H | H | CH₂ | C-H |
| C1.50 | H | CH₂CH₃ | CH₂ | C-H |
| C1.51 | H | CH₂C=CH₂ | CH₂ | C-H |
| C1.52 | H | CH₂C.CH | CH₂ | C-H |
| C1.53 | H | CH₂OCH₃ | CH₂ | C-H |
| C1.54 | H | CH₂OCH₂CH₃ | CH₂ | C-H |
| C1.55 | H | H | CH(CH₃) | C-H |
| C1.56 | H | CH₂CH₃ | CH(CH₃) | C-H |
| C1.57 | H | CH₂C=CH₂ | CH(CH₃) | C-H |
| C1.58 | H | CH₂C.CH | CH(CH₃) | C-H |
| C1.59 | H | CH₂OCH₃ | CH(CH₃) | C-H |
| C1.60 | H | CH₂OCH₂CH₃ | CH(CH₃) | C-H |
| C1.61 | H | H | CHF | C-H |
| C1.62 | H | CH₂CH₃ | CHF | C-H |
| C1.63 | H | CH₂C=CH₂ | CHF | C-H |
| C1.64 | H | CH₂C.CH | CHF | C-H |
| C1.65 | H | CH₂OCH₃ | CHF | C-H |
| C1.66 | H | CH₂OCH₂CH₃ | CHF | C-H |
| C1.67 | H | H | CH₂O | C-H |
| C1.68 | H | CH₂CH₃ | CH₂O | C-H |
| C1.69 | H | CH₂C=CH₂ | CH₂O | C-H |
| C1.70 | H | CH₂C.CH | CH₂O | C-H |
| C1.71 | H | CH₂OCH₃ | CH₂O | C-H |
| C1.72 | H | CH₂OCH₂CH₃ | CH₂O | C-H |
| C1.73 | F | H | CH₂ | C-H |
| C1.74 | F | CH₂CH₃ | CH₂ | C-H |
| C1.75 | F | CH₂C=CH₂ | CH₂ | C-H |
| C1.76 | F | CH₂C.CH | CH₂ | C-H |
| C1.77 | F | CH₂OCH₃ | CH₂ | C-H |
| C1.78 | F | CH₂OCH₂CH₃ | CH₂ | C-H |
| C1.79 | F | H | CH(CH₃) | C-H |
| C1.80 | F | CH₂CH₃ | CH(CH₃) | C-H |
| C1.81 | F | CH₂C=CH₂ | CH(CH₃) | C-H |
| C1.82 | F | CH₂C.CH | CH(CH₃) | C-H |
| C1.83 | F | CH₂OCH₃ | CH(CH₃) | C-H |
| C1.84 | F | CH₂OCH₂CH₃ | CH(CH₃) | C-H |
| C1.85 | F | H | CHF | C-H |
| C1.86 | F | CH₂CH₃ | CHF | C-H |
| C1.87 | F | CH₂C=CH₂ | CHF | C-H |
| C1.88 | F | CH₂C.CH | CHF | C-H |
| C1.89 | F | CH₂OCH₃ | CHF | C-H |
| C1.90 | F | CH₂OCH₂CH₃ | CHF | C-H |
| C1.91 | F | H | CH₂O | C-H |
| C1.92 | F | CH₂CH₃ | CH₂O | C-H |
| C1.93 | F | CH₂C=CH₂ | CH₂O | C-H |
| C1.94 | F | CH₂C.CH | CH₂O | C-H |
| C1.95 | F | CH₂OCH₃ | CH₂O | C-H |
| C1.96 | F | CH₂OCH₂CH₃ | CH₂O | C-H |
| C1.97 | H | H | CH₂ | C-Cl |
| C1.98 | H | CH₂CH₃ | CH₂ | C-Cl |
| C1.99 | H | CH₂C=CH₂ | CH₂ | C-Cl |
| C1.100 | H | CH₂C.CH | CH₂ | C-Cl |
| C1.101 | H | CH₂OCH₃ | CH₂ | C-Cl |
| C1.102 | H | CH₂OCH₂CH₃ | CH₂ | C-Cl |
| C1.103 | H | H | CH(CH₃) | C-Cl |
| C1.104 | H | CH₂CH₃ | CH(CH₃) | C-Cl |
| C1.105 | H | CH₂C=CH₂ | CH(CH₃) | C-Cl |
| C1.106 | H | CH₂C.CH | CH(CH₃) | C-Cl |
| C1.107 | H | CH₂OCH₃ | CH(CH₃) | C-Cl |
| C1.108 | H | CH₂OCH₂CH₃ | CH(CH₃) | C-Cl |
| C1.109 | H | H | CHF | C-Cl |
| C1.110 | H | CH₂CH₃ | CHF | C-Cl |
| C1.111 | H | CH₂C=CH₂ | CHF | C-Cl |
| C1.112 | H | CH₂C.CH | CHF | C-Cl |
| C1.113 | H | CH₂OCH₃ | CHF | C-Cl |
| C1.114 | H | CH₂OCH₂CH₃ | CHF | C-Cl |
| C1.115 | H | H | CH₂O | C-Cl |
| C1.116 | H | CH₂CH₃ | CH₂O | C-Cl |
| C1.117 | H | CH₂C=CH₂ | CH₂O | C-Cl |
| C1.118 | H | CH₂C.CH | CH₂O | C-Cl |
| C1.119 | H | CH₂OCH₃ | CH₂O | C-Cl |
| C1.120 | H | CH₂OCH₂CH₃ | CH₂O | C-Cl |
| C1.121 | F | H | CH₂ | C-Cl |
| C1.122 | F | CH₂CH₃ | CH₂ | C-Cl |
| C1.123 | F | CH₂C=CH₂ | CH₂ | C-Cl |
| C1.124 | F | CH₂C.CH | CH₂ | C-Cl |
| C1.125 | F | CH₂OCH₃ | CH₂ | C-Cl |
| C1.126 | F | CH₂OCH₂CH₃ | CH₂ | C-Cl |
| C1.127 | F | H | CH(CH₃) | C-Cl |
| C1.128 | F | CH₂CH₃ | CH(CH₃) | C-Cl |
| C1.129 | F | CH₂C=CH₂ | CH(CH₃) | C-Cl |
| C1.130 | F | CH₂C.CH | CH(CH₃) | C-Cl |
| C1.131 | F | CH₂OCH₃ | CH(CH₃) | C-Cl |
| C1.132 | F | CH₂OCH₂CH₃ | CH(CH₃) | C-Cl |
| C1.133 | F | H | CHF | C-Cl |
| C1.134 | F | CH₂CH₃ | CHF | C-Cl |
| C1.135 | F | CH₂C=CH₂ | CHF | C-Cl |
| C1.136 | F | CH₂C.CH | CHF | C-Cl |
| C1.137 | F | CH₂OCH₃ | CHF | C-Cl |
| C1.138 | F | CH₂OCH₂CH₃ | CHF | C-Cl |
| C1.139 | F | H | CH₂O | C-Cl |
| C1.140 | F | CH₂CH₃ | CH₂O | C-Cl |
| C1.141 | F | CH₂O=CH₂ | CH₂O | C-Cl |
| C1.142 | F | CH₂C.CH | CH₂O | C-Cl |
| C1.143 | F | CH₂OCH₃ | CH₂O | C-Cl |
| C1.144 | F | CH₂OCH₂CH₃ | CH₂O | C-Cl |

### Table C2

Table C2 provides 144 compounds of Formula C1 wherein X, R¹¹, A and J are as defined in Table C1 and R⁶ is ethyl.

### Table C3

Table C3 provides 144 compounds of Formula C1 wherein X, R¹¹, A and J are as defined in Table C1 and R⁶ is *n*-propyl.

### Table C4

Table C4 provides 144 compounds of Formula C1 wherein X, R¹¹, A and J are as defined in Table C1 and R⁶ is *iso-*propyl.

### Table C5

Table C5 provides 144 compounds of Formula C1 wherein X, R¹¹, A and J are as defined in Table C1 and R⁶ is *n*-butyl.

### Table C6

Table C6 provides 144 compounds of Formula C1 wherein X, R¹¹, A and J are as defined in Table C1 and R⁶ is *sec*-butyl.

### Table C7

Table C7 provides 144 compounds of Formula C1 wherein X, R¹¹, A and J are as defined in Table C1 and R⁶ is *iso*-butyl.

### Table C8

Table C8 provides 144 compounds of Formula C1 wherein X, R¹¹, A and J are as defined in Table C1 and R⁶ is *tert*-butyl.

### Table C9

Table C9 provides 144 compounds of Formula C1 wherein X, R¹¹, A and J are as defined in Table C1 and R⁶ is *n*-pentyl.

### Table C10

Table C10 provides 144 compounds of Formula C1 wherein X, R¹¹, A and J are as defined in Table C1 and R⁶ is 1-methylbutyl.

### Table C11

Table C11 provides 144 compounds of Formula C1 wherein X, R¹¹, A and J are as defined in Table C1 and R⁶ is 2-methylbutyl.

### Table C12

Table C12 provides 144 compounds of Formula C1 wherein X, R¹¹, A and J are as defined in Table C1 and R⁶ is 3-methylbutyl.

### Table C13

Table C13 provides 144 compounds of Formula C1 wherein X, R¹¹, A and J are as defined in Table C1 and R⁶ is neopentyl.

### Table C14

Table C14 provides 144 compounds of Formula C1 wherein X, R¹¹, A and J are as defined in Table C1 and R⁶ is 2,2-dimethylbutyl.

### Table C15

Table C15 provides 144 compounds of Formula C1 wherein X, R¹¹, A and J are as defined in Table C1 and R⁶ is 2,2,2-trifluoroethyl.

### Table C16

Table C16 provides 144 compounds of Formula C1 wherein X, R¹¹, A and J are as defined in Table C1 and R⁶ is 2,2-difluoro-2-methoxyethyl.

### Table C17

Table C17 provides 144 compounds of Formula C1 wherein X, R¹¹, A and J are as defined in Table C1 and R⁶ is 3,3,3-trifluoropropyl.

### Table C18

Table C18 provides 144 compounds of Formula C1 wherein X, R¹¹, A and J are as defined in Table C1 and R⁶ is *para*-fluorobenzyl.

### Table C19

Table C19 provides 144 compounds of Formula C1 wherein X, R¹¹, A and J are as defined in Table C1 and R⁶ is 2,4-dichlorophenyl.

### Table C20

Table C20 provides 144 compounds of Formula C1 wherein X, R¹¹, A and J are as defined in Table C1 and R⁶ is 2-chloro-4-trifluoromethylphenyl.

### Table D1

Table D1 provides 96 compounds of Formula D1 wherein X, R¹¹, A and J are as defined in Table D1 and R⁶ is methyl.

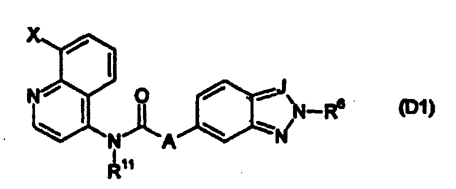

**Table D1**

| **Compound No** | **X** | **R**^{**11**} | **A** | **J** |
|---|---|---|---|---|
| D1.1 | H | H | CH₂ | C-H |
| D1.2 | H | CH₂CH₃ | CH₂ | C-H |
| D1.3 | H | CH₂C=CH₂ | CH₂ | C-H |
| D1.4 | H | CH₂C.CH | CH₂ | C-H |
| D1.5 | H | CH₂OCH₃ | CH₂ | C-H |
| D1.6 | H | CH₂OCH₂CH₃ | CH₂ | C-H |
| D1.7 | H | H | CH(CH₃) | C-H |
| D1.8 | H | CH₂CH₃ | CH(CH₃) | C-H |
| D1.9 | H | CH₂C=CH₂ | CH(CH₃) | C-H |
| D1.10 | H | CH₂C.CH | CH(CH₃) | C-H |
| D1.11 | H | CH₂OCH₃ | CH(CH₃) | C-H |
| D1.12 | H | CH₂OCH₂CH₃ | CH(CH₃) | C-H |
| D1.13 | H | H | CHF | C-H |
| D1.14 | H | CH₂CH₃ | CHF | C-H |
| D1.15 | H | CH₂C=CH₂ | CHF | C-H |
| D1.16 | H | CH₂C.CH | CHF | C-H |
| D1.17 | H | CH₂OCH₃ | CHF | C-H |
| D1.18 | H | CH₂OCH₂CH₃ | CHF | C-H |
| D1.19 | H | H | CH₂O | C-H |
| D1.20 | H | CH₂CH₃ | CH₂O | C-H |
| D1.21 | H | CH₂C=CH₂ | CH₂O | C-H |
| D1.22 | H | CH₂C.CH | CH₂O | C-H |
| D1.23 | H | CH₂OCH₃ | CH₂O | C-H |
| D1.24 | H | CH₂OCH₂CH₃ | CH₂O | C-H |
| D1.25 | F | H | CH₂ | C-H |
| D1.26 | F | CH₂CH₃ | CH₂ | C-H |
| D1.27 | F | CH₂C=CH₂ | CH₂ | C-H |
| D1.28 | F | CH₂C.CH | CH₂ | C-H |
| D1.29 | F | CH₂OCH₃ | CH₂ | C-H |
| D1.30 | F | CH₂OCH₂CH₃ | CH₂ | C-H |
| D1.31 | F | H | CH(CH₃) | C-H |
| D1.32 | F | CH₂CH₃ | CH(CH₃) | C-H |
| D1.33 | F | CH₂C=CH₂ | CH(CH₃) | C-H |
| D1.34 | F | CH₂C.CH | CH(CH₃) | C-H |
| D1.35 | F | CH₂OCH₃ | CH(CH₃) | C-H |
| D1.36 | F | CH₂OCH₂CH₃ | CH(CH₃) | C-H |
| D1.37 | F | H | CHF | C-H |
| D1.38 | F | CH₂CH₃ | CHF | C-H |
| D1.39 | F | CH₂C=CH₂ | CHF | C-H |
| D1.40 | F | CH₂C.CH | CHF | C-H |
| D1.41 | F | CH₂OCH₃ | CHF | C-H |
| D1.42 | F | CH₂OCH₂CH₃ | CHF | C-H |
| D1.43 | F | H | CH₂O | C-H |
| D1.44 | F | CH₂CH₃ | CH₂O | C-H |
| D1.45 | F | CH₂C=CH₂ | CH₂O | C-H |
| D1.46 | F | CH₂C.CH | CH₂O | C-H |
| D1.47 | F | CH₂OCH₃ | CH₂O | C-H |
| D1.48 | F | CH₂OCH₂CH₃ | CH₂O | C-H |
| D1.49 | H | H | CH₂ | C-Cl |
| D1.50 | H | CH₂CH₃ | CH₂ | C-Cl |
| D1.51 | H | CH₂C=CH₂ | CH₂ | C-Cl |
| D1.52 | H | CH₂C.CH | CH₂ | C-Cl |
| D1.53 | H | CH₂OCH₃ | CH₂ | C-Cl |
| D1.54 | H | CH₂OCH₂CH₃ | CH₂ | C-Cl |
| D1.55 | H | H | CH(CH₃) | C-Cl |
| D1.56 | H | CH₂CH₃ | CH(CH₃) | C-Cl |
| D1.57 | H | CH₂C=CH₂ | CH(CH₃) | C-Cl |
| D1.58 | H | CH₂C.CH | CH(CH₃) | C-Cl |
| D1.59 | H | CH₂OCH₃ | CH(CH₃) | C-Cl |
| D1.60 | H | CH₂OCH₂CH₃ | CH(CH₃) | C-Cl |
| D1.61 | H | H | CHF | C-Cl |
| D1.62 | H | CH₂CH₃ | CHF | C-Cl |
| D1.63 | H | CH₂C=CH₂ | CHF | C-Cl |
| D1.64 | H | CH₂C.CH | CHF | C-Cl |
| D1.65 | H | CH₂OCH₃ | CHF | C-Cl |
| D1.66 | H | CH₂OCH₂CH₃ | CHF | C-Cl |
| D1.67 | H | H | CH₂O | C-Cl |
| D1.68 | H | CH₂CH₃ | CH₂O | C-Cl |
| D1.69 | H | CH₂C=CH₂ | CH₂O | C-Cl |
| D1.70 | H | CH₂C.CH | CH₂O | C-Cl |
| D1.71 | H | CH₂OCH₃ | CH₂O | C-Cl |
| D1.72 | H | CH₂OCH₂CH₃ | CH₂O | C-Cl |
| D1.73 | F | H | CH₂ | C-Cl |
| D1.74 | F | CH₂CH₃ | CH₂ | C-Cl |
| D1.75 | F | CH₂C=CH₂ | CH₂ | C-Cl |
| D1.76 | F | CH₂C.CH | CH₂ | C-Cl |
| D1.77 | F | CH₂OCH₃ | CH₂ | C-Cl |
| D1.78 | F | CH₂OCH₂CH₃ | CH₂ | C-Cl |
| D1.79 | F | H | CH(CH₃) | C-Cl |
| D1.80 | F | CH₂CH₃ | CH(CH₃) | C-Cl |
| D1.81 | F | CH₂C=CH₂ | CH(CH₃) | C-Cl |
| D1.82 | F | CH₂C.CH | CH(CH₃) | C-Cl |
| D1.83 | F | CH₂OCH₃ | CH(CH₃) | C-Cl |
| D1.84 | F | CH₂OCH₂CH₃ | CH(CH₃) | C-Cl |
| D1.85 | F | H | CHF | C-Cl |
| D1.86 | F | CH₂CH₃ | CHF | C-Cl |
| D1.87 | F | CH₂C=CH₂ | CHF | C-Cl |
| D1.88 | F | CH₂C.CH | CHF | C-Cl |
| D1.89 | F | CH₂OCH₃ | CHF | C-Cl |
| D1.90 | F | CH₂OCH₂CH₃ | CHF | C-Cl |
| D1.91 | F | H | CH₂O | C-Cl |
| D1.92 | F | CH₂CH₃ | CH₂O | C-Cl |
| D1.93 | F | CH₂C=CH₂ | CH₂O | C-Cl |
| D1.94 | F | CH₂C.CH | CH₂O | C-Cl |
| D1.95 | F | CH₂OCH₃ | CH₂O | C-Cl |
| D1.96 | F | CH₂OCH₂CH₃ | CH₂O | C-Cl |

### Table D2

Table D2 provides 96 compounds of Formula D1 wherein X, R¹¹, A and J are as defined in Table D1 and R⁶ is ethyl.

### Table D3

Table D3 provides 96 compounds of Formula D1 wherein X, R¹¹, A and J are as defined in Table D1 and R⁶ is *n*-propyl.

### Table D4

Table D4 provides 96 compounds of Formula D1 wherein X, R¹¹, A and J are as defined in Table D1 and R⁶ is *iso*-propyl.

### Table D5

Table D5 provides 96 compounds of Formula D1 wherein X, R¹¹, A and J are as defined in Table D1 and R⁶ is *n*-butyl.

### Table D6

Table D6 provides 96 compounds of Formula D1 wherein X, R¹¹, A and J are as defined in Table D1 and R⁶ is *sec*-butyl.

### Table D7

Table D7 provides 96 compounds of Formula D1 wherein X, R¹¹, A and J are as defined in Table D1 and R⁶ is *iso*-butyl.

### Table D8

Table D8 provides 96 compounds of Formula D1 wherein X, R¹¹, A and J are as defined in Table D1 and R⁶ is *tert*-butyl.

### Table D9

Table D9 provides 96 compounds of Formula D1 wherein X, R¹¹, A and J are as defined in Table D1 and R⁶ is *n*-pentyl.

### Table D10

Table D10 provides 96 compounds of Formula D1 wherein X, R¹¹, A and J are as defined in Table D1 and R⁶ is 1-methylbutyl.

### Table D11

Table D11 provides 96 compounds of Formula D1 wherein X, R¹¹, A and J are as defined in Table D1 and R⁶ is 2-methylbutyl.

### Table D12

Table D12 provides 96 compounds of Formula D1 wherein X, R¹¹, A and J are as defined in Table D1 and R⁶ is 3-methylbutyl.

### Table D13

Table D13 provides 96 compounds of Formula D1 wherein X, R¹¹, A and J are as defined in Table D1 and R⁶ is neopentyl.

### Table D14

Table D14 provides 96 compounds of Formula D1 wherein X, R¹¹, A and J are as defined in Table D1 and R⁶ is 2,2-dimethylbutyl.

### Table D15

Table D15 provides 96 compounds of Formula D1 wherein X, R¹¹, A and J are as defined in Table D1 and R⁶ is 2,2,2-trifluoroethyl.

### Table D16

Table D16 provides 96 compounds of Formula D1 wherein X, R¹¹, A and J are as defined in Table D1 and R⁶ is 2,2-difluoro-2-methoxyethyl.

### Table D17

Table D17 provides 96 compounds of Formula D1 wherein X, R¹¹, A and J are as defined in Table D1 and R⁶ is 3,3,3-trifluoropropyl.

### Table D18

Table D18 provides 96 compounds of Formula D1 wherein X, R¹¹, A and J are as defined in Table D1 and R⁶ is *para*-fluorobenzyl.

### Table D19

Table D19 provides 96 compounds of Formula D1 wherein X, R¹¹, A and J are as defined in Table D1 and R⁶ is 2,4-dichlorophenyl.

### Table D20

Table D20 provides 96 compounds of Formula D1 wherein X, R¹¹, A and J are as defined in Table D1 and R⁶ is 2-chloro-4-trifluoromethylphenyl.

### Table E1

Table E1 provides 432 compounds of Formula E1 wherein R¹, D, E, R¹¹, A and J are as defined in Table A1 and R⁶ is methyl.

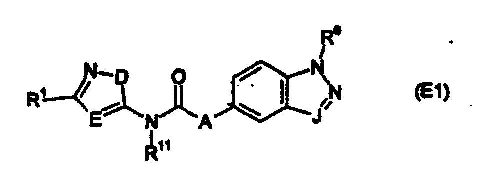

### Table E2

Table E2 provides 432 compounds of Formula E1 wherein R¹, D, E, R¹¹, A and J are as defined in Table A1 and R⁶ is ethyl.

### Table E3

Table E3 provides 432 compounds of Formula E1 wherein R¹, D, E, R¹¹, A and J are as defined in Table A1 and R⁶ is *n*-propyl.

### Table E4

Table E4 provides 432 compounds of Formula E1 wherein R¹, D, E, R¹¹, A and J are as defined in Table A1 and R⁶ is *iso*-propyl.

### Table E5

Table E5 provides 432 compounds of Formula E1 wherein R¹, D, E, R¹¹, A and J are as defined in Table A1 and R⁶ is *n*-butyl.

### Table E6

Table E6 provides 432 compounds of Formula E1 wherein R¹, D, E, R¹¹, A and J are as defined in Table A1 and R⁶ is *sec*-butyl.

### Table E7

Table E7 provides 432 compounds of Formula E1 wherein R¹, D, E, R¹¹, A and J are as defined in Table A1 and R⁶ is *iso*-butyl.

### Table E8

Table E8 provides 432 compounds of Formula E1 wherein R¹, D, E, R¹¹, A and J are as defined in Table A1 and R⁶ is *tert*-butyl.

### Table E9

Table E9 provides 432 compounds of Formula E1 wherein R¹, D, E, R¹¹, A and J are as defined in Table A1 and R⁶ is *n*-pentyl.

### Table E10

Table E10 provides 432 compounds of Formula E1 wherein R¹, D, E, R¹¹, A and J are as defined in Table A1 and R⁶ is 1-methylbutyl.

### Table E11

Table E11 provides 432 compounds of Formula E1 wherein R¹, D, E, R¹¹, A and J are as defined in Table A1 and R⁶ is 2-methylbutyl.

### Table E12

Table E12 provides 432 compounds of Formula E1 wherein R¹, D, E, R¹¹, A and J are as defined in Table A1 and R⁶ is 3-methylbutyl.

### Table E13

Table E13 provides 432 compounds of Formula E1 wherein R¹, D, E, R¹¹, A and J are as defined in Table A1 and R⁶ is neopentyl.

### Table E14

Table E14 provides 432 compounds of Formula E1 wherein R¹, D, E, R¹¹, A and J are as defined in Table A1 and R⁶ is 2,2-dimethylbutyl.

### Table E15

Table E15 provides 432 compounds of Formula E1 wherein R¹, D, E, R¹¹, A and J are as defined in Table A1 and R⁶ is 2,2,2-trifluoroethyl.

### Table E16

Table E16 provides 432 compounds of Formula E1 wherein R¹, D, E, R¹¹, A and J are as defined in Table A1 and R⁶ is 2,2-difluoro-2-methoxyethyl.

### Table E17

Table E17 provides 432 compounds of Formula E1 wherein R¹, D, E, R¹¹, A and J are as defined in Table A1 and R⁶ is 3,3,3-trifluoropropyl.

### Table E18

Table E18 provides 432 compounds of Formula E1 wherein R¹, D, E, R¹¹, A and J are as defined in Table A1 and R⁶ is *para*-fluorobenzyl.

### Table E19

Table E19 provides 432 compounds of Formula E1 wherein R¹, D, E, R¹¹, A and J are as defined in Table A1 and R⁶ is 2,4-dichlorophenyl.

### Table E20

Table E20 provides 432 compounds of Formula E1 wherein R¹, D, E, R¹¹, A and J are as defined in Table A1 and R⁶ is 2-chloro-4-trifluoromethylphenyl.

### Table E21

Table E21 provides 432 compounds of Formula E1 wherein R¹, D, E, R¹¹, A and J are as defined in Table A1 and R⁶ is 4-trifluoromethylphenyl.

### Table E22

Table E22 provides 432 compounds of Formula E1 wherein R¹, D, E, R¹¹, A and J are as defined in Table A1 and R⁶ is COCH₃.

### Table F1

Table F1 provides 432 compounds of Formula F1 wherein R¹, D, E, R¹¹, A and J are as defined in Table A1 and R⁶ is methyl.

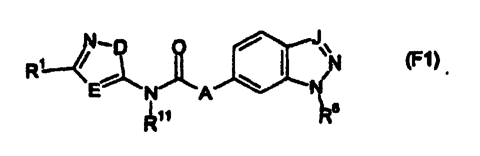

### Table F2

Table F2 provides 432 compounds of Formula F1 wherein R¹, D, E, R¹¹, A and J are as defined in Table A1 and R⁶ is ethyl.

### Table F3

Table F3 provides 432 compounds of Formula F1 wherein R¹, D, E, R¹¹, A and J are as defined in Table A1 and R⁶ is *n*-propyl.

### Table F4

Table F4 provides 432 compounds of Formula F1 wherein R¹, D, E, R¹¹, A and J are as defined in Table A1 and R⁶ is *iso*-propyl.

### Table F5

Table F5 provides 432 compounds of Formula F1 wherein R¹, D, E, R¹¹, A and J are as defined in Table A1 and R⁶ is *n*-butyl.

### Table F6

Table F6 provides 432 compounds of Formula F1 wherein R¹, D, E, R¹¹, A and J are as defined in Table A1 and R⁶ is *sec*-butyl.

### Table F7

Table F7 provides 432 compounds of Formula F1 wherein R¹, D, E, R¹¹, A and J are as defined in Table A1 and R⁶ is *iso*-butyl.

### Table F8

Table F8 provides 432 compounds of Formula F1 wherein R¹, D, E, R¹¹, A and J are as defined in Table A1 and R⁶ is *tert*-butyl.

### Table F9

Table F9 provides 432 compounds of Formula F1 wherein R¹, D, E, R¹¹, A and J are as defined in Table A1 and R⁶ is *n*-pentyl.

### Table F10

Table F10 provides 432 compounds of Formula F1 wherein R¹, D, E, R¹¹, A and J are as defined in Table A1 and R⁶ is 1-methylbutyl.

### Table F11

Table F11 provides 432 compounds of Formula F1 wherein R¹, D, E, R¹¹, A and J are as defined in Table A1 and R⁶ is 2-methylbutyl.

### Table F12

Table F12 provides 432 compounds of Formula F1 wherein R¹, D, E, R¹¹, A and J are as defined in Table A1 and R⁶ is 3-methylbutyl.

### Table F13

Table F13 provides 432 compounds of Formula F1 wherein R¹, D, E, R¹¹, A and J are as defined in Table A1 and R⁶ is neopentyl.

### Table F14

Table F14 provides 432 compounds of Formula F1 wherein R¹, D, E, R¹¹, A and J are as defined in Table A1 and R⁶ is 2,2-dimethylbutyl.

### Table F15

Table F15 provides 432 compounds of Formula F1 wherein R¹, D, E, R¹¹, A and J are as defined in Table A1 and R⁶ is 2,2,2-trifluoroethyl.

### Table F16

Table F16 provides 432 compounds of Formula F1 wherein R¹, D, E, R¹¹, A and J are as defined in Table A1 and R⁶ is 2,2-difluoro-2-methoxyethyl.

### Table F17

Table F17 provides 432 compounds of Formula F1 wherein R¹, D, E, R¹¹, A and J are as defined in Table A1 and R⁶ is 3,3,3-trifluoropropyl.

### Table F18

Table F18 provides 432 compounds of Formula F1 wherein R¹, D, E, R¹¹, A and J are as defined in Table A1 and R⁶ is *para*-fluorobenzyl.

### Table F19

Table F19 provides 432 compounds of Formula F1 wherein R¹, D, E, R¹¹, A and J are as defined in Table A1 and R⁶ is 2,4-dichlorophenyl.

### Table F20

Table F20 provides 432 compounds of Formula F1 wherein R¹, D, E, R¹¹, A and J are as defined in Table A1 and R⁶ is 2-chloro-4-trifluoromethylphenyl.

### Table F21

Table F21 provides 432 compounds of Formula F1 wherein R¹, D, E, R¹¹, A and J are as defined in Table A1 and R⁶ is 4-trifluoromethylphenyl.

### Table F22

Table F22 provides 432 compounds of Formula F1 wherein R¹, D, E, R¹¹, A and J are as defined in Table A1 and R⁶ is COCH₃.

### Table G1

Table G1 provides 144 compounds of Formula G1 wherein X, R¹¹, A and J are as defined in Table C1 and R⁶ is methyl.

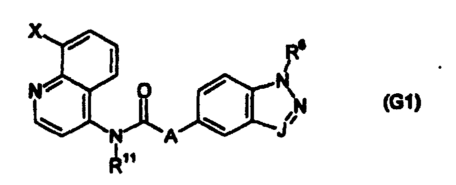

### Table G2

Table G2 provides 144 compounds of Formula G1 wherein X, R¹¹, A and J are as defined in Table C1 and R⁶ is ethyl.

### Table G3

Table G3 provides 144 compounds of Formula G1 wherein X, R¹¹, A and J are as defined in Table C1 and R⁶ is *n*-propyl.

### Table G4

Table G4 provides 144 compounds of Formula G1 wherein X, R¹¹, A and J are as defined in Table C1 and R⁶ is *iso*-propyl.

### Table G5

Table G5 provides 144 compounds of Formula G1 wherein X, R¹¹, A and J are as defined in Table C1 and R⁶ is *n*-butyl.

### Table G6

Table G6 provides 144 compounds of Formula G1 wherein X, R¹¹, A and J are as defined in Table C1 and R⁶ is *sec*-butyl.

### Table G7

Table G7 provides 144 compounds of Formula G1 wherein X, R¹¹, A and J are as defined in Table C1 and R⁶ is *iso*-butyl.

### Table G8

Table G8 provides 144 compounds of Formula G1 wherein X, R¹¹, A and J are as defined in Table C1 and R⁶ is *tert*-butyl.

### Table G9

Table G9 provides 144 compounds of Formula G1 wherein X, R¹¹, A and J are as defined in Table C1 and R⁶ is *n*-pentyl.

### Table G10

Table G10 provides 144 compounds of Formula G1 wherein X, R¹¹, A and J are as defined in Table C1 and R⁶ is 1-methylbutyl.

### Table G11

Table G11 provides 144 compounds of Formula G1 wherein X, R¹¹, A and J are as defined in Table C1 and R⁶ is 2-methylbutyl.

### Table G12

Table G12 provides 144 compounds of Formula G1 wherein X, R¹¹, A and J are as defined in Table C1 and R⁶ is 3-methylbutyl.

### Table G13

Table G13 provides 144 compounds of Formula G1 wherein X, R¹¹, A and J are as defined in Table C1 and R⁶ is neopentyl.

### Table G14

Table G14 provides 144 compounds of Formula G1 wherein X, R¹¹, A and J are as defined in Table C1 and R⁶ is 2,2-dimethylbutyl.

### Table G15

Table G15 provides 144 compounds of Formula G1 wherein X, R¹¹, A and J are as defined in Table C1 and R⁶ is 2,2,2-trifluoroethyl.

### Table G16

Table G16 provides 144 compounds of Formula G1 wherein X, R¹¹, A and J are as defined in Table C1 and R⁶ is 2,2-difluoro-2-methoxyethyl.

### Table G17

Table G17 provides 144 compounds of Formula G1 wherein X, R¹¹, A and J are as defined in Table C1 and R⁶ is 3,3,3-trifluoropropyl.

### Table G18

Table G18 provides 144 compounds of Formula G1 wherein X, R¹¹, A and J are as defined in Table C1 and R⁶ is *para*-fluorobenzyl.

### Table G19

Table G19 provides 144 compounds of Formula G1 wherein X, R¹¹, A and J are as defined in Table C1 and R⁶ is 2,4-dichlorophenyl.

### Table G20

Table G20 provides 144 compounds of Formula G1 wherein X, R¹¹, A and J are as defined in Table C1 and R⁶ is 2-chloro-4-trifluoromethylphenyl.

### Table H1

Table H1 provides 144 compounds of Formula H1 wherein X, R¹¹, A and J are as defined in Table C1 and R⁶ is methyl.

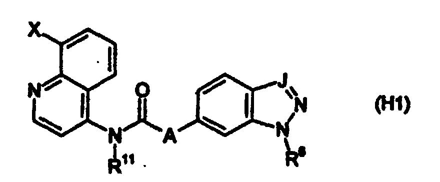

### Table H2

Table H2 provides 144 compounds of Formula H1 wherein X, R¹¹, A and J are as defined in Table C1 and R⁶ is ethyl.

### Table H3

Table H3 provides 144 compounds of Formula H1 wherein X, R¹¹, A and J are as defined in Table C1 and R⁶ is *n*-propyl.

### Table H4

Table H4 provides 144 compounds of Formula H1 wherein X, R¹¹, A and J are as defined in Table C1 and R⁶ is *iso*-propyl.

### Table H5

Table H5 provides 144 compounds of Formula H1 wherein X, R¹¹, A and J are as defined in Table C1 and R⁶ is *n*-butyl.

### Table H6

Table H6 provides 144 compounds of Formula H1 wherein X, R¹¹, A and J are as defined in Table C1 and R⁶ is *sec*-butyl.

### Table H7

Table H7 provides 144 compounds of Formula H1 wherein X, R¹¹, A and J are as defined in Table C1 and R⁶ is *iso*-butyl.

### Table H8

Table H8 provides 144 compounds of Formula H1 wherein X, R¹¹, A and J are as defined in Table C1 and R⁶ is *tert*-butyl.

### Table H9

Table H9 provides 144 compounds of Formula H1 wherein X, R¹¹, A and J are as defined in Table C1 and R⁶ is *n*-pentyl.

### Table H10

Table H10 provides 144 compounds of Formula H1 wherein X, R¹¹, A and J are as defined in Table C1 and R⁶ is 1-methylbutyl.

### Table H11

Table H11 provides 144 compounds of Formula H1 wherein X, R¹¹, A and J are as defined in Table C1 and R⁶ is 2-methylbutyl.

### Table H12

Table H12 provides 144 compounds of Formula H1 wherein X, R¹¹, A and J are as defined in Table C1 and R⁶ is 3-methylbutyl.

### Table H13

Table H13 provides 144 compounds of Formula H1 wherein X, R¹¹, A and J are as defined in Table C1 and R⁶ is neopentyl.

### Table H14

Table H14 provides 144 compounds of Formula H1 wherein X, R¹¹, A and J are as defined in Table C1 and R⁶ is 2,2-dimethylbutyl.

### Table H15

Table H15 provides 144 compounds of Formula H1 wherein X, R¹¹, A and J are as defined in Table C1 and R⁶ is 2,2,2-trifluoroethyl.

### Table H16

Table H16 provides 144 compounds of Formula H1 wherein X, R¹¹, A and J are as defined in Table C1 and R⁶ is 2,2-difluoro-2-methoxyethyl.

### Table H17

Table H17 provides 144 compounds of Formula H1 wherein X, R¹¹, A and J are as defined in Table C1 and R⁶ is 3,3,3-trifluoropropyl.

### Table H18

Table H18 provides 144 compounds of Formula H1 wherein X, R¹¹, A and J are as defined in Table C1 and R⁶ is *para*-fluorobenzyl.

### Table H19

Table H19 provides 144 compounds of Formula H1 wherein X, R¹¹, A and J are as defined in Table C1 and R⁶ is 2,4-dichlorophcnyl.

### Table H20

Table H20 provides 144 compounds of Formula H1 wherein X, R¹¹, A and J are as defined in Table C1 and R⁶ is 2-chloro-4-trifluoromethylphenyl.

### Table I1

Table I1 provides 288 compounds of Formula I1, wherein R¹, D, E, R², A and J are as defined in Table I1 and R⁶ is methyl.

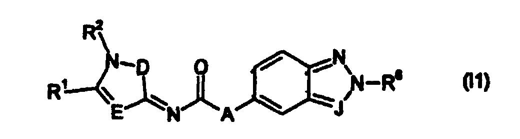

**Table I1**

| **Compound No.** | **R**^{**1**} | **D** | **E** | **R**^{**2**} | **A** | **J** |
|---|---|---|---|---|---|---|
| I1.1 | CH₃ | S | C-Cl | H | CH₂ | N |
| I1.2 | CH₃ | S | C-Cl | CH₂CH₃ | CH₂ | N |
| I1.3 | CH₃ | S | C-Cl | CH₂C=CH₂ | CH₂ | N |
| I1.4 | CH₃ | S | C-Cl | CH₂C.CH | CH₂ | N |
| I1.5 | CH₃ | S | C-Cl | CH₂OCH₃ | CH₂ | N |
| I1.6 | CH₃ | S | C-Cl | CH₂OCH₂CH₃ | CH₂ | N |
| I1.7 | CH₃ | S | C-Br | H | CH₂ | N |
| I1.8 | CH₃ | S | C-Br | CH₂CH₃ | CH₂ | N |
| I1.9 | CH₃ | S | C-Br | CH₂C=CH₂ | CH₂ | N |
| I1.10 | CH₃ | S | C-Br | CH₂C.CH | CH₂ | N |
| I1.11 | CH₃ | S | C-Br | CH₂OCH₃ | CH₂ | N |
| I1.12 | CH₃ | S | C-Br | CH₂OCH₂CH₃ | CH₂ | N |
| I1.13 | CH₂CH₃ | S | C-Cl | H | CH₂ | N |
| I1.14 | CH₂CH₃ | S | C-Cl | CH₂CH₃ | CH₂ | N |
| I1.15 | CH₂CH₃ | S | C-Cl | CH₂C=CH₂ | CH₂ | N |
| I1.16 | CH₂CH₃ | S | C-Cl | CH₂C.CH | CH₂ | N |
| I1.17 | CH₂CH₃ | S | C-Cl | CH₂OCH₃ | CH₂ | N |
| I1.18 | CH₂CH₃ | S | C-Cl | CH₂OCH₂CH₃ | CH₂ | N |
| I1.19 | CH₂CH₃ | S | C-Br | H | CH₂ | N |
| I1.20 | CH₂CH₃ | S | C-Br | CH₂CH₃ | CH₂ | N |
| I1.21 | CH₂CH₃ | S | C-Br | CH₂C=CH₂ | CH₂ | N |
| I1.22 | CH₂CH₃ | S | C-Br | CH₂C.CH | CH₂ | N |
| I1.23 | CH₂CH₃ | S | C-Br | CH₂OCH₃ | CH₂ | N |
| I1.24 | CH₂CH₃ | S | C-Br | CH₂OCH₂CH₃ | CH₂ | N |
| I1.25 | CH₃ | S | C-Cl | H | CH(CH₃) | N |
| I1.26 | CH₃ | S | C-Cl | CH₂CH₃ | CH(CH₃) | N |
| I1.27 | CH₃ | S | C-Cl | CH₂C=CH₂ | CH(CH₃) | N |
| I1.28 | CH₃ | S | C-Cl | CH₂C.CH | CH(CH₃) | N |
| I1.29 | CH₃ | S | C-Cl | CH₂OCH₃ | CH(CH₃) | N |
| I1.30 | CH₃ | S | C-Cl | CH₂OCH₂CH₃ | CH(CH₃) | N |
| I1.31 | CH₃ | S | C-Br | H | CH(CH₃) | N |
| I1.32 | CH₃ | S | C-Br | CH₂CH₃ | CH(CH₃) | N |
| I1.33 | CH₃ | S | C-Br | CH₂C=CH₂ | CH(CH₃) | N |
| I1.34 | CH₃ | S | C-Br | CH₂C.CH | CH(CH₃) | N |
| I1.35 | CH₃ | S | C-Br | CH₂OCH₃ | CH(CH₃) | N |
| I1.36 | CH₃ | S | C-Br | CH₂OCH₂CH₃ | CH(CH₃) | N |
| I1.37 | CH₂CH₃ | S | C-Cl | H | CH(CH₃) | N |
| I1.38 | CH₂CH₃ | S | C-Cl | CH₂CH₃ | CH(CH₃) | N |
| I1.39 | CH₂CH₃ | S | C-Cl | CH₂C=CH₂ | CH(CH₃) | N |
| I1.40 | CH₂CH₃ | S | C-Cl | CH₂C.CH | CH(CH₃) | N |
| I1.41 | CH₂CH₃ | S | C-Cl | CH₂OCH₃ | CH(CH₃) | N |
| I1.42 | CH₂CH₃ | S | C-Cl | CH₂OCH₂CH₃ | CH(CH₃) | N |
| I1.43 | CH₂CH₃ | S | C-Br | H | CH(CH₃) | N |
| I1.44 | CH₂CH₃ | S | C-Br | CH₂CH₃ | CH(CH₃) | N |
| I1.45 | CH₂CH₃ | S | C-Br | CH₂C=CH₂ | CH(CH₃) | N |
| I1.46 | CH₂CH₃ | S | C-Br | CH₂C.CH | CH(CH₃) | N |
| I1.47 | CH₂CH₃ | S | C-Br | CH₂OCH₃ | CH(CH₃) | N |
| I1.48 | CH₂CH₃ | S | C-Br | CH₂OCH₂CH₃ | CH(CH₃) | N |
| I1.49 | CH₃ | S | C-Cl | H | CHF | N |
| I1.50 | CH₃ | S | C-Cl | CH₂CH₃ | CHF | N |
| I1.51 | CH₃ | S | C-Cl | CH₂C=CH₂ | CHF | N |
| I1.52 | CH₃ | S | C-Cl | CH₂C.CH | CHF | N |
| I1.53 | CH₃ | S | C-Cl | CH₂OCH₃ | CHF | N |
| I1.54 | CH₃ | S | C-Cl | CH₂OCH₂CH₃ | CHF | N |
| I1.55 | CH₃ | S | C-Br | H | CHF | N |
| I1.56 | CH₃ | S | C-Br | CH₂CH₃ | CHF | N |
| I1.57 | CH₃ | S | C-Br | CH₂C=CH₂ | CHF | N |
| I1.58 | CH₃ | S | C-Br | CH₂C.CH | CHF | N |
| I1.59 | CH₃ | S | C-Br | CH₂OCH₃ | CHF | N |
| I1.60 | CH₃ | S | C-Br | CH₂OCH₂CH₃ | CHF | N |
| I1.61 | CH₂CH₃ | S | C-Cl | H | CHF | N |
| I1.62 | CH₂CH₃ | S | C-Cl | CH₂CH₃ | CHF | N |
| I1.63 | CH₂CH₃ | S | C-Cl | CH₂C=CH₂ | CHF | N |
| I1.64 | CH₂CH₃ | S | C-Cl | CH₂C.CH | CHF | N |
| I1.65 | CH₂CH₃ | S | C-Cl | CH₂OCH₃ | CHF | N |
| I1.66 | CH₂CH₃ | S | C-Cl | CH₂OCH₂CH₃ | CHF | N |
| I1.67 | CH₂CH₃ | S | C-Br | H | CHF | N |
| I1.68 | CH₂CH₃ | S | C-Br | CH₂CH₃ | CHF | N |
| I1.69 | CH₂CH₃ | S | C-Br | CH₂C=CH₂ | CHF | N |
| I1.70 | CH₂CH₃ | S | C-Br | CH₂C.CH | CHF | N |
| I1.71 | CH₂CH₃ | S | C-Br | CH₂OCH₃ | CHF | N |
| I1.72 | CH₂CH₃ | S | C-Br | CH₂OCH₂CH₃ | CHF | N |
| I1.73 | CH₃ | S | C-Cl | H | CH₂O | N |
| I1.74 | CH₃ | S | C-Cl | CH₂CH₃ | CH₂O | N |
| I1.75 | CH₃ | S | C-Cl | CH₂C=CH₂ | CH₂O | N |
| I1.76 | CH₃ | S | C-Cl | CH₂C.CH | CH₂O | N |
| I1.77 | CH₃ | S | C-Cl | CH₂OCH₃ | CH₂O | N |
| I1.78 | CH₃ | S | C-Cl | CH₂OCH₂CH₃ | CH₂O | N |
| I1.79 | CH₃ | S | C-Br | H | CH₂O | N |
| I1.80 | CH₃ | S | C-Br | CH₂CH₃ | CH₂O | N |
| I1.81 | CH₃ | S | C-Br | CH₂C=CH₂ | CH₂O | N |
| I1.82 | CH₃ | S | C-Br | CH₂C.CH | CH₂O | N |
| I1.83 | CH₃ | S | C-Br | CH₂OCH₃ | CH₂O | N |
| I1.84 | CH₃ | S | C-Br | CH₂OCH₂CH₃ | CH₂O | N |
| I1.85 | CH₂CH₃ | S | C-Cl | H | CH₂O | N |
| I1.86 | CH₂CH₃ | S | C-Cl | CH₂CH₃ | CH₂O | N |
| I1.87 | CH₂CH₃ | S | C-Cl | CH₂C=CH₂ | CH₂O | N |
| I1.88 | CH₂CH₃ | S | C-Cl | CH₂C.CH | CH₂O | N |
| I1.89 | CH₂CH₃ | S | C-Cl | CH₂OCH₃ | CH₂O | N |
| I1.90 | CH₂CH₃ | S | C-Cl | CH₂OCH₂CH₃ | CH₂O | N |
| I1.91 | CH₂CH₃ | S | C-Br | H | CH₂O | N |
| I1.92 | CH₂CH₃ | S | C-Br | CH₂CH₃ | CH₂O | N |
| I1.93 | CH₂CH₃ | S | C-Br | CH₂C=CH₂ | CH₂O | N |
| I1.94 | CH₂CH₃ | S | C-Br | CH₂C.CH | CH₂O | N |
| I1.95 | CH₂CH₃ | S | C-Br | CH₂OCH₃ | CH₂O | N |
| I1.96 | CH₂CH₃ | S | C-Br | CH₂OCH₂CH₃ | CH₂O | N |
| I1.97 | CH₃ | S | C-Cl | H | CH₂ | C-H |
| I1.98 | CH₃ | S | C-Cl | CH₂CH₃ | CH₂ | C-H |
| I1.99 | CH₃ | S | C-Cl | CH₂C=CH₂ | CH₂ | C-H |
| I1.100 | CH₃ | S | C-Cl | CH₂C.CH | CH₂ | C-H |
| I1.101 | CH₃ | S | C-Cl | CH₂OCH₃ | CH₂ | C-H |
| I1.102 | CH₃ | S | C-Cl | CH₂OCH₂CH₃ | CH₂ | C-H |
| I1.103 | CH₃ | S | C-Br | H | CH₂ | C-H |
| I1.104 | CH₃ | S | C-Br | CH₂CH₃ | CH₂ | C-H |
| I1.105 | CH₃ | S | C-Br | CH₂C=CH₂ | CH₂ | C-H |
| I1.106 | CH₃ | S | C-Br | CH₂C.CH | CH₂ | C-H |
| I1.107 | CH₃ | S | C-Br | CH₂OCH₃ | CH₂ | C-H |
| I1.108 | CH₃ | S | C-Br | CH₂OCH₂CH₃ | CH₂ | C-H |
| I1.109 | CH₂CH₃ | S | C-Cl | H | CH₂ | C-H |
| I1.110 | CH₂CH₃ | S | C-Cl | CH₂CH₃ | CH₂ | C-H |
| I1.111 | CH₂CH₃ | S | C-Cl | CH₂C=CH₂ | CH₂ | C-H |
| I1.112 | CH₂CH₃ | S | C-Cl | CH₂C.CH | CH₂ | C-H |
| I1.113 | CH₂CH₃ | S | C-Cl | CH₂OCH₃ | CH₂ | C-H |
| I1.114 | CH₂CH₃ | S | C-Cl | CH₂OCH₂CH₃ | CH₂ | C-H |
| I1.115 | CH₂CH₃ | S | C-Br | H | CH₂ | C-H |
| I1.116 | CH₂CH₃ | S | C-Br | CH₂CH₃ | CH₂ | C-H |
| I1.117 | CH₂CH₃ | S | C-Br | CH₂C=CH₂ | CH₂ | C-H |
| I1.118 | CH₂CH₃ | S | C-Br | CH₂C.CH | CH₂ | C-H |
| I1.119 | CH₂CH₃ | S | C-Br | CH₂OCH₃ | CH₂ | C-H |
| I1.120 | CH₂CH₃ | S | C-Br | CH₂OCH₂CH₃ | CH₂ | C-H |
| I1.121 | CH₃ | S | C-Cl | H | CH(CH₃) | C-H |
| I1.122 | CH₃ | S | C-Cl | CH₂CH₃ | CH(CH₃) | C-H |
| I1.123 | CH₃ | S | C-Cl | CH₂C=CH₂ | CH(CH₃) | C-H |
| I1.124 | CH₃ | S | C-Cl | CH₂C.CH | CH(CH₃) | C-H |
| I1.125 | CH₃ | S | C-Cl | CH₂OCH₃ | CH(CH₃) | C-H |
| I1.126 | CH₃ | S | C-Cl | CH₂OCH₂CH₃ | CH(CH₃) | C-H |
| I1.127 | CH₃ | S | C-Br | H | CH(CH₃) | C-H |
| I1.128 | CH₃ | S | C-Br | CH₂CH₃ | CH(CH₃) | C-H |
| I1.129 | CH₃ | S | C-Br | CH₂C=CH₂ | CH(CH₃) | C-H |
| I1.130 | CH₃ | S | C-Br | CH₂C.CH | CH(CH₃) | C-H |
| I1.131 | CH₃ | S | C-Br | CH₂OCH₃ | CH(CH₃) | C-H |
| I1.132 | CH₃ | S | C-Br | CH₂OCH₂CH₃ | CH(CH₃) | C-H |
| I1.133 | CH₂CH₃ | S | C-Cl | H | CH(CH₃) | C-H |
| I1.134 | CH₂CH₃ | S | C-Cl | CH₂CH₃ | CH(CH₃) | C-H |
| I1.135 | CH₂CH₃ | S | C-Cl | CH₂C=CH₂ | CH(CH₃) | C-H |
| I1.136 | CH₂CH₃ | S | C-Cl | CH₂C.CH | CH(CH₃) | C-H |
| I1.137 | CH₂CH₃ | S | C-Cl | CH₂OCH₃ | CH(CH₃) | C-H |
| I1.138 | CH₂CH₃ | S | C-Cl | CH₂OCH₂CH₃ | CH(CH₃) | C-H |
| I1.139 | CH₂CH₃ | S | C-Br | H | CH(CH₃) | C-H |
| I1.140 | CH₂CH₃ | S | C-Br | CH₂CH₃ | CH(CH₃) | C-H |
| I1.141 | CH₂CH₃ | S | C-Br | CH₂C=CH₂ | CH(CH₃) | C-H |
| I1.142 | CH₂CH₃ | S | C-Br | CH₂C.CH | CH(CH₃) | C-H |
| I1.143 | CH₂CH₃ | S | C-Br | CH₂OCH₃ | CH(CH₃) | C-H |
| I1.144 | CH₂CH₃ | S | C-Br | CH₂OCH₂CH₃ | CH(CH₃) | C-H |
| I1.145 | CH₃ | S | C-Cl | H | CHF | C-H |
| I1.146 | CH₃ | S | C-Cl | CH₂CH₃ | CHF | C-H |
| I1.147 | CH₃ | S | C-Cl | CH₂C=CH₂ | CHF | C-H |
| I1.148 | CH₃ | S | C-Cl | CH₂C.CH | CHF | C-H |
| I1.149 | CH₃ | S | C-Cl | CH₂OCH₃ | CHF | C-H |
| I1.150 | CH₃ | S | C-Cl | CH₂OCH₂CH₃ | CHF | C-H |
| I1.151 | CH₃ | S | C-Br | H | CHF | C-H |
| I1.152 | CH₃ | S | C-Br | CH₂CH₃ | CHF | C-H |
| I1.153 | CH₃ | S | C-Br | CH₂C=CH₂ | CHF | C-H |
| I1.154 | CH₃ | S | C-Br | CH₂C.CH | CHF | C-H |
| I1.155 | CH₃ | S | C-Br | CH₂OCH₃ | CHF | C-H |
| I1.156 | CH₃ | S | C-Br | CH₂OCH₂CH₃ | CHF | C-H |
| I1.157 | CH₂CH₃ | S | C-Cl | H | CHF | C-H |
| I1.158 | CH₂CH₃ | S | C-Cl | CH₂CH₃ | CHF | C-H |
| I1.159 | CH₂CH₃ | S | C-Cl | CH₂C=CH₂ | CHF | C-H |
| I1.160 | CH₂CH₃ | S | C-Cl | CH₂C.CH | CHF | C-H |
| I1.161 | CH₂CH₃ | S | C-Cl | CH₂OCH₃ | CHF | C-H |
| I1.162 | CH₂CH₃ | S | C-Cl | CH₂OCH₂CH₃ | CHF | C-H |
| I1.163 | CH₂CH₃ | S | C-Br | H | CHF | C-H |
| I1.164 | CH₂CH₃ | S | C-Br | CH₂CH₃ | CHF | C-H |
| I1.165 | CH₂CH₃ | S | C-Br | CH₂C=CH₂ | CHF | C-H |
| I1.166 | CH₂CH₃ | S | C-Br | CH₂C.CH | CHF | C-H |
| I1.167 | CH₂CH₃ | S | C-Br | CH₂OCH₃ | CHF | C-H |
| I1.168 | CH₂CH₃ | S | C-Br | CH₂OCH₂CH₃ | CHF | C-H |
| I1.169 | CH₃ | S | C-Cl | H | CH₂O | C-H |
| I1.170 | CH₃ | S | C-Cl | CH₂CH₃ | CH₂O | C-H |
| I1.171 | CH₃ | S | C-Cl | CH₂C=CH₂ | OH₂O | C-H |
| I1.172 | CH₃ | S | C-Cl | CH₂C.CH | CH₂O | C-H |
| I1.173 | CH₃ | S | C-Cl | CH₂OCH₃ | CH₂O | C-H |
| I1.174 | CH₃ | S | C-Cl | CH₂OCH₂CH₃ | CH₂O | C-H |
| I1.175 | CH₃ | S | C-Br | H | CH₂O | C-H |
| I1.176 | CH₃ | S | C-Br | CH₂CH₃ | CH₂O | C-H |
| I1.177 | CH₃ | S | C-Br | CH₂C=CH₂ | CH₂O | C-H |
| I1.178 | CH₃ | S | C-Br | CH₂C.CH | CH₂O | C-H |
| I1.179 | CH₃ | S | C-Br | CH₂OCH₃ | CH₂O | C-H |
| I1.180 | CH₃ | S | C-Br | CH₂OCH₂CH₃ | CH₂O | C-H |
| I1.181 | CH₂CH₃ | S | C-Cl | H | CH₂O | C-H |
| I1.182 | CH₂CH₃ | S | C-Cl | CH₂CH₃ | CH₂O | C-H |
| I1.183 | CH₂CH₃ | S | C-Cl | CH₂C=CH₂ | CH₂O | C-H |
| I1.184 | CH₂CH₃ | S | C-Cl | CH₂C.CH | CH₂O | C-H |
| I1.185 | CH₂CH₃ | S | C-Cl | CH₂OCH₃ | CH₂O | C-H |
| I1.186 | CH₂CH₃ | S | C-Cl | CH₂OCH₂CH₃ | CH₂O | C-H |
| I1.187 | CH₂CH₃ | S | C-Br | H | CH₂O | C-H |
| I1.188 | CH₂CH₃ | S | C-Br | CH₂CH₃ | CH₂O | C-H |
| I1.189 | CH₂CH₃ | S | C-Br | CH₂C=CH₂ | CH₂O | C-H |
| I1.190 | CH₂CH₃ | S | C-Br | CH₂C.CH | CH₂O | C-H |
| I1.191 | CH₂CH₃ | S | C-Br | CH₂OCH₃ | CH₂O | C-H |
| I1.192 | CH₂CH₃ | S | C-Br | CH₂OCH₂CH₃ | CH₂O | C-H |
| I1.193 | CH₃ | S | C-Cl | H | CH₂ | C-Cl |
| I1.194 | CH₃ | S | C-Cl | CH₂CH₃ | CH₂ | C-Cl |
| I1.195 | CH₃ | S | C-Cl | CH₂C=CH₂ | CH₂ | C-Cl |
| I1.196 | CH₃ | S | C-Cl | CH₂C.CH | CH₂ | C-Cl |
| Il.197 | CH₃ | S | C-Cl | CH₂OCH₃ | CH₂ | C-Cl |
| I1.198 | CH₃ | S | C-Cl | CH₂OCH₂CH₃ | CH₂ | C-Cl |
| I1.199 | CH₃ | S | C-Br | H | CH₂ | C-Cl |
| I1.200 | CH₃ | S | C-Br | CH₂CH₃ | CH₂ | C-Cl |
| I1.201 | CH₃ | S | C-Br | CH₂C=CH₂ | CH₂ | C-Cl |
| I1.202 | CH₃ | S | C-Br | CH₂C.CH | CH₂ | C-Cl |
| I1.203 | CH₃ | S | C-Br | CH₂OCH₃ | CH₂ | C-Cl |
| I1.204 | CH₃ | S | C-Br | CH₂OCH₂CH₃ | CH₂ | C-Cl |
| I1.205 | CH₂CH₃ | S | C-Cl | H | CH₂ | C-Cl |
| I1.206 | CH₂CH₃ | S | C-Cl | CH₂CH₃ | CH₂ | C-Cl |
| I1207 | CH₂CH₃ | S | C-Cl | CH₂C=CH₂ | CH₂ | C-Cl |
| I1.208 | CH₂CH₃ | S | C-Cl | CH₂C.CH | CH₂ | C-Cl |
| I1.209 | CH₂CH₃ | S | C-Cl | CH₂OCH₃ | CH₂ | C-Cl |
| I1.210 | CH₂CH₃ | S | C-Cl | CH₂OCH₂CH₃ | CH₂ | C-Cl |
| I1.211 | CH₂CH₃ | S | C-Br | H | CH₂ | C-Cl |
| I1.212 | CH₂CH₃ | S | C-Br | CH₂CH₃ | CH₂ | C-Cl |
| I1.213 | CH₂CH₃ | S | C-Br | CH₂C=CH₂ | CH₂ | C-Cl |
| I1.214 | CH₂CH₃ | S | C-Br | CH₂C.CH | CH₂ | C-Cl |
| I1.215 | CH₂CH₃ | S | C-Br | CH₂OCH₃ | CH₂ | C-Cl |
| I1.216 | CH₂CH₃ | S | C-Br | CH₂OCH₂CH₃ | CH₂ | C-Cl |
| I1.217 | CH₃ | S | C-Cl | H | CH(CH₃) | C-Cl |
| I1.218 | CH₃ | S | C-Cl | CH₂CH₃ | CH(CH₃) | C-Cl |
| I1.219 | CH₃ | S | C-Cl | CH₂C=CH₂ | CH(CH₃) | C-Cl |
| I1.220 | CH₃ | S | C-Cl | CH₂C.CH | CH(CH₃) | C-Cl |
| I1.221 | CH₃ | S | C-Cl | CH₂OCH₃ | CH(CH₃) | C-Cl |
| I1.222 | CH₃ | S | C-Cl | CH₂OCH₂CH₃ | CH(CH₃) | C-Cl |
| I1.223 | CH₃ | S | C-Br | H | CH(CH₃) | C-Cl |
| I1.224 | CH₃ | S | C-Br | CH₂CH₃ | CH(CH₃) | C-Cl |
| I1.225 | CH₃ | S | C-Br | CH₂C=CH₂ | CH(CH₃) | C-Cl |
| I1.226 | CH₃ | S | C-Br | CH₂C.CH | CH(CH₃) | C-Cl |
| I1.227 | CH₃ | S | C-Br | CH₂OCH₃ | CH(CH₃) | C-Cl |
| I1.228 | CH₃ | S | C-Br | CH₂OCH₂CH₃ | CH(CH₃) | C-Cl |
| I1.229 | CH₂CH₃ | S | C-Cl | H | CH(CH₃) | C-Cl |
| I1.230 | CH₂CH₃ | S | C-Cl | CH₂CH₃ | CH(CH₃) | C-Cl |
| I1.231 | CH₂CH₃ | S | C-Cl | CH₂C=CH₂ | CH(CH₃) | C-Cl |
| I1.232 | CH₂CH₃ | S | C-Cl | CH₂C.CH | CH(CH₃) | C-Cl |
| I1.233 | CH₂CH₃ | S | C-Cl | CH₂OCH₃ | CH(CH₃) | C-Cl |
| I1.234 | CH₂CH₃ | S | C-Cl | CH₂OCH₂CH₃ | CH(CH₃) | C-Cl |
| I1.235 | CH₂CH₃ | S | C-Br | H | CH(CH₃) | C-Cl |
| I1.236 | CH₂CH₃ | S | C-Br | CH₂CH₃ | CH(CH₃) | C-Cl |
| I1.237 | CH₂CH₃ | S | C-Br | CH₂C=CH₂ | CH(CH₃) | C-Cl |
| I1.238 | CH₂CH₃ | S | C-Br | CH₂C.CH | CH(CH₃) | C-Cl |
| I1.239 | CH₂CH₃ | S | C-Br | CH₂OCH₃ | CH(CH₃) | C-Cl |
| I1.240 | CH₂CH₃ | S | C-Br | CH₂OCH₂CH₃ | CH(CH₃) | C-Cl |
| I1.241 | CH₃ | S | C-Cl | H | CHF | C-Cl |
| I1.242 | CH₃ | S | C-Cl | CH₂CH₃ | CHF | C-Cl |
| I1.243 | CH₃ | S | C-Cl | CH₂C=CH₂ | CHF | C-Cl |
| I1.244 | CH₃ | S | C-Cl | CH₂C.CH | CHF | C-Cl |
| I1.245 | CH₃ | S | C-Cl | CH₂OCH₃ | CHF | C-Cl |
| I1.246 | CH₃ | S | C-Cl | CH₂OCH₂CH₃ | CHF | C-Cl |
| I1.247 | CH₃ | S | C-Br | H | CHF | C-Cl |
| I1.248 | CH₃ | S | C-Br | CH₂CH₃ | CHF | C-Cl |
| I1.249 | CH₃ | S | C-Br | CH₂C=CH₂ | CHF | C-Cl |
| I1.250 | CH₃ | S | C-Br | CH₂C.CH | CHF | C-Cl |
| I1.251 | CH₃ | S | C-Br | CH₂OCH₃ | CHF | C-Cl |
| I1.252 | CH₃ | S | C-Br | CH₂OCH₂CH₃ | CHF | C-Cl |
| I1.253 | CH₂CH₃ | S | C-Cl | H | CHF | C-Cl |
| I1.254 | CH₂CH₃ | S | C-Cl | CH₂CH₃ | CHF | C-Cl |
| I1.255 | CH₂CH₃ | S | C-Cl | CH₂C=CH₂ | CHF | C-Cl |
| I1.256 | CH₂CH₃ | S | C-Cl | CH₂C.CH | CHF | C-Cl |
| I1.257 | CH₂CH₃ | S | C-Cl | CH₂OCH₃ | CHF | C-Cl |
| I1.258 | CH₂CH₃ | S | C-Cl | CH₂OCH₂CH₃ | CHF | C-Cl |
| I1.259 | CH₂CH₃ | S | C-Br | H | CHF | C-Cl |
| I1.260 | CH₂CH₃ | S | C-Br | CH₂CH₃ | CHF | C-Cl |
| I1261 | CH₂CH₃ | S | C-Br | CH₂C=CH₂ | CHF | C-Cl |
| I1.262 | CH₂CH₃ | S | C-Br | CH₂C.CH | CHF | C-Cl |
| I1.263 | CH₂CH₃ | S | C-Br | CH₂OCH₃ | CHF | C-Cl |
| I1.264 | CH₂CH₃ | S | C-Br | CH₂OCH₂CH₃ | CHF | C-Cl |
| I1.265 | CH₃ | S | C-Cl | H | CH₂O | C-Cl |
| I1.266 | CH₃ | S | C-Cl | CH₂CH₃ | CH₂O | C-Cl |
| I1.267 | CH₃ | S | C-Cl | CH₂C=CH₂ | CH₂O | C-Cl |
| I1.268 | CH₃ | S | C-Cl | CH₂C.CH | CH₂O | C-Cl |
| I1.269 | CH₃ | S | C-Cl | CH₂OCH₃ | CH₂O | C-Cl |
| I1.270 | CH₃ | S | C-Cl | CH₂OCH₂CH₃ | CH₂O | C-Cl |
| I1.271 | CH₃ | S | C-Br | H | CH₂O | C-Cl |
| I1.272 | CH₃ | S | C-Br | CH₂CH₃ | CH₂O | C-Cl |
| I1.273 | CH₃ | S | C-Br | CH₂C=CH₂ | CH₂O | C-Cl |
| I1.274 | CH₃ | S | C-Br | CH₂C.CH | CH₂O | C-Cl |
| I1.275 | CH₃ | S | C-Br | CH₂OCH₃ | CH₂O | C-Cl |
| I1.276 | CH₃ | S | C-Br | CH₂OCH₂CH₃ | CH₂O | C-Cl |
| I1.277 | CH₂CH₃ | S | C-Cl | H | CH₂O | C-Cl |
| I1.278 | CH₂CH₃ | S | C-Cl | CH₂CH₃ | CH₂O | C-Cl |
| I1.279 | CH₂CH₃ | S | C-Cl | CH₂C=CH₂ | CH₂O | C-Cl |
| I1.280 | CH₂CH₃ | S | C-Cl | CH₂C.CH | CH₂O | C-Cl |
| I1.281 | CH₂CH₃ | S | C-Cl | CH₂OCH₃ | CH₂O | C-Cl |
| I1.282 | CH₂CH₃ | S | C-Cl | CH₂OCH₂CH₃ | CH₂O | C-Cl |
| I1.283 | CH₂CH₃ | S | C-Br | H | CH₂O | C-Cl |
| I1.284 | CH₂CH₃ | S | C-Br | CH₂CH₃ | CH₂O | C-Cl |
| I1.285 | CH₂CH₃ | S | C-Br | CH₂C=CH₂ | CH₂O | C-Cl |
| I1.286 | CH₂CH₃ | S | C-Br | CH₂C.CH | CH₂O | C-Cl |
| I1.287 | CH₂CH₃ | S | C-Br | CH₂OCH₃ | CH₂O | C-Cl |
| I1.288 | CH₂CH₃ | S | C-Br | CH₂OCH₂CH₃ | CH₂O | C-Cl |

### Table I2

Table I2 provides 288 compounds of Formula I1 wherein X, R², A and J are as defined in Table I1 and R⁶ is ethyl.

### Table I3

Table I3 provides 288 compounds of Formula I1 wherein X, R², A and J are as defined in Table I1 and R⁶ is *n*-propyl.

### Table I4

Table I4 provides 288 compounds of Formula I1 wherein X, R², A and J are as defined in Table I1 and R⁶ is *iso*-propyl.

### Table I5

Table I5 provides 288 compounds of Formula I1 wherein X, R², A and J are as defined in Table I1 and R⁶ is *n*-butyl.

### Table I6

Table I6 provides 288 compounds of Formula I1 wherein X, R², A and J are as defined in Table I1 and R⁶ is *sec*-butyl.

### Table I7

Table I7 provides 288 compounds of Formula I1 wherein X, R², A and J are as defined in Table I1 and R⁶ is *iso*-butyl.

### Table I8

Table I8 provides 288 compounds of Formula I1 wherein X, R², A and J are as defined in Table I1 and R⁶ is *tert*-butyl.

### Table 19

Table I9 provides 288 compounds of Formula I1 wherein X, R², A and J are as defined in Table I1 and R⁶ is *n*-pentyl.

### Table I10

Table I10 provides 288 compounds of Formula I1 wherein X, R², A and J are as defined in Table I1 and R⁶ is 1-methylbutyl.

### Table I11

Table I11 provides 288 compounds of Formula I1 wherein X, R², A and J are as defined in Table I1 and R⁶ is 2-methylbutyl.

### Table I12

Table I12 provides 288 compounds of Formula I1 wherein X, R², A and J are as defined in Table II and R⁶ is 3-methylbutyl.

### Table I13

Table I13 provides 288 compounds of Formula I1 wherein X, R², A and J are as defined in Table I1 and R⁶ is neopentyl.

### Table I14

Table I14 provides 288 compounds of Formula I1 wherein X, R², A and J are as defined in Table I1 and R⁶ is 2,2-dimethylbutyl.

### Table I15

Table I15 provides 288 compounds of Formula I1 wherein X, R², A and J are as defined in Table I1 and R⁶ is 2,2,2-trifluoroethyl.

### Table I16

Table I16 provides 288 compounds of Formula I1 wherein X, R², A and J are as defined in Table I1 and R⁶ is 2,2-difluoro-2-methoxyethyl.

### Table I17

Table I17 provides 288 compounds of Formula I1 wherein X, R², A and J are as defined in Table I1 and R⁶ is 3,3,3-trifluoropropyl.

### Table I18

Table I18 provides 288 compounds of Formula I1 wherein X, R², A and J are as defined in Table I1 and R⁶ is *para*-fluorobenzyl.

### Table I19

Table I19 provides 288 compounds of Formula I1 wherein X, R², A and J are as defined in Table I1 and R⁶ is 2,4-dichlorophenyl.

### Table I20

Table I20 provides 288 compounds of Formula I1 wherein X, R², A and J are as defined in Table I1 and R⁶ is 2-chloro-4-trifluoromethylphenyl.

### Table J1

Table J1 provides 288 compounds of Formula J1, wherein R¹, D, E, R², A and J are as defined in Table I1 and R⁶ is methyl.

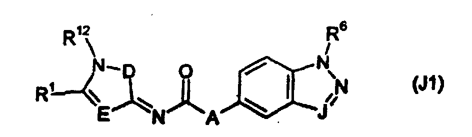

### Table J2

Table J2 provides 288 compounds of Formula J1 wherein R¹, D, E, R², A and J are as defined in Table I1 and R⁶ is ethyl.

### Table J3

Table J3 provides 288 compounds of Formula J1 wherein R¹, D, E, R², A and J are as defined in Table I1 and R⁶ is *n*-propyl.

### Table J4

Table J4 provides 288 compounds of Formula J1 wherein R¹, D, E, R², A and J are as defined in Table I1 and R⁶ is *iso*-psopyl.

### Table J5

Table J5 provides 288 compounds of Formula J1 wherein R¹, D, E, R², A and J are as defined in Table I1 and R⁶ is *n*-butyl.

### Table J6

Table J6 provides 288 compounds of Formula J1 wherein R¹, D, E, R², A and J are as defined in Table J1 and R⁶ is *sec*-butyl.

### Table J7

Table J7 provides 288 compounds of Formula J1 wherein R¹, D, E, R², A and J are as defined in Table I1 and R⁶ is *iso*-butyl.

### Table J8

Table J8 provides 288 compounds of Formula J1 wherein R¹, D, E, R², A and J are as defined in Table I1 and R⁶ is *tert*-butyl.

### Table J9

Table J9 provides 288 compounds of Formula J1 wherein R¹, D, E, R², A and J are as defined in Table I1 and R⁶ is *n*-pentyl.

### Table J10

Table J10 provides 288 compounds of Formula J1 wherein R¹, D, E, R², A and J are as defined in Table I1 and R⁶ is 1-methylbutyl.

### Table J11

Table J11 provides 288 compounds of Formula J1 wherein R¹, D, E, R², A and J are as defined in Table I1 and R⁶ is 2-methylbutyl.

### Table J12

Table J12 provides 288 compounds of Formula J1 wherein R¹, D, E, R², A and J are as defined in Table I1 and R⁶ is 3-methylbutyl.

### Table J13

Table J13 provides 288 compounds of Formula J1 wherein R¹, D, E, R², A and J are as defined in Table I1 and R⁶ is neopentyl.

### Table J14

Table J14 provides 288 compounds of Formula J1 wherein R¹, D, E, R², A and J are as defined in Table I1 and R⁶ is 2,2-dimethylbutyl.

### Table J15

Table J15 provides 288 compounds of Formula J1 wherein R¹, D, E, R², A and J are as defined in Table I1 and R⁶ is 2,2,2-trifluoroethyl.

### Table J16

Table J16 provides 288 compounds of Formula J1 wherein R¹, D, E, R², A and J are as defined in Table I1 and R⁶ is 2,2-difluoro-2-methoxyethyl.

### Table J17

Table J17 provides 288 compounds of Formula J1 wherein R¹, D, E, R², A and J are as defined in Table I1 and R⁶ is 3,3,3-trifluoropropyl.

### Table J18

Table J18 provides 288 compounds of Formula J1 wherein R¹, D, E, R², A and J are as defined in Table II and R⁶ is *para*-fluorobenzyl.

### Table J19

Table J19 provides 288 compounds of Formula J1 wherein R¹, D, E, R², A and J are as defined in Table I1 and R⁶ is 2,4-dichlorophenyl.

### Table J20

Table J20 provides 288 compounds of Formula J1 wherein R¹, D, E, R², A and J are as defined in Table I1 and R⁶ is 2-chloro-4-trifluoromethylphenyl.

### Table K1

Table K1 provides 288 compounds of Formula K1, wherein R¹, D, E, R², A and J are as defined in Table I1 and R⁶ is methyl.

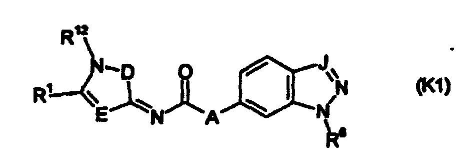

### Table K2

Table K2 provides 288 compounds of Formula K1 wherein R¹, D, E, R², A and J are as defined in Table I1 and R⁶ is ethyl.

### Table K3

Table K3 provides 288 compounds of Formula K1 wherein R¹, D, E, R², A and J are as defined in Table I1 and R⁶ is *n*-propyl.

### Table K4

Table K4 provides 288 compounds of Formula K1 wherein R¹, D, E, R², A and J are as defined in Table I1 and R⁶ is *iso*-propyl.

### Table K5

Table K5 provides 288 compounds of Formula K1 wherein R¹, D, E, R², A and J are as defined in Table I1 and R⁶ is *n*-butyl.

### Table K6

Table K6 provides 288 compounds of Formula K1 wherein R¹, D, E, R², A and J are as defined in Table I1 and R⁶ is *sec*-butyl.

### Table K7

Table K7 provides 288 compounds of Formula K1 wherein R¹, D, E, R², A and J are as defined in Table I1 and R⁶ is *iso*-butyl.

### Table K8

Table K8 provides 288 compounds of Formula K1 wherein R¹, D, E, R², A and J are as defined in Table I1 and R⁶ is *tert*-butyl.

### Table K9

Table K9 provides 288 compounds of Formula K1 wherein R¹, D, E, R², A and J are as defined in Table I1 and R⁶ is *n*-pentyl.

### Table K10

Table K10 provides 288 compounds of Formula K1 wherein R¹, D, E, R², A and J are as defined in Table I1 and R⁶ is 1-methylbutyl.

### Table K11

Table K11 provides 288 compounds of Formula K1 wherein R¹, D, E, R², A and J are as defined in Table I1 and R⁶ is 2-methylbutyl.

### Table K12

Table K12 provides 288 compounds of Formula K1 wherein R¹, D, E, R², A and J are as defined in Table I1 and R⁶ is 3-methylbutyl.

### Table K13

Table K13 provides 288 compounds of Formula K1 wherein R¹, D, E, R², A and J are as defined in Table I1 and R⁶ is neopentyl.

### Table K14

Table K14 provides 288 compounds of Formula K1 wherein R¹, D, E, R², A and J are as defined in Table I1 and R⁶ is 2,2-dimethylbutyl.

### Table K15

Table K15 provides 288 compounds of Formula K1 wherein R¹, D, E, R², A and J are as defined in Table I1 and R⁶ is 2,2,2-trifluoroethyl.

### Table K16

Table K16 provides 288 compounds of Formula K1 wherein R¹, D, E, R², A and J are as defined in Table I1 and R⁶ is 2,2-difluoro-2-methoxyethyl.

### Table K17

Table K17 provides 288 compounds of Formula K1 wherein R¹, D, E, R², A and J are as defined in Table I1 and R⁶ is 3,3,3-trifluoropropyl.

### Table K18

Table K18 provides 288 compounds of Formula K1 wherein R¹, D, E, R², A and J are as defined in Table I1 and R⁶ is *para*-fluorobenzyl.

### Table K19

Table K19 provides 288 compounds of Formula K1 wherein R¹, D, E, R², A and J are as defined in Table I1 and R⁶ is 2,4-dichlorophenyl.

### Table K20

Table K20 provides 288 compounds of Formula K1 wherein R¹, D, E, R², A and J are as defined in Table Il and R⁶ is 2-chloro-4-trifluoromethylphenyl.

### Table L1

Table L1 provides 192 compounds of Formula L1, wherein R¹, D, E, R², A and J are as defined in Table L1 and R⁶ is methyl.

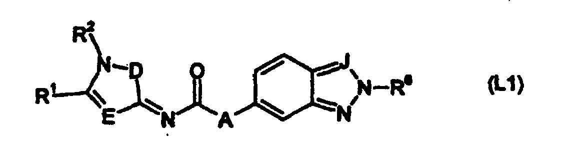

**Table L1**

| **Compound No.** | **R**^{**1**} | **D** | **E** | **R**^{**2**} | **A** | **J** |
|---|---|---|---|---|---|---|
| L1.1 | CH₃ | S | C-Cl | H | CH₂ | C-H |
| L1.2 | CH₃ | S | C-Cl | CH₂CH₃ | CH₂ | C-H |
| L1.3 | CH₃ | S | C-Cl | CH₂C=CH₂ | CH₂ | C-H |
| L1.4 | CH₃ | S | C-Cl | CH₂C.CH | CH₂ | C-H |
| L1.5 | CH₃ | S | C-Cl | CH₂OCH₃ | CH₂ | C-H |
| L1.6 | CH₃ | S | C-Cl | CH₂OCH₂CH₃ | CH₂ | C-H |
| L1.7 | CH3 | S | C-Br | H | CH₂ | C-H |
| L1.8 | CH₃ | S | C-Br | CH₂CH₃ | CH₂ | C-H |
| L1.9 | CH₃ | S | C-Br | CH₂C=CH₂ | CH₂ | C-H |
| L1.10 | CH₃ | S | C-Br | CH₂C.CH | CH₂ | C-H |
| L1.11 | CH₃ | S | C-Br | CH₂OCH₃ | CH₂ | C-H |
| L1.12 | CH₃ | S | C-Br | CH₂OCH₂CH₃ | CH₂ | C-H |
| L1.13 | CH₂CH₃ | S | C-Cl | H | CH₂ | C-H |
| L1.14 | CH₂CH₃ | S | C-Cl | CH₂CH₃ | CH₂ | C-H |
| L1.15 | CH₂CH₃ | S | C-Cl | CH₂C=CH₂ | CH₂ | C-H |
| L1.16 | CH₂CH₃ | S | C-Cl | CH₂C.CH | CH₂ | C-H |
| L1.17 | CH₂CH₃ | S | C-Cl | CH₂OCH₃ | CH₂ | C-H |
| L1.18 | CH₂CH₃ | S | C-Cl | CH₂OCH₂CH₃ | CH₂ | C-H |
| L1.19 | CH₂CH₃ | S | C-Br | H | CH₂ | C-H |
| L1.20 | CH₂CH₃ | S | C-Br | CH₂CH₃ | CH₂ | C-H |
| L1.21 | CH₂CH₃ | S | C-Br | CH₂C=CH₂ | CH₂ | C-H |
| L1.22 | CH₂CH₃ | S | C-Br | CH₂C.CH | CH₂ | C-H |
| L1.23 | CH₂CH₃ | S | C-Br | CH₂OCH₃ | CH₂ | C-H |
| L1.24 | CH₂CH₃ | S | C-Br | CH₂OCH₂CH₃ | CH₂ | C-H |
| L1.25 | CH₃ | S | C-Cl | H | CH(CH₃) | C-H |
| L1.26 | CH₃ | S | C-Cl | CH₂CH₃ | CH(CH₃) | C-H |
| L1.27 | CH₃ | S | C-Cl | CH₂C=CH₂ | CH(CH₃) | C-H |
| L1.28 | CH₃ | S | C-Cl | CH₂C.CH | CH(CH₃) | C-H |
| L1.29 | CH₃ | S | C-Cl | CH₂OCH₃ | CH(CH₃) | C-H |
| L1.30 | CH₃ | S | C-Cl | CH₂OCH₂CH₃ | CH(CH₃) | C-H |
| L1.31 | CH₃ | S | C-Br | H | CH(CH₃) | C-H |
| L1.32 | CH₃ | S | C-Br | CH₂CH₃ | CH(CH₃) | C-H |
| L1.33 | CH₃ | S | C-Br | CH₂C=CH₂ | CH(CH₃) | C-H |
| L1.34 | CH₃ | S | C-Br | CH₂C.CH | CH(CH₃) | C-H |
| L1.35 | CH₃ | S | C-Br | CH₂OCH₃ | CH(CH₃) | C-H |
| L1.36 | CH₃ | S | C-Br | CH₂OCH₂CH₃ | CH(CH₃) | C-H |
| L1.37 | CH₂CH₃ | S | C-Cl | H | CH(CH₃) | C-H |
| L1.38 | CH₂CH₃ | S | C-Cl | CH₂CH₃ | CH(CH₃) | C-H |
| L1.39 | CH₂CH₃ | S | C-Cl | CH₂C=CH₂ | CH(CH₃) | C-H |
| L1.40 | CH₂CH₃ | S | C-Cl | CH₂C.CH | CH(CH₃) | C-H |
| L1.41 | CH₂CH₃ | S | C-Cl | CH₂OCH₃ | CH(CH₃) | C-H |
| L1.42 | CH₂CH₃ | S | C-Cl | CH₂OCH₂CH₃ | CH(CH₃) | C-H |
| L1.43 | CH₂CH₃ | S | C-Br | H | CH(CH₃) | C-H |
| L1.44 | CH₂CH₃ | S | C-Br | CH₂CH₃ | CH(CH₃) | C-H |
| L1.45 | CH₂CH₃ | S | C-Br | CH₂C=CH₂ | CH(CH₃) | C-H |
| L1.46 | CH₂CH₃ | S | C-Br | CH₂C.CH | CH(CH₃) | C-H |
| L1.47 | CH₂CH₃ | S | C-Br | CH₂OCH₃ | CH(CH₃) | C-H |
| L1.48 | CH₂CH₃ | S | C-Br | CH₂OCH₂CH₃ | CH(CH₃) | C-H |
| L1.49 | CH₃ | S | C-Cl | H | CHF | C-H |
| L1.50 | CH₃ | S | C-Cl | CH₂CH₃ | CHF | C-H |
| L1.51 | CH₃ | S | C-Cl | CH₂C=CH₂ | CHF | C-H |
| L1.52 | CH₃ | S | C-Cl | CH₂C.CH | CHF | C-H |
| L1.53 | CH₃ | S | C-Cl | CH₂OCH₃ | CHF | C-H |
| L1.54 | CH₃ | S | C-Cl | CH₂OCH₂CH₃ | CHF | C-H |
| L1.55 | CH₃ | S | C-Br | H | CHF | C-H |
| L1.56 | CH₃ | S | C-Br | CH₂CH₃ | CHF | C-H |
| L1.57 | CH₃ | S | C-Br | CH₂C=CH₂ | CHF | C-H |
| L1.58 | CH₃ | S | C-Br | CH₂C.CH | CHF | C-H |
| L1.59 | CH₃ | S | C-Br | CH₂OCH₃ | CHF | C-H |
| L1.60 | CH₃ | S | C-Br | CH₂OCH₂CH₃ | CHF | C-H |
| L1.61 | CH₂CH₃ | S | C-Cl | H | CHF | C-H |
| L1.62 | CH₂CH₃ | S | C-Cl | CH₂CH₃ | CHF | C-H |
| L1.63 | CH₂CH₃ | S | C-Cl | CH₂C=CH₂ | CHF | C-H |
| L1.64 | CH₂CH₃ | S | C-Cl | CH₂C.CH | CHF | C-H |
| L1.65 | CH₂CH₃ | S | C-Cl | CH₂OCH₃ | CHF | C-H |
| L1.66 | CH₂CH₃ | S | C-Cl | CH₂OCH₂CH₃ | CHF | C-H |
| L1.67 | CH₂CH₃ | S | C-Br | H | CHF | C-H |
| L1.68 | CH₂CH₃ | S | C-Br | CH₂CH₃ | CHF | C-H |
| L1.69 | CH₂CH₃ | S | C-Br | CH₂C=CH₂ | CHF | C-H |
| L1.70 | CH₂CH₃ | S | C-Br | CH₂C.CH | CHF | C-H |
| L1.71 | CH₂CH₃ | S | C-Br | CH₂OCH₃ | CHF | C-H |
| L1.72 | CH₂CH₃ | S | C-Br | CH₂OCH₂CH₃ | CHF | C-H |
| L1.73 | CH₃ | S | C-Cl | H | CH₂O | C-H |
| L1.74 | CH₃ | S | C-Cl | CH₂CH₃ | CH₂O | C-H |
| L1.75 | CH₃ | S | C-Cl | CH₂C=CH₂ | CH₂O | C-H |
| L1.76 | CH₃ | S | C-Cl | CH₂C.CH | CH₂O | C-H |
| L1.77 | CH₃ | S | C-Cl | CH₂OCH₃ | CH₂O | C-H |
| L1.78 | CH₃ | S | C-Cl | CH₂OCH₂CH₃ | CH₂O | C-H |
| L1.79 | CH₃ | S | C-Br | H | CH₂O | C-H |
| L1.80 | CH₃ | S | C-Br | CH₂CH₃ | CH₂O | C-H |
| L1.81 | CH₃ | S | C-Br | CH₂C=CH₂ | CH₂O | C-H |
| L1.82 | CH₃ | S | C-Br | CH₂C.CH | CH₂O | C-H |
| L1.83 | CH₃ | S | C-Br | CH₂OCH₃ | CH₂O | C-H |
| L1.84 | CH₃ | S | C-Br | CH₂OCH₂CH₃ | CH₂O | C-H |
| L1.85 | CH₂CH₃ | S | C-Cl | H | CH₂O | C-H |
| L1.86 | CH₂CH₃ | S | C-Cl | CH₂CH₃ | CH₂O | C-H |
| L1.87 | CH₂CH₃ | S | C-Cl | CH₂C=CH₂ | CH₂O | C-H |
| L1.88 | CH₂CH₃ | S | C-Cl | CH₂C.CH | CH₂O | C-H |
| L1.89 | CH₂CH₃ | S | C-Cl | CH₂OCH₃ | CH₂O | C-H |
| L1.90 | CH₂CH₃ | S | C-Cl | CH₂OCH₂CH₃ | CH₂O | C-H |
| L1.91 | CH₂CH₃ | S | C-Br | H | CH₂O | C-H |
| L1.92 | CH₂CH₃ | S | C-Br | CH₂CH₃ | CH₂O | C-H |
| L1.93 | CH₂CH₃ | S | C-Br | CH₂C=CH₂ | CH₂O | C-H |
| L1.94 | CH₂CH₃ | S | C-Br | CH₂C.CH | CH₂O | C-H |
| L1.95 | CH₂CH₃ | S | C-Br | CH₂OCH₃ | CH₂O | C-H |
| L1.96 | CH₂CH₃ | S | C-Br | CH₂OCH₂CH₃ | CH₂O | C-H |
| L1.97 | CH₃ | S | C-Cl | H | CH₂ | C-Cl |
| L1.98 | CH₃ | S | C-Cl | CH₂CH₃ | CH₂ | C-Cl |
| L1.99 | CH₃ | S | C-Cl | CH₂C=CH₂ | CH₂ | C-Cl |
| L1.100 | CH₃ | S | C-Cl | CH₂C.CH | CH₂ | C-Cl |
| L1.101 | CH₃ | S | C-Cl | CH₂OCH₃ | CH₂ | C-Cl |
| L1.102 | CH₃ | S | C-Cl | CH₂OCH₂CH₃ | CH₂ | C-Cl |
| L1.103 | CH₃ | S | C-Br | H | CH₂ | C-Cl |
| L1.104 | CH₃ | S | C-Br | CH₂CH₃ | CH₂ | C-Cl |
| L1.105 | CH₃ | S | C-Br | CH₂C=CH₂ | CH₂ | C-Cl |
| L1.106 | CH₃ | S | C-Br | CH₂C.CH | CH₂ | C-Cl |
| L1.107 | CH₃ | S | C-Br | CH₂OCH₃ | CH₂ | C-Cl |
| L1.108 | CH₃ | S | C-Br | CH₂OCH₂CH₃ | CH₂ | C-Cl |
| L1.109 | CH₂CH₃ | S | C-Cl | H | CH₂ | C-Cl |
| L1.110 | CH₂CH₃ | S | C-Cl | CH₂CH₃ | CH₂ | C-Cl |
| L1.111 | CH₂CH₃ | S | C-Cl | CH₂C=CH₂ | CH₂ | C-Cl |
| L1.112 | CH₂CH₃ | S | C-Cl | CH₂C.CH | CH₂ | C-Cl |
| L1.113 | CH₂CH₃ | S | C-Cl | CH₂OCH₃ | CH₂ | C-Cl |
| L1.114 | CH₂CH₃ | S | C-Cl | CH₂OCH₂CH₃ | CH₂ | C-Cl |
| L1.115 | CH₂CH₃ | S | C-Br | H | CH₂ | C-Cl |
| L1.116 | CH₂CH₃ | S | C-Br | CH₂CH₃ | CH₂ | C-Cl |
| L1.117 | CH₂CH₃ | S | C-Br | CH₂C=CH₂ | CH₂ | C-Cl |
| L1.118 | CH₂CH₃ | S | C-Br | CH₂C.CH | CH₂ | C-Cl |
| L1.119 | CH₂CH₃ | S | C-Br | CH₂OCH₃ | CH₂ | C-Cl |
| L1.120 | CH₂CH₃ | S | C-Br | CH₂OCH₂CH₃ | CH₂ | C-Cl |
| L1.121 | CH₃ | S | C-Cl | H | CH(CH₃) | C-Cl |
| L1.122 | CH₃ | S | C-Cl | CH₂CH₃ | CH(CH₃) | C-Cl |
| L1.123 | CH₃ | S | C-Cl | CH₂C=CH₂ | CH(CH₃) | C-Cl |
| L1.124 | CH₃ | S | C-Cl | CH₂C.CH | CH(CH₃) | C-Cl |
| L1.125 | CH₃ | S | C-Cl | CH₂OCH₃ | CH(CH₃) | C-Cl |
| L1.126 | CH₃ | S | C-Cl | CH₂OCH₂CH₃ | CH(CH₃) | C-Cl |
| L1.127 | CH₃ | S | C-Br | H | CH(CH₃) | C-Cl |
| L1.128 | CH₃ | S | C-Br | CH₂CH₃ | CH(CH₃) | C-Cl |
| L1.129 | CH₃ | S | C-Br | CH₂C=CH₂ | CH(CH₃) | C-Cl |
| L1.130 | CH₃ | S | C-Br | CH₂C.CH | CH(CH₃) | C-Cl |
| L1.131 | CH₃ | S | C-Br | CH₂OCH₃ | CH(CH₃) | C-Cl |
| L1.132 | CH₃ | S | C-Br | CH₂OCH₂CH₃ | CH(CH₃) | C-Cl |
| L1.133 | CH₂CH₃ | S | C-Cl | H | CH(CH₃) | C-Cl |
| L1.134 | CH₂CH₃ | S | C-Cl | CH₂CH₃ | CH(CH₃) | C-Cl |
| L1.135 | CH₂CH₃ | S | C-Cl | CH₂C=CH₂ | CH(CH₃) | C-Cl |
| L1.136 | CH₂CH₃ | S | C-Cl | CH₂C.CH | CH(CH₃) | C-Cl |
| L1.137 | CH₂CH₃ | S | C-Cl | CH₂OCH₃ | CH(CH₃) | C-Cl |
| L1.138 | CH₂CH₃ | S | C-Cl | CH₂OCH₂CH₃ | CH(CH₃) | C-Cl |
| L1.139 | CH₂CH₃ | S | C-Br | H | CH(CH₃) | C-Cl |
| L1.140 | CH₂CH₃ | S | C-Br | CH₂CH₃ | CH(CH₃) | C-Cl |
| L1.141 | CH₂CH₃ | S | C-Br | CH₂C=CH₂ | CH(CH₃) | C-Cl |
| L1.142 | CH₂CH₃ | S | C-Br | CH₂C.CH | CH(CH₃) | C-Cl |
| L1.143 | CH₂CH₃ | S | C-Br | CH₂OCH₃ | CH(CH₃) | C-Cl |
| L1.144 | CH₂CH₃ | S | C-Br | CH₂OCH₂CH₃ | CH(CH₃) | C-Cl |
| L1.145 | CH₃ | S | C-Cl | H | CHF | C-Cl |
| L1.146 | CH₃ | S | C-Cl | CH₂CH₃ | CHF | C-Cl |
| L1.147 | CH₃ | S | C-Cl | CH₂C=CH₂ | CHF | C-Cl |
| L1.148 | CH₃ | S | C-Cl | CH₂C.CH | CHF | C-Cl |
| L1.149 | CH₃ | S | C-Cl | CH₂OCH₃ | CHF | C-Cl |
| L1.150 | CH₃ | S | C-Cl | CH₂OCH₂CH₃ | CHF | C-Cl |
| L1.151 | CH₃ | S | C-Br | H | CHF | C-Cl |
| L1.152 | CH₃ | S | C-Br | CH₂CH₃ | CHF | C-Cl |
| L1.153 | CH₃ | S | C-Br | CH₂C=CH₂ | CHF | C-Cl |
| L1.154 | CH₃ | S | C-Br | CH₂C.CH | CHF | C-Cl |
| L1.155 | CH₃ | S | C-Br | CH₂OCH₃ | CHF | C-Cl |
| L1.156 | CH₃ | S | C-Br | CH₂OCH₂CH₃ | CHF | C-Cl |
| L1.157 | CH₂CH₃ | S | C-Cl | H | CHF | C-Cl |
| L1.158 | CH₂CH₃ | S | C-Cl | CH₂CH₃ | CHF | C-Cl |
| L1.159 | CH₂CH₃ | S | C-Cl | CH₂C=CH₂ | CHF | C-Cl |
| L1.160 | CH₂CH₃ | S | C-Cl | CH₂C.CH | CHF | C-Cl |
| L1.161 | CH₂CH₃ | S | C-Cl | CH₂OCH₃ | CHF | C-Cl |
| L1.162 | CH₂CH₃ | S | C-Cl | CH₂OCH₂CH₃ | CHF | C-Cl |
| L1.163 | CH₂CH₃ | S | C-Br | H | CHF | C-Cl |
| L1.164 | CH₂CH₃ | S | C-Br | CH₂CH₃ | CHF | C-Cl |
| L1.165 | CH₂CH₃ | S | C-Br | CH₂C=CH₂ | CHF | C-Cl |
| L1.166 | CH₂CH₃ | S | C-Br | CH₂C.CH | CHF | C-Cl |
| L1.167 | CH₂CH₃ | S | C-Br | CH₂OCH₃ | CHF | C-Cl |
| L1.168 | CH₂CH₃ | S | C-Br | CH₂OCH₂CH₃ | CHF | C-Cl |
| L1.169 | CH₃ | S | C-Cl | H | CH₂O | C-Cl |
| L1.170 | CH₃ | S | C-Cl | CH₂CH₃ | CH₂O | C-Cl |
| L1.171 | CH₃ | S | C-Cl | CH₂C=CH₂ | CH₂O | C-Cl |
| L1.172 | CH₃ | S | C-Cl | CH₂C.CH | CH₂O | C-Cl |
| L1.173 | CH₃ | S | C-Cl | CH₂OCH₃ | CH₂O | C-Cl |
| L1.174 | CH₃ | S | C-Cl | CH₂OCH₂CH₃ | CH₂O | C-Cl |
| L1.175 | CH₃ | S | C-Br | H | CH₂O | C-Cl |
| L1.176 | CH₃ | S | C-Br | CH₂CH₃ | CH₂O | C-Cl |
| L1.177 | CH₃ | S | C-Br | CH₂C=CH₂ | CH₂O | C-Cl |
| L1.178 | CH₃ | S | C-Br | CH₂C.CH | CH₂O | C-Cl |
| L1.179 | CH₃ | S | C-Br | CH₂OCH₃ | CH₂O | C-Cl |
| L1.180 | CH₃ | S | C-Br | CH₂OCH₂CH₃ | CH₂O | C-Cl |
| L1.181 | CH₂CH₃ | S | C-Cl | H | CH₂O | C-Cl |
| L1.182 | CH₂CH₃ | S | C-Cl | CH₂CH₃ | CH₂O | C-Cl |
| L1.183 | CH₂CH₃ | S | C-Cl | CH₂C=CH₂ | CH₂O | C-Cl |
| L1.184 | CH₂CH₃ | S | C-Cl | CH₂C.CH | CH₂O | C-Cl |
| L1.185 | CH₂CH₃ | S | C-Cl | CH₂OCH₃ | CH₂O | C-Cl |
| L1.186 | CH₂CH₃ | S | C-Cl | CH₂OCH₂CH₃ | CH₂O | C-Cl |
| L1.187 | CH₂CH₃ | S | C-Br | H | CH₂O | C-Cl |
| L1.188 | CH₂CH₃ | S | C-Br | CH₂CH₃ | CH₂O | C-Cl |
| L1.189 | CH₂CH₃ | S | C-Br | CH₂C=CH₂ | CH₂O | C-Cl |
| L1.190 | CH₂CH₃ | S | C-Br | CH₂C.CH | CH₂O | C-Cl |
| L1.191 | CH₂CH₃ | S | C-Br | CH₂OCH₃ | CH₂O | C-Cl |
| L1.192 | CH₂CH₃ | S | C-Br | CH₂OCH₂CH₃ | CH₂O | C-Cl |

### Table L2

Table L2 provides 192 compounds of Formula L1 wherein R¹, D, E, R², A and J are as defined in Table L1 and R⁶ is ethyl.

### Table L3

Table L3 provides 192 compounds of Formula L1 wherein R¹, D, E, R², A and J are as defined in Table L1 and R⁶ is *n*-propyl.

### Table L4

Table L4 provides 192 compounds of Formula L1 wherein R¹, D, E, R², A and J are as defined in Table L1 and R⁶ is *iso*-propyl.

### Table L5

Table L5 provides 192 compounds of Formula L1 wherein R¹, D, E, R², A and J are as defined in Table L1 and R⁶ is *n*-butyl.

### Table L6

Table L6 provides 192 compounds of Formula L1 wherein R¹, D, E, R², A and J are as defined in Table L1 and R⁶ is *sec*-butyl.

### Table L7

Table L7 provides 192 compounds of Formula L1 wherein R¹, D, E, R², A and J are as defined in Table L1 and R⁶ is *iso*-butyl.

### Table L8

Table L8 provides 192 compounds of Formula L1 wherein R¹, D, E, R², A and J are as defined in Table L1 and R⁶ is *tert*-butyl.

### Table L9

Table L9 provides 192 compounds of Formula L1 wherein R¹, D, E, R², A and J are as defined in Table L1 and R⁶ is *n*-pentyl.

### Table L10

Table L10 provides 192 compounds of Formula L1 wherein R¹, D, E, R², A and J are as defined in Table L1 and R⁶ is 1-methylbutyl.

### Table L11

Table L11 provides 192 compounds of Formula L1 wherein R¹, D, E, R², A and J are as defined in Table L1 and R⁶ is 2-methylbutyl.

### Table L12

Table L12 provides 192 compounds of Formula L1 wherein R¹, D, E, R², A and J are as defined in Table L1 and R⁶ is 3-methylbutyl.

### Table L13

Table L13 provides 192 compounds of Formula L1 wherein R¹. D, E, R², A and J are as defined in Table L1 and R⁶ is neopentyl.

### Table L14

Table L14 provides in compounds of Formula L1 wherein R¹, D, E, R², A and J are as defined in Table L1 and R⁶ is 2,2-dimethylbutyl.

### Table L15

Table L15 provides 192 compounds of Formula L1 wherein R¹, D, E, R², A and J are as defined in Table L1 and R⁶ is 2,2,2-trifluoroethyl.

### Table L16

Table L16 provides 192 compounds of Formula L1 wherein R¹, D, E, R², A and J are as defined in Table L1 and R⁶ is 2,2-difluoro-2-methoxyethyl.

### Table L17

Table L17 provides 192 compounds of Formula L1 wherein R¹, D, E, R², A and J are as defined in Table L1 and R⁶ is 3,3,3-trifluoropropyl.

### Table L18

Table L18 provides 192 compounds of Formula L1 wherein R¹, D, E, R², A and J are as defined in Table L1 and R⁶ is *para*-fluorobenzyl.

### Table L19

Table L19 provides 192 compounds of Formula L1 wherein R¹, D, E, R², A and J are as defined in Table L1 and R⁶ is 2,4-dichlorophenyl.

### Table L20

Table L20 provides 192 compounds of Formula L1 wherein R¹, D, E, R², A and J are as defined in Table L1 and R⁶ is 2-chloro-4-trifluoromethylphenyl.

The following abbreviations are used throughout this description:

| | |
|---|---|
| m.p. = melting point | ppm = parts per million |
| s = singlet | br = broad |
| d = doublet | dd = doublet of doublets |
| t = triplet | q = quartet |
| m = multiplet | |

Table 1 shows selected NMR data, all with CDCl₃, as the solvent (unless otherwise stated; if a mixture of solvents is present, this is indicated as, for example (CDCl₃ / *d*_{*6*}-DMSO)), (no attempt is made to list all characterising data in all cases) for compounds of Tables A1 to L20.

**TABLE 1**

| **Compound No.** | ^{**1**}**H NMR (CDCl**_{**3**} **unless otherwise stated)** |
|---|---|
| A3.1 | 1.0(t,3H); 2.15(m,2,H); 2.35(s,3H); 4.0(s,2H); 4.7(t,2H); 7.35(dd,1H); 7.85(d,1H); 7.95(d,1H); 8.15(br,1H). |
| A1.1 | 2.35(s,3H); 4.0(s,2H); 4.5(s,3H); 7.35(dd,1H); 7.8(d,1H); 7.9(d,1H); 8.05(br,1H). |
| A18.1 | (*d*₆-DMSO): 2.35(s,3H); 4.05(s,2H); 5.8(s,2H); 7.0(m,2H); 7.4(m,3H); 7.8(m,2H); 10.9(br,1H). |
| A15.1 | 2.4(s,3H); 4.0(s,2H); 5.3(q,2H); 7.4(dd,1H); 7.85(d,1H); 7.95(d,1H); 8.15(br,1H). |
| A9.1 | 0.9(t,3H); 1.35(m,4H); 2.15(m,2H); 2.4(s,3H); 4.05(s,2H); 4.7(t,2H); 7.35(dd,1H); 7.85(d,1H); 7.9(d,1H); 8.15(br,1H). |
| A20.1 | 2.4(s,3H); 4.05(s,2H); 7.45(dd,1H); 7.75(dd,1H); 7.95(m,3H); 8.05(m,1H); 8.2(br,1H). |
| A7.1 | 0.85(t,3H); 1.7(d,3H); 2.0-2.2(m,2H); 2.35(s,3H); 4.0(s,2H); 4.9(m,1H); 7.3(dd,1H); 7.85(d,1H); 7.9(d,1H); 8.15(br,1H). |
| A2.1 | 1.75(t,3H); 2.35(s,3H); 4.0(s,2H); 4.8(q,2H); 7.35(dd,1H); 7.8(br,1H); 7.9(d,1H); 8.1(br,1H). |
| A3.103 | 1.0(t,3H); 1.25(t,3H); 2.15(m,2H); 2.85(q,2H); 3.95(s,2H); 4.7(t,2H); 7.35(m,1H); 7.85(br,1H); 7.95(d,1H); 7.95(br,1H); 8.2(d,1H); 8.35(d,1H). |
| A13.1 | 1.05(s.9H); 2.4(s,3H); 4.05(s,2H); 4.55(s,2H); 7.35(dd,1H); 7.85(br,1H); 7.95(d,1H); 8.15(br,1H). |
| A3.2 | 0.95(t,3H); 1.2(t,3H); 2.13(m,2H); 2.5(s,3H); 3.65(br,2H), 3.8(br,2H); 4.65(t,2H); 7.15(br.d,1H); 7.5(br,1H); 7.78(dd,1H). |
| A3.6 | 0.95(t,3H); 1.2(t,3H); 2.1(m,2H); 2.5(s,3H); 3.6(q,2H), 3.7(br,2H); 4.7(t,2H); 5.1(br,2H); 7.15(br,1H); 7.55(br,1H); 7.8(d,1H). |
| A21.1 | 2.4(s,3H); 4.1(s,2H); 7.5(m,1H), 7.8-7.9(m,4H); 8.5(br.d,2H); 10.35(s,1H). |
| A16.1 | 2.39(s,3H); 3.61(s,3H); 4.03(s,2H); 5.19(t,2H); 7.40(dd,1H); 7.88(s,1H); 7.97(d,1H); 8.16(s,1H). |
| A10.1 | 1.0(d,6H); 2.03(q,2H); 2.37(s,3H); 2.37(m.1H); 4.0(s,2H); 4.77(dd,2H); 7.34(dd,1H); 7.83(s,1H); 7.92(d,1H); 8.16(s,1H). |
| A6.1 | 1.0(d,6H); 2.37(s,3H); 2.54(septet,1H); 4.03(s,2H); 4.56(d,2H); 7.36(dd,1H); 7.84(s,1H); 7.94(d,1H). |
| I3.2 | 0.95(t,3H); 1.4(t,3H); 2.12(m,2H); 2.5(s,3H); 3.9(q,2H); 4.2(s,2H); 4.65(t,2H); 7.42(dd,1H); 7.8(br,1H); 7.81(br,1H). |
| I3.6 | 0.95(t,3H); 1.15(t,3H); 2.1(m,2H); 2.55(s,3H); 3.5(q,2H); 4.2(s,2H); 4.7(t,2H), 5.2(s,2H); 7.4(dd,1H); 7.8(m,2H). |
| E1.1 | 2.35(s,3H); 4.1(s,2H); 4.3(s,3H); 7.55(m,2H); 8.0(d,1H); 8.3(br.s,1H). |
| E15.1 | 2.4(s,3H); 4.05(s,2H); 5.25(q,2H); 7.4(dd,1H); 7.6(d,1H); 8.15(d,1H); 8.3(br,1H). |
| F15.1 | 2.4(s,3H); 4.1(s,2H); 5.25(q,1H); 7.6(m,2H); 8.1(br.s,1H); 8.25(br,1H). |
| E22.1 | 2.40(s,3H); 3.03(s,3H); 4.10(s,2H); 7.52(dd,2H); 8.19(d,1H); 8.2(br,1H); 8.3(d,1H). |
| F1.1 | 2.35(s,3H); 4.1(s,2H); 4.3(s,3H); 7.3(d,1H); 7.55(d,1H); 8.1(d,1H); 8.3(br.s,1H). |
| F22.1 | 2.40(s,3H); 3.03(s,3H); 4.09(s,2H); 7.66(dd,2H); 8.10(d,1H); 8.2(br,1H); 8.34(d,1H). |
| E6.145 | 0.94(d,6H); 2.36(m,1H); 2.36(s,3H); 3.98(s,2H); 4.22(d,2H), 7.32(m,1H); 7.48(m,1H); 7.70(m,1H); 8.02(s,1H); 8.1(br,1H). |
| A6.145 | 0.96(d,6H); 2.36(s,3H); 2.42(m,1H); 3.96(s,2H); 4.24(d,2H), 7.21(m,1H); 7.64(s,1H); 7.80(d,1H); 7.92(s,1H); 8.14(br,1H). |
| E6.150 | 0.92(d,6H); 1.14(t,3H); 2.33(m,1H); 2.50(s,3H); 3.60(q,2H); 3.72(br,2H); 4.16(d,2H); 5.10(br,2H); 7.13(m,1H); 7.37(m,2H); 7.90(s,1H). |
| J6.102 | 0.92(d,6H); 1.17(t,3H); 2.34(m,1H); 2.56(s,3H); 3.50(q,2H); 4.12-4.18(m,4H); 5.20(s,2H); 7.32-7.44(m,2H); 7.70(s,1H); 7.92(s,1H). |
| A6.150 | 0.94(d,6H); 1.19(t,3H); 2.38(m,1H); 2.50(s,3H); 3.60(q,2H); 3.68(br,2H); 4.18(d,2H); 5.10(br,2H); 7.02(d,1H); 7.32(br,1H); 7.62(d,1H); 7.80(s,1H). |
| I6.102 | 0.92(d,6H);1.15(t,3H); 2.39(m,1H); 2.54(s,3H); 3.50(q,2H); 4.14-4.20(m,4H); 5.20(s,2H); 7.32(m,1H); 7.60-7.68(m,2H); 7.82(s,1H). |

The compounds of the invention may be made in a variety of ways. For example, as shown in Scheme 1, compounds (Ia) wherein Y is O and A is alkylene, alkenylene, alkylenoxy, alkylenamino or alkylenethio may be prepared by reacting amine (II) with an appropriate carboxylic ester (III) (X¹ = alkoxy or aryloxy) or with a carboxylic acid (III, X¹= OH) optionally in the presence of a known coupling agent such as 1,3-dicyclohexylcarbodiimide or 1-(3-dimethylaminopropyl)-3-ethylcarbodiimide. Alternatively the carboxylic acid (III, X¹ = OH) may first be converted to an acid chloride, anhydride or chloroformate suitable for reaction with an amine to form an amide; such procedures are well known to those skilled in the art and are described, for example in J. March, Advanced Organic Chemistry, Third Edition, John Wiley and Sons, New York, 1985, pps 370-376 and references therein.

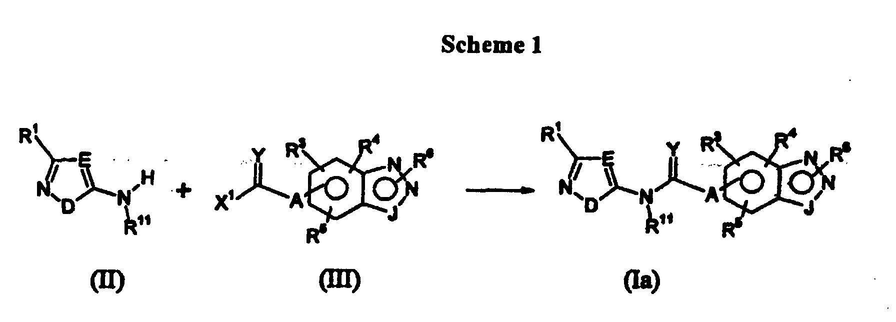

Therefore in a further aspect the invention provides a process for preparing a compound of formula (I) where Y is oxygen comprising reacting a compound of formula (II) with a compound of formula (III) where Y is oxygen and X¹ is a leaving group (such as alkoxy, aryloxy, OH, chloro or ClC(O)O or such that the compound of formula (III) is a dianhydride).

Compounds of formula (II) are known compounds, or may be prepared from commercially available starting materials by methods described in the literature (see, for example, C. Oliver Kappe, Robert Flammang, and Curt Wentrup, Heterocycles, Vol. 37, No. 3, 1615, (1994), A. Adams and R. Slack, J. Chem. Soc., 3061, (1959) and Ronald E Hackler, Kenneth W. Burow, Jr., Sylvester V. Kaster and David I. Wicldser, J. Heterocyclic Chem, 26, 1575, (1989), Ronald E Hackler, Glen P Jourdan, Peter L Johnson, Brian R Thoreen and Jack G Samaritoni, PCT Int. Appl., WO9304580 A1, and references therein).

The syntheses of substituted benzotriazoles and indazoles is described in the chemical literature (see, for example Alan R. Kalritzky and Charles W. Rees, Comprehensive Heterocyclic Chemistry, Pergamon Press, 1984; Christoph Ruchardt and Volker Hassmann, Liebis Ann. Chem., 1980, 908; Jean-Jacques Vandenbossche and Alain Lagrange, Eur. Pat. Appl., EP904769; F. Halley and X. Sava, Synth. Commun. 1997, 27 (7), 1199; Donald R. James, Don R. Baker, Steven D. Mielich, William J. Michaely, Steven Fitzjohn, Christopher G. Knudsen, Christopher Mathews and John M. Gerdes, PCT Int. Appl., WO9318008 A1, Donald Richard James and Raymond Anthony Felix, PCT Int. Appl., WO9425446 Al) and similar methods may be used to prepare compounds of formula (III) starting from known appropriately substituted anilines. Alternatively, halobenzotriazoles and haloindazoles (especially where the halogen is bromo or iodo), which may be prepared by the above methods, may be converted into compounds of formula (III) via procedures involving transition-metal catalysed transformations analogous to those described by Nitya G. Kundu et al., Tetrahedron, 53 (39), 13397, (1997), Takao Sakamoto et al. Heterocycles, 36 (11), 2509 (1993), Motoi Kawatsura and John F. Hartwig, JACS, 1999, 121, 1473, and others.

A compound of formula (Ia), wherein Y is sulphur, may be prepared by reacting a compound of formula (Ia), wherein Y is oxygen, with a suitable thionating agent such as 2,4-bis(4-methoxyphenyl)-1,3-dithia-2,4-diphosphetane-2,4-disulfide (Lawesson's reagent), 2,4-bis(methylthio)-1,3-dithia-2,4-diphosphetane-2,4-disulfide (Davy reagent methyl), 2,4-bis(*para*-tolyl)-1,3-dithia-2,4-diphosphetane-2,4-disulfide (Davy reagent *p*-tolyl) or phosphorus pentasulfide in a suitable solvent such as toluene or fluorobenzene.

Therefore in a further aspect the invention provides a process for preparing a compound of formula (I) where Y is sulphur comprising preparing a compound of formula (I) where Y is oxygen (by reacting a compound of formula (II) with a compound of formula (III) where Y is oxygen and X¹ is a leaving group) and then reacting this compound with a thionating agent

A compound of formula (IV) may be made from a compound of formula (V) by treatment with N,N-dimethylformamide dialkyl acetal in a suitable solvent such as toluene or N,N-dimethylformamide. Frequently this reaction produces a mixture of E and Z isomers which are sometimes separable by standard techniques such as flash column chromatography and recrystallisation. This invention covers isolated isomers together with mixtures of isomers.

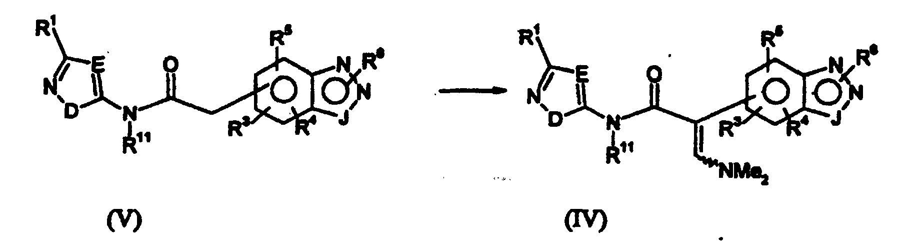

A compound of formula (IV) may be treated subsequently with an amine (HNRaRb) to give a compound of formula (VI); a compound of formula (V) may be treated in an analogous manner with a trialkylorthoformate (HC(ORd)₃) to afford a compound of formula (VII):

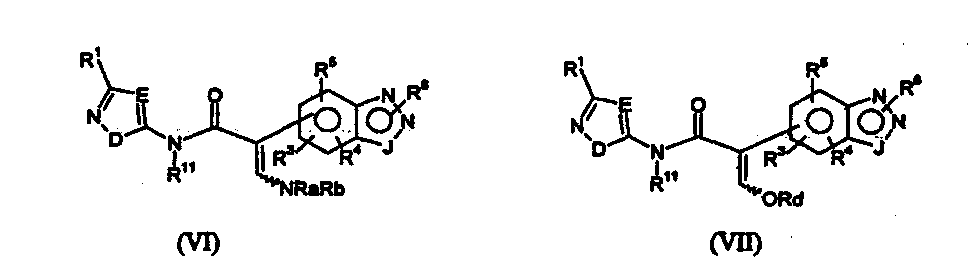

A compound of formula (Ia), wherein R¹¹ is hydrogen, may be treated with an optionally substituted alkylating agent (such as an alkyl halide, chloromethylether, dialkyl sulfate or trialkyloxonium salt) optionally in the presence of a base to give an additional compound of formula (Ia) wherein R¹¹ is optionally substituted alkyl, which forms a further part of this invention. When Y is O, this reaction usually produces a mixture of isomeric products of formulae (Ia) and (Ib). In contrast, when Y is S, a compound of formula (Ic) is the predominant product.

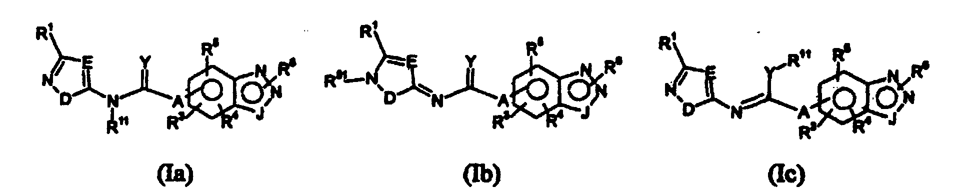

Those skilled in the art will recognise that analogous reactions involving sulfenylation, sulfonylation and acylation are possible when Y is O.

A compound of formula (Ia), where R¹¹ is alkoxymethyl or acyloxymethyl, may also be prepared from a compound of formula (Ia), where R¹¹ is hydrogen, by sequential reaction with formaldehyde and an alkylating or acylating agent.

Additional compounds of formula (Ia) may be prepared from a compound of formula (I), where R¹¹ is hydrogen, by reaction with hydroxymethylbenzotriazole or benzotriazoles and aldehydes according to the method of A.R.Katritzky et al., J. Org. Chem., (1993), 58, 2086.

A compound of formula (Ic) where Y = S may be reacted with an alcohol, amine, O-alkylhydroxyl-amine or hydrazine, optionally in the presence of a mercuric salt (such as mercuric chloride), according to known procedures to give a compound of formula (1d) or (Ie) respectively: 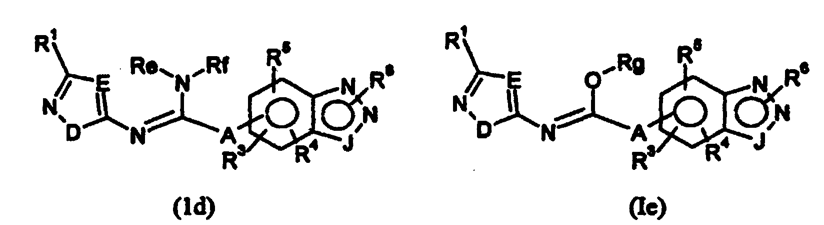 where Re is H, optionally substituted alkyl, alkenyl or alkynyl, Rf is substituted alkyl, alkenyl alkynyl, amino, substituted amino or alkoxy and Rg is optionally substituted alkyl, alkenyl or alkynyl.

A compound of formula (1d) where Re is hydrogen may be treated with an optionally substituted alkylating agent (such as an alkyl halide, chloromethylether, dialkyl sulfate or trialkyloxonium salt) optionally in the presence of a base to give a compound of formula (If).

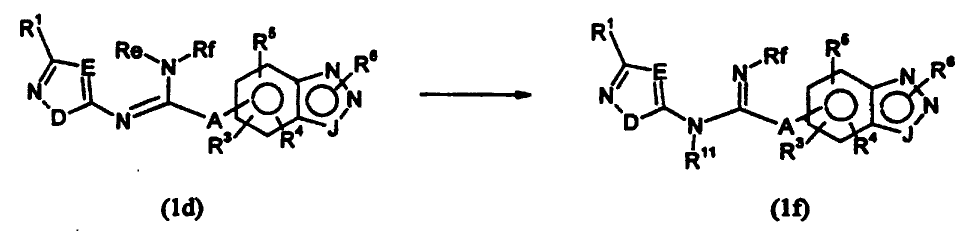

Heteroaryl N-oxides can be produced by methods known to the skilled person.

It will be appreciated that compounds of fomula (Ia), (Ib), (Ic), (Id), (Ie), (If), (IV), (V), (VI) and (VII) are all compounds of formula (I).

The compounds of formula (I) can be used to combat and control infestations of insect pests such as Lepidoptera, Diptera, Hemiptera, Thysanoptera, Orthoptera, Dictyoptera, Coleoptera, Siphonaptera, Hymenoptera and Isoptera and also other invertebrate pests, for example, acarine, nematode and mollusc pests. Insects, acarines, nematodes and molluscs are hereinafter collectively referred to as pests. The pests which may be combated and controlled by the use of the invention compounds include those pests associated with agriculture (which term includes the growing of crops for food and fibre products), horticulture and animal husbandry, companion animals, forestry and the storage of products of vegetable origin (such as fruit, grain and timber); those pests associated with the damage of man-made structures and the transmission of diseases of man and animals; and also nuisance pests (such as flies).

Examples of pest species which may be controlled by the compounds of formula (I) include: *Myzus persicae* (aphid), *Aphis gossypii* (aphid), *Aphis fabae* (aphid), *Lygus* spp. (capsids), *Dysdercus* spp. (capsids), *Nilaparvata lugens* (planthopper), *Nephotettixc incticeps* (leafhopper), *Nezara* spp. (stinkbugs), *Euschistus* spp. (stinkbugs), *Leptocorisa* spp. (stinkbugs), *Frankliniella occidentalis* (thrip), Thrips spp. (thrips), *Leptinotarsa decemlineata* (Colorado potato beetle), *Anthonomus grandis* (boll weevil), *Aonidiella* spp. (scale insects), *Trialeurodes* spp. (white flies), *Bemisia tabaci* (white fly), *Ostrinia nubilalis* (European corn borer), *Spodoptera littoralis* (cotton leafworm), *Heliothis virescens* (tobaoco budworm), *Helicoverpa armigera* (cotton bollworm), *Helicoverpa zea* (cotton bollworm), *Sylepta derogata* (cotton leaf roller), *Pieris brassicae* (white butterfly), *Plutella xylostella* (diamond back moth), *Agrotis* spp. (cutworms), *Chilo suppressalis* (rice stem borer), *Locusta* *migratoria* (locust), *Chortiocetes terminifera* (locust), *Diabrotica* spp. (rootworms), *Panonychus ulmi* (European red mite), *Panonychus citri* (citrus red mite), *Tetranychus urticae* (two-spotted spider mite), *Tetranychus cinnabarinus* (carmine spider mite), *Phyllocoptruta oleivora* (citrus rust mite), *Polyphagotarsonemus latus* (broad mite), *Brevipalpus* spp. (flat mites), *Boophilus microplus* (cattle tick), *Dermacentor variabilis* (American dog tick), *Ctenocephalides felis* (cat flea), *Liriomyza* spp. (leafminer), *Musca domestica* (housefly), *Aedes aegypti* (mosquito), *Anopheles* spp. (mosquitoes), *Culex* spp. (mosquitoes), *Lucillia* spp. (blowflies), *Blattella germanica* (cockroach), *Periplaneta americana* (cockroach), *Blatta orientalis* (cockroach), termites of the Mastotermitidae (for example *Mastotermes* spp.), the Kalotermitidae (for example *Neotermes* spp.), the Rhinotermitidae (for example *Coptotermes formosanus, Reticulitermes flavipes, R. speratu, R. virginicus, R. hesperus*, and *R. santonensis*) and the Termitidae (for example *Globitermes sulphureus*), *Solenopsis geminata* (fire ant), *Monomorium pharaonis* (pharaoh's ant), *Damalinia* spp. and *Linognathus* spp. (biting and sucking lice), *Meloidogyne* spp. (root knot nematodes), *Globodera* spp. and *Heterodera* spp. (cyst nematodes), *Pratylenchus* spp. (lesion nematodes), *Rhodopholus* spp. (banana burrowing nematodes), *Tylenchulus* spp.(citrus nematodes), *Haemonchus contortus* (barber pole worm), *Caenorhabditis elegans* (vinegar eelworm), *Trichostrongylus* spp. (gastro intestinal nematodes) and *Deroceras reticulatum* (slug).

The compounds of formula (I) are also active fungicides and may be used to control one or more of the following pathogens: *Pyricularia oryzae* (*Magnaporthe grisea*) on rice and wheat and other *Pyricularia* spp. on other hosts; *Puccinia recondita, Puccinia striiformis* and other rusts on wheat, *Puccinia hordei, Puccinia striiformis* and other rusts on barley, and rusts on other hosts (for example turf, rye, coffee, pears, apples, peanuts, sugar beet, vegetables and ornamental plants); *Erysiphe cichoracearum* on cucurbits (for example melon); *Erysiphe graminis* (powdery mildew) on barley, wheat, rye and turf and other powdery mildews on various hosts, such as *Sphaerotheca macularis* on hops, *Sphaerotheca fusca* (*Sphaerotheca fuliginea*) on cucurbits (for example cucumber), *Leveillula taurica* on tomatoes, aubergine and green pepper, *Podosphaera leucotricha* on apples and *Uncinula necator* on vines; *Cochliobolus* spp., *Helminthosporium spp., Drechslera* spp. (*Pyrenophora* spp.), *Rhynchosporium* spp., *Mycosphaerella graminicola (Septoria tritici)* and *Phaeosphaeria nodorum* (*Stagonospora nodorum* or *Septoria nodorum*), *Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. (*Pleospora* spp.) on apples, pears, onions and other hosts; summer diseases (for example bitter rot (*Glomerella cingulata*), black rot or frogeye leaf spot (*Botryosphaeria obtusa*), Brooks fruit spot (*Mycosphaerella pomi*), Cedar apple rust (*Gymnosporangium juniperi-virginianae*), sooty blotch (*Gloeodes pomigena*), flyspeck (*Schizothyrium pomi*) and white rot (*Botryosphaeria dothidea*)) on apples and pears; *Plasmopara viticola* on vines; other downy mildews, such as *Bremia lactucae* on lettuce, *Peronospora* spp. on soybeans, tobacco, onions and other hosts, *Pseudoperonospora humuli* on hops and *Pseudoperonospora cubensis* on cucurbits; *Pythium* spp. (including *Pythium ultimum*) on turf and other hosts; *Phytophthora infestans* on potatoes and tomatoes and other *Phytophthora* spp. on vegetables, strawberries, avocado, pepper, ornamentals, tobacco, cocoa and other hosts; *Thanatephorus cucumeris* on rice and turf and other *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Diaporthe* spp. on citrus, soybean, melon, pears, lupin and other hosts; *Elsinoe* spp. on citrus, vines, olives, pecans, roses and other hosts; *Verticillium* spp. on a range of hosts including hops, potatoes and tomatoes; *Pyrenopeziza* spp. on oil-seed rape and other hosts; *Oncobasidium theobromae* on cocoa causing vascular streak dieback; *Fusarium* spp., *Typhula* spp., *Microdochium nivale, Ustilago* spp., *Urocystis* spp., *Tilletia* spp. and *Claviceps purpurea* on a variety of hosts but particularly wheat, barley, turf and maize; *Ramularia* spp. on sugar beet, barley and other hosts; post-harvest diseases particularly of fruit (for example *Penicillium digitatum, Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, Guignardia bidwellii, Phellinus igniarus, Phomopsis viticola*, *Pseudopeziza tracheiphila* and *Stereum hirsutum*; other pathogens on trees (for example *Lophodermium seditiosum*) or lumber, notably *Cephaloascus fragrans, Ceratocystis* spp., *Ophiostoma piceae, Penicillium* spp., *Trichoderma pseudokoningii, Trichoderma viride*, *Trichoderma harzianum, Aspergillus niger, Leptographium lindbergi* and *Aureobasidium pullulans*; and fungal vectors of viral diseases (for example *Polymyxa graminis* on cereals as the vector of barley yellow mosaic virus (BYMV) and *Polymyxa betae* on sugar beet as the vector of rhizomania).

A compound of formula (I) may move acropetally, basipetally or locally in plant tissue to be active against one or more fungi. Moreover, a compound of formula (I) may be volatile enough to be active in the vapour phase against one or more fungi on the plant.

The invention therefore provides a method of combating and controlling insects, acarines, nematodes or molluscs which comprises applying an insecticidally, acaricidally, nematicidally or molluscicidally effective amount of a compound of formula (I), or a composition comaining a compound of formula (I), to a pest, a locus of pest, or to a plant susceptible to attack by a pest, and a method of combating and controlling fungi which comprises applying a fungicidally effective amount of a compound of formula (I), or a composition containing a compound of formula (I), to a plant, to a seed of a plant, to the locus of the plant or seed, to soil or to any other growth medium (for example a nutrient solution). The compounds of formula (I) are preferably used against insects, acarines, nematodes or fungi.

The term "plant" as used herein includes seedlings, bushes and trees. Furthermore, the fungicidal method of the invention includes protectant, curative, systemic, eradicant and antisporulant treatments.

As fungicides, the compounds of formula (I) are preferably used for agricultural, horticultural and turfgrass purposes in the form of a composition.

In order to apply a compound of formula (I) as an insecticide, acaricide, nematicide or molluscicide to a pest, a locus of pest, or to a plant susceptible to attack by a pest, or, as a fungicide to a plant, to a seed of a plant, to the locus of the plant or seed, to soil or to any other growth medium, a compound of formula (I) is usually formulated into a composition which includes, in addition to the compound of formula (I), a suitable inert diluent or carrier and, optionally, a surface active agent (SFA). SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (I). The composition is generally used for the control of pests or fungi such that a compound of formula (I) is applied at a rate of from 0.1g to 10kg per hectare, preferably from 1g to 6kg per hectare, more preferably from 1g to 1kg per hectare.

When used in a seed dressing, a compound of formula (I) is used at a rate of 0.0001g to 10g (for example 0.001g or 0.05g), preferably 0.005g to 10g, more preferably 0.005g to 4g, per kilogram of seed.

In another aspect the present invention provides an insecticidal, acaricidal, nematicidal, molluscicidal or fungicidal composition comprising an insecticidally, acaricidally, nematicidally, molluscicidally or fungicidally effective amount of a compound of formula (I) and a suitable carrier or diluent therefor. The composition is preferably an insecticidal, acaricidal, nematicidal or fungicidal composition.

In a still further aspect the invention provides a method of combating and controlling pests or fungi at a locus which comprises treating the pests or fungi or the locus of the pests or fungi with an insecticidally, acaricidally, nematicidally, molluscicidally or fungicidally effective amount of a composition comprising a compound of formula (I). The compounds of formula (I) are preferably used against insects, acarines, nematodes or fungi.

The compositions can be chosen from a number of formulation types, including durable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (I).

Dustable powders (DP) may be prepared by mixing a compound of formula (I) with one or more solid diluents (for example natural clays, kaolin, pyrophyllite, bentomte, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder. Soluble powders (SP) may be prepared by mixing a compound of formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (I) and one or more powdered solid diluents or carriers, or from pre-formed blank granules by absorbing a compound of formula (I) (or a solution thereof, in a suitable agent) in a porous granular material (such as pumice, attapulgite clays, fuller's earth, kieselguhr, diatomaceous earths or ground corn cobs) or by adsorbing a compound of formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (I) either as a liquid (if it is not a liquid at room temperature, it may be melted at a reasonable temperature, typically below 70°C) or in solution (by dissolving it in an appropriate solvent) and then emulsifiying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (I). SCs may be prepared by ball or bead milling the solid compound of formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (I) may be dry milled and added to water, containing agents hereinbefore described, to produce the desired end product.

Aerosol formulations comprise a compound of formula (I) and a suitable propellant (for example *n*-butane). A compound of formula (I) may also be dissolved or dispersed in a suitable medium (for example water or a water miscible liquid, such as *n*-propanol) to provide compositions for use in non-pressurised, hand-actuated spray pumps.

A compound of formula (I) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (I) and, optionally, a carrier or diluent therefor. The polymeric shell may be produced by either an interfacial polycondensation reaction or by a coacervation procedure. The compositions may provide for controlled release of the compound of formula (I) and they may be used for seed treatment. A compound of formula (I) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (I)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (I)).

A compound of formula (I) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be surface SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-*iso*propyl-naphthalene sulphonates), ether sulphates, alcohol ether sulphates (for example sodium laureth-3-sulphate), ether carboxylates (for example sodium laurcth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (I) may be applied by any of the known means of applying pesticidal or fungicidal compounds. For example, it may be applied, formulated or unformulated, to the pests or to a locus of the pests (such as a habitat of the pests, or a growing plant liable to infestation by the pests) or to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (I) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (I) (for example 0.0001 to 10%, by weight) depending upon the purpose for which they are to be used.

A compound of formula (I) may be used in mixtures with fertilisers (for example nitrogen-, potassium- or phosphorus-containing fertilisers). Suitable formulation types include granules of fertiliser. The mixtures suitably contain up to 25% by weight of the compound of formula (I).

The invention therefore also provides a fertiliser composition comprising a fertiliser and a compound of formula (I).

The compositions of this invention may contain other compounds having biological activity, for example micronutrients or compounds having similar or complementary fungicidal activity or which possess plant growth regulating, herbicidal, insecticidal, nematicidal or acaricidal activity.

By including another fungicide, the resulting composition may have a broader spectrum of activity or a greater level of intrinsic activity than the compound of formula (I) alone. Further the other fungicide may have a synergistic effect on the fungicidal activity of the compound of formula (I).

The compound of formula (I) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (I); or help to overcome or prevent the development of resistance to individual components. The particular additional active ingredient will depend upon the intended utility of the composition. Examples of suitable pesticides include the following:
a) Pyrethroids, such as permethrin, cypermethrin, fenvalerate, esfenvalerate, deltamethrin, cyhalothrin (in particular lambda-cyhalothrin), bifenthrin, fenpropathrin, cyfluthrin, tefluthrin, fish safe pyrethroids (for example ethofenprox), natural pyrethrin, tetramethrin, s-bioallethrin, fenfluthrin, prallethrin or 5-benzyl-3-furylmethyl-(E)-(1R,3S)-2,2-dimethyl-3-(2-oxothiolan-3-ylidenemethyl)cyclopropane carboxylate;
b) Organophosphates, such as, profenofos, sulprofos, acephate, methyl parathion, azinphos-methyl, demeton-s-methyl, heptenophos, thiometon, fenamiphos, monocrotophos, profenofos, triazophos, methamidophos, dimethoate, phosphamidon, malathion, chlorpyrifos, phosalone, terbufos, fensulfothion, fonofos, phorate, phoxim, pirimiphos-methyl, pirimiphos-ethyl, fenitrothion, fosthiazate or diazinon;
c) Carbamates (including aryl carbamates), such as pirimicarb, triazamate, cloethocarb, carbofuran, furathiocarb, ethiofencarb, aldicarb, thiofurox, carbosulfan, bendiocarb, fenobucarb, propoxur, methomyl or oxamyl;
d) Benzoyl ureas, such as diflubenzuron, triflumuron, hexaflumuron, flufenoxuron or chlorfluazuron;
e) Organic tin compounds, such as cyhexatin, fenbutatin oxide or azocyclotin;
f) Pyrazoles, such as tebufenpyrad and fenpyroximate;
g) Macrolides, such as avermectins or milbemycins, for example abamectin, emamectin benzoate, ivermectin, milbemycin, spinosad or azadirachtin;
h) Hormones or pheromones;
i) Organochlorine compounds such as endosulfan, benzene hexachloride, DDT, chlordane or dieldrin;
j) Amidines, such as chlordimeform or amitraz;
k) Fumigant agents, such as chloropicrin, dichloropropane, methyl bromide or metam;
l) Chloronicotinyl compounds such as imidacloprid, thiacloprid, acetamiprid, nitenpyram or thiamethoxam;
m) Diacylhydrazines, such as tebufenozide, chromafenozide or methoxyfenozide;
n) Diphenyl ethers, such as diofenolan or pyriproxifen;
o) Indoxacarb;
p) Chlorfenapyr; or
q) Pymetrozine.

In addition to the major chemical classes of pesticide listed above, other pesticides having particular targets may be employed in the composition, if appropriate for the intended utility of the composition. For instance, selective insecticides for particular crops, for example stemborer specific insecticides (such as cartap) or hopper specific insocticides (such as buprofezin) for use in rice may be employed. Alternatively insecticides or acaricides specific for particular insect species/stages may also be included in the compositions (for example acaricidal ovo-larvicides, such as clofentezine, flubenzimine, hexythiazox or tetradifon; acaricidal motilicides, such as dicofol or propargite; acaricides, such as bromopropylate or chlorobenzilate; or growth regulators, such as hydramethylnon, cyromazine, methoprene, chlorfluazuron or diflubenzuron).

Examples of fungicidal compounds which may be included in the composition of the invention are (*E*)-*N*-methyl-2-[2-(2,5-dimethylphenoxymethyl)phenyl]-2-methoxyiminoacetamide (SSF-129), 4-bromo-2-cyano-*N,N*-dimethyl-6-trifluoromethylbenzimidazole-1-sulphonamide, α-[*N*-(3-chloro-2,6-xylyl)-2-methoxyacetamido]-γ-butyrolactone, 4-chloro-2-cyano-*N,N*-dimethyl-5-*p*-tolylimidazole-1-sulfonamide (IKF-916, cyamidazosulfamid), 3-5-dichloro-*N*-(3-chloro-1-ethyl-1-methyl-2-oxopropyl)-4-methylbenzamide (RH-7281, zoxamide), *N*-allyl-4,5,-dimethyl-2-trimethylsilylthiophene-3-carboxamide (MON65500), *N*-(1-cyano-1,2-dimethylpropyl)-2-(2,4-dichlorophenoxy)propionamide (AC382042), *N*-(2-methoxy-5-pyridyl)-cyclopropane carboxamide, acibenzolar (CGA245704), alanycarb, aldimorph, anilazine, azaconazole, azoxystrobin, benalaxyl, benomyl, biloxazol, bitertanol, blasticidin S, bromuconazole, bupirimate, captafol, captan, carbendazim, carbendazim chlorhydrate, carboxin, carpropamid, carvone, CGA41396, CGA41397, chinomethionate, chlorothalonil, chlorozolinate, clozylacon, copper containing compounds such as copper oxychloride, copper oxyquinolate, copper sulphate, copper tallate and Bordeaux mixture, cymoxanil, cyproconazole, cyprodinil, debacarb, di-2-pyridyl disulphide 1,1'-dioxide, dichlofluanid, diclomezine, dicloran, diethofencarb, difenoconazole, difenzoquat, diflumetorim, *O,O*-di-*iso*-propyl-*S*-benzyl thiophosphate, dimefluazole, dimetconazole, dimethomorph, dimethirimol, diniconazole, dinocap, dithianon, dodecyl dimethyl ammonium chloride, dodemorph, dodine, doguadine, edifenphos, epoxiconazole, ethirimol, ethyl(*Z*)-*N*-benzyl-*N*([methyl(methylthioethylideneaminooxycarbonyl)amino]thio)-β-alaninate, etridiazole, famoxadone, fenamidone (RPA407213), fenarimol, fenbuconazole, fenfuram, fenhexamid (KBR2738), fenpiclonil, fenpropidin, fenpropimorph, fentin acetate, fentin hydroxide, ferbam, ferimzone, fluazmam, fludioxonil, flumetover, fluoroimide, fluquinconazole, flusilazole, flutolanil, flutriafol, folpet, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb (SZX0722), isopropanyl butyl carbamate, isoprothiolane, kasugamycin, kresoxim-methyl, LY186054, LY211795, LY248908, mancozeb, maneb, mefenoxam, mepanipyrim, mepronil, metalaxyl, metconazole, metiram, metiram-zinc, metominostrobin, myclobutanil, neoasozin, nickel dimethyldithiocarbamate, nitrothal-*iso*propyl, nuarimol, ofurace, organomercury compounds, oxadixyl, oxasulfuron, oxolinic acid, oxpoconazole, oxycarboxin, pefurazoate, penconazole, pencycuron, phenazin oxide, phosetyl-Al, phosphorus acids, phthalide, picoxystrobin (ZA1963), polyoxin D, polyram, probenazole, prochloraz, procymidone, propamocarb, propiconazole, propineb, propionic acid, pyrazophos, pyrifenox, pyrimethanil, pyroquilon, pyroxyfur, pyrrolnitrin, quaternary ammonium compounds, quinomethionate, quinoxyfen, quintozene, sipconazole (F-155), sodium pentachlorophenate, spiroxamine, streptomycin, sulphur, tebuconazole, tecloftalam, tecnazene, tetaconazole, thiabendazole, thifluzamid, 2-(thiocyanomethylthio)benzothiazole, thiophanate-methyl, thiram, timibenconazole, tolclofos-methyl, tolylfluanid, triadimefon, triadimenol, triazbutil, triazoxide, tricyclazole, tridemorph, trifloxystrobin (CGA279202), triforine, triflumizole, triticonazole, validamycin A, vapam, vinclozolin, zineb and ziram.

The compounds of formula (I) may be mixed with soil, peat or other rooting media for the protection of plants against seed-borne, soil-borne or foliar fungal diseases.

Examples of suitable synergists for use in the compositions include piperonyl butoxide, sesamex, safroxan and dodecyl imidazole.

Suitable herbicides and plant-growth regulators for inclusion in the compositions will depend upon the intended target and the effect required.

An example of a rice selective herbicide which may be included is propanil. An example of a plant growth regulator for use in cotton is PIX™.

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The invention is illustrated by the following Examples.

### EXAMPLE 1

This Example illustrates the preparation of N-(4-chloro-3-methylisothiazol-5-yl)-(2-propylbenzotriazol-5-yl)acetamide (Compound No.A3.1).

### Step 1 - Preparation of methyl 4-aminophenylacetate.

4-Aminophenylacetic acid (100g) was suspended in methanol (1000ml) and gaseous hydrogen chloride was passed through until the mixture was saturated. The mixture was heated at 50°C for 2hours and was then allowed to cool to room temperature. The solvent was evaporated *in vacuo* and the residue was taken up in aqueous sodium bicarbonate solution and extracted with ethyl acetate. The organic extract was washed with brine, dried over anhydrous magnesium sulfate, filtered and the filtrate evaporated *in vacuo* to afford methyl 4-aminophenylacetate as a pale brown liquid.

¹H NMR (CDCl₃) δ ppm: 3.5(s,2H); 3.7(s,3H); 6.65(m,2H); 7.05(m,2H).

### Step 2 - Preparation of methyl 4-acetamido-3-nitrophenylacetate.

Methyl 4-aminophenylacetate (110g, 0.667mol) was added dropwise to acetic anhydride (600ml), maintaining the temperature of the reaction mixture below 30°C by external cooling. Once the addition was complete the mixture was cooled to below 10°C and concentrated nitric acid (85 ml) was added dropwise. Once the addition of the acid was complete, the mixture was allowed to warm to room temperature and poured onto a mixture of ice and water. The precipitated solid was collected by filtration and dried to give methyl 4-acetamido-3-nitrophenylacetate (120g) as a pale yellow solid.

¹H NMR (CDCl₃) δ ppm: 2.3(s,3H); 3.65(s,2H); 3.7(s,3H); 7.55(dd,1H); 8.1(d,1H); 8.7(d,1H); 10.3(b,1H).

### Step 3 - Preparation of methyl 4-acetamido-3-aminophenylacetate.

Methyl 4-acctainido-3-nitrophenylacetate (40g, 0.159mol)) was dissolved in methanol (400ml) and hydrogenated at 5bar over a 5% palladium on carbon catalyst. The catalyst was removed by filtration and the filtrate evaporated *in vacuo* to give methyl 4-acetamido-3-aminophenylacetate.

### Step 4 - Preparation of methyl (benzotriazol-5-yl)acetate.

Methyl 4-acetamido-3-aminophenylacetate (77g, 0.373mol) was taken up in a mixture of acetic acid (210ml) and water (210ml) and cooled to below 5°C. A solution of sodium nitrite (38.63g, 0.559mol) in water (200ml) was added dropwise, and once the addition was complete the mixture was stirred for 4hours, warming slowly to room temperature. The mixture was diluted with ethyl acetate and the insoluble material removed by filtration. The filtrate was washed with brine, dried over anhydrous magnesium sulfate, filtered and the solvent evaporated *in vacuo*, to give a viscous residue (51g) which was then dissolved in a solution of methanol (400ml) previously saturated with hydrogen chloride and the mixture stirred for 2hours. The solvent was removed *in vacuo*, the residue taken up in water, neutralised with saturated aqueous sodium bicarbonate solution and extracted with ethyl acetate. The organic extract was washed with brine, dried over anhydrous magnesium sulfate, filtered and the filtrate evaporated *in vacuo* to afford methyl 5-benzotriazoleacetate (35g) as a reddish liquid which crystallised on standing.

¹H NMR (CDCl₃) δ ppm: 3.7(s,3H); 3.8(s, 2H); 7.35(d,1H); 7.8(b,2H); 13.6(b,1H).

### Step 5 - Preparation of methyl (2-propylbenzotriazol-5-yl)acetate.

A solution of methyl 5-benzotriazoleacetate (10g, 0.0523mol) in N,N-dimethylformamide was added dropwise to a chilled (ice-bath) suspension of sodium hydride (60% dispersion in oil, 2.3g, 0.0575mol) in N,N-dimethylformamide. When the addition was complete, the ice-bath was removed and the mixture stirred for 1hour. N-Propyl iodide (9.78g, 0.0575mol) was added dropwise, and once the addition was complete the mixture was stirred at room temperature for 3hours. The reaction mixture was poured onto ice/water, and extracted with ethyl acetate. The organic extract was washed with brine, dried over anhydrous magnesium sulfate, filtered and the filtrate evaporated *in vacuo*. The residue was further purified by column chromatography to afford methyl (2-propylbenzotriazol-5-yl)acetate (4.8g).

¹H NMR (CDCl₃) δ ppm: 0.9(t,3H); 2.1(m,2H); 3.7(s,3H); 3.8(s,2H); 4.65(t,2H); 7.3(dd,1H); 7.7(d,1H); 7.8(d,1H).

### Step 6 - Preparation of (2-propylbenzotriazol-5-yl)acetic acid.

The acetate prepared in Step 5 above (4.8g, 0.0206mol) was dissolved in methanol (60ml) and potassium hydroxide flakes (1.27g, 0.0226mol) added. The mixture was refluxed for 2hours, then cooled and the solvent evaporated *in vacuo*. The residual solid was dissolved in water and extracted with diethyl ether. The aqueous solution was acidified to pH 1 by addition of dilute aqueous hydrochloric acid and extracted with ethyl acetate. The ethyl acetate extract was washed with brine, dried over anhydrous magnesium sulfate, filtered and the filtrate evaporated *in vacuo* to afford (2-propylbenzotriazol-5-yl)aoetic acid (3.6g) as a colourless solid.

### Step 7

The acid prepared in Step 6 (0.438g, 0.002mol) was dissolved in dichloromethane (5ml) and one drop of N,N-dimethyl formamide was added. Oxalyl chloride (0.307g. 0.0022mol) was added dropwise, and the mixture stirred at room temperature for 2hours. The solvent was removed *in vacuo*, the residue taken up in xylene and added to a mixture of 5-amino-4-chloro-3-methylisothiazole (0.446g, 0.003mol) in xylene and heated at reflux for 2hours. The mixture was cooled to room temperature and the solvent evaporated *in vacuo*. The residue was taken up in ethyl acetate, washed sequentially with saturated aqueous sodium bicarbonate solution and brine, dried over anhydrous magnesium sulfate, filtered and the filtrate evaporated *in vacuo*. The residue was further purified by flash column chromatography, eluting with 2:1 hexane : ethyl acetate, to give the title compound (0.34g) as a sandy-coloured solid.

### EXAMPLE 2

This Example illustrates the preparation of N-(4-chloro-3-methylisothiazol-5-yl)-N-(ethoxymethyl)-(2-propylbenzotriazol-5-yl)acetamide (Compound No. A3.6) and N-[2-ethoxymethyl-4-chloro-3-methylisothiazolin-5-ylidene]-(2-propylbenzo-triazol-5-yl)acetamide(Compound No. I3.6).

Lithium bis(trimethylsilyl)amide (1.0 M solution in tetrahydrofuran, 3.3ml, 0.0033mol) was added dropwise to a solution of N-(4-chloro-3-methylisothiazol-5-yl)-(2-propylbenzotriazol-5-yl)acetamide (Compound No.A3.1) (0.699g, 0.002mol) in tetrahydrofuran (10ml) and once the addition was complete the mixture was stirred at room temperature for 30minutes. Chloromethylethyl ether (0.620g, 0.0066mol) was added and the mixture was stirred at room temperature for 2hours. The mixture was poured into water, neutralised, and extracted with ethyl acetate. The organic extract was dried over anhydrous magnesium sulfate, filtered and the filtrate was evaporated *in vacuo*. The residue was further purified by flash column chromatography, eluting initially with 2:1 hexane: ethyl acetate and then with ethyl acetate to give N-(4-chloro-3-methylisothiazol-5-yl)-N-(ethoxymethyl)-(2-propylbenzotriazol-5-yl)acetamide (0.135g) as an orange gum and N-[2-ethoxymethyl-4-chloro-3-methylisothiazolin-5-ylidene]-(2-propylbenzotriazol-5-yl) acetamide (0.128g) as a colourless solid, m.p. 108-109 °C.

### EXAMPLE 3

This Example illustrates the preparation of N-(4-chloro-3-methylisothiazol-5-yl)-[2-(2-methylpropyl)indazol-5-yl]acetamide (Compound No.A6.145).

### Step 1 - Preparation of 4-bromo-2-methylacetanilide.

Acetic anhydride (15.2ml, 0.16mol) was added dropwise to a stirred mixture of 4-bromo-2-methylaniline (10.0g, 0.054mol) and sodium acetate (2.52g, 0.056mol) in toluene (150ml). Once the addition was complete the mixture was warmed to 85°C and stirred for 40minutes. The mixture was cooled to room temperature, diluted with ethyl acetate and washed with water. The organic solution was evaporated *in vacuo*, a further quantity of toluene was added and then the solvent was evaporated to give 4-bromo-2-methylacetanilide (12.3g), as a colourless solid.

### Step 2 - Preparation of 5-bromoindazole.

*tert*-Butyl nitrite (8.34g, 0.081mol) was added dropwise, over 30minutes, to a suspension of 4-bromo-2-methylacetanilide (12.3g) in toluene (300ml) at 65°C and the mixture was stirred at 65°C for 45minutes and then at 90°C for 3hours. The mixture was cooled to room temperature and the solvent was removed *in vacuo*. The residue was purified by flash column chromatography on silica gel eluting with 2:1 diethyl ether:hexane to give 5-bromoindarole (4.12g), as a sand-coloured solid.

¹H NMR (CDCl₃) δ: 7.27(m,1H); 7.40-7.54(m,2H); 7.94(m,1H); 8.06(br,1H)ppm.

### Step 3 - Preparation of 5-bromo-1-(2-methylpropyl)indazole and 5-bromo-2-(2-methylpropyl)indazole.

Sodium hydride (80% suspension in oil, 0.7g, 0.023mol) was suspended in *N,N*-dimethylformamide (DMF) (10ml) and then a solution of 5-bromoindazole (4.1g, 0.021mol) in DMF (40ml) was added dropwise over 30minutes. Once the addition was complete the mixture was stirred at ambient temperature for 75minutes and then a solution of 1-bromo-2-methylpropane (3.7g, 0.027mol) in DMF (25ml) was added over 10minutes. The mixture was stirred for 5hours and was then allowed to stand at ambient temperature overnight. The mixture was poured into water, extracted with diethyl ether and then the organic extract was washed further with water, dried over anhydrous magnesium sulfate, filtered and the filtrate was evaporated *in vacuo*. The residue was purified by flash column chromatography on silica gel eluting with 1:1 diethyl ether:hexane to give 5-bromo-1-(2-methylpropyl)indazole (2.67g) and 5-bromo-2-(2-methylpropyl)indazole (1.49g), each as an orange oil.

5-Bromo-1-(2-methylpropyl)indazole:
¹H NMR (CDCl₃) δ: 0.92(d,6H); 2.32(m,1H); 4.16(d,2H); 7.28(m,1H); 7.44(m,1H); 7.86(m,1H); 7.92(s,1H)ppm.

5-Bromo-2-(2-methylpropyl)indazole:
¹H NMR (CDCl₃) δ: 0.94(d,6H); 2.38(m,1H); 4.18(d,2H); 7.32(m,1H); 7.58(m,1H); 7.82(m,2H)ppm.

### Step 4 - Preparation of ethyl [2-(2-methylpropyl)indazol-5-yl]acetate.

5-Bromo-2-(2-methylpropyl)indazole (1.41g, 0.0056mol) and palladium acetate (0.063g, 0.00028mol) were stirred in toluene (10ml) under an atmosphere of nitrogen. Ethyl trimethylsilylacetate (2.35ml, 0.0115mol), potassium *tert*-butoxide (0.66g, 0.059mol) and a 10% w/v solution of tri-*tert*-butylphosphine in toluene (0.97ml, 0.00048mol) were added sequentially and then the mixture was heated to 90°C for 105minutes. The mixture was cooled to room temperature, diluted with diethyl ether and filtered through diatomaceous earth and the filtrate was evaporated *in vacuo*. The residue was purified by flash column chromatography on silica gel eluting with 2:1 diethyl ether : hexane to give ethyl[2-(2-methylpropyl)indazol-5-yl]acetate (1.85g),as a yellow oil.

¹H NMR (CDCl₃) δ: 0.96(d,6H); 127(t,3H); 2.39(m,1H); 3.68(s,2H); 4.12-4.22(m,4H); 7.22(m,1H); 7.54(m,1H); 7.68(m,1H); 7.84(s,1H) ppm.

### Step 5 - Preparation of N-(4-chloro-3-methylisothiazol-5-yl)-[2-(2-methylpropyl)indazol-5-yl]acetamide.

Sodium methoxide (0.42g, 0.0078mol) was added to a solution of 5-amino-4-chloro-3-methylisothiazole (0.46g, 0.0031mol) in tetrahydrofuran (THF) (15ml) and the mixture was stirred at ambient temperature for 15minutes. A solution of ethyl[2-(2-methylpropyl)indazol-5-yl]acetate (0.80g, 0.0031mol) in THF was added dropwise and, once the addition was complete, the mixture was stirred at ambient temperature for 3hours. The mixture was partitioned between ethyl acetate and saturated aqueous ammonium chloride solution and then the organic extract was dried over anhydrous magnesium sulfate, filtered and the filtrate was evaporated *in vacuo*. The residue was triturated with diethyl ether to give N-(4-chloro-3-methylisothiazol-5-yl)-[2-(2-methylpropyl)indazol-5-yl]acetamide (0.78g) as a pale-brown solid (m.p. 133-135°C).

### EXAMPLE 4

This Example illustrates the preparation of N-(4-chloro-3-methylisothiazol-5-yl)-N-(ethoxymethyl)-[2-(2-methylpropyl)indazol-5-yl]acetamide (Compound No.A6.150) and N-[2-ethoxymethyl-4-chloro-3-methylisothiazolin-5-ylidene]-[2-(2-methylpropyl)indazol-5-yl) acetamide (Compound No.I6.102).

N-(4-chloro-3-methylisothiazol-5-yl)-[2-(2-methylpropyl)indazol-5-yl]acetamide (0.50g, 0.0014mol) was dissolved in dichloromethane (15ml) and the solution was cooled to 5°C. Chloromethyl ethyl ether (0.32ml, 0.0035mol), 50% aqueous sodium hydroxide solution (0.56ml) and benzyltriethylammonium chloride (0.018g) were added sequentially and then the mixture was stirred for 2hours. The mixture was diluted with dichloromethane, washed with water, dried over anhydrous magnesium sulfate, filtered and the filtrate was evaporated *in vacuo*. The residue was purified by flash column chromatography on silica gel, initially eluting with 3:1 diethyl ether : hexane, then with diethyl ether and finally with ethyl acetate to give N-(4-chloro-3-methylisothiazol-5-yl)-N-(ethoxymethyl)-[2-(2-methylpropyl)indazol-5-yl]acetamide (0.16g) as a yellow oil and N-[2-ethoxymethyl-4-chloro-3-methylisothiazolin-5-ylidene]-[2-(2-methylpropyl)indazol-5-yl) acetamide (0.20g) as a cream-coloured solid (m.p.105-106°C).

### EXAMPLE 5

This Example illustrates the pesticidal/insecticidal properties of compounds of formula (I). The activities of samples of individual compounds of formula (I) or of mixtures of compounds of formula (I) were determined using a variety of pests. The pests were treated with a liquid composition containing 500 parts per million (ppm) by weight of a compound. Each composition was made by dissolving the compound in an acetone and ethanol (50:50 by volume) mixture and diluting the solution with water containing 0.05% by volume of a wetting agent, SYNPERONIC NP8, until the liquid composition contained the required concentration of the compound.

Samples used were as shown in Table 2.

**Table 2**

| **Sample No.** | **Component A** **(Compound No.)** | **Component B** **(Compound No.)** | **Molar Ratio** **A : B** |
|---|---|---|---|
| 1 | A3.1 | | |
| 2 | A1.1 | | |
| 3 | F1.1 | E1.1 | 1 : 3 |
| 4 | A18.1 | | |
| 5 | E18.1 | F18.1 | 1 : 1 |
| 6 | F15.1 | E15.1 | 8 : 92 |
| 7 | F15.1 | E15.1 | 1 : 2 |
| 8 | A15.1 | | |
| 9 | A9.1 | | |
| 10 | E9.1 | F9.1 | 1 : 1 |
| 11 | A20.1 | | |
| 12 | E20.1 | F20.1 | 1 : 1 |
| 13 | A7.1 | | |
| 14 | E7.1 | F7.1 | 1 : 1 |
| 15 | A2.1 | | |
| 16 | E2.1 | F2.1 | 1 : 1 |
| 17 | A13.1 | | |
| 18 | E13.1 | F13.1 | 3 : 2 |
| 19 | A3.2 | | |
| 20 | A3.6 | | |
| 21 | A21.1 | | |
| 22 | F21.1 | | |
| 23 | E21.1 | F21.1 | 1 : 1 |
| 24 | E3.1 | F3.1 | 1 : 1 |
| 25 | E21.1 | F21.1 | 9 : 1 |
| 26 | F21.1 | | |
| 27 | E22.1 | | |
| 29 | A16.1 | | |
| 30 | A10.1 | | |
| 31 | A6.1 | | |
| 32 | A3.103 | | |
| 33 | I3.2 | | |
| 34 | I3.6 | | |
| 35 | E6.145 | | |
| 36 | A6.145 | | |
| 37 | E6.150 | | |
| 38 | J6.102 | | |
| 39 | A6.150 | | |
| 40 | I6.102 | | |

The test procedure adopted with regard to each pest, was essentially the same and comprised supporting a number of the pests on a medium which was usually a substrate, a host plant or a foodstuff on which the pests feed, and treating either or both the medium and the pests with a composition. Pest mortality was assessed usually between two and five days after treatment.

The results of the tests against peach aphid (*Myzus persicae*) are presented below. In this test Chinese cabbage leaves were infested with aphids, the infested leaves were sprayed with the test composition, and the mortality assessed after three days. The results indicate a grading of mortality (score) designated as 9,5 or 0 wherein 9 indicates 80-100% mortality, 5 indicates 40-79% mortality and 0 indicates less than 40% mortality.

Sample Nos. 2, 13, 15, 19, 20, 30, 31, 32, 36, 37, 39 and 40 each gave a mortality score of 9 whilst Sample Nos. 8, 9, 11, 17, 29, 34, 35 and 38 each gave a score of 5.

In addition, in a similar test against two-spotted spider mites (*Tetranychus urticae*) Sample Nos. 1, 8, 11, 12, 13, 17, 18, 19, 20 and 30 each gave a mortality score of 9 whilst Sample No. 9 gave a score of 5.

### EXAMPLE 6

This Example illustrates the fungiddal properties of compoimds of formula (I). The compounds were tested against a variety of foliar fungal diseases of plants. The technique employed was as follows.

Plants were grown in John Innes Potting Compost (No.1 or 2) in 4cm diameter, 3.5cm depth minipots. The test samples were individually formulated as a solution either in acetone or acetone/ethanol (1:1 by volume) which was diluted in deionised water to a concentration of 100ppm (that is, 1mg of compound in a final volume of 10ml) immediately before use. When foliar sprays were applied to monocotyledonous crops, TWEEN 20 (0.1% by volume) wasadded. TWEEN is a registered trade mark.

Individual compounds of formula (I), or mixtures of compounds of formula (1), were applied as a foliar (Folr) application (where the chemical solution was applied to the foliage of the test plants by spraying the plant to maximum droplet retention.)

These tests were carried out against *Uncinula necator* (UNCINE), on vines; *Phytophthora infestans lycopersici* (PHYTIN) on tomatoes; *Puccinia recondita* (PUCCRT), on wheat; and *Pyricularia oryzae* (PYRIOR) on rice. Each treatment was applied to two or more replicate plants for *Phytophthora infestans lycopersici* and *Uncinula necator*. For tests on *Puccinia recondita* and *Pyricularia oryzae* two replicate pots each containing 6 to 10 plants were used for each treatment. The plants were inoculated one day before (Erad) or one day after (Prot) chemical application. The *Phytophthora infestans lycopersici, Puccinia recondita* and *Pyricularia oryzae* plants were inoculated with a calibrated fungal spore suspension. The *Uncinula necator* plants were inoculated using a 'blowing' inoculation technique.

After chemical application and inoculation, the plants were incubated under high humidity conditions and then put into an appropriate environment to allow infection to proceed, until the disease was ready for assessment The time period between chemical application and assessment varied from five to fourteen days according to the disease and environment. However, each individual disease was assessed after the same time period for all samples.

Assessments were performed on each of two leaves on each of the replicate plants for *Phytophthora infestans lycopersici*. Assessments were performed on a single leaf of each of the replicate plants for *Uncinula necator.* For *Pucdnia recondita and Pyricularia recondita* assessments were earned out collectively on the plants in each replicate pot.

The disease level present (that is, the percentage leaf area covered by actively sporulating disease) was assessed visually. For each treatment, the assessed values for all its replicates were meaned to provide mean disease values. Untreated control plants were assessed in the same manner. The data were then processed by me method, described hereinafter, to provide PRCO (Percentage Reduction from Control) values.

An example of a typical calculation is as follows:
Mean disease level for treatment A = 25%
Mean disease level on untreated controls = 85%
$\text{PRCO = 100 -} \frac{\text{{ Mean disease level for treatment A }}}{\text{{Mean disease level on untreated controls}}} \text{x 100} \text{= 100 - (} \frac{\text{25}}{\text{85}} \text{x 100) = 70.6}$

The PRCO is then rounded to the nearest whole number; therefore, in this particular example, the PRCO result is 71.

It is possible for negative PRCO values to be obtained.

PRCO results are shown below in Table 3, where the Sample Nos. are as defined in Example 5.

**TABLE 3**

| **Sample No.** | **PHYTIN** **Prot** | **PUCCRT** **Prot** | **PYRIOR** **Prot** | **UNCINE** **Erad** |
|---|---|---|---|---|
| 1 | 100 | 100 | 100 | |
| 2 | | | 87 | |
| 3 | | | 40 | |
| 4 | | | 92 | |
| 6 | 75 | 16 | 6 | 11 |
| 7 | | | 96 | |
| 8 | | | 100 | |
| 9 | 100 | 99 | 100 | |
| 11 | | | 100 | |
| 12 | | | 98 | |
| 19 | 81 | 87 | | |
| 30 | 100 | 100 | | 100 |
| 31 | 100 | 100 | | 100 |
| 33 | 30 | 36 | | |
| 34 | 81 | | 93 | 100 |

## Claims

1. A compound of formula (I): 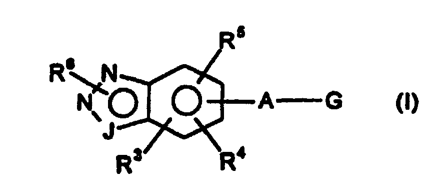 wherein G is either 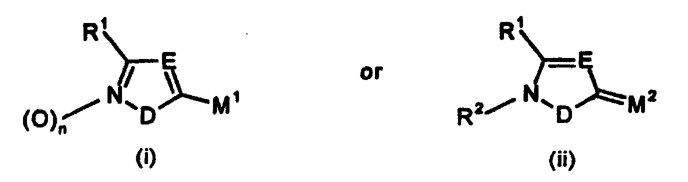 where M¹ or M² is bonded to A; n is 0 or 1; A is optionally substituted C₁₋₆ alkylene, optionally substituted C₂₋₆ alkenylene, optionally substituted C₂₋₆ alkynylene, optionally substituted C₁₋₆ alkylenoxy, optionally substituted oxy(C₁₋₆)alkylene, optionally substituted C₁₋₆ alkylenethio, optionally substituted thio(C₁₋₆)alkylene, optionally substituted C₁₋₆ alkylenamino, optionally substituted amino(C₁₋₆)alkylene, optionally substituted [C₁₋₆ alkyleneoxy(C₁₋₆)alkylene], optionally substituted - [C₁₋₆ alkylenethio(C₁₋₆)alkylene], optionally substituted [C₁₋₆ alkylenesulfinyl(C₁₋₆)alkylene], optionally substituted [C₁₋₆ alkylenesulfonyl(C₁₋₆)alkylene] or optionally substituted [C₁₋₆ alkyleneamino(C₁₋₆)alkylene]; when G is (i), D is S, NR⁷, CR⁸=CR⁹, CR⁸=N, CR⁸=N(O), N=CR⁹ or N(O)=CR⁹; when G is (ii), D is S or NR⁷; E is N, N-oxide or CR¹⁰; M¹ is OC(=Y), N(R¹¹)C(=Y), N=C(OR¹²), N=C(SR¹³) or N=C(NR¹⁴R¹⁵) where O or N is the atom of attachment to the ring containing D and E; M² is N-C(=Y) where N is the atom of attachment to the ring containing D and E; Y is O, S or NR¹⁶; J is N or CR¹⁷; R¹ is hydrogen, halogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₁₋₆ alkylthio, optionally substituted C₃₋₇ cycloalkyl, cyano, nitro or SF₅; R² is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀ alkylaminocarbonyl, optionally substituted di(C₁₋₁₀)alkylaminocarbonyl, optionally substituted phenoxycarbonyl, optionally substituted C₁₋₆ alkylthio, optionally substituted C₁₋₆ alkylsulfinyl, optionally substituted C₁₋₆ alkylsulfonyl, optionally substituted arylthio, optionally substituted arylsulfinyl, optionally substituted arylsulfonyl or R¹⁸R¹⁹NS; R³, R⁴ and R⁵ are, independently, hydrogen, halogen, optionally substituted C₁₋₆ alkyl, optionally substituted C₁₋₆ alkoxy, optionally substituted C₁₋₆ alkylthio, optionally substituted C₁₋₆ alkylsulfinyl, optionally substituted C₁₋₆ alkylsulfonyl, cyano, nitro, optionally substituted C₁₋₆ alkylcarbonyl, optionally substituted C₁₋₆ alkoxycarbonyl or SF₅; R⁶ is hydrogen, cyano, optionally substituted C₁₋₂₀ alkyl, optionally substituted C₂₋₂₀ alkenyl(C₁₋₆)alkyl, optionally substituted C₂₋₂₀ alkynyl(C₁₋₆)alkyl, optionally substituted C₃₋₇ cycloalkyl, optionally substituted C₅₋₆ cycloalkenyl, formyl, optionally substituted C₁₋₂₀ alkoxycarbonyl, optionally substituted C₁₋₂₀ alkylcarbonyl, aminocarbonyl, optionally substituted C₁₋₂₀ alkylaminocarbonyl, optionally substituted di(C₁₋₂₀)alkylaminocarbonyl, optionally substituted aryloxycarbonyl, optionally substituted arylcarbonyl, optionally substituted arylaminocarbonyl, optionally substituted N-alkyl-N-arylaminocarbonyl, optionally substituted diarylaminocarbonyl, optionally substituted heteroaryloxycarbonyl, optionally substituted heteroarylcarbonyl, optionally substituted heteroarylaminocarbonyl, optionally substituted alkylheteroarylaminocarbonyl, optionally substituted diheteroarylaminocarbonyl, optionally substituted phenyl, optionally substituted heteroaryl, optionally substituted heterocyclyl, optionally substituted C₁₋₂₀ alkylsulfonyl or optionally substituted arylsulfonyl; R⁷ is C₁₋₆ alkyl; R⁸ and R⁹ are, independently, hydrogen, halogen, cyano, nitro, optionally substituted C₁₋₆ alkyl, optionally substituted C₂₋₆ alkenyl, optionally substituted C₂₋₆ alkynyl or optionally substituted C₁₋₆ alkoxy; R¹⁰ is hydrogen, halogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₁₋₆ alkylthio, optionally substituted - C₁₋₆ alkylsulfinyl, optionally substituted C₁₋₆ alkylsulfonyl, cyano, nitro, formyl, R²⁰ON=C(R²¹), optionally substituted C₁₋₆ alkylcarbonyl, optionally substituted C₁₋₆ alkoxycarbonyl or SF₅; or R¹ and R¹⁰ together with the atoms to which they are attached may be joined to form a five, six or seven-membered saturated or unsaturated ring carbocylic or heterocyclic ring which may contain one or two hetero atoms selected from O, N and S and which may be optionally substituted by C₁₋₆ alkyl, C₁₋₆ haloalkyl or halogen; R¹¹ is hydrogen, optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀alkylaminocarbonyl, optionally substituted di(C₁₋₁₀)alkylaminocarbonyl, optionally substituted phenoxycarbonyl, optionally substituted C₁₋₆ alkylthio, optionally substituted C₁₋₆ alkylsulfinyl, optionally substituted C₁₋₆ alkylsulfonyl, optionally substituted arylthio, optionally substituted arylsulfinyl, optionally substituted arylsulfonyl or R²²R²³NS; R¹² is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀ alkylaminocarbonyl, optionally substituted di(C₁₋₁₀)alkylaminocarbonyl, , amino, optionally substituted C₁₋₆ alkylamino, optionally substituted di(C₁₋₆)alkylamino, optionally substituted phenoxycarbonyl, tri(C₁₋₄)alkylsilyl, aryldi(C₁₋₄)alkylsilyl, (C₁₋₄)alkyldiarylsilyl or triarylsilyl; R¹³ is optionally substituted C₁₋₁₀ alkyl, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, optionally substituted C₁₋₁₀ alkylaminocarbonyl, optionally substituted di(C₁₋₁₀)alkylaminocarbonyl or optionally substituted phenoxycarbonyl); R¹⁴ and R¹⁵ are, independently optionally substituted C₁₋₁₀ alkyl, optionally substituted C₁₋₆ alkoxy, optionally substituted [C₂₋₆ alkenyl(C₁₋₆)alkyl], optionally substituted [C₂₋₆ alkynyl(C₁₋₆)alkyl], optionally substituted C₃₋₇ cycloalkyl, optionally substituted C₁₋₁₀ alkylcarbonyl, optionally substituted C₁₋₁₀ alkoxycarbonyl, formyl, optionally substituted C₁₋₁₀alkylaminocarbonyl, optionally substituted di(C₁₋₁₀)alkylaminocarbonyl, hydroxy, amino, optionally substituted C₁₋₆ alkylamino, optionally substituted di(C₁₋₆)alkylamino, or optionally substituted phenoxycarbonyl; R¹⁶ is hydrogen, cyano, nitro, optionally substituted C₁₋₆ alkyl, optionally substituted C₃₋₇ cycloalkyl, optionally substituted (C₂₋₆)alkenyl(C₁₋₆)alkyl, optionally substituted (C₂₋₆)alkynyl(C₁₋₆)alkyl, optionally substituted phenyl, optionally substituted heteroaryl, optionally substituted C₁₋₆ alkylcarbonyl, optionally substituted C₁₋₆ alkoxycarbonyl, optionally substituted C₁₋₆ alkylamino, optionally substituted di(C₁₋₆)alkylamino, optionally substituted C₁₋₆ alkylcarbonylamino, optionally substituted C₁₋₆ alkoxycarbonylamino, optionally substituted C₁₋₆ alkoxy, optionally substituted C₁₋₆ alkylthio, optionally substituted C₁₋₆ alkylsulfinyl, optionally substituted C₁₋₆ alkylsulfonyl, optionally substituted arylthio, optionally substituted arylsulfinyl, optionally substituted arylsulfonyl or C₁₋₆ acyloxy; R¹⁷ is hydrogen, halogen, nitro, cyano, optionally substituted C₁₋₈ alkyl, optionally substituted C₂₋₆ alkenyl, optionally substituted C₂₋₆ alkynyl, optionally substituted C₃₋₇ cycloalkyl, optionally substituted C₁₋₆ alkoxycarbonyl, optionally substituted C₁₋₆ alkylcarbonyl, optionally substituted C₁₋₆ alkylaminocarbonyl, optionally substituted di(C₁₋₆)alkylaminocarbonyl, optionally substituted phenyl or optionally substituted heteroaryl; R¹⁸ and R¹⁹ are, independently, optionally substituted C₁₋₆ alkyl or R¹⁸ and R¹⁹ together with the N atom to which they are attached form a five, six or seven-membered heterocyclic ring which may contain one or two further hetero atoms selected from O, N or S and which may be optionally substituted by one or two C₁₋₆ alkyl groups; R²⁰ is hydrogen, optionally substituted phenyl, optionally substituted phenyl(C₁₋₄)alkyl or optionally substituted C₁₋₂₀ alkyl; R²¹ is hydrogen, optionally substituted phenyl or optionally substituted C₁₋₆ alkyl; and R²² and R²³ are, independently, optionally substituted C₁₋₆ alkyl or R²² and R²³ together with the N atom to which they are attached form a five, six or seven-membered heterocyclic ring which may contain one or two further hetero atoms selected from O, N or S and which may be optionally substituted by one or two C₁₋₆ alkyl groups; wherein the optional substituents, when present, on the alkylene, alkenylene or alkynylene moieties are, subject to valency constraints, one or more of halogen, C₁₋₆ alkyl, C₁₋₆ haloalkyl, C₁₋₆ cyanoalkyl, C₁₋₆ alkoxy(C₁₋₆) alkyl, C₁₋₆ alkoxy, cyano, =O, =NR²⁴ and =CR²⁵R²⁶, wherein R²⁴ is C₁₋₆ alkyl, C₁₋₆ haloalkyl, OR²⁷ or R²⁸R²⁹N; where R²⁵ and R²⁶ are, independently, hydrogen, C₁₋₆ alkyl, C₁₋₆ alkoxy, C₁₋₆ haloalkyl, cyano, C₁₋₆ alkoxycarbonyl, C₁₋₆ alkylcarbonyl or R³⁰R³¹N; R²⁷ is C₁₋₆ alkyl, C₁₋₆ haloalkyl or phenyl(C₁₋₂)alkyl; R²⁸ and R²⁹ are, independently, hydrogen, C₁₋₈ alkyl, C₃₋₇ cycloalkyl, C₂₋₆ alkenyl(C₁₋₆)alkyl, C₂₋₆ alkynyl(C₁₋₆)alkyl, C₂₋₆ haloalkyl, C₁₋₆ alkoxy(C₁₋₆)alkyl, C₁₋₆ alkoxycarbonyl(C₁₋₆)alkyl, carboxy(C₁₋₆)alkyl or phenyl(C₁₋₂)alkyl or R²⁸ and R²⁹ together with the N atom to which they are attached form a five, six or seven-membered heterocyclic ring which may contain one or two further hetero atoms selected from O, N or S and which may be optionally substituted by one or two C₁₋₆ alkyl groups; R³⁰ and R³¹ are, independently, hydrogen, C₁₋₈ alkyl, C₃₋₇ cycloalkyl, C₂₋₆ alkenyl(C₁₋₆)alkyl, C₂₋₆ alkynyl(C₁₋₆)alkyl, C₂₋₆ haloalkyl, C₁₋₆ alkoxy-(C₁₋₆)alkyl, C₁₋₆ alkoxycarbonyl(C₁₋₆)alkyl, carboxy(C₁₋₆)alkyl or phenyl(C₁₋₂)alkyl; or R³⁰ and R³¹ together with the N atom to which they are attached form a five, six or seven-membered heterocyclic ring which may contain one or two further hetero atoms selectedfrom O, N or S and which may be optionally substituted by one or two C₁₋₆ alkyl groups; and wherein each alkyl moiety is a straight or branched chain and, when present, the optional substituents on alkyl are one or more of halogen, nitro, cyano, HO₂C, C₁₋₁₀ alkoxy (itself optionally substituted by C₁₋₁₀ alkoxy), aryl-(C₁₋₄)alkoxy, C₁₋₁₀ alkylthio, C₁₋₁₀ alkylcarbonyl, C₁₋₁₀ alkoxycarbonyl, C₁₋₆ alkylaminocarbonyl, di(C₁₋₆ alkyl)aminocarbonyl, (C₁₋₆)alkylcarbonyloxy, optionally substituted phenyl, heteroaryl, aryloxy, arylcarbonyloxy, heteroaryloxy, heterocyclyl, heterocyclyloxy, C₃₋₇ cycloalkyl (itself optionally substituted with (C₁₋₆)alkyl or halogen), C₃₋₇ cycloalkyloxy, C₅₋₇ cycloalkenyl, C₁₋₆ alkylsulfonyl, C₁₋₆ alkylsulfinyl, tri(C₁₋₄)alkylsilyl, tri(C₁₋₄)alkylsilyl-(C₁₋₆)alkoxy, aryldi(C₁₋₄)alkylsilyl, (C₁₋₄)alkyldiarylsilyl, triarylsilyl, =N-OR' and =N-NR'R"; where R' and R" are, independently, C₁₋₆ alkyl or C₁₋₆ haloalkyl; and where the alkenyl and alkynyl moieties are in the form of straight or branched chains, and the alkenyl moieties, where appropriate, can be of either the (E)- or (Z)-configuration; and, when present, the optional substituents on alkenyl or alkynyl are one or more of halogen, aryl and C₃₋₇ cycloalkyl; and wherein acyl is optionally substituted C₁₋₆ alkylcarbonyl, optionally substituted C₂₋₆ alkenylcarbonyl, optionally substituted C₂₋₆ alkynylcarbonyl, optionally substituted arylcarbonyl or optionally substituted heteroarylcarbonyl; and wherein halogen is fluorine, chlorine, bromine or iodine; haloalkyl groups are alkyl groups which are optionally substituted with one or more of the same or different halogen atoms; aryl is phenyl, naphthyl, anthracyl, fluorenyl and indenyl; heteroaryl is an aromatic ring containing up to 10 atoms including one or more heteroatoms selected from O, S and N; heterocycle and heterocyclyl are a non-aromatic ring containing up to 10 atoms including one or more heteroatoms selected from O, S and N; cycloalkyl is cyclopropyl, cyclopentyl and cyclohexyl and the optional substituents for cycloalkyl are halogen, cyano and C₁₋₃ alkyl; cycloalkenyl is cyclopentenyl and cyclohexenyl and the optional substituents for cycloalkenyl are C₁₋₃ alkyl, halogen and cyano; carbocyclic rings are aryl, cycloalkyl and cycloalkenyl groups; and for substituted phenyl moieties, heterocyclyl and heteroaryl groups the substituents are independently selected from one or more of halogen, C₁₋₆ alkyl, C₁₋₆ haloalkyl, C₁₋₆ alkoxy(C₁₋₆)alkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₂₋₆ alkenyl, C₂₋₆ haloalkenyl, C₂₋₆ alkynyl, C₃₋₇ cycloalkyl, nitro, cyano, CO₂H, C₁₋₆ alkylcarbonyl, C₁₋₆ alkoxycarbonyl, R³²R³³N or R³⁴R³⁵NC(O) wherein R³², R³³, R³⁴ and R³⁵ are, independently, hydrogen or C₁₋₆ alkyl; and wherein dialkylamino substituents include those where the dialkyl groups together with the N atom to which they are attached form a five, six or seven-membered heterocyclic ring which may contain one or two further hetero atoms selected from O, N or S and which may be optionally substituted by one or two C₁₋₆ alkyl groups; and when heterocyclic rings are formed by joining two groups on an N atom, the resulting rings are pyrrolidine, piperidine, thiomorpholine and morpholine each of which may be substituted by one or two (C₁₋₆)alkyl groups.

2. A compound as claimed in claim 1 of formula (IA): 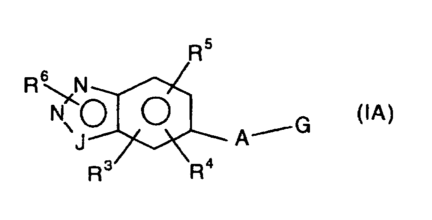 wherein A, G, J, R³, R⁴, R⁵ and R⁶ are as claimed in claim 1.

3. A compound of formula (I) as claimed in claim 1 or a compound of formula (IA) as claimed in claim 2 wherein, when G is (i) as defined in claim 1, D is S or CR⁸=CR⁹, where R⁸ and R⁹ are, independently, hydrogen, halogen, C₁₋₆ alkyl, C₁₋₆ haloalkyl C₂₋₆ alkenyl, C₂₋₆ alkynyl, C₁₋₆ alkoxy or C₁₋₆ haloalkoxy; when G is (ii) as defined in claim 1, D is S; E is N or CR¹⁰ where R¹⁰ is hydrogen, halogen, C₁₋₆ alkyl, C₁₋₆ haloalkyl, - C₁₋₆ alkoxy, C₁₋₆ haloalkoxy, C₁₋₆ alkoxy (C₁₋₆)alkyl, C₁₋₆ alkylthio or SF₅; or R¹ and R¹⁰ together with the atoms to which they are attached form a benzene ring optionally substituted by C₁₋₆ alkyl, C₁₋₆ haloalkyl or halogen; and M¹ is N(R¹¹)C(=O) where R¹¹ is hydrogen, C₁₋₆ alkyl, C₁₋₆ alkoxy(C₁₋₆)alkyl, benzyloxymethyl or benzoyloxymethyl.

4. A compound of formula (I) as claimed in claim 1 or 3 or a compound of formula (IA) as claimed in claim 2 or 3 wherein A is C₁₋₄ alkylene, -C(O)- or C₁₋₄ alkyleneoxy.

5. A compound of formula (I) as claimed in claim 1, 3 or 4 or a compound of formula (IA) as claimed in claim 2, 3 or 4 wherein G is (i) as defined in claim 1 and n is 0.

6. A compound of formula (I) as claimed in claim 1, 3, 4 or 5 or a compound of formula (IA) as claimed in claim 2, 3, 4 or 5 wherein R³, R⁴ and R⁵ are, independently, hydrogen, C₁₋₃ alkyl or halogen.

7. A compound of formula (I) as claimed in claim 1, 3, 4, 5 or 6 or a compound of formula (IA) as claimed in claim 2, 3, 4, 5 or 6 wherein R⁶ is C₁₋₁₀ alkyl or C₁₋₁₀ haloalkyl (each one of which may be substituted with a C₁₋₆ alkyloxime, C₁₋₆ haloalkyloxime, C₁₋₆ alkylhydrazone or C₁₋₆ haloalkylhydrazone group) or C₁₋₆ cyanoalkyl, C₂₋₆ alkenyl(C₁₋₆)alkyl, C₂₋₆ alkynyl(C₁₋₆)alkyl, C₃₋₇ cycloalkyl, C₃₋₇ halocycloalkyl, C₃₋₇ cyanocycloalkyl, C₁₋₃ alkyl(C₃₋₇)cycloalkyl, C₁₋₃ alkyl(C₃₋₇)halocycloalkyl, C₅₋₆ cycloalkenyl, C₃₋₇ cycloalkyl(C₁₋₆)alkyl, C₅₋₆ cycloalkenyl(C₁₋₆)alkyl, C₂₋₆ haloalkenyl(C₁₋₆)alkyl, C₁₋₆ cyanoalkenyl(C₁₋₆)alkyl, C₁₋₆ alkoxy(C₁₋₆)alkyl, C₃₋₆ alkenyloxy(C₁₋₆)alkyl, C₃₋₆ alkynyloxy(C₁₋₆)alkyl, aryloxy(C₁₋₆)alkyl, C₁₋₆ carboxyalkyl, C₁₋₆ alkylcarbonyl(C₁₋₆)alkyl, C₂₋₆ alkenylcarbonyl(C₁₋₆)alkyl, C₂₋₆ alkynylcarbonyl(C₁₋₆)alkyl, C₁₋₆ alkoxycarbonyl(C₁₋₆)alkyl, C₃₋₆ alkenyloxycarbonyl(C₁₋₆)alkyl, C₃₋₆ alkynyloxycarbonyl(C₁₋₆)alkyl, aryloxycarbonyl(C₁₋₆)alkyl, C₁₋₆ alkylthio(C₁₋₆)alkyl, C₁₋₆ alkylsulfinyl(C₁₋₆)alkyl, C₁₋₆ alkylsulfonyl(C₁₋₆)alkyl, aminocarbonyl(C₁₋₆)alkyl, aminocarbonyl(C₂₋₆)alkenyl, aminocarbonyl(C₂₋₆)alkynyl, C₁₋₆alkylaminocarbonyl(C₁₋₆)alkyl, di(C₁₋₆)alkylaminocarbonyl(C₁₋₆)alkyl, C₁₋₆alkylaminocarbonyl(C₂₋₆)alkenyl(C₁₋₆)alkyl, di(C₁₋₆)alkylaminocarbonyl(C₂₋₆)alkenyl(C₁₋₆)alkyl, alkylaminocarbonyl(C₂₋₆)alkynyl(C₁₋₆)alkyl, di(C₁₋₆)alkylaminocarbonyl(C₂₋₆)alkynyl(C₁₋₆)alkyl, phenyl (optionally substituted by halo, nitro, cyano, C₁₋₆ alkyl, C₁₋₆ haloalkyl, C₁₋₆ alkoxy or C₁₋₆ haloalkoxy), phenyl(C₁₋₄)alkyl (wherein the phenyl group is optionally substituted by halo, nitro, cyano, C₁₋₆ alkyl, C₁₋₆ haloalkyl, C₁₋₆ alkoxy or C₁₋₆ haloalkoxy), phenyl(C₂₋₄)alkenyl(C₁₋₆)alkyl, (wherein the phenyl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy), heteroaryl (optionally substituted by halo, nitro, cyano, C₁₋₆ alkyl, C₁₋₆ haloalkyl, C₁₋₆ alkoxy or C₁₋₆ haloalkoxy), heterocyclyl (wherein the heterocyclyl group is optionally substituted by halo, nitro, cyano, C₁₋₆ alkyl, C₁₋₆ haloalkyl, C₁₋₆ alkoxy or C₁₋₆ haloalkoxy), heteroaryl(C₁₋₄)alkyl (wherein the heteroaryl group is optionally substituted by halo, nitro, cyano, C₁₋₆ alkyl, C₁₋₆ haloalkyl, C₁₋₆ alkoxy, C₁₋₆ alkylsulfonyl or C₁₋₆ haloalkoxy)or heterocyclyl(C₁₋₄)alkyl (wherein the heterocyclyl group is optionally substituted by halo, nitro, cyano, C₁₋₆ alkyl, C₁₋₆ haloalkyl, C₁₋₆ alkoxy or C₁₋₆ haloalkoxy).

8. A compound of formula (I) as claimed in claim 1, 3, 4, 5, 6 or 7 or a compound of formula (IA) as claimed in claim 2, 3, 4, 5, 6 or 7 wherein J is N or CR¹⁷ where R¹⁷ is hydrogen, C₁₋₆ alkyl, C₁₋₆ haloalkyl, cyano, halogen or nitro.

9. A process for preparing a compound of formula (I) as claimed in claim 1 comprising
(a) where Y is oxygen, reacting a compound of formula (II) 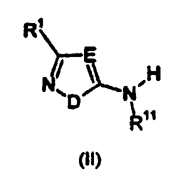 where D, E, R¹ and R¹¹ are as defined in claim 1 with a compound of formula (III) 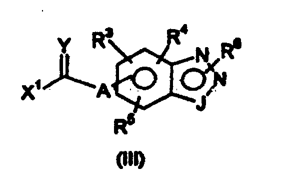 where Y is oxygen, X¹ is a leaving group and A, J, R³, R⁴, R⁵ and R⁶ are as defined in claim 1; or
(b) where Y is sulphur, preparing a compound of formula (I) as claimed in claim 1 where Y is oxygen using the method described in (a) and then reacting this compound with a thionating agent.

10. A fungicidal, insecticidal, acaricidal, molluscicidal or nematicidal composition comprising a fungicidally, insecticidally, acaricidally, molluscicidally or nematicidally effective amount of a compound of formula (I) as claimed in claim 1 and a carrier or diluent therefor.

11. A method of combating and controlling fungi comprising applying to a plant, to a seed of a plant, to the locus of the plant or seed or to the soil a fungicidally effective amount of either a compound of formula (I) as claimed in claim 1.

12. A method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I) as claimed in claim 1.

## Patentansprüche

1. Verbindung der Formel (I): 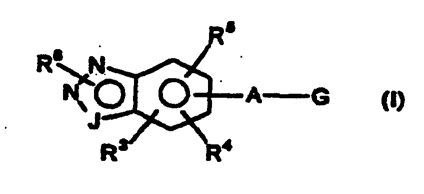 worin G entweder 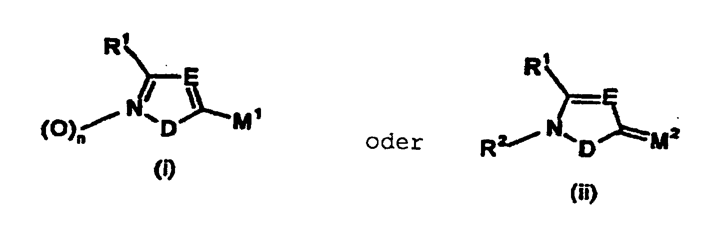 ist, worin M¹ oder M² an A gebunden ist; n 0 oder 1 ist; A gegebenenfalls substituiertes C₁₋₆-Alkylen, gegebenenfalls substituiertes C₂₋₆-Alkenylen, gegebenenfalls substituiertes C₂₋₆-Alkinylen, gegebenenfalls substituiertes C₁₋₆-Alkylenoxy, gegebenenfalls substituiertes Oxy(C₁₋₆)alkylen, gegebenenfalls substituiertes C₁₋₆-Alkylenethio, gegebenenfalls substituiertes Thio(C₁₋₆)alkylen, gegebenenfalls substituiertes C₁₋₆-Alkylenamino, gegebenenfalls substituiertes Amino(C₁₋₆)alkylen, gegebenenfalls substituiertes [C₁₋₆-Alkylenoxy(C₁₋₆)alkylen], gegebenenfalls substituiertes [C₁₋₆-Alkylenthio(C₁₋₆)-alkylen], gegebenenfalls substituiertes [C₁₋₆-Alkylensulfinyl-(C₁₋₆)alkylen], gegebenenfalls substituiertes [C₁₋₆-Alkylensulfonyl(C₁₋₆)alkylen] oder gegebenenfalls substituiertes [C₁₋₆-Alkylenamino(C₁₋₆)alkylen] ist; wenn G (i) ist, D S, NR⁷, CR⁸=CR⁹, CR⁸=N, CR⁸=N(O), N=CR⁹ oder N(O)=CR⁹ ist; wenn G (ii) ist, D S oder NR⁷ ist; E N, N-Oxid oder CR¹⁰ ist; M¹ OC(=Y), N(R¹¹)C(=Y), N=C(OR¹²), N=C(SR¹³) oder N=C(NR¹⁴R¹⁵) ist, worin O oder N das Atom der Bindung an den Ring ist, der D und E enthält; M² N-C(=Y) ist, worin N das Atom der Bindung an den Ring ist, der D und E enthält; Y O, S oder NR¹⁶ ist; J N oder CR¹⁷ ist; R¹ Wasserstoff, Halogen, gegebenenfalls substituiertes C₁₋₆-Alkyl, gegebenenfalls substituiertes C₂₋₆-Alkenyl, gegebenenfalls substituiertes C₂₋₆-Alkinyl, gegebenenfalls substituiertes C₁₋₆-Alkoxy, gegebenenfalls substituiertes C₁₋₆-Alkylthio, gegebenenfalls substituiertes C₃₋₇-Cycloalkyl, Cyano, Nitro oder SF₅ ist; R² gegebenenfalls substituiertes C₁₋₁₀-Alkyl, gegebenenfalls substituiertes [C₂₋₆-Alkenyl(C₁₋₆)alkyl], gegebenenfalls substituiertes [C₂₋₆-Alkinyl(C₁₋₆)alkyl], gegebenenfalls substituiertes C₃₋₇-Cycloalkyl, gegebenenfalls substituiertes C₁₋₁₀-Alkylcarbonyl, gegebenenfalls substituiertes C₁₋₁₀-Alkoxycarbonyl, Formyl, gegebenenfalls substituiertes C₁₋₁₀-Alkylaminocarbonyl, gegebenenfalls substituiertes Di(C₁₋₁₀)alkylaminocarbonyl, gegebenenfalls substituiertes Phenoxycarbonyl, gegebenenfalls substituiertes C₁₋₆-Alkylthio, gegebenenfalls substituiertes C₁₋₆-Alkylsulfinyl, gegebenenfalls substituiertes C₁₋₆-Alkylsulfonyl, gegebenenfalls substituiertes Arylthio, gegebenenfalls substituiertes Arylsulfinyl, gegebenenfalls substituiertes Arylsulfonyl oder R¹⁸R¹⁹NS ist; R³, R⁴ und R⁵ unabhängig Wasserstoff, Halogen, gegebenenfalls substituiertes C₁₋₆-Alkyl, gegebenenfalls substituiertes C₁₋₆-Alkoxy, gegebenenfalls substituiertes C₁₋₆-Alkylthio, gegebenenfalls substituiertes C₁₋₆-Alkylsulfinyl, gegebenenfalls substituiertes C₁₋₆-Alkylsulfonyl, Cyano, Nitro, gegebenenfalls substituiertes C₁₋₆-Alkylcarbonyl, gegebenenfalls substituiertes C₁₋₆-Alkoxycarbonyl oder SF₅ sind; R⁶ Wasserstoff, Cyano, gegebenenfalls substituiertes C₁₋₂₀-Alkyl, gegebenenfalls substituiertes C₂₋₂₀-Alkenyl(C₁₋₆)alkyl, gegebenenfalls substituiertes C₂₋₂₀-Alkinyl(C₁₋₆)alkyl, gegebenenfalls substituiertes C₃₋₇-Cycloalkyl, gegebenenfalls substituiertes C₅₋₆-Cycloalkenyl, Formyl, gegebenenfalls substituiertes C₁₋₂₀-Alkoxycarbonyl, gegebenenfalls substituiertes C₁₋₂₀-Alkylcarbonyl, Aminocarbonyl, gegebenenfalls substituiertes C₁₋₂₀-Alkylaminocarbonyl, gegebenenfalls substituiertes Di(C₁₋₂₀)alkylaminocarbonyl, gegebenenfalls substituiertes Aryloxycarbonyl, gegebenenfalls substituiertes Arylcarbonyl, gegebenenfalls substituiertes Arylaminocarbonyl, gegebenenfalls substituiertes N-Alkyl-N-arylaminocarbonyl, gegebenenfalls substituiertes Diarylaminocarbonyl, gegebenenfalls substituiertes Heteroaryloxycarbonyl, gegebenenfalls substituiertes Heteroarylcarbonyl, gegebenenfalls substituiertes Heteroarylaminocarbonyl, gegebenenfalls substituiertes Alkylheteroarylaminocarbonyl, gegebenenfalls substituiertes Diheteroarylaminocarbonyl, gegebenenfalls substituiertes Phenyl, gegebenenfalls substituiertes Heteroaryl, gegebenenfalls substituiertes Heterocyclyl, gegebenenfalls substituiertes C₁₋₂₀-Alkylsulfonyl oder gegebenenfalls substituiertes Arylsulfonyl ist; R⁷ C₁₋₆-Alkyl ist; R⁸ und R⁹ unabhängig Wasserstoff, Halogen, Cyano, Nitro, gegebenenfalls substituiertes C₁₋₆-Alkyl, gegebenenfalls substituiertes C₂₋₆-Alkenyl, gegebenenfalls substituiertes C₂₋₆-Alkinyl oder gegebenenfalls substituiertes C₁₋₆-Alkoxy sind; R¹⁰ Wasserstoff, Halogen, gegebenenfalls substituiertes C₁₋₆-Alkyl, gegebenenfalls substituiertes C₂₋₆-Alkenyl, gegebenenfalls substituiertes C₂₋₆-Alkinyl, gegebenenfalls substituiertes C₁₋₆-Alkoxy, gegebenenfalls substituiertes C₁₋₆-Alkylthio, gegebenenfalls substituiertes C₁₋₆-Alkylsulfinyl, gegebenenfalls substituiertes C₁₋₆-Alkylsulfonyl, Cyano, Nitro, Formyl, R²⁰ON=C(R²¹), gegebenenfalls substituiertes C₁₋₆-Alkylcarbonyl, gegebenenfalls substituiertes C₁₋₆-Alkoxycarbonyl oder SF₅ ist; oder R¹ und R¹⁰ zusammen mit den Atomen, an die sie gebunden sind, verbunden sein können, um einen 5-, 6- oder 7-gliedrigen gesättigten oder ungesättigten carbocyclischen oder heterocyclischen Ring zu bilden, der ein oder zwei Heteroatome enthalten kann, ausgewählt aus O, N und S, und der gegebenenfalls mit C₁₋₆-Alkyl, C₁₋₆-Halogenalkyl oder Halogen substituiert sein kann; R¹¹ Wasserstoff, gegebenenfalls substituiertes C₁₋₁₀-Alkyl, gegebenenfalls substituiertes [C₂₋₆-Alkenyl(C₁₋₆)alkyl], gegebenenfalls substituiertes [C₂₋₆-Alkinyl(C₁₋₆)alkyl], gegebenenfalls substituiertes C₃₋₇-Cycloalkyl, gegebenenfalls substituiertes C₁₋₁₀-Alkylcarbonyl, gegebenenfalls substituiertes C₁₋₁₀-Alkoxycarbonyl, Formyl, gegebenenfalls substituiertes C₁₋₁₀-Alkylaminocarbonyl, gegebenenfalls substituiertes Di(C₁₋₁₀)alkylaminocarbonyl, gegebenenfalls substituiertes Phenoxycarbonyl, gegebenenfalls substituiertes C₁₋₆-Alkylthio, gegebenenfalls substituiertes C₁₋₆-Alkylsulfinyl, gegebenenfalls substituiertes C₁₋₆-Alkylsulfonyl, gegebenenfalls substituiertes Arylthio, gegebenenfalls substituiertes Arylsulfinyl, gegebenenfalls substituiertes Arylsulfonyl oder R²²R²³NS ist; R¹² gegebenenfalls substituiertes C₁₋₁₀-Alkyl, gegebenenfalls substituiertes [C₂₋₆-Alkenyl(C₁₋₆)alkyl], gegebenenfalls substituiertes [C₂₋₆-Alkinyl(C₁₋₆)alkyl], gegebenenfalls substituiertes C₃₋₇-Cycloalkyl, gegebenenfalls substituiertes C₁₋₁₀-Alkylcarbonyl, gegebenenfalls substituiertes C₁₋₁₀-Alkoxycarbonyl, Formyl, gegebenenfalls substituiertes C₁₋₁₀-Alkylaminocarbonyl, gegebenenfalls substituiertes Di(C₁₋₁₀)alkylaminocarbonyl, Amino, gegebenenfalls substituiertes C₁₋₆-Alkylamino, gegebenenfalls substituiertes Di(C₁₋₆)alkylamino, gegebenenfalls substituiertes Phenoxycarbonyl, Tri(C₁₋₄)alkylsilyl, Aryldi(C₁₋₄)alkylsilyl, (C₁₋₄)-Alkyldiarylsilyl oder Triarylsilyl ist; R¹³ gegebenenfalls substituiertes C₁₋₁₀-Alkyl, gegebenenfalls substituiertes [C₂₋₆-Alkenyl(C₁₋₆)alkyl], gegebenenfalls substituiertes [C₂₋₆-Alkinyl(C₁₋₆)alkyl], gegebenenfalls substituiertes C₃₋₇-Cycloalkyl, gegebenenfalls substituiertes C₁₋₁₀-Alkylcarbonyl, gegebenenfalls substituiertes C₁₋₁₀-Alkoxycarbonyl, gegebenenfalls substituiertes C₁₋₁₀-Alkylaminocarbonyl, gegebenenfalls substituiertes Di(C₁₋₁₀)alkylaminocarbonyl oder gegebenenfalls substituiertes Phenoxycarbonyl ist; R¹⁴ und R¹⁵ unabhängig gegebenenfalls substituiertes C₁₋₁₀-Alkyl, gegebenenfalls substituiertes C₁₋₆-Alkoxy, gegebenenfalls substituiertes [C₂₋₆-Alkenyl(C₁₋₆)alkyl], gegebenenfalls substituiertes [C₂₋₆-Alkinyl(C₁₋₆)alkyl], gegebenenfalls substituiertes C₃₋₇-Cycloalkyl, gegebenenfalls substituiertes C₁₋₁₀-Alkylcarbonyl, gegebenenfalls substituiertes C₁₋₁₀-Alkoxycarbonyl, Formyl, gegebenenfalls substituiertes C₁₋₁₀-Alkylaminocarbonyl, gegebenenfalls substituiertes Di(C₁₋₁₀)-alkylaminocarbonyl, Hydroxy, Amino, gegebenenfalls substituiertes C₁₋₆-Alkylamino, gegebenenfalls substituiertes Di(C₁₋₆)alkylamino oder gegebenenfalls substituiertes Phenoxycarbonyl sind; R¹⁶ Wasserstoff, Cyano, Nitro, gegebenenfalls substituiertes C₁₋₆-Alkyl, gegebenenfalls substituiertes C₃₋₇-Cycloalkyl, gegebenenfalls substituiertes (C₂₋₆)Alkenyl(C₁₋₆)alkyl, gegebenenfalls substituiertes (C₂₋₆)Alkinyl(C₁₋₆)alkyl, gegebenenfalls substituiertes Phenyl, gegebenenfalls substituiertes Heteroaryl, gegebenenfalls substituiertes C₁₋₆-Alkylcarbonyl, gegebenenfalls substituiertes C₁₋₆-Alkoxycarbonyl, gegebenenfalls substituiertes C₁₋₆-Alkylamino, gegebenenfalls substituiertes Di(C₁₋₆)alkylamino, gegebenenfalls substituiertes C₁₋₆-Alkylcarbonylamino, gegebenenfalls substituiertes C₁₋₆-Alkoxycarbonylamino, gegebenenfalls substituiertes C₁₋₆-Alkoxy, gegebenenfalls substituiertes C₁₋₆-Alkylthio, gegebenenfalls substituiertes C₁₋₆-Alkylsulfinyl, gegebenenfalls substituiertes C₁₋₆-Alkylsulfonyl, gegebenenfalls substituiertes Arylthio, gegebenenfalls substituiertes Arylsulfinyl, gegebenenfalls substituiertes Arylsulfonyl oder C₁₋₆-Acyloxy ist; R¹⁷ Wasserstoff, Halogen, Nitro, Cyano, gegebenenfalls substituiertes C₁₋₈-Alkyl, gegebenenfalls substituiertes C₂₋₆-Alkenyl, gegebenenfalls substituiertes C₂₋₆-Alkinyl, gegebenenfalls substituiertes C₃₋₇-Cycloalkyl, gegebenenfalls substituiertes C₁₋₆-Alkoxycarbonyl, gegebenenfalls substituiertes C₁₋₆-Alkylcarbonyl, gegebenenfalls substituiertes C₁₋₆-Alkylaminocarbonyl, gegebenenfalls substituiertes Di(C₁₋₆)alkylaminocarbonyl, gegebenenfalls substituiertes Phenyl oder gegebenenfalls substituiertes Heteroaryl ist; R¹⁸ und R¹⁹ unabhängig gegebenenfalls substituiertes C₁₋₆-Alkyl sind oder R¹⁸ und R¹⁹ zusammen mit dem N-Atom, an das sie gebunden sind, einen 5-, 6- oder 7-gliedrigen heterocyclischen Ring bilden, der ein oder zwei weitere Heteroatome enthalten kann, ausgewählt aus 0, N und S, und der gegebenenfalls mit einer oder zwei C₁₋₆-Alkylgruppen substituiert sein kann; R²⁰ Wasserstoff, gegebenenfalls substituiertes Phenyl, gegebenenfalls substituiertes Phenyl(C₁₋₄)alkyl oder gegebenenfalls substituiertes C₁₋₂₀-Alkyl ist; R²¹ Wasserstoff, gegebenenfalls substituiertes Phenyl oder gegebenenfalls substituiertes C₁₋₆-Alkyl ist; und R²² und R²³ unabhängig gegebenenfalls substituiertes C₁₋₆-Alkyl sind oder R²² und R²³ zusammen mit dem N-Atom, an das sie gebunden sind, einen 5-, 6- oder 7-gliedrigen heterocyclischen Ring bilden, der ein oder zwei weitere Heteroatome enthalten kann, ausgewählt aus O, N und S, und der gegebenenfalls mit einer oder zwei C₁₋₆-Alkylgruppen substituiert sein kann; worin die optionalen Substituenten, wenn vorhanden, an den Alkylen-, Alkenylen- oder Alkinylen-Einheiten, vorbehaltlich Valenzbeschränkungen, eines oder mehrere sind aus: Halogen, C₁₋₆-Alkyl, C₁₋₆-Halogenalkyl, C₁₋₆-Cyanoalkyl, C₁₋₆-Alkoxy(C₁₋₆)alkyl, C₁₋₆-Alkoxy, Cyano, =O, =NR²⁴ und =CR²⁵R²⁶, worin R²⁴ C₁₋₆-Alkyl, C₁₋₆-Halogenalkyl, OR²⁷ oder R²⁸R²⁹N ist; worin R²⁵ und R²⁶ unabhängig Wasserstoff, C₁₋₆-Alkyl, C₁₋₆-Alkoxy, C₁₋₆-Halogenalkyl, Cyano, C₁₋₆-Alkoxycarbonyl, C₁₋₆-Alkylcarbonyl oder R³⁰R³¹N sind; R²⁷ C₁₋₆-Alkyl, C₁₋₆-Halogenalkyl oder Phenyl(C₁₋₂)alkyl ist; R²⁸ und R²⁹ unabhängig Wasserstoff, C₁₋₈-Alkyl, C₃₋₇-Cycloalkyl, C₂₋₆-Alkenyl(C₁₋₆)alkyl, C₂₋₆-Alkinyl(C₁₋₆)alkyl, C₂₋₆-Halogenalkyl, C₁₋₆-Alkoxy(C₁₋₆)alkyl, C₁₋₆-Alkoxycarbonyl(C₁₋₆)-alkyl, Carboxy(C₁₋₆)alkyl oder Phenyl(C₁₋₂)alkyl sind oder R²⁸ und R²⁹ zusammen mit dem N-Atom, an das sie gebunden sind, einen 5-, 6- oder 7-gliedrigen heterocyclischen Ring bilden, der ein oder zwei weitere Heteroatome enthalten kann, ausgewählt aus O, N und S, und der gegebenenfalls mit einer oder zwei C₁₋₆-Alkylgruppen substituiert sein kann; R³⁰ und R³¹ unabhängig Wasserstoff, C₁₋₈-Alkyl, C₃₋₇-Cycloalkyl, C₂₋₆-Alkenyl(C₁₋₆)alkyl, C₂₋₆-Alkinyl(C₁₋₆)alkyl, C₂₋₆-Halogenalkyl, C₁₋₆-Alkoxy(C₁₋₆)alkyl, C₁₋₆-Alkoxycarbonyl(C₁₋₆)alkyl, Carboxy(C₁₋₆)alkyl oder Phenyl(C₁₋₂)alkyl sind; oder R³⁰ und R³¹ zusammen mit dem N-Atom, an das sie gebunden sind, einen 5-, 6- oder 7-gliedrigen heterocyclischen Ring bilden, der ein oder zwei weitere Heteroatome enthalten kann, ausgewählt aus O, N und S, und der gegebenenfalls mit einer oder zwei C₁₋₆-Alkylgruppen substituiert sein kann; und worin jede Alkyleinheit eine lineare oder verzweigte Kette ist und die optionalen Substituenten, wenn vorhanden, an Alkyl eines oder mehrere sind aus: Halogen, Nitro, Cyano, HO₂C, C₁₋₁₀-Alkoxy (selbst gegebenenfalls substituiert mit C₁₋₁₀-Alkoxy), Aryl(C₁₋₄)-alkoxy, C₁₋₁₀-Alkylthio, C₁₋₁₀-Alkylcarbonyl, C₁₋₁₀-Alkoxycarbonyl, C₁₋₆-Alkylaminocarbonyl, Di(C₁₋₆-alkyl)-aminocarbonyl, (C₁₋₆)-Alkylcarbonyloxy, gegebenenfalls substituiertes Phenyl, Heteroaryl, Aryloxy, Arylcarbonyloxy, Heteroaryloxy, Heterocyclyl, Heterocyclyloxy, C₃₋₇-Cycloalkyl (selbst gegebenenfalls substituiert mit (C₁₋₆)-Alkyl oder Halogen), C₃₋₇-Cycloalkyloxy, C₅₋₇-Cycloalkenyl, C₁₋₆-Alkylsulfonyl, C₁₋₆-Alkylsulfinyl, Tri(C₁₋₄)alkylsilyl, Tri(C₁₋₄)alkylsilyl-(C₁₋₆)alkoxy, Aryldi(C₁₋₄)alkylsilyl, (C₁₋₄)Alkyldiarylsilyl, Triarylsilyl, =N-OR' und =N-NR'R"; worin R' und R" unabhängig C₁₋₆-Alkyl oder C₁₋₆-Halogenalkyl sind; und worin die Alkenyl- und Alkinyl-Einheiten in Form von linearen oder verzweigten Ketten sind und die Alkenyl-Einheiten, nach Zweckmäßigkeit, entweder die (E)- oder (Z)-Konfiguration haben können; und worin die optionalen Substituenten, wenn vorhanden, an Alkenyl oder Alkinyl eines oder mehrere sind aus: Halogen, Aryl und C₃₋₇-Cycloalkyl; und worin Acyl gegebenenfalls substituiertes C₁₋₆-Alkylcarbonyl, gegebenenfalls substituiertes C₂₋₆-Alkenylcarbonyl, gegebenenfalls substituiertes C₂₋₆-Alkinylcarbonyl, gegebenenfalls substituiertes Arylcarbonyl oder gegebenenfalls substituiertes Heteroarylcarbonyl ist; und worin Halogen Fluor, Chlor, Brom oder Iod ist; Halogenalkylgruppen Alkylgruppen sind, die gegebenenfalls mit einem oder mehreren der gleichen oder unterschiedlichen Halogenatome substituiert sind; Aryl Phenyl, Naphthyl, Anthracyl, Fluorenyl und Indenyl ist; Heteroaryl ein aromatischer Ring ist, der bis zu 10 Atome einschließt, einschließlich eines oder mehrerer Heteroatome, ausgewählt aus O, S und N; Heterocyclus und Heterocyclyl ein nichtaromatischer Ring sind, der bis zu 10 Atome enthält, einschließlich eines oder mehrerer Heteroatome, ausgewählt aus O, S und N; Cycloalkyl Cyclopropyl, Cyclopentyl und Cyclohexyl ist und die optionalen Substituenten für Cycloalkyl Halogen, Cyano und C₁₋₃-Alkyl sind; Cycloalkenyl Cyclopentenyl und Cyclohexenyl ist und die optionalen Substituenten für Cycloalkenyl C₁₋₃-Alkyl, Halogen und Cyano sind; carbocyclische Ringe Aryl-, Cycloalkyl- und Cycloalkenyl-Gruppen sind; und für substituierte Phenyleinheiten, Heterocyclyl- und Heteroaryl-Gruppen die Substituenten unabhängig ausgewählt sind aus einem oder mehreren aus: Halogen, C₁₋₆-Alkyl, C₁₋₆-Halogenalkyl, C₁₋₆-Alkoxy(C₁₋₆)alkyl, C₁₋₆-Alkoxy, C₁₋₆-Halogenalkoxy, C₁₋₆-Alkylthio, C₁₋₆-Halogenalkylthio, C₁₋₆-Alkylsulfinyl, C₁₋₆-Halogenalkylsulfinyl, C₁₋₆-Alkylsulfonyl, C₁₋₆-Halogenalkylsulfonyl, C₂₋₆-Alkenyl, C₂₋₆-Halogenalkenyl, C₂₋₆-Alkinyl, C₃₋₇-Cycloalkyl, Nitro, Cyano, CO₂H, C₁₋₆-Alkylcarbonyl, C₁₋₆-Alkoxycarbonyl, R³²R³³N oder R³⁴R³⁵NC(O), worin R³², R³³, R³⁴ und R³⁵ unabhängig Wasserstoff oder C₁₋₆-Alkyl sind; und worin Dialkylaminosubstituenten diejenigen einschließen, worin die Dialkylgruppen zusammen mit dem N-Atom, an das sie gebunden sind, einen 5-, 6- oder 7-gliedrigen heterocyclischen Ring bilden, der ein oder zwei weitere Heteroatome enthalten kann, ausgewählt aus O, N und S, und der gegebenenfalls mit einer oder zwei C₁₋₆-Alkylgruppen substituiert sein kann; und worin die resultierenden Ringe, wenn heterocyclische Ringe durch Verbindung zweier Gruppen an einem N-Atom gebildet werden, Pyrrolidin, Piperidin, Thiomorpholin und Morpholin sind, von denen jedes mit einer oder zwei C₁₋₆-Alkylgruppen substituiert sein kann.

2. Verbindung gemäß Anspruch 1 der Formel (IA): 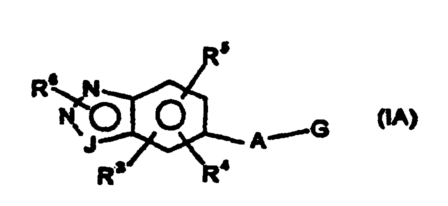 worin A, G, J, R³, R⁴, R⁵ und R⁶ gemäß Anspruch 1 sind.

3. Verbindung der Formel (I) gemäß Anspruch 1 oder Verbindung der Formel (IA) gemäß Anspruch 2, worin dann, wenn G (i) wie in Anspruch 1 definiert ist, D S oder CR⁸=CR⁹ ist, worin R⁸ und R⁹ unabhängig Wasserstoff, Halogen, C₁₋₆-Alkyl, C₁₋₆-Halogenalkyl, C₂₋₆-Alkenyl, C₂₋₆-Alkinyl, C₁₋₆-Alkoxy oder C₁₋₆-Halogenalkoxy sind; dann, wenn G (ii) wie in Anspruch 1 definiert ist, D S ist; E N oder CR¹⁰ ist, worin R¹⁰ Wasserstoff, Halogen, C₁₋₆-Alkyl, C₁₋₆-Halogenalkyl, C₁₋₆-Alkoxy, C₁₋₆-Halogenalkoxy, C₁₋₆-Alkoxy(C₁₋₆)alkyl, C₁₋₆-Alkylthio oder SF₅ ist; oder R¹ und R¹⁰ zusammen mit den Atomen, an die sie gebunden sind, einen Benzolring bilden, der gegebenenfalls mit C₁₋₆-Alkyl, C₁₋₆-Halogenalkyl oder Halogen substituiert ist; und M¹ N(R¹¹)C(=O) ist, worin R¹¹ Wasserstoff, C₁₋₆-Alkyl, C₁₋₆-Alkoxy(C₁₋₆)alkyl, Benzyloxymethyl oder Benzoyloxymethyl ist.

4. Verbindung der Formel (I) gemäß Anspruch 1 oder 3 oder Verbindung der Formel (IA) gemäß Anspruch 2 oder 3, worin A C₁₋₄-Alkylen, -C(O)- oder C₁₋₄-Alkylenoxy ist.

5. Verbindung der Formel (I) gemäß Anspruch 1, 3 oder 4 oder Verbindung der Formel (IA) gemäß Anspruch 2, 3 oder 4, worin G (i) wie in Anspruch 1 definiert ist und n 0 ist.

6. Verbindung der Formel (I) gemäß Anspruch 1, 3, 4 oder 5 oder Verbindung der Formel (IA) gemäß Anspruch 2, 3, 4 oder 5, worin R³, R⁴ und R⁵ unabhängig Wasserstoff, C₁₋₃-Alkyl oder Halogen sind.

7. Verbindung der Formel (I) gemäß Anspruch 1, 3, 4, 5 oder 6 oder Verbindung der Formel (IA) gemäß Anspruch 2, 3, 4, 5 oder 6, worin R⁶ C₁₋₁₀-Alkyl oder C₁₋₁₀-Halogenalkyl (von denen jedes mit einer C₁₋₆-Alkyloxim-, C₁₋₆-Halogenalkyloxim-, C₁₋₆-Alkylhydrazon- oder C₁₋₆-Halogenalkylhydrazongruppe substituiert sein kann) oder C₁₋₆-Cyanoalkyl, C₂₋₆-Alkenyl(C₁₋₆)alkyl, C₂₋₆-Alkinyl(C₁₋₆)alkyl, C₃₋₇-Cycloalkyl, C₃₋₇-Halogencycloalkyl, C₃₋₇-Cyanocycloalkyl, C₁₋₃-Alkyl(C₃₋₇)cycloalkyl, C₁₋₃-Alkyl(C₃₋₇)halogencycloalkyl, C₅₋₆-Cycloalkenyl, C₃₋₇-Cycloalkyl(C₁₋₆)alkyl, C₅₋₆-Cycloalkenyl(C₁₋₆)alkyl, C₂₋₆-Halogenalkenyl(C₁₋₆)alkyl, C₁-6-Cyanoalkenyl(C₁₋₆)alkyl, C₁₋₆-Alkoxy(C₁₋₆)alkyl, C₃₋₆-Alkenyloxy(C₁₋₆)alkyl, C₃₋₆-Alkinyloxy(C₁₋₆)alkyl, Aryloxy(C₁₋₆)alkyl, C₁₋₆-Carboxyalkyl, C₁₋₆-Alkylcarbonyl(C₁₋₆)alkyl, C₂₋₆-Alkenylcarbonyl(C₁₋₆)alkyl, C₂₋₆-Alkinylcarbonyl(C₁₋₆)alkyl, C₁₋₆-Alkoxycarbonyl(C₁₋₆)alkyl, C₃₋₆-Alkenyloxycarbonyl(C₁₋₆)alkyl, C₃₋₆-Alkinyloxycarbonyl(C₁₋₆)alkyl, Aryloxycarbonyl(C₁₋₆)alkyl, C₁₋₆-Alkylthio(C₁₋₆)alkyl, C₁₋₆-Alkylsulfinyl(C₁₋₆)alkyl, C₁₋₆-Alkylsulfonyl(C₁₋₆)alkyl, Aminocarbonyl(C₁₋₆)alkyl, Aminocarbonyl(C₂₋₆)alkenyl, Aminocarbonyl(C₂₋₆)alkinyl, C₁₋₆-Alkylaminocarbonyl(C₁₋₆)alkyl, Di(C₁-₆)alkylaminocarbonyl(C₁₋₆)alkyl, C₁₋₆-Alkylaminocarbonyl(C₂-₆)alkenyl(C₁₋₆)alkyl, Di(C₁₋₆)alkylaminocarbonyl(C₂-₆) alkenyl(C₁₋₆)alkyl, Alkylaminocarbonyl(C₂₋₆)alkinyl(C₁₋₆)alkyl, Di(C₁₋₆)alkylaminocarbonyl(C₂₋₆)alkinyl(C₁₋₆)alkyl, Phenyl (gegebenenfalls substituiert mit Halogen, Nitro, Cyano, C₁₋₆-Alkyl, C₁₋₆-Halogenalkyl, C₁₋₆-Alkoxy oder C₁₋₆-Halogenalkoxy), Phenyl(C₁₋₄)alkyl (worin die Phenylgruppe gegebenenfalls mit Halogen, Nitro, Cyano, C₁₋₆-Alkyl, C₁₋₆-Halogenalkyl, C₁₋₆-Alkoxy oder C₁₋₆-Halogenalkoxy substituiert ist), Phenyl(C₂₋₄)alkenyl(C₁₋₆)alkyl (worin die Phenylgruppe gegebenenfalls mit Halogen, Nitro, Cyano, C₁₋₆-Alkyl, C₁₋₆-Halogenalkyl, C₁₋₆-Alkoxy, C₁₋₆-Alkylsulfonyl oder C₁₋₆-Halogenalkoxy substituiert ist), Heteroaryl (gegebenenfalls substituiert mit Halogen, Nitro, Cyano, C₁₋₆-Alkyl, C₁₋₆-Halogenalkyl, C₁₋₆-Alkoxy oder C₁₋₆-Halogenalkoxy), Heterocyclyl (worin die Heterocyclylgruppe gegebenenfalls mit Halogen, Nitro, Cyano, C₁₋₆-Alkyl, C₁₋₆-Halogenalkyl, C₁₋₆-Alkoxy oder C₁₋₆-Halogenalkoxy substituiert ist), Heteroaryl(C₁₋₄)alkyl (worin die Heteroarylgruppe gegebenenfalls mit Halogen, Nitro, Cyano, C₁₋₆-Alkyl, C₁₋₆-Halogenalkyl, C₁₋₆-Alkoxy, C₁₋₆-Alkylsulfonyl oder C₁₋₆-Halogenalkoxy substituiert ist) oder Heterocyclyl(C₁₋₄)alkyl (worin die Heterocyclylgruppe gegebenenfalls mit Halogen, Nitro, Cyano, C₁₋₆-Alkyl, C₁₋₆-Halogenalkyl, C₁₋₆-Alkoxy oder C₁₋₆-Halogenalkoxy substituiert ist) ist.

8. Verbindung der Formel (I) gemäß Anspruch 1, 3, 4, 5, 6 oder 7 oder Verbindung der Formel (IA) gemäß Anspruch 2, 3, 4, 5, 6 oder 7, worin J N oder CR¹⁷ ist, worin R¹⁷ Wasserstoff, C₁₋₆-Alkyl, C₁₋₆-Halogenalkyl, Cyano, Halogen oder Nitro ist.

9. Verfahren zur Herstellung einer Verbindung der Formel (I) gemäß Anspruch 1, umfassend
(a) wenn Y Sauerstoff ist, Umsetzen einer Verbindung der Formel (II) 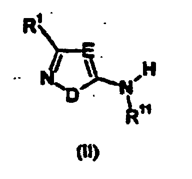 worin D, E, R¹ und R¹¹ wie in Anspruch 1 definiert sind, mit einer Verbindung der Formel (III) 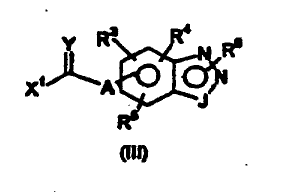 worin Y Sauerstoff ist, X¹ eine Abgangsgruppe ist und A, J, R³, R⁴, R⁵ und R⁶ wie in Anspruch 1 definiert sind; oder
(b) wenn Y Schwefel ist, Herstellen,einer Verbindung der Formel (I) gemäß Anspruch 1, worin Y Sauerstoff ist, unter Verwendung des in (a) beschriebenen Verfahrens und anschließend Umsetzen dieser Verbindung mit einem Thionierungsmittel.

10. Fungizide, insektizide, akarizide, molluskizide oder nematizide Zusammensetzung, die eine fungizid, insektizid, akarizid oder nematizid wirksame Menge einer Verbindung der Formel (I) gemäß Anspruch 1 und einen Träger oder ein Verdünnungsmittel dafür umfasst.

11. Verfahren zur Bekämpfung und Kontrolle von Pilzen, umfassend das Ausbringen einer fungizid wirksamen Menge einer Verbindung der Formel (I) gemäß Anspruch 1 auf eine Pflanze, einen Samen einer Pflanze, den Locus der Pflanze oder des Samens oder auf die Erde.

12. Verfahren zur Bekämpfung und Kontrolle von Insekten, Milben, Nematoden oder Mollusken, welches das Ausbringen einer insektizid, akarizid, nematizid oder molluskizid wirksamen Menge einer Verbindung der Formel (I) gemäß Anspruch 1 auf einen Schädling, einen Locus eines Schädlings oder eine Pflanze, die für einen Befall durch einen Schädling anfällig ist, umfasst.

## Revendications

1. Composé de formule (I): 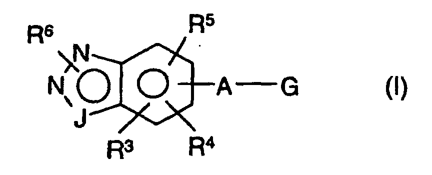 où G est 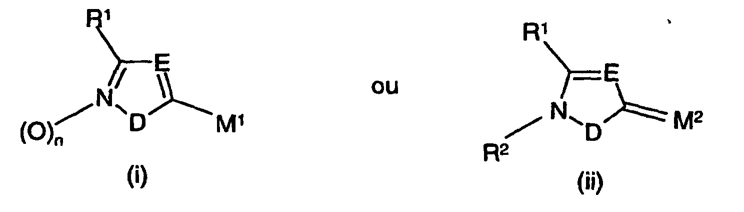 où M¹ ou M² est lié à A ; n est 0 ou 1 ; A est alkylène en C₁₋₆ éventuellement substitué, alcénylène en C₂₋₆ éventuellement substitué, alcynylène en C₂₋₆ éventuellement substitué, alkylène en C₁₋₆-oxy éventuellement substitué, oxy-alkylène en C₁₋₆ éventuellement substitué, alkylène en C₁₋₆-thio éventuellement substitué, thio-alkylène en C₁₋₆ éventuellement substitué, alkylène en C₁₋₆-amino éventuellement substitué, aminoalkylène en C₁₋₆ éventuellement substitué, [alkylène en C₁₋₆-oxy-alkylène en C₁₋₆] éventuellement substitué, [alkylène en C₁₋₆-thio-alkylène en C₁₋₆] éventuellement substitué, [alkylène en C₁₋₆-sulfinyl-alkylène en C₁₋₆] éventuellement substitué, [alkylène en C₁₋₆-sulfonyl-alkylène en C₁₋₆] éventuellement substitué ou [alkylène en C₁₋₆-amino-alkylène en C₁₋₆] éventuellement substitué ; quand G est (i), D est S, NR⁷, CR⁸=CR⁹, CR⁸=N, CR⁸=N(O), N=CR⁹ ou N(O)=CR⁹ ; quand G est (ii), D est S ou NR⁷ ; E est N,N-oxyde ou CR¹⁰ ; M¹ est OC(=Y), N(R¹¹)C(=Y), N=C(OR¹²), N=C(SR¹³) ou N=C(NR¹⁴R¹⁵) où O ou N est l'atome de liaison au cycle contenant D et E ; M² est N-C(=Y) où N est l'atome de liaison au cycle contenant D et E ; Y est O, S ou NR¹⁶ ; J est N ou CR¹⁷ ; R¹ est hydrogène, halogène, alkyle en C₁₋₆ éventuellement substitué, alcényle en C₂₋₆ éventuellement substitué, alcynyle en C₂₋₆ éventuellement substitué, alcoxy en C₁₋₆ éventuellement substitué, alkyle en C₁₋₆-thio éventuellement substitué, cycloalkyle en C₃₋₇ éventuellement substitué, cyano, nitro ou SF₅; R² est alkyle en C₁₋₁₀ éventuellement substitué, [alcényle en C₂₋₆-alkyle en C₁₋₆] éventuellement substitué, [alcynyle en C₂₋₆-alkyle en C₁₋₆] éventuellement substitué, cycloalkyle en C₃₋₇ éventuellement substitué, alkyle en C₁₋₁₀-carbonyle éventuellement substitué, alcoxy en C₁₋₁₀-carbonyle éventuellement substitué, formyle, alkyle en C₁₋₁₀-aminocarbonyle éventuellement substitué, dialkyle en C₁₋₁₀-aminocarbonyle éventuellement substitué, phénoxycarbonyle éventuellement substitué, alkyle en C₁₋₆-thio éventuellement substitué, alkyle en C₁₋₆-sulfinyle éventuellement substitué, alkyle en C₁₋₆-sulfonyle éventuellement substitué, arylthio éventuellement substitué, arylsulfinyle éventuellement substitué, arylsulfonyle éventuellement substitué ou R¹⁸R¹⁹NS ; R³, R⁴ et R⁵ sont, indépendamment, hydrogène, halogène, alkyle en C₁₋₆ éventuellement substitué, alcoxy en C₁₋₆ éventuellement substitué, alkyle en C₁₋₆-thio éventuellement substitué, alkyle en C₁₋₆-sulfinyle éventuellement substitué, alkyle en C₁₋₆-sulfonyle éventuellement substitué, cyano, nitro, alkyle en C₁₋₆-carbonyle éventuellement substitué, alcoxy en C₁₋₆-carbonyle éventuellement substitué ou SF₅ ; R⁶ est hydrogène, cyano, alkyle en C₁₋₂₀ éventuellement substitué, alcényle en C₂₋₂₀-alkyle en C₁₋₆ éventuellement substitué, alcynyle en C₂₋₂₀-alkyle en C₁₋₆ éventuellement substitué, cycloalkyle en C₃₋₇ éventuellement substitué, cycloalcényle en C₅₋₆ éventuellement substitué, formyle, alcoxy en C₁₋₂₀-carbonyle éventuellement substitué, alkyle en C₁₋₂₀-carbonyle éventuellement substitué, aminocarbonyle, alkyle en C₁₋₂₀-aminocarbonyle éventuellement substitué, dialkyle en C₁₋₂₀-aminocarbonyle éventuellement substitué, aryloxycarbonyle éventuellement substitué, arylcarbonyle éventuellement substitué, arylaminocarbonyle éventuellement substitué, N-alkyl-N-arylaminocarbonyle éventuellement substitué, diarylaminocarbonyle éventuellement substitué, hétéroaryloxycarbonyle éventuellement substitué, hétéroarylcarbonyle éventuellement substitué, hétéroarylaminocarbonyle éventuellement substitué, alkylhétéroarylaminocarbonyle éventuellement substitué, dihétéroarylaminocarbonyle éventuellement substitué, phényle éventuellement substitué, hétéroaryle éventuellement substitué, hétérocyclyle éventuellement substitué, alkyle en C₁₋₂₀-sulfonyle éventuellement substitué ou arylsulfonyle éventuellement substitué ; R⁷ est alkyle en C₁₋₆ ; R⁸ et R⁹ sont, indépendamment, hydrogène, halogène, cyano, nitro, alkyle en C₁₋₆ éventuellement substitué, alcényle en C₂₋₆ éventuellement substitué, alcynyle en C₂₋₆ éventuellement substitué ou alcoxy en C₁₋₆ éventuellement substitué ; R¹⁰ est hydrogène, halogène, alkyte en C₁₋₆ éventuellement substitué, alcényle en C₂₋₆ éventuellement substitué, alcynyle en C₂₋₆ éventuellement substitué, alcoxy en C₁₋₆ éventuellement substitué, alkyle en C₁₋₆-thio éventuellement substitué, alkyle en C₁₋₆-sulfinyle éventuellement substitué, alkyle en C₁₋₆-sulfonyle éventuellement substitué, cyano, nitro, formyle, R²⁰ON=C(R²¹), alkyle en C₁₋₆-carbonyle éventuellement substitué, alcoxy en C₁₋₆-carbonyle éventuellement substitué ou SF₅ ; ou bien R¹ et R¹⁰ peuvent être joints avec les atomes auxquels ils sont liés pour former un cycle carbocyclique ou hétérocyclique saturé ou insaturé à 5, 6 ou 7 chaînons qui peut contenir un ou deux hétéroatomes choisis parmi O, N et S et qui peut être éventuellement substitué par alkyle en C₁₋₆, halogénoalkyle en C₁₋₆ ou halogène ; R¹¹ est hydrogène, alkyle en C₁₋₁₀ éventuellement substitué, [alcényle en C₂₋₆-alkyle en C₁₋₆] éventuellement substitué, [alcynyle en C₂₋₆-alkyle en C₁₋₆] éventuellement substitué, cycloalkyle en C₃₋₇ éventuellement substitué, alkyle en C₁₋₁₀-carbonyle éventuellement substitué, alcoxy en C₁₋₁₀-carbonyle éventuellement substitué, formyle, alkyle en C₁₋₁₀-aminocarbonyle éventuellement substitué, dialkyle en C₁₋₁₀-aminocarbonyle éventuellement substitué, phénoxycarbonyle éventuellement substitué, alkyle en C₁₋₆-thio éventuellement substitué, alkyle en C₁₋₆-sulfinyle éventuellement substitué, alkyle en C₁₋₆-sulfonyle éventuellement substitué, arylthio éventuellement substitué, arylsulfinyle éventuellement substitué, arylsulfonyle éventuellement substitué ou R²²R²³NS ; R¹² est alkyle en C₁₋₁₀ éventuellement substitué, [alcényle en C₂₋₆-alkyle en C₁₋₆] éventuellement substitué, [alcynyle en C₂₋₆-alkyle en C₁₋₆] éventuellement substitué, cycloalkyle en C₃₋₇ éventuellement substitué, alkyle en C₁₋₁₀-carbonyle éventuellement substitué, alkcoxy en C₁₋₁₀-carbonyle éventuellement substitué, formyle, alkyle en C₁₋₁₀-aminocarbonyle éventuellement substitué, dialkyle en C₁₋₁₀-aminocarbonyle éventuellement substitué, amino, alkyle en C₁₋₆-amino éventuellement substitué, dialkyle en C₁₋₆-amino éventuellement substitué, phénoxycarbonyle éventuellement substitué, trialkyle en C₁₋₄-silyle, aryl-dialkyle en C₁₋₄-silyle, alkyle en C₁₋₄-diarylsilyle ou triarylsilyle ; R¹³ est alkyle en C₁₋₁₀ éventuellement substitué, [alcényle en C₂₋₆-alkyle en C₁₋₆] éventuellement substitué, [alcynyle en C₂₋₆-alkyle en C₁₋₆] éventuellement substitué, cycloalkyle en C₃₋₇ éventuellement substitué, alkyle en C₁₋₁₀-carbonyle éventuellement substitué, alcoxy en C₁₋₁₀-carbonyle éventuellement substitué, alkyle en C₁₋₁₀-aminocarbonyle éventuellement substitué, dialkyle en C₁₋₁₀-aminocarbonyle éventuellement substitué ou phénoxycarbonyle éventuellement substitué ; R¹⁴ et R¹⁵ sont, indépendamment, alkyle en C₁₋₁₀ éventuellement substitué, alcoxy en C₁₋₆ éventuellement substitué, [alcényle en C₂₋₆-alkyle en C₁₋₆] éventuellement substitué, [alcynyle en C₂₋₆-alkyle en C₁₋₆] éventuellement substitué, cycloalkyle en C₃₋₇ éventuellement substitué, alkyle en C₁₋₁₀-carbonyle éventuellement substitué, alcoxy en C₁₋₁₀-carbonyle éventuellement substitué, formyle, alkyle en C₁₋₁₀-aminocarbonyle éventuellement substitué, dialkyle en C₁₋₁₀-aminocarbonyle éventuellement substitué, hydroxyle, amino, alkyle en C₁₋₆-amino éventuellement substitué, dialkyle en C₁₋₆-amino éventuellement substitué ou phénoxycarbonyle éventuellement substitué ; R¹⁶ est hydrogène, cyano, nitro, alkyle en C₁₋₆ éventuellement substitué, cycloalkyle en C₃₋₇ éventuellement substitué, alcényle en C₂₋₆-alkyle en C₁₋₆ éventuellement substitué, alcynyle en C₂₋₆-alkyle en C₁₋₆ éventuellement substitué, phényle éventuellement substitué, hétéroaryle éventuellement substitué, alkyle en C₁₋₆-carbonyle éventuellement substitué, alcoxy en C₁₋₆-carbonyle éventuellement substitué, alkyle en C₁₋₆-amino éventuellement substitué, dialkyle en C₁₋₆-amino éventuellement substitué, alkyle en C₁₋₆-carbonylamino éventuellement substitué, alcoxy en C₁₋₆-carbonylamino éventuellement substitué, alcoxy en C₁₋₆ éventuellement substitué, alkyle en C₁₋₆-thio éventuellement substitué, alkyle en C₁₋₆-sulfinyle éventuellement substitué, alkyle en C₁₋₆-sulfonyle éventuellement substitué, arylthio éventuellement substitué, arylsulfinyle éventuellement substitué, arylsulfonyle éventuellement substitué ou acyloxy en C₁₋₆ ; R¹⁷ est hydrogène, halogène, nitro, cyano, alkyle en C₁₋₈ éventuellement substitué, alcényle en C₂₋₆ éventuellement substitué, alcynyle en C₂₋₆ éventuellement substitué, cycloalkyle en C₃₋₇ éventuellement substitué, alcoxy en C₁₋₆-carbonyle éventuellement substitué, alkyle en C₁₋₆-carbonyle éventuellement substitué, alkyle en C₁₋₆-aminocarbonyle éventuellement substitué, dialkyle en C₁₋₆-aminocarbonyle éventuellement substitué, phényle éventuellement substitué ou hétéroaryle éventuellement substitué ; R¹⁸ et R¹⁹ sont, indépendamment, alkyle en C₁₋₆ éventuellement substitué ou R¹⁸ et R¹⁹ forment avec l'atome N auquel ils sont liés un cycle hétérocyclique à 5, 6 ou 7 chaînons qui peut contenir un ou deux autres hétéroatomes choisis parmi O, N et S et qui peut éventuellement être substitué par un ou deux groupes alkyle en C₁₋₆ ; R²⁰ est hydrogène, phényle éventuellement substitué, phényl-alkyle en C₁₋₄ éventuellement substitué ou alkyle en C₁₋₂₀ éventuellement substitué ; R²¹ est hydrogène, phényle éventuellement substitué ou alkyle en C₁₋₆ éventuellement substitué ; et R²² et R²³ sont, indépendamment, alkyle en C₁₋₆ éventuellement substitué ou bien R²² et R²³ forment avec l'atome N auquel ils sont liés un cycle hétérocyclique à 5, 6 ou 7 chaînons qui peut contenir un ou deux autres hétéroatomes choisis parmi O, N et S et qui peut être éventuellement substitué par un ou deux groupes alkyle en C₁₋₆ ; où les substituants éventuels, quand ils sont présents, sur des entités alkylène, alcénylène ou alcynylène sont, selon les contraintes de valence, un ou plusieurs substituants parmi halogène, alkyle en C₁₋₆, halogénoalkyle en C₁₋₆, cyanoalkyle en C₁₋₆, alcoxy en C₁₋₆-alkyle en C₁-C₆, alcoxy en C₁₋₆, cyano, =O, =NR²⁴ et =CR²⁵R²⁶ où R²⁴ est alkyle en C₁₋₆, halogénoalkyle en C₁₋₆, OR²⁷ ou R²⁸R²⁹N ; où R²⁵ et R²⁶ sont, indépendamment, hydrogène, alkyle en C₁₋₆, alcoxy en C₁₋₆, halogénoalkyle en C₁₋₆, cyano, alcoxy en C₁₋₆-carbonyle, alkyle en C₁₋₆-carbonyle ou R³⁰R³¹N ; R²⁷ est alkyle en C₁₋₆, halogénoalkyle en C₁₋₆ ou phényl-alkyle en C₁₋₂ ; R²⁸ et R²⁹ sont, indépendamment, hydrogène, alkyle en C₁₋₈, cycloalkyle en C₃₋₇, alcényle en C₂₋₆-alkyle en C₁₋₆, alcynyle en C₂₋₆-alkyle en C₁₋₆, halogénoalkyle en C₂₋₆, alcoxy en C₁₋₆-alkyle en C₁₋₆, alcoxy en C₁₋₆-carbonyle-alkyle en C₁₋₆, carboxy-alkyle en C₁₋₆ ou phényl-alkyle en C₁₋₂ ou bien R²⁸ et R²⁹ forment avec l'atome N auquel ils sont liés un cycle hétérocyclique à 5, 6 ou 7 chaînons qui peut contenir un ou deux autres hétéroatomes choisis parmi O, N et S et qui peut être éventuellement substitué par un ou deux groupes alkyle en C₁₋₆ ; R³⁰ et R³¹ sont, indépendamment, hydrogène, alkyle en C₁₋₈, cycloalkyle en C₃₋₇, alcényle en C₂₋₆-alkyle en C₁₋₆, alcynyle en C₂₋₆-alkyle en C₁₋₆, halogénoalkyle en C₂₋₆, alcoxy en C₁₋₆-alkyle en C₁₋₆, alcoxy en C₁₋₆-carbonyl-alkyle en C₁₋₆, carboxy-alkyle en C₁₋₆ ou phényl-alkyle en C₁₋₂ ; ou bien R³⁰ et R³¹ forment avec l'atome N auquel ils sont liés un cycle hétérocyclique à 5, 6 ou 7 chaînons qui peut contenir un ou deux autres hétéroatomes choisis parmi O, N et S et qui peut être éventuellement substitué par un ou deux groupes alkyle en C₁₋₆ ; et où chaque entité alkyle est une chaîne linéaire ou ramifiée et, quand ils sont présents, les substituants éventuels sur alkyle sont un ou plusieurs substituants parmi halogène, nitro, cyano, HO₂C, alcoxy en C₁₋₁₀ (lui-même éventuellement substitué par alcoxy en C₁₋₁₀), aryl-alcoxy en C₁₋₄, alkyle en C₁₋₁₀-thio, alkyle en C₁₋₁₀-carbonyle, alcoxy en C₁₋₁₀-carbonyle, alkyle en C₁₋₆-aminocarbonyle, dialkyle en C₁₋₆-aminocarbonyle, alkyle en C₁₋₆-carbonyloxy, phényle éventuellement substitué, hétéroaryle, aryloxy, arylcarbonyloxy, hétéroaryloxy, hétérocyclyte, hétérocyclyloxy, cycloalkyle en C₃₋₇ (lui-même éventuellement substitué par alkyle en C₁₋₆ ou halogène), cycloalkyle en C₃₋₇-oxy, cycloalcényle en C₅₋₇, alkyle en C₁₋₆-sulfonyle, alkyle en C₁₋₆-sulfinyle, trialkyle en C₁₋₄-silyle, trialkyle en C₁₋₄-silyl-alcoxy en C₁₋₆, aryl-dialkyle en C₁₋₄-silyle, alkyle en C₁₋₄-diarylsilyle, triarylsilyle, =N-OR' et =N-NR'R" ; où R' et R" sont, indépendamment, alkyle en C₁₋₆ ou halogénoalkyle en C₁₋₆ ; et où les entités alcényle et alcynyle sont sous forme de chaînes linéaires ou ramifiées, et les entités alcényle, quand cela est approprié, peuvent être de configuration (E) ou (Z) ; et, quand ils sont présents, les substituants éventuels sur alcényle ou alcynyle sont un ou plusieurs substituants parmi halogène, aryle et cycloalkyle en C₃₋₇ ; et où acyle est alkyle en C₁₋₆-carbonyle éventuellement substitué, alcényle en C₂₋₆-carbonyle éventuellement substitué, alcynyle en C₂₋₆-carbonyle éventuellement substitué, arylcarbonyle éventuellement substitué ou hétéroarylcarbonyle éventuellement substitué ; et où halogène est fluor, chlore, brome ou iode ; les groupes halogénoalkyle sont des groupes alkyle qui sont éventuellement substitués par un ou plusieurs atomes d'halogène identiques ou différents ; aryle est phényle, naphtyle, anthracyle, fluorényle et indényle ; hétéroaryle est un cycle aromatique contenant jusqu'à 10 atomes incluant un ou plusieurs hétéroatomes choisis parmi O, S et N ; hétérocycle et hétérocyclyle sont un cycle non aromatique contenant jusqu'à 10 atomes incluant un ou plusieurs hétéroatomes choisis parmi O, S et N ; cycloalkyle est cyclopropyle, cyclopentyle et cyclohexyle et les substituants éventuels pour cycloalkyle sont halogène, cyano et alkyle en C₁₋₃ ; cycloalcényle est cyclopentényle et cyclohexényle et les substituants éventuels pour cycloalcényle sont alkyle en C₁₋₃, halogène et cyano ; les cycles carbocycliques sont des groupes aryle, cycloalkyle et cycloalcényle ; et pour les entités phényle substituées, les groupes hétérocyclyle et hétéroaryle substitués, les substituants sont choisis indépendamment parmi un ou plusieurs des substituants halogène, alkyle en C₁₋₆, halogénoalkyle en C₁₋₆, alcoxy en C₁₋₆-alkyle en C₁₋₆, alcoxy en C₁₋₆, halogénoalcoxy en C₁₋₆, alkyle en C₁₋₆-thio, halogénoalkyle en C₁₋₆-thio, alkyle en C₁₋₆-sulfinyle, halogénoalkyle en C₁₋₆-sulfinyle, alkyle en C₁₋₆-sulfonyle, halogénoalkyle en C₁₋₆-sulfonyle, alcényle en C₂₋₆, halogénoalcényle en C₂₋₆, alcynyle en C₂₋₆, cycloalkyle en C₃₋₇, nitro, cyano, CO₂H, alkyle en C₁₋₆-carbonyle, alcoxy en C₁₋₆-carbonyle, R³²R³³N ou R³⁴R³⁵NC(O) où R³², R³³, R³⁴ et R³⁵ sont, indépendamment, hydrogène ou alkyle en C₁₋₆ ; et où les substituants dialkylamino incluent ceux où les groupes dialkyle forment avec l'atome N auquel ils sont liés un cycle hétérocyclique à 5, 6 ou 7 chaînons qui peut contenir un ou deux autres hétéroatomes choisis parmi O, N et S et qui peut être éventuellement substitué par un ou deux groupes alkyle en C₁₋₆ ; et quand des cycles hétérocycliques sont formés par la jonction de deux groupes sur un atome N, les cycles résultants sont pyrrolidine, pipéridine, thiomorpholine et morpholine qui peuvent être substitués chacun par un ou deux groupes alkyle en C₁₋₆.

2. Composé selon la revendication 1, de formule (IA) : 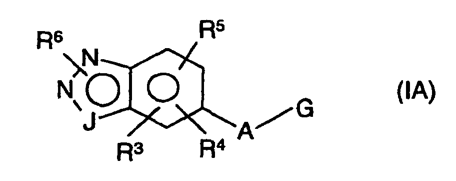 où A, G, J, R³, R⁴, R⁵ et R⁶ sont selon la revendication 1.

3. Composé de formule (I) selon la revendication 1 ou composé de formule (IA) selon la revendication 2 où, quand G est (i) tel que défini dans la revendication 1, D est S ou CR⁸=CR⁹, où R⁸ et R⁹ sont, indépendamment, hydrogène, halogène, alkyle en C₁₋₆, halogénoalkyle en C₁₋₆, alcényle en C₂₋₆, alcynyle en C₂₋₆, alcoxy en C₁₋₆ ou halogénoalcoxy en C₁₋₆ ; quand G est (ii) tel que défini dans la revendication 1, D est S ; E est N ou CR¹⁰ où R¹⁰ est hydrogène, halogène, alkyle en C₁₋₆, halogénoalkyle en C₁₋₆, alcoxy en C₁₋₆, halogénoalcoxy en C₁₋₆, alcoxy en C₁₋₆-alkyle en C₁₋₆, alkyle en C₁₋₆-thio ou SF₅ ; ou bien R¹ et R¹⁰ forment avec les atomes auxquels ils sont liés un cycle benzénique éventuellement substitué par alkyle en C₁₋₆, halogénoalkyle en C₁₋₆ ou halogène ; et M¹ est N(R¹¹)C(=O) où R¹¹ est hydrogène, alkyle en C₁₋₆, alcoxy en C₁₋₆alkyle en C₁₋₆, benzyloxyméthyle ou benzoyloxyméthyle.

4. Composé de formule (I) selon la revendication 1 ou 3 ou composé de formule (IA) selon la revendication 2 ou 3 où A est alkylène en C₁₋₄, -C(O)- ou alkylène en C₁₋₄-oxy.

5. Composé de formule (I) selon la revendication 1, 3 ou 4 ou composé de formule (IA) selon la revendication 2, 3 ou 4 où G est (i) tel que défini dans la revendication 1 et n est 0.

6. Composé de formule (I) selon la revendication 1, 3, 4 ou 5 ou composé de formule (IA) selon la revendication 2, 3, 4 ou 5 où R³, R⁴ et R⁵ sont, indépendamment, hydrogène, alkyle en C₁₋₃ ou halogène.

7. Composé de formule (I) selon la revendication 1, 3, 4, 5 ou 6 ou composé de formule (IA) selon la revendication 2, 3, 4, 5 ou 6 où R⁶ est alkyle en C₁₋₁₀ ou halogénoalkyle en C₁₋₁₀ (qui peuvent être substitués chacun par un groupe alkyle en C₁₋₆-oxime, halogénoalkyle en C₁₋₆-oxime, alkyle en C₁₋₆-hydrazone ou halogénoalkyle en C₁₋₆-hydrazone) ou cyanoalkyle en C₁₋₆, alcényle en C₂₋₆-alkyle en C₁₋₆, alcynyle en C₂₋₆-alkyle en C₁₋₆, cycloalkyle en C₃₋₇, halogénocycloalkyle en C₃₋₇, cyano-cycloalkyle en C₃₋₇, alkyle en C₁₋₃-cycloalkyle en C₃₋₇, alkyle en C₁₋₃-halogénocycloalkyle en C₃₋₇, cycloalcényle en C₅₋₆, cycloalkyle en C₃₋₇-alkyle en C₁₋₆, cycloalcényle en C₅₋₆-alkyle en C₁₋₆, halogénoalcényle en C₂₋₆-alkyle en C₁₋₆, cyanoalcényle en C₂₋₆-alkyle en C₁₋₆, alcoxy en C₁₋₆-alkyle en C₁₋₆, alcényle en C₃₋₆-oxy-alkyle en C₁₋₆, alcynyle en C₃₋₆-oxy-alkyle en C₁₋₆, aryloxy-alkyle en C₁₋₆, carboxy-alkyle en C₁₋₆, alkyle en C₁₋₆-carbonyl-alkyle en C₁₋₆, alcényle en C₂₋₆-carbonyl-alkyle en C₁₋₆, alcynyle en C₂₋₆-carbonyl-alkyle en C₁₋₆, alcoxy en C₁₋₆-carbonyl-alkyle en C₁₋₆, alcényle en C₃₋₆-oxy-carbonylalkyle en C₁₋₆, alcynyle en C₃₋₆-oxycarbonyl-alkyle en C₁₋₆, aryloxycarbonylalkyle en C₁₋₆, alkyle en C₁₋₆-thio-alkyle en C₁₋₆, alkyle en C₁₋₆-sulfinyl-alkyle en C₁₋₆, alkyle en C₁₋₆-sulfonyl-alkyle en C₁₋₆, aminocarbonyl-alkyle en C₁₋₆, aminocarbonyl-alcényle en C₂₋₆, aminocarbonyl-alcynyle en C₂₋₆, alkyle en C₁₋₆-aminocarbonyl-alkyle en C₁₋₆, dialkyle en C₁₋₆-aminocarbonyl-alkyle en C₁₋₆, alkyle en C₁₋₆-aminocarbonyl-alcényle en C₂₋₆-alkyle en C₁₋₆, dialkyle en C₁₋₆-aminocarbonyl-alcényle en C₂₋₆-alkyle en C₁₋₆, alkylaminocarbonylalcynyle en C₂₋₆-alkyle en C₁₋₆, dialkyle en C₁₋₆-aminocarbonyl-alcynyle en C₂₋₆-alkyle en C₁₋₆, phényle (éventuellement substitué par halogéno, nitro, cyano, alkyle en C₁₋₆, halogénoalkyle en C₁₋₆, alcoxy en C₁₋₆ ou halogénoalcoxy en C₁₋₆), phényl-alkyle en C₁₋₄ (où le groupe phényle est éventuellement substitué par halogéno, nitro, cyano, alkyle en C₁₋₆, halogénoalkyle en C₁₋₆, alcoxy en C₁₋₆ ou halogénoalcoxy en C₁₋₆), phényl-alcényle en C₂₋₄-alkyle en C₁₋₆ (où le groupe phényle est éventuellement substitué par halogéno, nitro, cyano, alkyle en C₁₋₆, halogénoalkyle en C₁₋₆, alcoxy en C₁₋₆, alkyle en C₁₋₆-sulfonyle ou halogénoalcoxy en C₁₋₆), hétéroaryle (éventuellement substitué par halogéno, nitro, cyano, alkyle en C₁₋₆, halogénoalkyle en C₁₋₆, alcoxy en C₁₋₆ ou halogénoalcoxy en C₁₋₆), hétérocyclyle (où le groupe hétérocyclyle est éventuellement substitué par halogéno, nitro, cyano, alkyle en C₁₋₆, halogénoalkyle en C₁₋₆, alcoxy en C₁₋₆ ou halogénoalcoxy en C₁₋₆), hétéroaryl-alkyle en C₁₋₄ (où le groupe hétéroaryle est éventuellement substitué par halogéno, nitro, cyano, alkyle en C₁₋₆, halogénoalkyle en C₁₋₆, alcoxy en C₁₋₆, alkyle en C₁₋₆-sulfonyle ou halogénoalcoxy en C₁₋₆) ou hétérocyclyl-alkyle en C₁₋₄ (où le groupe hétérocyclyle est éventuellement substitué par halogéno, nitro, cyano, alkyle en C₁₋₆, halogénoalkyle en C₁₋₆, alcoxy en C₁₋₆ ou halogénoalcoxy en C₁₋₆).

8. Composé de formule (I) selon la revendication 1, 3, 4, 5, 6 ou 7 ou composé de formule (IA) selon la revendication 2, 3, 4, 5, 6 ou 7 où J est N ou CR¹⁷ où R¹⁷ est hydrogène, alkyle en C₁₋₆, halogénoalkyle en C₁₋₆, cyano, halogène ou nitro.

9. Procédé pour préparer un composé de formule (I) selon la revendication 1 comprenant (a) où Y est l'oxygène, la réaction d'un composé de formule (II) 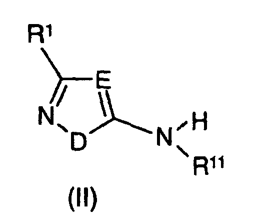 où D, E, R¹ et R¹¹ sont tels que définis dans la revendication 1 avec un composé de formule (III) 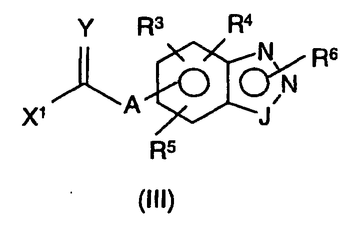 où Y est l'oxygène, X¹ et un groupe partant et A, J, R³, R⁴, R⁵ et R⁶ sont tels que définis dans la revendication 1 ; ou bien
(b) où Y est le soufre, la préparation d'un composé de formule (I) selon la revendication 1 où Y est l'oxygène au moyen du procédé décrit en (a) puis la réaction de ce composé avec un agent de thionation.

10. Composition fongicide, insecticide, acaricide, molluscicide ou nématicide comprenant une quantité efficace du point de vue fongicide, insecticide, acaricide, molluscicide ou nématicide d'un composé de formule (I) selon la revendication 1 et un support ou un diluant pour celui-ci.

11. Procédé pour combattre et maîtriser les champignons comprenant l'application à une plante, à une graine d'une plante, au site de la plante ou de la graine ou au sol d'une quantité efficace du point de vue fongicide d'un composé de formule (I) selon la revendication 1.

12. Procédé pour combattre et maîtriser les insectes, les acariens, les nématodes ou les mollusques qui comprend l'application à un nuisible, à un site d'un nuisible ou à une plante susceptible d'être attaquée par un nuisible d'une quantité efficace du point de vue insecticide, acaricide, nématicide ou molluscicide d'un composé de formule (I) selon la revendication 1.
